(19) Europäisches Patentamt
European Patent Office
Office européen des brevets

(11) EP 2 216 335 B1

(12) EUROPEAN PATENT SPECIFICATION

(45) Date of publication and mention of the grant of the patent:
**12.03.2014 Bulletin 2014/11**

(21) Application number: **07816752.5**

(22) Date of filing: **05.11.2007**

(51) Int Cl.:
***A61K 31/37*** *(2006.01)* ***A61K 31/4025*** *(2006.01)*
***A61P 31/06*** *(2006.01)* ***A61P 31/18*** *(2006.01)*
***C07D 493/22*** *(2006.01)* ***C07D 493/04*** *(2006.01)*
***C07D 493/14*** *(2006.01)*

(86) International application number:
**PCT/CN2007/003139**

(87) International publication number:
**WO 2009/059452 (14.05.2009 Gazette 2009/20)**

# (54) TETRACYCLODIPYRANYL COUMARINS AND THE ANTI-HIV AND ANTI-TUBERCULOSIS USES THEREOF

TETRACYCLODIPYRANYLCOUMARINE UND DIE ANTI-HIV- UND ANTI-TUBERKULOSE-ANWENDUNGEN DAVON

TÉTRACYCLODIPYRANYL-COUMARINES ET LEURS UTILISATIONS ANTI-VIH ET ANTITUBERCULOSE

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**11.08.2010 Bulletin 2010/32**

(73) Proprietor: **Institute of Mataria Medica, Chinese Academy of Medical Sciences**
**Beijing 100050 (CN)**

(72) Inventors:
- **LIU, Gang**
  **Beijing 100050 (CN)**
- **XUE, Hai**
  **Beijing 100050 (CN)**
- **MA, Tao**
  **Beijing 100050 (CN)**
- **CHEN, Ziwei**
  **Beijing 100050 (CN)**
- **WANG, Lin**
  **Beijing 100050 (CN)**

(74) Representative: **Hart-Davis, Jason et al**
**Cabinet Beau de Loménie**
**158, rue de l'Université**
**75340 Paris Cedex 07 (FR)**

(56) References cited:
**CN-A- 1 548 439** **CN-A- 101 100 470**
**US-A- 5 859 050** **US-B1- 6 277 879**
**US-B2- 6 670 383**

- **XUE H ET AL: "Highly suppressing wild-type HIV-1 and Y181C mutant HIV-1 strains by 10-chloromethyl-11-demethyl-12-oxo-calanol ide A with druggable profile", JOURNAL OF MEDICINAL CHEMISTRY 2010 AMERICAN CHEMICAL SOCIETY USA LNKD- DOI: 10.1021/JM901653E, vol. 53, no. 3, 2010, pages 1397-1401, XP002622243, ISSN: 0022-2623**
- **ZHOU, CHUNMEI ET AL.: 'Synthesis and anti-HIV activity of (±)-calanolide A and its analogs.' ACTA PHARMACEUTICA SINICA vol. 34, no. 9, 1999, pages 673 - 678, XP008134186**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 216 335 B1

## Description

### Field of the invention

The present invention relates to the compounds that inhibit human immunodeficiency virus type 1 (HIV-1) infection and possess anti-Mycobacterium Tuberculosis (TB) activities. The present invention also relates to the synthesis of the compounds and to the use of such compounds as effective candidate drugs for treatment of HIV-1 and/or TB infections in patients. More specifically, this invention relates to certain tetracyclic dipyrano-coumarin derivatives that possess both anti-HIV-1 and anti-TB activities.

### Background of the invention

Acquired immunodeficiency syndrome (AIDS) caused by HIV infection is a malignant infectious disease affecting human health, and one of the leading causes of morbidity and mortility in human. Since the first case of AIDS patient was reported in USA in 1981 , the FDA has approved total 22 different drugs for the treatment of AIDS patients in clinic. Even though the successful combination treatment of highly active anti-retroviral therapy (HAART) was used, most of the AIDS patients still can not be cured up to now. Because the current launched anti-HIV drugs are expensive and toxic, it is difficult for the administration of the HIV-infected patients for a long term therapy. Furthermore, the emergence of the drug resistance is frequently occurred leading that the therapy has to be ceased. Thus it is a long-sought goal of countries and pharmacologists to develop highly effective drugs against not only the wild strains but also the resistant strains of HIV with low toxic and inexpensive properties.

In 1992, researchers of national cancer institute (NCI) of USA reported 8 novel dipyrano-coumarin derivatives containing tricyclic and tetracyclic rings with anti-HIV-1 activities (J. MED. CHEM. 1992, 35, 2735), which were first isolated from a tropical rainforest plant (*calophllum lunigerum*) in Malaysia. Among them, the most active (+)-calanolide A (**2**) exihibited an $EC_{50}$ value of 0.1 μM and $TC_{50}$ value of 20μM, repectively. The therapeutic index (TI) is within 16-279 (J. MED. CHEM. 1996, 39, 1303). When the concentration reached at 0.1 μM, (+)-calanolide A not only inhibited HIV-1 replication but also protected CEM-SS cells (human T-lymphoblastic cells) from HIV-1 attack. (+)-calanolide A is active against the AZT- -resistant strains (G-9106) of HIV-1 as well as the TIBO of pyridinone-resistant A17 strains. Experimental studies further demonstrated that (+)-calanolide A synergized well with the first line of nucleosides reverse transcriptase inhibitors (NRTIs) against HIV-1 such as AZT or other NNRTIs against HIV in present clinical practice. Conseqently, (+)-calanolide A is confirmed as a novel HIV-1 non-nucleosides reverse transcriptase inhibitor.

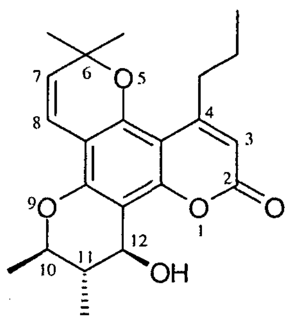

**2**

Recent investigation *in vitro* also demonstrated naturally occurring anti-HIV-1 drug *calophyllum* compounds to be active against TB (Bioorg. Med. Chem., 2004, 1199-1207). (+)-Calanolide A inhibited H37Rv strain growth with $MIC_{90}$ of 3.13 μg/mL. Many patients with AIDS died as a result from a variety of infections, in which infections caused by Mycobacterium Tuberculosis often occurred. To find a novel active compound which can directly inhibit HIV virus and anti-TB has important significance in study of prolonging life of the patients with AIDS and reducing the death rate of the patients caused by TB infection.

In the treatment of the patients with infections of HIV and TB, current strategies, such as the therapies of HAART and antituberculosis drugs, can always result in the related side effects including nausea, vomiting, rash, abnormal liver function and the like, the therapy for HIV has to be ceased. Discontinuing treatment results in a higher probability of the virus infections. Thus, to find a pharmaceutical agent which can be used for treatment of HIV and TB infections in patients has importante significance.

Due to the low content of naturally occurring *culophyllum* compounds in plants, the amount of these compounds which can be extracted from the plants is very limited. It would pose a risk of destroying the environment to obtain a

large amount of the compounds. Chenera and Kucherenko et al. reported the synthesis routes of racemic calanolide A, respectively (J Org Chem, 1993, 58, 5605; Tetrahedron Lett, 1995, 36, 5475). The present inventors also reported the total synthesis of racemic calanolide A and 11-demethyl calanolide A (Acta Pharmaceutica Sinica, 1999, 34 (9): 673; Chinese Chem. Lett. 1997, 8: 859 , Chinese Chem. Lett. 1998, 9: 433). In 2003, the present inventors have applied for Chinese patent (Application Number: 03123628, 6, CN154849A) which described a new synthesis method by using PPA as a reaction reagent to selectively provide a key intermediate of tricyclic coumarin in large scale. In fact, it is important to identify compounds with enhanced activity by modifying the structure of compound 2, and reducing chiral centre of the structure based on the current research results.

In a further research, the present inventors found that compound **3** gave a similar activity level against HIV as compared to the parent native compound (+)-calanolide A *in vitro*;yet compound 3 has two chiral centre carbons less, and is simpler and more convenient to synthesize. Compound **3** can be obtained from phloroglucinol as starting material through three reaction steps. Through the further structural development to compound 3, the invention has been accomplished.

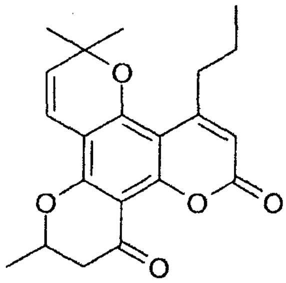

3

US patent application number 5 859 050 A relates to preparation methods of calanolide A and its analogues comprising a significant number of synthetic steps. These analogues have reported activities against HIV- of about 1 uM.

US patent number 6 670 383 B2 relates to compounds and compositions useful in treating or preventing conditions and diseases related to mycobacterium infection.

US patent number 6 277 879 B1 relates to calanolide analogues having reported activities against HIV-1 of about 1 uM.

The article by XUE H. et al. entitled "Highly suppressing wild-type HIV-1 and Y181C mutant HIV-1 strains by 10-chloromethy-11-demethyl-12-oxo-calanolide A with druggable profile", published in the Jomal of Medicinal Chemistry, vol.53, no.3, 2010, pp 1397-1401, relates to calanolide A derivatives useful against HIV-1.

## Disclosure of the invention

In one aspect, the present invention relates to a compound of general formula of (I), (H) or (III), or an optically active form, pharmaceutically acceptable salt or solvate thereof, which is used as drug.

In another aspect, the present invention also relates to a process for preparing the compound of general formula (I), (II) or (III), or an optically active form, pharmaceutically acceptable salt or solvate thereof, which is used as drug.

In yet another aspect, the present invention also relates to a pharmaceutical composition comprising the compound of general formula (I), (II) or (III), an optically active form, pharmaceutically acceptable salt or solvate thereof and a pharmaceutically acceptable carrier or excipient.

In yet another aspect, the present invention also relates to use of the compound of general formula (I), (II) or (III) for the manufacture of a medicament for prevention or treatment of disease resulting from HIV-1 infection including AIDS and disease resulting from Mycobacterium Tuberculosis (TB) infection.

In addition, the present invention also relates to the compounds of formula I for use in a method for treating HIV infection and TB infection.

Specifically, in the first aspect, the invention relates to a compound of general formula (I):

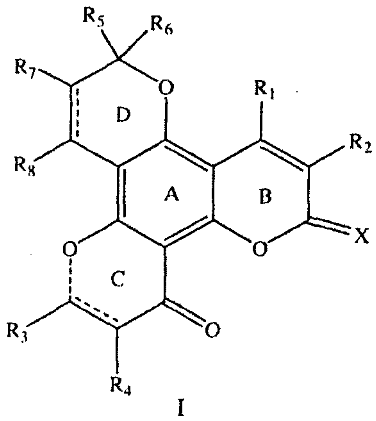

I

or an optical isomer, pharmaceutically acceptable salt or solvate thereof, as defined in the appended claims.

Preferable compounds of general fomula I and related various intermediate and by-products in their synthesis procedure are those wherein X is O, $R_5$ and $R_6$ are methyl, in ring C is carbon-carbon single bond, and in ring D is a carbon-carbon double bond.

Among those compounds mentioned above, more preferably, $R_1$ is $C_{1-6}$ alkyl, $R_2$ is H or halogen, $R_4$ is H; wherein $R_3$ may be dimethyl.

As disclosed herein $R_1$ and $R_2$ may, together with the carbon atom to which they are attached,form an additional 5-member or 6-member ring.

As disclosed herein, $R_1$ is $C_{1-6}$ alkyl, $R_2$ is H, $R_3$ and $R_4$ may be together with each other or together with the carbon atom to which they are attached, form an additional 3-member or 6-member ring.

As disclosed herein, the compounds may also be the compounds of general formula (I) whereinX is O, both of $R_5$ and $R_6$ are methyl, in ring C is a carbon-carbon double bond. In another aspect preferably, the compounds of general formula (I) are those in which X is S, $R_4$ is H, both of $R_5$ and $R_6$ are methyl, in ring C is a carbon-carbon single bond.

Furthermore, as disclosed herein the compound of general formula (I) may also be those in which $R_1$ is $C_{1-6}$ alkyl, $R_2$ is H or halogen, $R_3$ is methyl, $R_4$ is H, one of $R_5$ and $R_6$ is H and the other is $C_{1-6}$ alkyl, alkenyl, alkoxyl, or aryl, in ring C is a carbon-carbon single bond.

Furthermore, when the tetracyclic coumarin compound of claim 1 in which $R_3$ is $CH_2Br$ group is reacted with primary amines, a rearrangement reaction occurs leading to ring C being opened while the structure of tricyclic ring A, B, and D is unchanged. Therefore, the compounds then turn into the structure of the general formula 1'

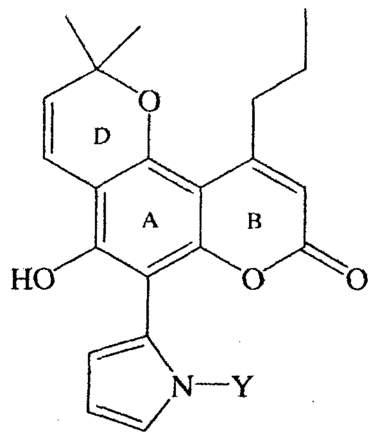

1'

wherein Y is selected from the group consisting of primary amine groups and substituted groups, such as $C_{1-10}$ alkyl chain, different aromatic or unaromatic ring as well as heterocyclic ring , such as isopropyl-, 2-methylene pyridine and 2-methylene furan.

The present disclosure also relates to a compound of general formula (II):

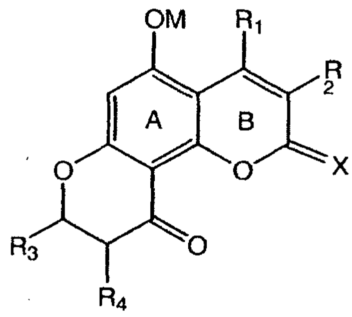

**(II )**

or an optical isomer, pharmaceutically acceptable salt or solvate thereof, wherein:

$R_1$ is cyano, $C_{1-6}$ alkyl, $C_{1-6}$ alkyl substituted by one or more halogen, $C_{3-6}$ cycloalkyl, $C_{1-6}$ alkyl substituted by phenyl, or $C_{1-6}$ alkyl substituted by heterocyclyl, wherein the said phenyl group or heterocyclic group may each be unsubstituted or substituted by one or more substituents selected from the group consisting of $C_{1-6}$ alkyl, $C_{1-6}$ alkoxyl, nitro, amino, cyano, halogen;
$R_2$ is H, halogen, cyano, $C_{1-6}$ alkyl, $C_{1-6}$ alkyl substituted by one or more halogen, $C_{1-6}$ alkyl substituted by phenyl, or $C_{1-6}$ alkyl substituted by heterocyclyl, wherein the said phenyl group or heterocyclic group may each be unsubstituted or substituted selectively by one or more substituents selected from the group consisting of $C_{1-6}$ alkyl, $C_{1-6}$ alkoxyl, nitro, amino, cyano, halogen; or $R_1$ and $R_2$ together with the ring B may form a 5- or 6-member saturated alicyclic ring;
$R_3$ is H, $C_{1-6}$ alkyl, aryl, or a methylene substituted by one or more substituents selected from the group consisting of halogen, cyano, azido, amino, substituted amino, and substituted aminoamide, substituted sulfonylamide, substituted ureido, substituted thioureido , substituted guanidino groups and substituted groups selected from aryl or alkyl;
$R_4$ is H, methyl or ethyl, with the proviso that when $R_1$ is phenyl and $R_2$ is H, $R_3$ and $R_4$ are methyl groups where $R_3$ and $R_4$ are trans or cis; when $R_4$ is H and ═══ in ring C is a carbon-carbon single bond, $R_3$ can be a dimethyl group; furthermore, $R_3$ and $R_4$ may, together with the carbon atom $C_{10}$ and $C_{11}$ to which they are each attached, form an additional 3-member and/or 6-member saturated carbon ring;
M is H, $C_{1-4}$ alkylacyl (alkanoyl), p-toluenesulfonyl group, or $C_{1-4}$ alkyl.

The present disclosure also relates to a compound of general formula (III):

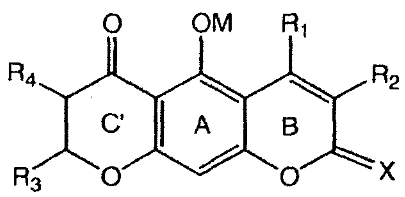

**(III)**

or an optical isomer, pharmaceutically acceptable salt or solvate thereof,
wherein:

$R_1$ is cyano, $C_{1-6}$ alkyl, $C_{1-6}$ alkyl substituted by one or more halogen, $C_{3-6}$ cycloalkyl, $C_{1-6}$ alkyl substituted by heterocyclyl, or $C_{1-6}$ alkyl substituted by phenyl, wherein the said phenyl group or heterocyclyl group may each be unsubstituted or substituted selectively by one or more substituents selected from the group consisting of $C_{1-6}$ alkyl, $C_{1-6}$ alkoxyl, nitro, amino, cyano, halogen;
$R_2$ is H, halogen, cyano, $C_{1-6}$ alkyl, $C_{1-6}$ alkyl substituted by one or more halogen, $C_{1-6}$ alkyl substituted by phenyl, or $C_{1-6}$ alkyl substituted by heterocyclyl, wherein the said phenyl group or heterocyclyl group may each be unsubstituted or substituted selectively by one or more substituents selected from the group consisting of $C_{1-6}$ alkyl, $C_{1-6}$ alkoxyl, nitro, amino, cyano, halogen; or $R_1$ and $R_2$ together with the ring B may form a 5- or 6-member saturated

alicyclic ring;

$R_3$ is H, $C_{1-6}$ alkyl, aryl, or a methylene group substituted by one or more substituents selected from the group consisting of halogen, cyano, azido, amino, substituted amino, and substituted aminoamide, substituted sulfonylamide, substituted ureido, substituted thioureido , substituted guanidino groups and substituted groups selected from aryl or alkyl;

$R_4$ is H, methyl or ethyl; with the proviso that, when $R_1$ is phenyl and $R_2$ is H, $R_3$ and $R_4$ are methyl groups where $R_3$ and $R_4$ are trans or cis; when $R_4$ is H and 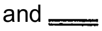 in ring C is a carbon-carbon single bond, $R_3$ may be a dimethyl group; furthermore, $R_3$ and $R_4$ may, together with the carbon atom $C_{10}$ and $C_{11}$ to which they are each attached, form an additional 3-member and/or 6-member saturated carbon ring;

M is H, $C_{1-4}$ alkylacyl, *p*-toluenesulfonyl group or $C_{1-4}$ alkyl.

## Definitions

As used herein, the term "optical isomers" is meant to include, when there exists an unsymmetrical carbon center in the molecule , any enantiomers, diastereomers, mixture of enantimer and diastereomers in any ratios, and racemic mixture thereof and the like.

As used herein, the term "pharmaceutically acceptable salts" means any base additional salts of a compound, which may be formed by a carboxy group present in the compound in complex with alkali metal or alkaline-earth metal such as sodium, kalium, lithium, calcium, magnesium, aluminium, etc; or with ammonia and/or organic amine group; or any acid additional salts, which may be formed by an amino group present in the compound with inorganic acids or organic acids, the inorganic acids include sulfuric acid, phosphonic acid, hydrochloric acid, hydrobromic acid, nitric acid and so on; the organic acids include oxalic acid, citric acid, maleic acid, fumaric acid, malic acid, tartaric acid, sulfonic acid and so on. The pharmaceutically acceptable salts are not limited to any particular salt.

As used herein, the term "solvate" describes a molecular complex comprising a compound of a general fomula described above and an organic solvent or water, wherein the complex contains therein the organic solvent or water molecule(s); or having water or solvent molecules(s) embedded in the crystal lattices of the compound that are detectable by the analytical technology in the art. Pharmaceutically acceptable solvents suitable for purposes of the present invention include those which do not affect the biological activity of the compounds (such as water, ethanol, acetic acid, N,N-dimethylformamaide (DMF), dimethyl sulfoxide (DMSO), or any other solvents known very well or easily confirmed by those skilled in the art. The compound of the present invention can form hydrate or other solvate. It is known that hydrate is formed during freeze-drying the compound and water together, and solvate is formed by condensation of a solution of the compound in a suitable organic solvent. Consequently, both the hydrate and the solvate are also included in the present invention.

In the present invention, the term "alkyl" means both branched- and straight- chain saturated aliphatic hydrocarbon groups having the specified number of carbon atoms.

The term "alkoxyl" means an alkyl group of indicated number of carbon atoms attached through an oxygen bridge.

The term "halogen" or "halo" indicates fluoro, chloro, bromo,or iodo as substituent group; when halogen is as a substituent group, the number of substituent may be one, two or three.

The term "aryl" includes phenyl, substituted phenyl (wherein the substituted group comprises one, two or three substituent selected from the group consisting of $C_{1-6}$ alkyl, $C_{1-6}$ alkoxyl, cyano, nitro, or halogen).

The term "heterocyclic" as used in the present invention represents a stable 5- to 7-membered monocyclic ring, that is either saturated or unsaturated, and is consisted of carbon atoms and one to four heteroatom selected from N, O, and S; wherein the nitrogen and sulfur heteroatoms may optionally be oxidized and the nitrogen heteroatoms may optionally be quaternized. Preferably, the heterocyclic ring is indicated as six-membered ring such as pyridine, piperidine, piperazine, morpholine and thiomorpholine, etc.

In the present invention, when the structure formulas contain "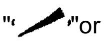"or "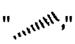" or in the presence of similar sign, it is indicated that the covalent bond is located above the paper or below the paper, respectively; note that unless specified, this type of configuration is meant to be relative. Similarly, when "cis" or "trans" is referred to, they only represent the relative orientation between groups, unless specified.

In the present invention, the compounds having the sign of "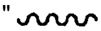" in ring C represents compounds that have not been chirally resolved and are a mixture of two (or more than two) chiral isomers.

The compounds of formula (I) may also appear as either their protected forms or derivatives including those that are not specified herein. These forms or derivatives are readily apparent to those skilled in this art.

In yet another aspect, the present invention also relates to a process for preparing the compound of general formula (I).

In addition, the present invention relates to a process for preparing a tetracyclic coumarin compound of general formula below (4 and 4'), which is characterized in that the compound with ring D is obtained from tricyclic coumarin

compound with rings A, B and C under microwave irradiation as the chemical reaction below:

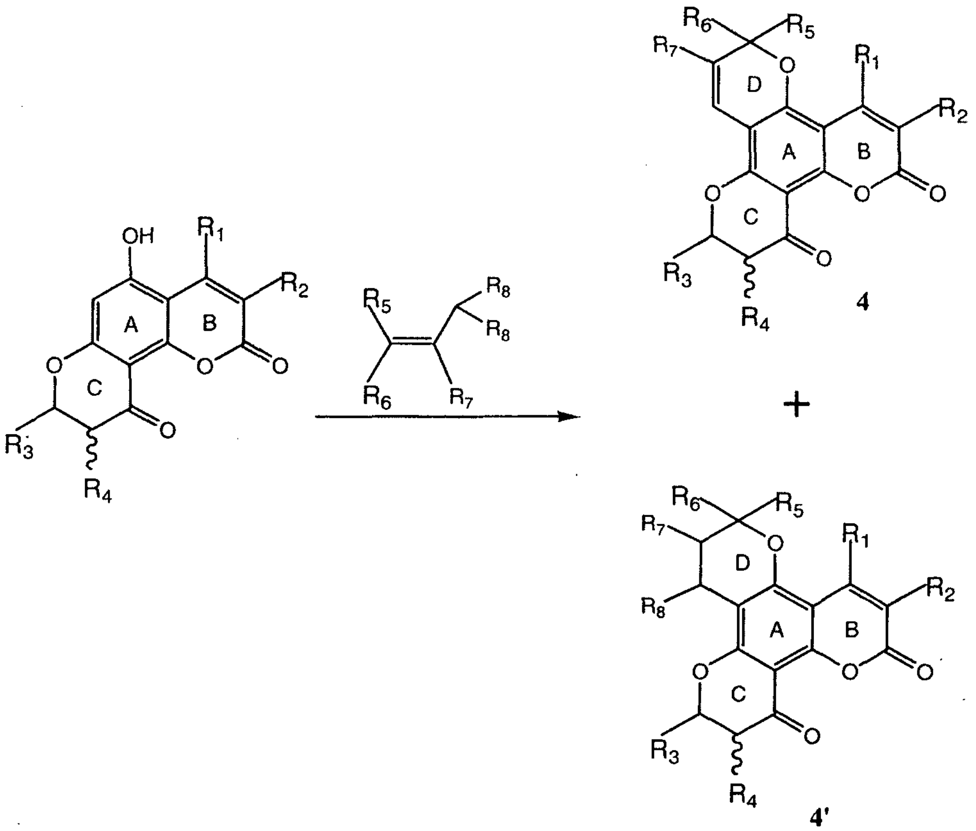

4

4'

wherein $R_1$-$R_8$ are as defined in relation to general formula (I).

The present invention also relates to a process for preparing tetracyclocoumarin compound **4**, which is characterized in that tricyclic coumarin compound **14** with rings A, B and D is reacted with acyl halide of a, β-unsaturated acid to form ring C:

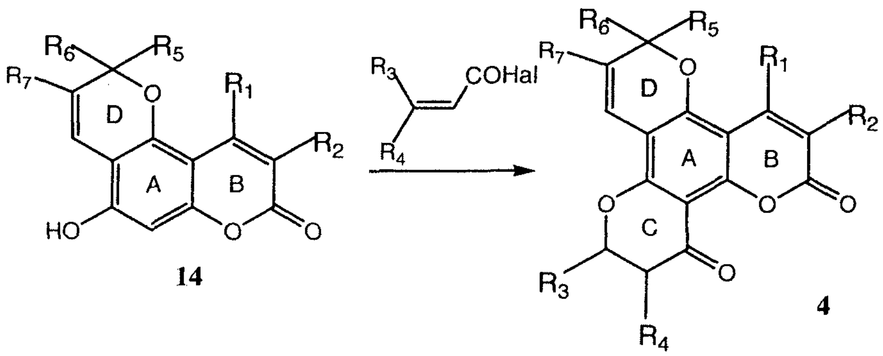

14

4

wherein $R_1$-$R_7$ are as defined in relation to general formula (I).

The present invention further relates to a process for preparing the compound of general formula (I) in which X is S, which is characterized in that the compound is synthesized through the construction of ring D from tricyclic coumarin compound with rings A, B and C according to the process above:

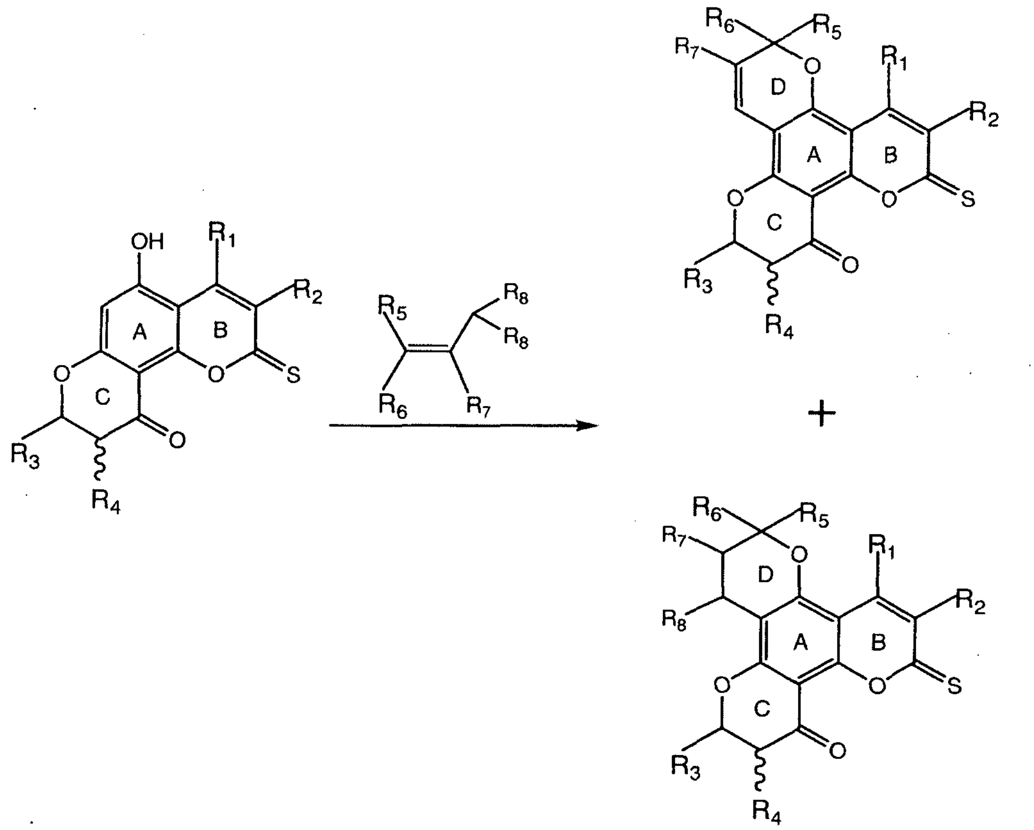

wherein $R_1$-$R_8$ are each as defined the same as above description.

Particularly, the present invention provides a procedure for synthesis of the tetrayclic coumarin compounds through the following reaction schemes 1-6.

The purpose of the present invention is to provide a procedure (scheme 1) for synthesis of compound **4** through an improved Pechman reaction to offer coumarin (6) from phloroglucinol as starting material, characterized in that methanol saturated with dry HCl gas is used to replace usual sulfuric acid catalyst. Good yields are achieved at the room temperature for the condensation reaction of phloroglucinol with $\alpha$-substituted-$\alpha$-alkylacyl (alkanoyl) ethyl acetates, such as ethyl propionylacetate, ethyl butyrylacetate, ethyl benzoylacetate in the methanol containing HCl gas. According to a previous method (CN1548439A), reaction of the obtained compound **6** with crotonic acid in PPA, which acts as both a solvent and a catalyst for cyclization, gives chromenes (**5**a) in high yield and a minor by-product (**5**b). The additional characteristic is to construct ring D from compound **5**a for synthesizing tetracyclic compound (4) by the aid of microwave catalysis, a new synthetic means, as shown in the following reaction scheme 1:

Reaction scheme (**1**):

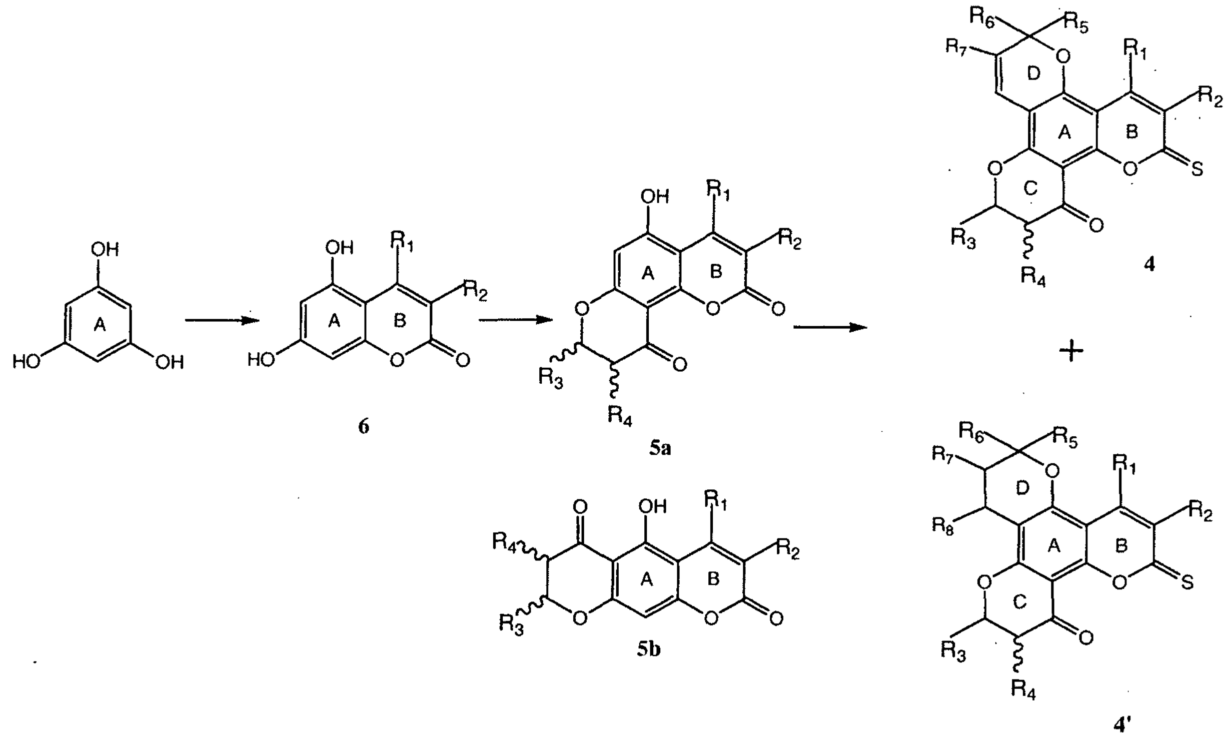

6 5a 5b 4 4'

All compounds mentioned above are racemates. $R_1$-$R_8$ are as defined the same as above description.

Wherein the α-substituted-α-alkanoyl ethyl acetates used in Pechman reaction are obtained with the methods of:

(1) A chlorine atom is introduced at the position $R_2$ of the coumarin (**6**) by the reaction (Cabon O., Buisson D., Larcheveque M., et al. Tetrahedron Asymmetry, 1995, 6 (9): 2199-2210). Further replacement of the chlorine atom with the related reagents can introduce more diversity at the position $R_2$, such as replacement of the chlorine atom through the nucleophilic reagents.

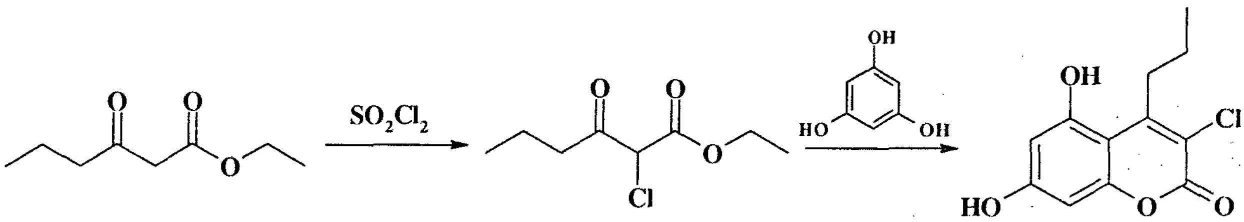


(2) Reaction of malonic acid with acetone resulted in Meldrum's acid (Lermer L., Neeland E. G, Ounsworth J. P., et al. Can J Chem, 1992, 70 (5): 1427-1445). Condensation of the Meldrum's acid with any organic acid offered diversity at the α-position of the Meldrum's acid. Further, diversity is introduced at the $R_1$ position by reaction of phloroglucinol.

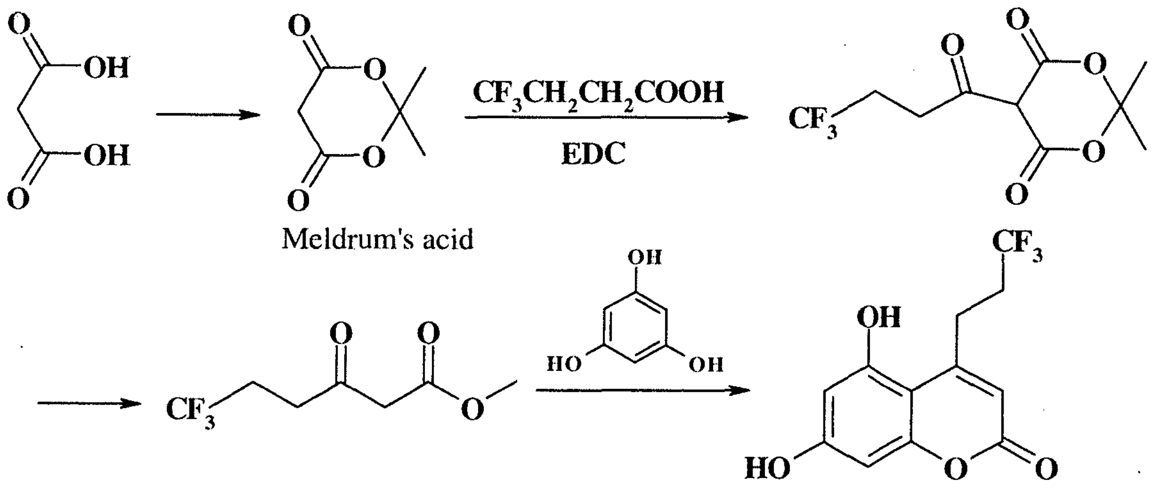


(3) Another characteristic of the present invention is to provide a chloroethyl group at the $R_1$ position by the improved Pechman reaction with α-chloro-α-substituted-α-alkanoyl acetic ester, such as

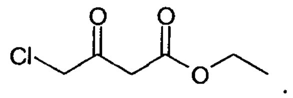

Chlorine atom of the chloroethyl group at the $R_1$ position can be replaced by various reactions to introduce more diversity, such as nucleophilic substituted reaction (as shown scheme below), Grignard reagent reaction, wittig reaction, and so on.

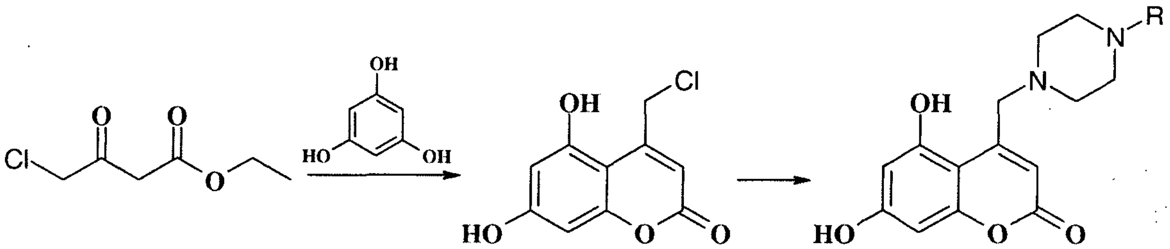

The present invention uses the building blocks shown below for introducing diversity at $R_1$ and/or $R_2$ position and has investigated the structure activity relationships (SARs) for anti-HIV-1 and anti-TB thereof.

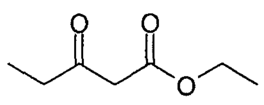
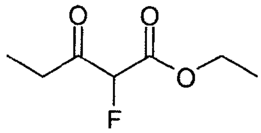
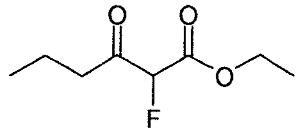
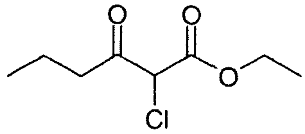

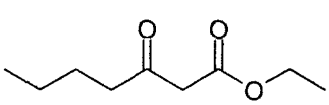
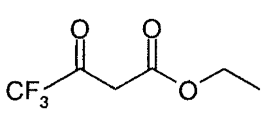
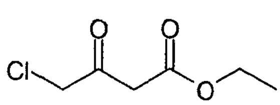
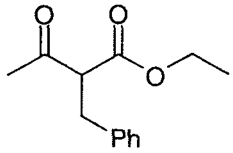

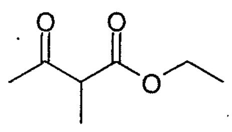
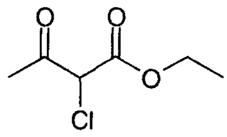
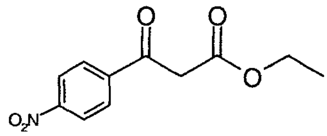
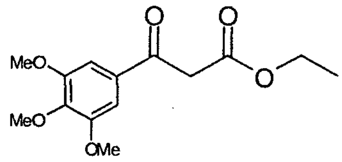

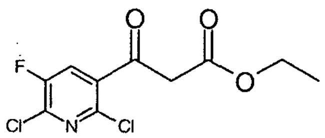
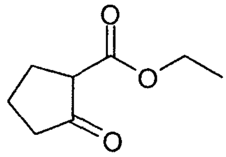
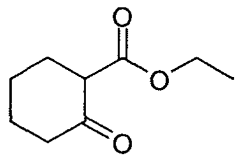
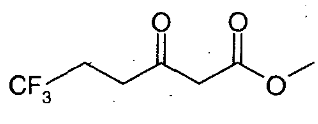

2. The synthetic method of the compound of general formula I in which X is S is described as reaction scheme 2. After the compound (**6**) is obtained as described above, both hydroxyl groups of **6** are protected by groups such as *p*-methylphenylsulfonyl group to give compound **8**. **8** reacts with a thionating reagent to give compound **9** in which the C=O group is replaced by C=S group at the C2 position. Removal of two protective groups gave compound **10**. Accordingly, construction of ring C (**11**) and ring D (**4**) is completed subsequently. The reaction is shown in the following reaction scheme 2:

Reaction scheme 2

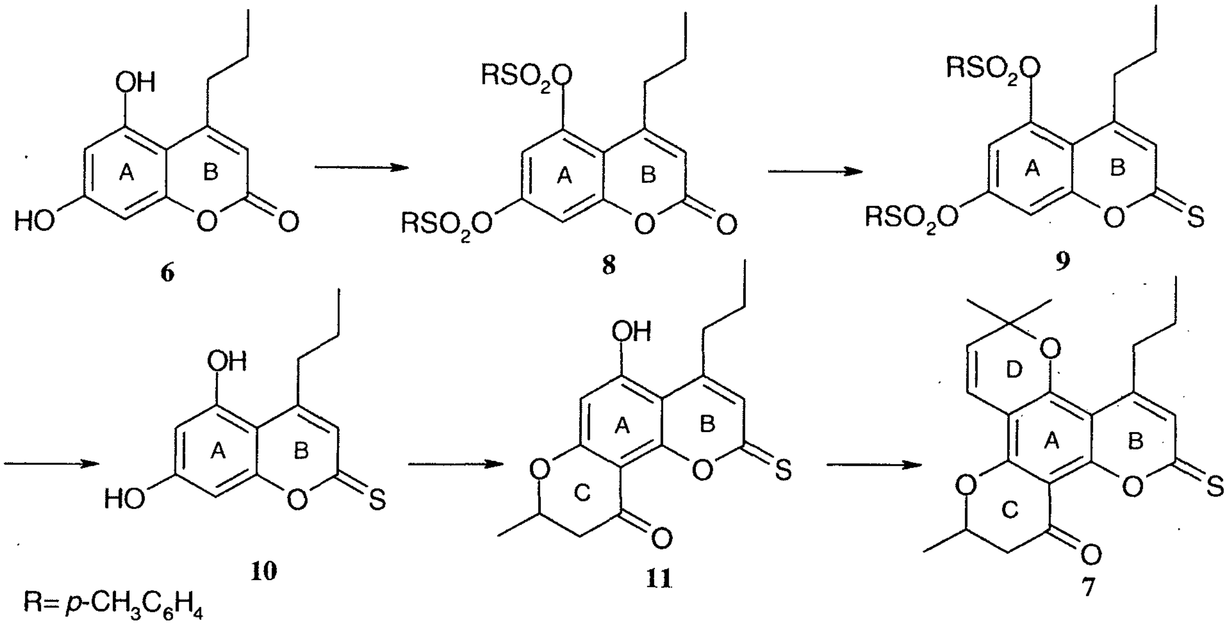

**6** **8** **9** **10** **11** **7**

R= *p*-$CH_3C_6H_4$

3. Another purpose of the present invention is to provide a novel synthetic strategy for the compound of **4** (reaction scheme 3), characterized in that the two protecting groups of compound **8** are selectively removed under different reaction conditions. The 5-OH is selectively free at first to give compound **12,** subsequently ring D is constructed to offer compound **13.** Removal of the remaining protecting group gains 7-OH compound of **14,** which allows constructing the ring C at the last step. To address this novel synthetic strategy, it is valuable with its regioselectivity of reaction for large-scale synthesis of chemical library in a parallel manner synthesis.

Reaction scheme 3

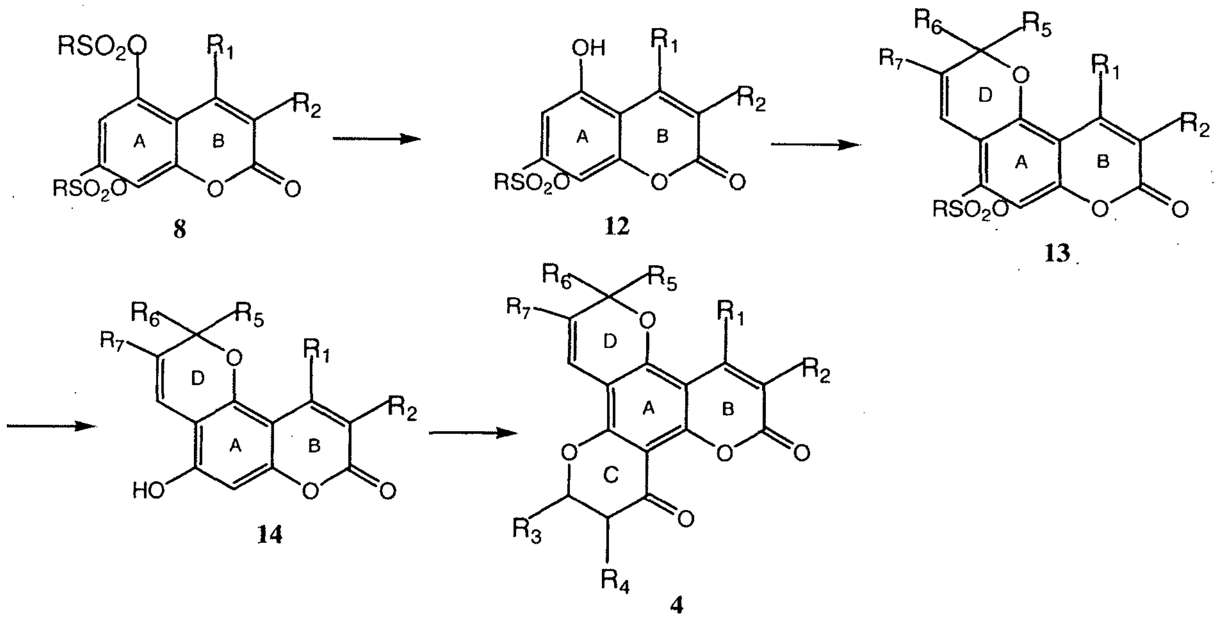

8 12 13 14 4

Wherein, $R_1$-$R_7$ are as defined above.

The synthesis method of the present invention is detailed as follows.

4. The specific reaction conditions of the first synthesis method of the present invention are indicated in reaction scheme 4. The compound **6** is synthesized from phloroglucinol as starting material reacted with β-keto-ethylacetate in dry HCl gas saturated methanol. The tricyclic ring compound **5** can be obtained via the described procedure above. Ring D is finally constructed to give compound **4** by microwave catalysis. When campared to the conventional method, this method has the advantages of significantly shortened reaction time, increased yield, simplified experimental operation, and being suitable for extensive parallel reaction. The reaction is shown in the following reaction scheme 4:

Reaction scheme 4:

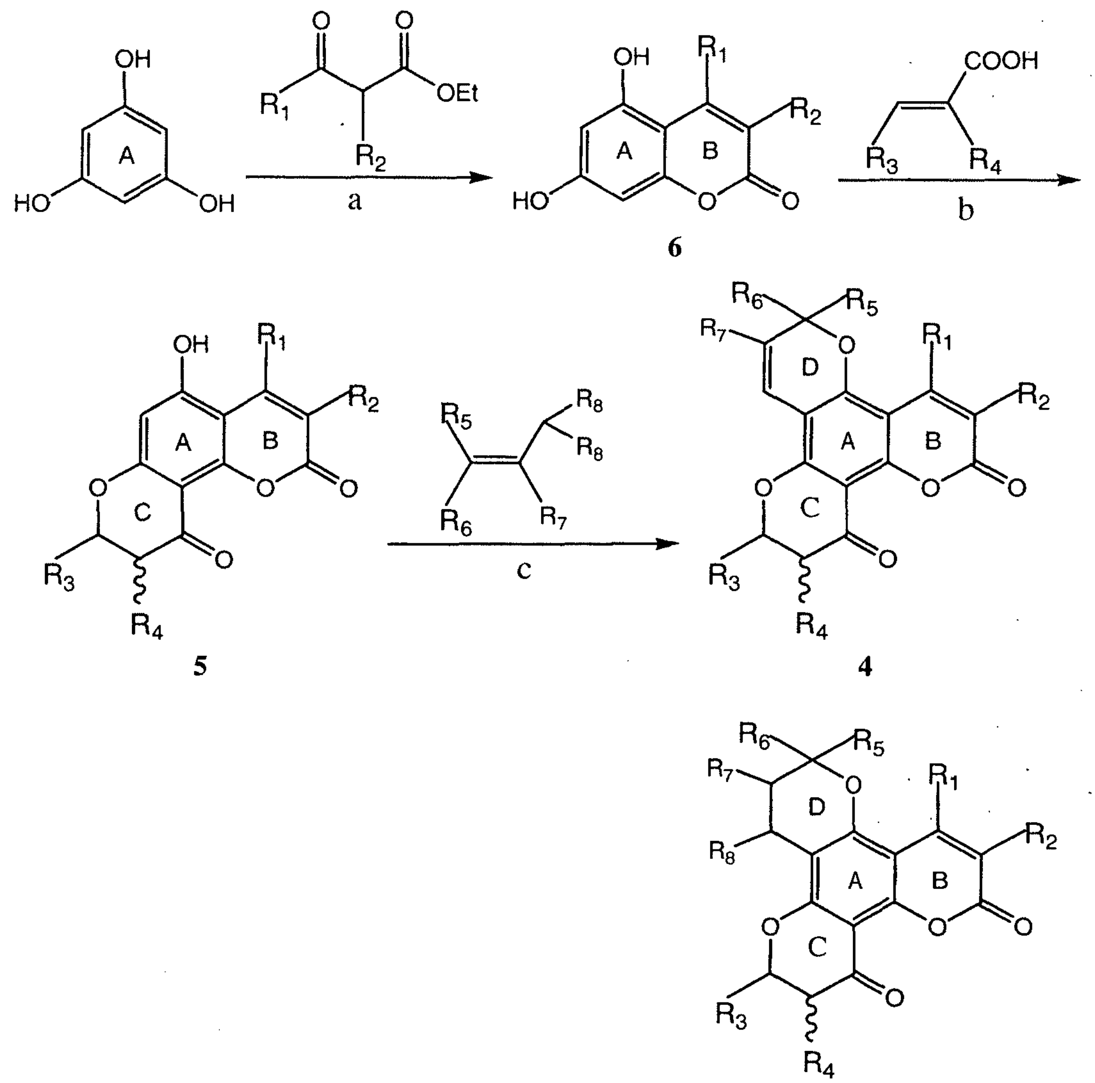


6

5

4

4'

Wherein, $R_1$-$R_8$ are as defined as above.

In the reaction *a*, compound **6** is obtained from phloroglucinol according to the literature method (J. Med. Chem. 1996, 39: 1303-1313). For example, compound **6** is produced by Pechmann reaction of phloroglucinol with $\alpha$-substituted-$\alpha$-alkanoyl acetic esters, such as acetoacetic ester in the presence of phosphoric acid, hydrochloric acid, or sulfuric acid as catalyst. The present inventors found that compound **6** can be obtained by using methanol saturated with dry HCl gas as reaction system. The reaction can be completed at room temperature, without heating. All of the compound **6** produced is directly crystallized out of the reaction system. Thus, the target compound can be obtained by filtration, and no more purification step is necessary. Consequently, it is a preferable method. Suitable reagents for this first reaction step (a) acetoacetic ester reagents bearing corresponding $R_1$, $R_2$ substitutents, for example, ethyl acetylacetate, ethyl propionylacetate, ethyl butyrylacetate, $\gamma$-trifloro-ethyl butyrylacetate, ethyl pentanoylacetate, ethyl 4-substituted-acetylacetate, ethyl 2-substituted-acetylacetate, ethyl 2-substituted- butyrylacetate, ethyl substituted-benzoylacetate containing ethyl benzylacetate, 2-oxo-cyclopentanoic acid ethyl ester, 2-oxo-cyclohexanoic acid ethyl ester, and so on. The molar ratio between the reagents and phloroglucinol ranges from 1:1 to 1:2, with a preferable ratio being 1:1 ; the amount of the solvent used in the medium can be an amount just sufficient to completely dissolve the starting solid.

The reagents used in the second reaction step *b* consist of various corresponding substituted $\alpha$, $\beta$-unsaturated organic acid, such as crotonic acid, 3,3-dimethyl acroleic acid and the like, which is reacted with coumarin compound **6** to give tricyclic compound **5** with ring A, B and C. The molar ratio between $\alpha$, $\beta$-unsaturated organic acid and compound **6** is 1 ~ 1.5: 1, with a preferable range of 1 ~ 1.2: 1 and a more preferable range of 1.05: 1. Using PPA as a catalyst, the reaction temperature is maintained at 50C ~ 100°C, preferable 90°C. The reaction time is 0.5-24h, preferable 1-10h, and more preferably 3-5h. The reaction conditions of b are expemplified in the CN1548439A, but the invention is not limited to those specific conditions.

The reagents used in the third reaction step c consist of substituted propenal acetals with corresponding $R_5$ and $R_6$ groups, such as 1,1-diethoxyl-3-methyl-2-butene, 1,1-diethoxyl-2-butene, 1,41-diethoxyl-2-propene, etc. The acetals are reacted with tricylic compound (5a) to obtain the final product of tetracyclic coumarin compounds **4.** The molar ratio between the tricyclic intermediate (**5**a) and 1,1-diethoxyl-3-methyl-2-butene is 2~6:1, preferably 3~5:1, and more preferably 4:1. The present invention found that the reaction is significantly accelerated by microwave irradiation, thus the reaction time is apparently shortened. The microwave power is set up at 50-300 watt, preferable 100-200 watt, and more preferably 150 watt. The reaction temperature is 80-200°C, preferable 100-150°C, and more preferably 120°C. The irradiation time is between 10-40 minutes, preferably 20 minutes.

5. The present invention also relates to a novel synthesis method of compound (**7**). The characteristic of this method is that two hydroxyl groups of the compound (**6**) are protected by groups such as *p*-methylphenylsulfonyl group to give compound (**8**), and then the replacement of the C=O group with a C=S group by using a thionating reagent to obtain compound (**9**) as indicated in the reaction scheme 5.

Reaction scheme 5

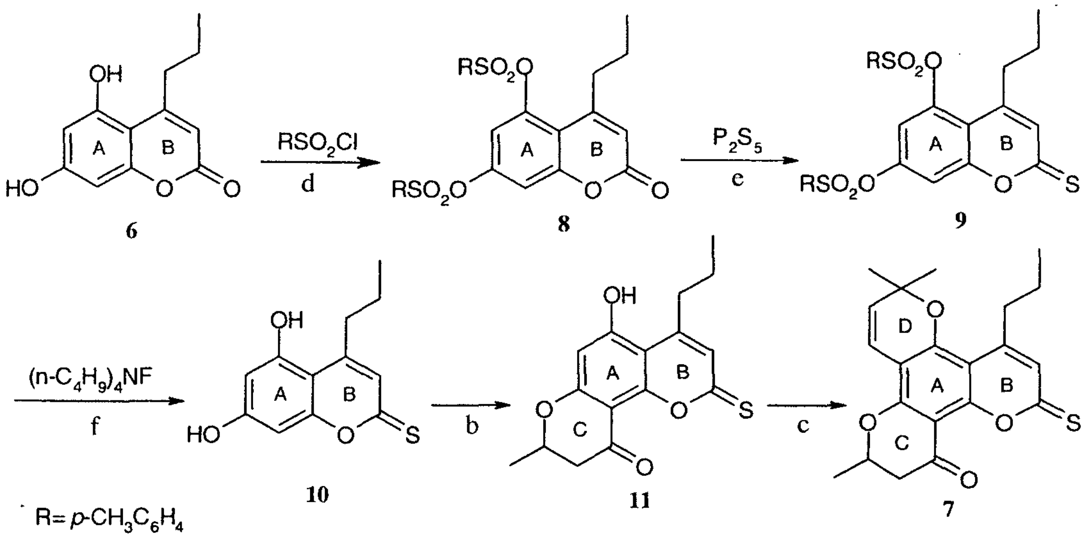


6, 8, 9, 10, 11, 7

The purpose of the first reaction step (*d*) is to protect both hydroxyl groups of compound (**6**) with some sulfonyl chlorides. The preferred protection reagents are methylsulfonyl chloride and paramethylphenylsulfonyl chloride; more preferably, paramelthylphenylsulfonyl chloride. The reaction molar ratio between sulfonyl chloride and compound (**6**) is 4~10:1, preferably 5~8:1, and more preferably 6:1.

The second reaction step e is carried out by employing thionating reagent such as $P_2S_5$ or Lawesson's reagent. A preferred reagent is $P_2S_5$. The reaction molar ratio between $P_2S_5$ and compound (**6**) is 5-20: 1, preferably 8~15:1, and more preferably 10:1. Toluene and dimethyl benzene or other aromatic solvents are used as reaction solvent. A preferable solvent is dimethyl benzene. The reaction temperature is 60°C to the refluxing temperature of the solvent used. The refluxing temperature of the solvent is preferred. Reaction time depends on the specific reaction; generally, it is 9-21 hours, and preferably 12 hours.

The purpose of the third reaction step *f* is to deprotect 5,7-ditosyl protective groups (phenolic hydroxyl group) of compound **9** by using tetrabutyl ammonium salts, such as tetrabutyl ammonium fluoride (TBAF), tetrabutyl ammonium chloride. (TBAC), tetrabutyl ammonium bromide (TBAB). TBAF is preferred. The reaction molar ratio between TBAF and compound **9** may be selected depending on the specified case of the reaction. A preferred reaction molar ratio is about 2:1 and the reaction solvent is aprotic solvent with THF as a preferred solvent. The reaction temperature is from room temperature to the refluxing temperature of the solvent used. The refluxing temperature of the refluxing solvent used is preferred. The reaction time is 3-20 hours, preferably 6~15, and more preferably about 10 hours.

Both reaction steps of *b* and c are as the same as the description above.

In the preceding reaction scheme 5, a specific compound is used as example. Apparently, when the corresponding groups of $R_1$-$R_6$ are as defined the same as mentioned above, the preceding synthetic objects can also be carried out.

6. The present invention further provids a novel synthetic strategy of the compounds of general formula (**4**). The

first protective group at the 7-position of the compound (**8**) is selectively deprotected to give 5-OH of compound (**12**). The ring D is then constructed. The second protective group is subsequently removed to give 9-OH of compound (**14**). The ring C is built lastly as indicated in reaction scheme 6.

Reaction scheme 6

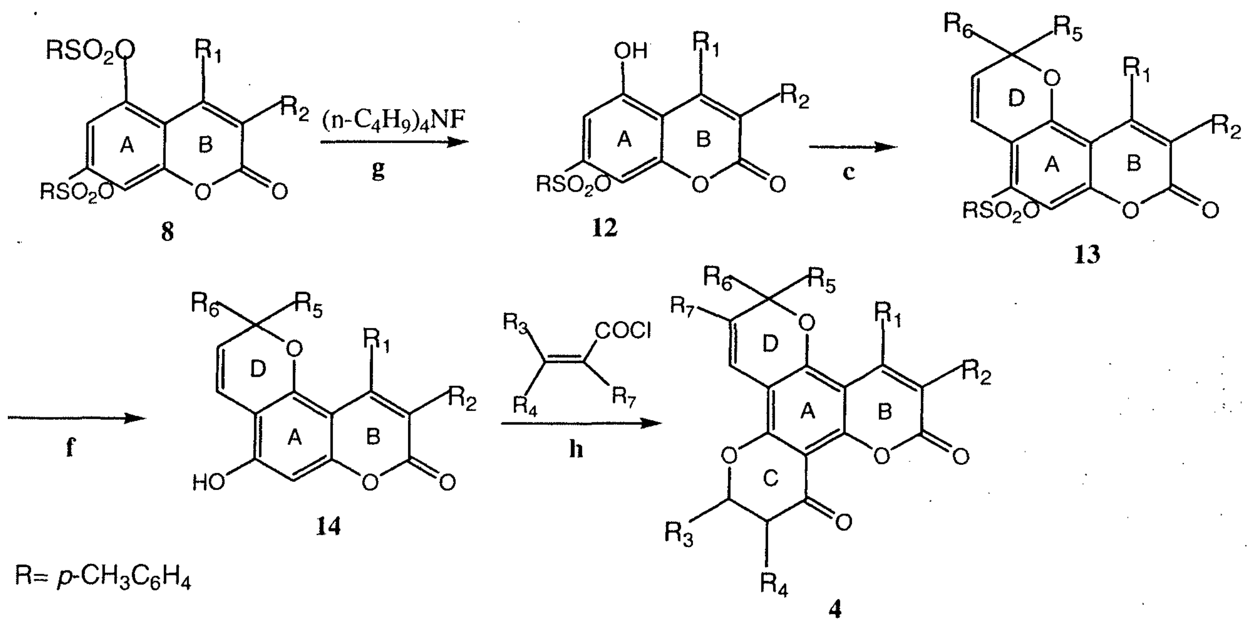


8 12 13 14 4

R= $p$-$CH_3C_6H_4$

wherein $R_1$-$R_8$ are as defined above.

The reaction step g is to deprotect one protective group of compound **8**. The two groups can be selectively removed with different reaction conditions. At room temperature, the protective group at the 5-position is deprotected in priority by using the reagent, solvent and reaction time referred to the reaction step *f* of synthetic scheme 5.

The second reaction step c is as the same as the previous description.

The third reaction step *f* is as the same as the previous description and its reaction temperature is the refluxing temperature of the solvent used.

The forth reaction step *h* utilizes acyl halide of α-β-unsaturated acid, such as acyl chloride of α-β-unsaturated acid. The molar ratio between the acyl chloride and compound (**14**) is 1~6:1, and preferably 4:1. Lewis acid is used as catalyst and $BF_3$/ethyl ether solution is preferable. The reaction temperature is room temperature to the refluxing temperature of the solvent used, and is preferably the refluxing temperature of the solvent used. The reaction time is 1-4 hours, preferably 2 hours.

In addition, when $R_3$ is bromomethyl group, it can be reacted with primary or secondary amines in THF at room temperature. The reaction molar ratio between amine and the corresponding bromide is 2:1. The reaction time is about 12 hours. As shown in the following reaction scheme, it illustrates that, when the reagent is a secondary amine, the reaction gives compound **15** accordingly. When the reagent is a primary amine, the structure of tricyclic ring A, B, D is unchanged and ring C happens to be opened by a rearrangement reaction to give the compound **16** and a byproduct **17**, which has a 3-member saturated cyclic ring formed by $R_3$ at position C10 and $R_4$ at position C11, together with the carbon atom to which they are attached.

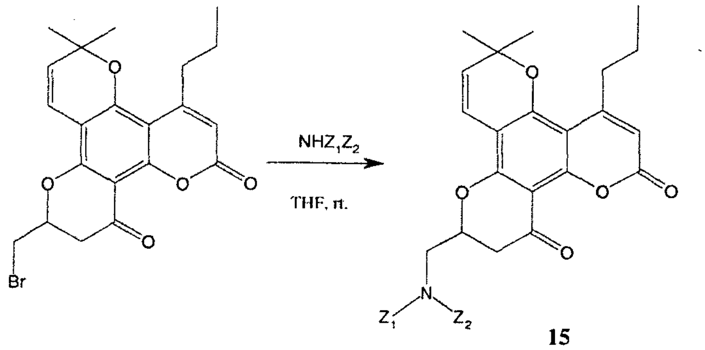


15

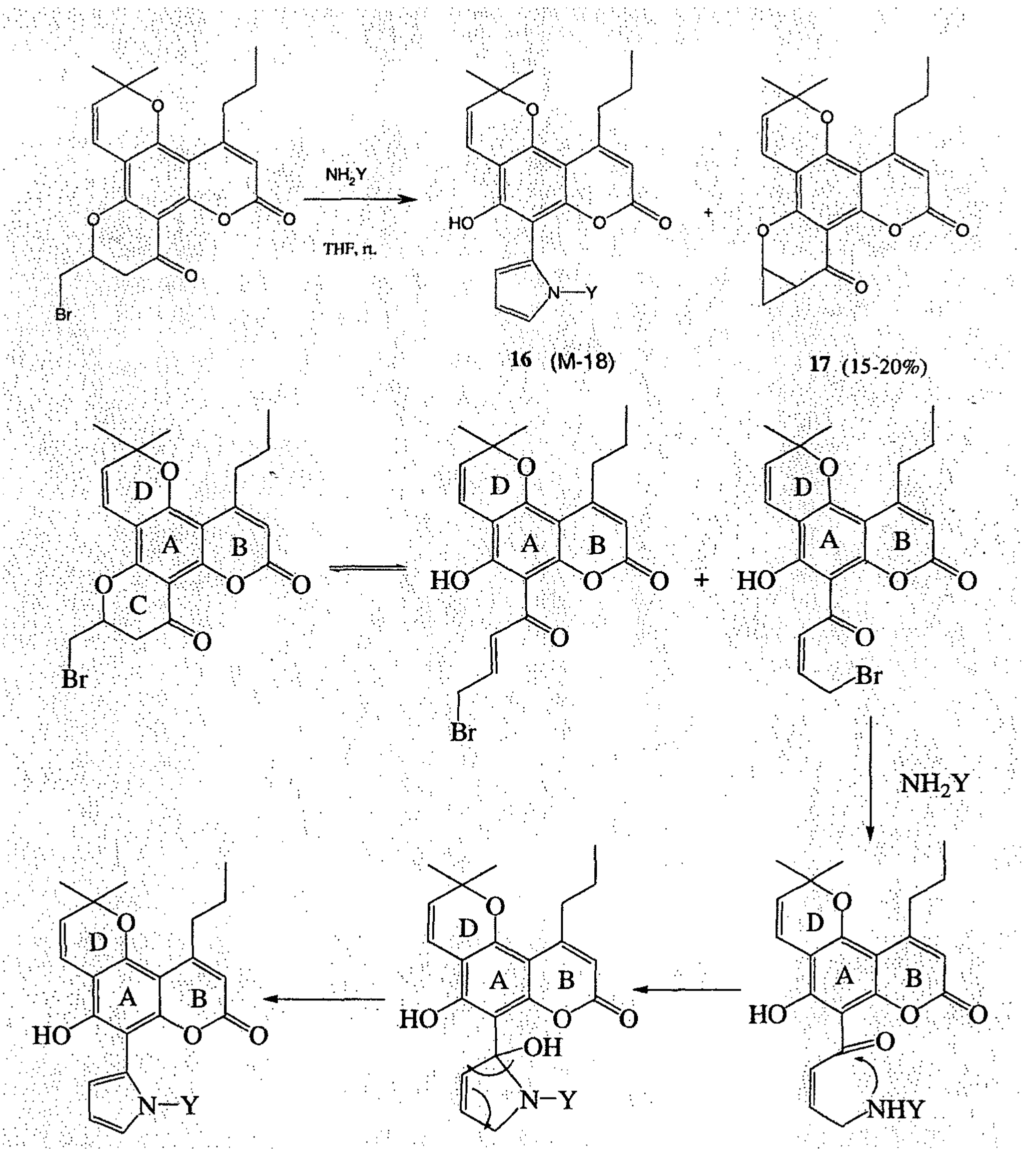


16 (M-18)

17 (15-20%)

Y comes from various primary amines having substituted groups, such as C1-10 aliphatic chains, various aromatic or non-aromantic rings as well as heterocyclic rings. Each of Z1 and Z2 is halogen, cyano, hydroxyl, azido, benzene substituted by amino, C1-10 aliphatic chain or C1-6 alkyl, amide substituted by aryl, sulfonyl amide, ureido, thioureido and guanidino group and so on.

Because of all of the reactions mentioned above possess the characteristic of the rather mild reaction conditions, the shorten reaction time and the stable yield, the synthesis procedure in this invention is compatible with the synthesis of the chemical library by employing combinatorial method. Therefore, the procedure for synthesizing chemical library

is also included in the scope of the present invention.

It is believed that one skilled in the art can modify the preceding procedure in order to improve the yield. They also can use the conventional knowledge in the art to confirm synthetic route or scheme or procedure for example, selection of the reactants, solvents and temperatures of the reaction. This modifications or variations are included in the scope of the present invention. It also can use a different conventional procedure in order to avoid some side reaction and/or improve the yield of product. This method of conventional protective groups can see for example "T. Greene, Protecting Croups in Organic. Synthesis" (the Fourth Edition, John Wiley & Sons, Inc.)

The present invention relates to the pharmaceutical compositions containing the compounds at an effective, nontoxic dosage.

The present invention provides pharmaceuticals containing a therapeutically effective amount of the compounds and one or more pharmaceutically acceptable carriers and/or excipients. The carriers include saline solution, buffered saline solution, glucose, water, glycerin, ethanol and mixtures thereof, which will be illustrated in more detail in the following text. When it is necessary, the pharmaceutical composition also includes a small amount of moisture agent or emulsifying agent, or pH buffered solution. The composition can be a liquid solution, suspension, emulsion, tablet, pill, capsule, substained release formulation or powder. The composition may utilize traditional binder and carrier such as glycery tricarboxylate to prepare suppository. The formulation for oral administration concludes standard carrier such as pharmaceutically gradely mannitol, lactose, starch, magnesium stearate, sodium saccharin, cellulose and magnesium carbonate and so on. The preparation of the pharmaceutical involves mixing, granulating and compressing or solubiling components, according to the need. In the additional route, the pharmaceutical composition may be prepared in nanoparticles.

The pharmaceutical carrier used may be solid or liquid.

The carriers or excipients may include time-delayed substannce known in the art, such as glyceryl monostearate or glyceryl distearate, and may also include wax, ethylcellulose, hydroxypropyl methyl cellulose, and isobutenic methyl ester and so on. When the formulation is used for oral administration, 0.01% Tween 80 in PHOSALPG-50 (condensate of phospholipid and $\alpha$-propylene-glycol (1,2-propanediol), A Nattermann & Cie.GmhH ), in which is used for the preparation of acceptable oral formulation of other compounds. The formulations for oral administration are suitable for all of the compounds in the present invention.

The present disclosure also relates to a method for treatment of HIV infection and/or TB infection.

The present invention relates to the compounds and use of said compounds in the manufacture of a medicament for treating viral infections caused by a broad-spectrum of viruses in mammals. In particular, the present invention is suitable for treating viral infection caused by retrovirus. More specifically, the present invention relates to the compounds which selectively inhibit HIV replication.

The present invention relates to the compounds and use of said compounds in the manufacture of a medicament for treatment of mycrobacteria infection caused by TB in mammals. In particular, the present invention is also suitable for the treatment of tuberculosis disease caused by TB.

The compounds of the present invention can be used in a variety of pharmaceutical forms. If solid carrier is used, the pharmaceutical in formulation can be a tablet form, powder form put into hard capsule, pill form, pastille form or (sugar) lozenge form. The amount of solid carrier can be variable, and preferably will be selected from about 25 mg to about 1.0 g per dosage. If liquid carrier is used, the formulation will be in the form of syrup, emulsion, soft capsule or sterile injectable solution or suspention in non-aqueous suspention in the ampule or the vial.

A various of release systems is known and may use for administration of the compound or any formulations thereof. These formulations include tablet, capsule, injectable solution, capsule in liposome, microparticler, microcapsule and so on.

The method of administrations include but not limited to subcutaneous, intracutaneous, intramuscular, intraperitoneal, intravenous, hypodermatic, iutranasal, intrapulmonary, epidural, intraocular route, but preferably oral administration. The compound may be adminstered by any convenient or other suitable route, such as by injection, or bolus, to be absorbed via epithelial route or mucosal route (eg. oral mucosa, rectal mucosa and intestinal mucosa and so on) or through a carrier or matrix-loaded drug, or adminstered together with other biological active agents. The administrations of drugs may be the whole or the topical administration. When it is used for the treatment or prevention of nasal-, bronchial-, or lung- diseases, preferable administration is oral, nasal, or bronchial, aerosol, or sprayer.

**Examples:**

The following examples are illustrative of the invention but do not deem to limit their scope.

**Experimental**

Melting points were uncorrected and were determined with Yanaco micromelting point apparatus. Microwave

reaction was conducted with a CEM Discover Explorer Microwave Reaction Synthesizer. MS spectra were recorded with an Finnigan LCQ-Advantage spectrometer. Infrared spectra were recorded with an Impaco 400FI-IR spectrometer (KBr). $^1$ H NMR spectra were recorded with ARX-300, -400 NMR spectrometers. Elemental analyses were performed with a Carlo-Erba 1106 elemental analyzer. High resolution MS spectra were recorded with a Agilent LC-MSD/TOF mass spectrometer.

Only the Examples marked "of the invention" are part of the present invention.

Example 1

4,6,6,10-tetranicthyl-2H,6H,12H-benzo[1,2-b:3,4-b':5,6-b"]-tripyranyl-2,1,2-dione (4-1, $R_1$=$R_3$=$R_5$=$R_6$=$CH_3$, $R_2$=$R_4$=H)

(1) 4-methyl-5,7-dihydroxy-coumarin (6-1, $R_1$=$CH_3$, $R_2$=H)

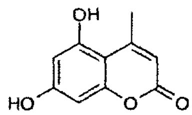

To a mixture of 7.5g (0.046mol) phloroglucinol and 6.0g (0.046mol) acetoacetic ester was added 50 ml methanol saturated with dry hydrochloride gas. The reaction mixture was stirred until phloroglucinol was dissolved under the room temperature, the reaction sulotion was kept three days at room temperature. The solid product was collected by filtration to obtain 8.5g of the title compound as a white powder. Yield, 96%; m.p.282-284°C.
$^1$H-NMR (300MHz, DMSO-$d_6$, ppm): 10.497 (s, 1H, OH), 10.275 (s, 1H, OH), 6.241 (d, 1H. J=2.4Hz, 8-H.), 6.147 (d, 1H, J=2.4Hz, 6-H), 5.822 (s, 1H, 3-H), 2.468 (s, 3H, 4-$CH_3$);
ESI-MS (*m/z*): 193.1 $[M+H]^+$ (MW=192.17);

(2) benzo[1,2-b:3,4-b']-dipyranyl-5-hydroxyl-4,8-dimethyl-2,10-dione (5a-1, $R_1$=$R_3$=$CH_3$ ,$R_2$=$R_4$=H) or benzo[1,2-b:3,4-b']-dipyranyl-5-hydroxyl-4,8-dimethyl-2,6-dione (5b-1, $R_1$=$R_3$=$CH_3$, $R_2$=$R_4$=H).

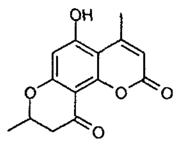

**5a-1**

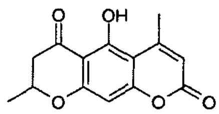

**5b-1**

To a fine mixture of 1.92g (10mmol) 4-methyl-5,7-dihydroxyl-coumarin and 0.92g (10.7 mmol) crotonic acid was added 50 ml polyphosphate (PPA) with stirring at 90□ for 3 hours. The reaction mixture was poured into ice-water with vigorous stirring. A yellow solid powder obtained and was filtrated, washed with water and dried. The solid obtained was separated with silica-gel column chromatography with petroleum ether/ethyl acetate as the eluent to give the 1.6g of the title compound **5a-1** (yield, 62% , m.p.264-266°C) as a white powder and **5b-1** (yield, 10% , m.p.210-211°C) as a white powder.

Compound **5a-1** $^1$H NMR (300MHz, DMSO-$d_6$, ppm): 11.687 (s, 1H, OH), 6.277 (s, 1H, 6-H), 6.056 (s, 1H, 3-H), 4.637 (m, 1H, 8-H), 2.680 (dd, 1H, J=5.4Hz, 16.2Hz, 9-He), 2.592 (dd, 1H, J=2.4Hz, 16.2Hz, 9-Ha), 2.478 (s, 3H, 4-$CH_3$), 1.388 (d, 3H, J=6.3Hz, 8-$CH_3$); ESI-MS (*m/z*): 261.1 $[M+H]^+$ (MW=260.25) ;

Compound **5b-1** $^1$H NMR (300MHz, DMSO-$d_6$, ppm): 13.724 (s, 1H, OH), 6.450 (s, , 1H, 10-H), 5.971 (s, 1H, 3-H), 4.620 (m, 1H, 8-H), 2.780 (m, 2H, 7-$CH_2$), 2.620 (s, 3H, 4-$CH_3$), 1.550 (d, 3H, J=6.3Hz, 8-$CH_3$); ESI-MS (*m/z*): 261.1 $[M+H]^+$ (MW=260.25) ;

(3) 4,6,6,10-tetramethyl-2H,6H,12H-benzo[1,2-b:3,4-b':5,6-b"]-tripyranyl-2,12-dione (4-1, $R_1$=$R_3$=$R_5$=$R_6$=$CH_3$, $R_2$=$R_4$=H)

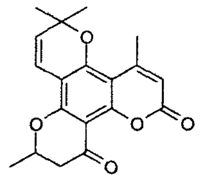

To a solution of benzo[1,2-b:3,4-b']-dipyranyl-5-hydroxyl-4,8-dimethyl-2,10-dione (**5a-1**) (26mg, 0.1mmol) in 5 ml toluene. 1,1-diethoxyl-3-methyl-2-butene (63mg, 0.4mmol) in 1ml pyridine was added. The reaction solution was irradiated with microwave at 150 watt, 120°C for 20 min. The resulting reaction was diluted with dichloromethane (DCM) and then washed with 5% HCl (3×20ml), saturated $NaHCO_3$ (3×20ml) and NaCl (3×20ml) sequentially. The above DCM solution was dried over dry $MgSO_4$ and concentrated in vacuo to give viscous solid, which was purified by silica-gel column chromatography with petroleum ether/ethyl acetate as the eluent to afford 27 mg of the title compound in 83% yield as a off-white powder, m.p.213-215°C.
$^1$NMR (300MHz, DMSO-$d_6$, ppm): 6.640 (d, 1H, J=9.9Hz, 8-H), 6.030 (s, 1H, 3-H), 5.600 (d, 1H, J=9.9Hz, 7-H), 4.640 (m, 1H, 10-H), 2.690 (m, 2H, 11-$CH_2$), 2.560 (s, 3H, 4-$CH_3$), 1.550, 1.540 (2s, 6H, $CH_3$), 1.520 (d, 3H, J=6.3Hz, 10-$CH_3$); ESI-MS (*m/z*): 327.1 $[M+H]^+$ (MW=326.35) ; I
R (KBr , $cm^{-1}$) : 1726, 1685, 1608, 1558, 1336, 1245, 1118, 1082, 885;
Element analysis: Calculated for $C_{19}H_{18}O_5$ (%) : C, 69.93; H, 5.56. Found (%) : C, 69.63; H, 5.53.

Example 2,

3,4,6,6,10-pentamethyl-2H,6H,12H-benzo[1,2-b:3,4-b':5,6-b"]-tripyranyl-2,12-dione (4-2, $R_1$=$R_2$=$R_3$=$R_5$=$R_6$=$CH_3$, $R_4$=H)

(1) 3,4-dimethyl-5,7-dihydroxy-coumarin (6-2, $R_1$=$R_2$=$CH_3$)

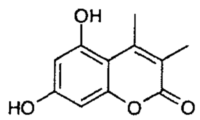

Using the procedure the same as described in the preparative method of compound (6-1), except for using 7.5g (0.046mol) pholoroglucinol and 6.63g (0.046mol) 2-mehyl-acetoacetic ester as starting material to obtain 9.2g of the title compound in 97% yield as a white powder crystalline. Yield, 97% , m.p.235-237°C.
$^1$H-NMR (400MHz, DMSO-$d_6$, ppm): 10.377 (s, 1H, 7-OH), 10.105 (s, 1H, 5-OH), 6.249 (d, 1H, J=2.4Hz, 8-H), 6.127 (d, 1H, J=2.4Hz, 6-H), 2.503 (s, 3H, 4-$CH_3$), 1.982 (s, 3H, 3-C3); ESI-MS (*m/z*): 207.1 $[M+H]^+$ (MW=206.20);

(2) benzo[1,2-b:3,4-b']-dipyranyl-5-hydroxyl-3,4,8-trimethyl-2,10-dione (5a-2, $R_1$=$R_2$=R=$CH_3$, $R_4$=H)

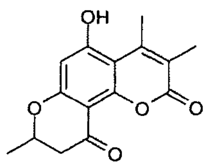

Using the procedure the same as described in the preparative method of compound 5a-1, except for using 2.06g (10mmol) 3,4-dimethyl-5,7-dihydroxyl-coumarin (6-2) and 0.92g (10.7mmol) crotonic acid as starting material to obtain 2.25g ot the title compound in 82% yield as a yellowish powder. m.p.168-170°C.
$^1$H NMR (300MHz, DMSO-$d_6$, ppm): 11.570 (s, 1H, OH), 6.263 (s, 1H, 6-H), 4.617 (m, 1H, J=6.3Hz, 3.9Hz, 11.1Hz, 8-H), 2.636 (dd, 1H, J=11.1Hz, 16.2Hz, 9-He), 2.543 (dd, 1H, J=3.9Hz, 16.2Hz, 9-Ha), 2.489 (s, 3H, 4-$CH_3$), 2.010 (s, 3H, 3-$CH_3$), 1.380 (d, 3H, J=6.3Hz, 8-$CH_3$);

ESI-MS (*m/z*): 275.1 [M+H]+ (MW=274.27);

Element analysis: Calculated for $C_{15}H_{14}O_5 \cdot \frac{4}{3} H_2O$ (%): C, 60.39; H, 5.63. Found (%): C, 60.21; H, 5.59.

(3) 3,4,6,6,10-pentamethyl-2H,6H,12H-benzo[1,2-b:3,4-b':5,6-b"]-tripyranyl-2,12-dione (4-2, $R_1=R_2=R_3=R_5=R_6=CH_3$, $R_4=H$)

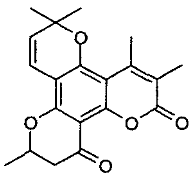

Using the same procedure as described in the preparative method of compound 4-1, except for using 27mg (0.1mmol) benzo[1,2-b:3,4-b']-dipyranyl-5-hydroxyl-3,4,8-trimethyl-2,10-dione (5a-2) and 63mg (0.4mmol) 1,1-diethoxyl-3-methyl-2-butene as starting material to obtain 27 mg of the title compound in 79% yield as off-white solid, m.p.142-144°C. $^1$H-NMR (400MHz, DMSO-$d_6$): 6.557 (d, 1H, J=10.0Hz, 8-H), 5.762 (d, 1H, J=10.0Hz, 7-H), 4.717 (m, 1H, 10-H), 2.696 (dd, 1H, J=12.4Hz, 16.4Hz, 11-He), 2.594 (dd, 1H, J=2.8Hz, 16.4Hz, 11-Ha), 2.515 (s, 3H, $CH_3$), 2.029 (s, 3H, $CH_3$), 1.433 (s, 6H, 6-$CH_3$), 1.478 (d, 3H, J=6.0Hz, 10-$CH_3$);

ESI-MS (*m/z*): 341.1 [M+H]+ (MW=340.38);

Element analysis : Calculated for $C_{20}H_{20}O_5 \cdot \frac{5}{6} H_2O$ C, 67.59; H, 6.14. Found (%): C, 67.37; H, 6.04.

Example 3

4,6,6,10-tetramethyl-3-chloro-2H,6H,12H-benzo[1,2-b:3,4-b':5,6-b"]-tripyranyl-2,12-di one (4-3, $R_1=R_3=R_5=R_6=CH_3$, $R_2=Cl$, $R_4=H$)

(1) 3-chloro-4-methyl-5,7-dihydroxy-coumarin (6-3, $R_1=CH_3$, $R_2=Cl$)

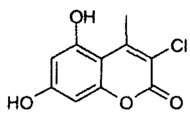

Using the same procedure as described in the preparative method of compound (6-1), except for using 7.5g (0.046mol) phloroglucinol and 5.57g (0.046mol) 2-chloro acetoacetic ester as starting material to obtain 9.8g of the title compound in 94% yield as a white powder crystalline. m.p. >300°C.
$^1$H-NMR (400MHz, DMSO-$d_6$, ppm): 10.762 (s, 1H, 7-OH), 10.433 (s, 1H, 5-OH), 6.312 (d, 1H, J=2.8Hz, 8-H), 6.195 (d, 1H, J=2.8Hz, 6-H), 2.682 (s, 3H, 4-$CH_3$);
ESI-MS (*m/z*): 227.1 [M+H]+ (MW=226.62)

(2) benzo[1,2-b:3,4-b']-dipyranyl-5-hydroxyl-4,8-dimethyl-3-chloro-2,10-dione (5a-3, $R_1=R_3=CH_3$, $R_2=Cl$, $R_4=H$) and benzo[1,2-b:3,4-b']-dipyranyl-5-hydroxyl-4,8-dimethyl-3-chloro-2,6-dione (5b-3, $R_1=R_3=CH_3$, $R_2=Cl$, $R_4=H$).

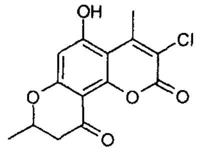

5a-3

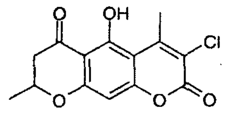

5b-3

Using the same procedure as described in the preparative method of compound 5a-1, except for using 2.26g (10mmol) 3-chloro-4-methyl-5,7-dihydroxy-coumarin (6-3) and 0.92g (10.7mmol) of crotonic acid as starting material to obtain 2.3g of the title compound 5a-3 in 78% yield as a white powder m.p.151-153°C as well as 0.28 g of the title compound 5b-3 in 10% yield as a white powder m.p.179-180°C.

Compound 5a-3: $^1$H NMR (300MHz, DMSO-$d_6$, ppm): 11.931 (s, 1H, OH), 6.329 (s, 1H, 6-H), 4.655 (m, 1H, 8-H), 2.713 (s, 3H, 4-$CH_3$), 2.658 (dd, 1H, J=11.4Hz, 16.5Hz, 9-He), 2.599 (dd, 1H, J=3.9Hz, 16.5Hz, 9-Ha), 1.394 (d, 3H, J=6.3Hz, 8-$CH_3$);
ESI-MS (*m*/*z*): 295.1 $[M+H]^+$ (MW=294.69);
Element analysis: Calculated for $C_{14}H_{11}ClO_5$ (%): C, 57.06; H, 3.76. Found (%): C, 56.88; H, 3.80.

Compound 5b-3: $^1$H-NMR (400MHz, DMSO-$d_6$, ppm): 13.986 (s, 1H, OH), 6.527 (s, 1H, 10-H), 4.765 (m, 1H, 8-H), 2.999 (dd, 1H, J=12.4Hz, 17.2Hz, 7-He), 2.821 (dd, 1H, J=3.2Hz, 17.2Hz, 7-Ha), 2.720 (s, 3H, 4-$CH_3$), 1.443 (d, 3H, J=6.4Hz, 8-$CH_3$);
ESI-MS (*m*/*z*): 295.1 $[M+H]^+$ (MW=294.69);

(3) 4,6,6,10-tetramethyl-3-chloro-2H,6H,12H-benzo(1,2-b:3,4-b':5,6-b"]-tripyranyl-2,12-dione (4-3, $R_1$=$R_3$=$R_5$=$R_6$=$CH_3$, $R_2$=Cl, $R_4$=H)

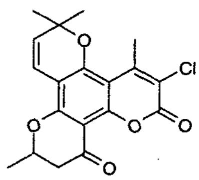

Using the same procedure as described in the preparative method of compound 4-1, except for using 29mg (0.1mmol) benzo[1,2-b:3,4-b']-dipyranyl-5-hydroxyl-4,8-dimethyl-3-chloro-2,10-dione (5a-3) and 63mg (0.4mmol) 1,1.-diethoxyl-3-methyl-2-butene as starting material to obtain 31 mg of the title compound in 86% yield as off-white powder, m.p.141-143°C.
$^1$H-NMR (400MHz, DMSO-$d_6$): 6.585 (d, 1H, J=10.0Hz, 8-H), 5.81.5 (d, 1H, J=10.0Hz, 7-H), 4.733 (m, 1H, 10-H), 2.775 (dd, 1H, J=12.0Hz, 16.4Hz, 11-He), 2.706 (s, 3H, 4-$CH_3$), 2.641 (dd, 1H, J=3.2Hz, 16.4Hz, 11-Ha), 1.510, 1.474 (2s, 6H, 6-$CH_3$), 1.452 (d, 3H, J=6.4Hz, 10-$CH_3$);
ESI-MS (*m*/*z*): 361.1 $[M+H]^+$ (MW=360.80);

Element analysis: Calculated for $C_{19}H_{17}ClO_5 \cdot \frac{2}{3} H_2O$ C, 61.21; H, 4.96. Found (%): C, 61.36; H, 5.12.

Example 4

4,6,6,10-tetramethyl-3-benzyl-2H,6H,12H-benzo[1,2-b:3,4-b':5,6-b"]-tripyranyl-2,12-d ione (4-4, $R_1$=$R_3$=$R_5$=$R_6$=$CH_3$, $R_2$=$CH_2C_6H_5$, $R_4$=H)

(1) 3-benzyl-4-methyl-5,7-dihydroxy-coumarin (6-4, $R_1$=$CH_3$ , $R_2$=$CH_2C_6H_5$)

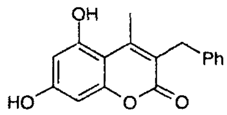

Using the same procedure as described in the preparative method of compound (6-1), except for using 7.5g (0.046mol) phloroglucinol and 10.2g (0.046mol) 2-benzyl acetoacetic ester as starting material to obtain 12.1g of the title compound in 92% yield as a white powder crystalline. m.p. 256-257°C.
$^1$H-NMR (400MHz, DMSO-$d_6$, ppm): 10.469 (s, 1H, 7-OH), 10.202 (s, 1H, 5-OH), 7.228 (m, 5H, Ph), 6.271 (d, 1H, J=2.4Hz, 8-H), 6.165 (d, 1H, J=2.4Hz, 6-H), 3.874 (s, 2H, 3-$CH_2$), 2.513 (s, 3H, 4-$CH_3$);
ESI-MS (*m*/*z*): 283.0 $[M+H]^+$ (MW=282.30) ;

(2) benzo[1,2-b:3,4-b']-dipyranyl-5-hydroxyl-3-benzyl-4,8-dimethyl-2,10-dione (5a-4, $R_1$=$R_3$=$CH_3$, $R_2$=$CH_2C_6H_5$, $R_4$=H) and benzo[1,2-b:3,4-b']-dipyranyl-5-hydroxyl-4,8-diimethyl-3-benzyl-2,6-dione (5b-4, $R_1$=$R_3$=$CH_3$, $R_2$=$CH_2C_6H_5$, $R_4$=H).

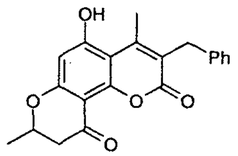

5a-4

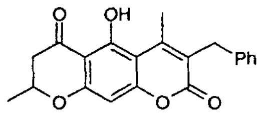

5b-4

Using the same procedure as described in the preparative method of compound 5a-1 in example 1, except for using 2.82g (10mmol) 2-benzyl-4-methyl-5,7-dihydroxyl-coumarin (6-4) and 0.92g (10.7mmol) crotonic acid as starting material to obtain 2.3g of the title compound 5a-4 in 66% yield as a white powder m.p.1.72-174°C as well as 0.6 g of the title compound 5b-4 in 17% yield as a white powder m.p.198-199°C.

Compound 5a-4: $^1$H-NMR (300MHz, DMSO-$d_6$, ppm): 7.230 (m, 5H, 3-$CH_2$-<u>Ph</u>), 6.304 (s, 1H, 6-H), 4.645 (m, 1H, 8-H), 3.913 (s, 2H, 3-C<u>H</u>$_2$-Ph), 2.587 (m, 2H, 9-CH2), 2.517 (s, 3H, 4-$CH_3$), 1.387 (d, 3H, J=6.6Hz, 8-$CH_3$);
ESI-MS (*m*/*z*): 351.1 $[M+H]^+$ (MW=350.37);

Elemental analysis: Calculated for $C_{21}H_{18}O_5\cdot\frac{1}{2}H_2O$ C, 70.19; H, 5.32. Found (%): C, 70.32; H, 5.02.

Compound 5b-4: $^1$H-NMR (300MHz, DMSO-$d_6$, ppm): 13.958 (s, 1H, 5-OH), 7.239 (m, 5H, 3-$CH_2$-<u>Ph</u>), 6.468 (s, 1H, 10-H), 4.739 (ddq, 1H, J=6.3Hz, 3.3Hz, 12.3Hz, 8-H), 3.916 (s, 2H, 3-C<u>H</u>$_2$-Ph), 2.982 (dd, 1H, J=17.4Hz, 12.3Hz, 7-He), 2.799 (dd, 1H, J=17.4Hz, 3.3Hz, 7-Ha), 2.565 (s, 3H, 4-$CH_3$), 1.437 (d, 3H, J=6.3Hz, 8-$CH_3$);
ESI-MS (*m*/*z*): 351.1 $[M+H]^+$ (MW=350.37);
Elemental analysis : Calculated for $C_{21}H_{18}O_5$ (%) : C, 71.99; H, 5.18. found (%): C, 71.87; H, 5.15.

(3) 4,6,6,10-tetramethyl-3-benzyl-2H,6H,12H-benzo[1,2-b:3,4-b':5,6-b"]-tripyranyl-2,12-dione (4-4, $R_1$=$R_3$=$R_5$=$R_6$=$CH_3$, $R_2$=$CH_2C_6H_5$, $R_4$=H)

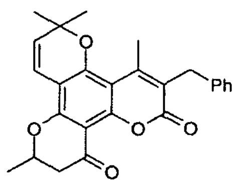

Using the same procedure as described in the preparative method of compound 4-1, except for using 35mg (0.1mmol) benzo[1,2-b:3,4-b']-dipyranyl-5-hydroxyl-3-benzyl-4,8-dimethyl-2,10-dione (5a-4) and 63mg (0.4mmol) 1,1-diethoxyl-3-methyl-2-butene as starting material to obtain 34 mg of the title compound in 82% yield as off-white powder,

m.p.158-159°C.
$^1$H-NMR (400MHz, DMSO-$d_6$): 7.199 (m, 5H, 3-$CH_2$-Ph), 6.587 (d, 1H, J=10.0Hz, 8-H), 5.776 (d, 1H, J=10.0Hz, 7-H), 4.708 (m, 1H, 10-H), 3.951 (s, 2H, 3-$CH_2$), 2.726 (dd, 1H, J=12.0Hz, 16.8Hz, 11-He), 2.631 (dd, 1H, J=3.2Hz, 16.8Hz, 11-Ha), 2.550 (s, 3H, 4-$CH_3$), 1.486, 1.455 (2s, 6H, 6-$CH_3$), 1.448 (d, 3H, J=5.6Hz, 10-$CH_3$);
ESI-MS (*m*/*z*): 417.1 $[M+H]^+$ (MW=416.48);

Elemental analysis: Calculated for $C_{26}H_{24}O_5 \cdot \frac{2}{3} H_2O$ C, 72.88; H, 5.96. found (%) : C, 72.57; H, 5.86.

Example 5

6,6,10-trimethyl-4-chloromethylene-2H,6H,12H-benzo[1,2-b:3,4-b':5,6-b"]-tripyranyl-2 ,12-dione (4-5, $R_1$=$ClCH_2$, $R_2$=$R_4$=H, $R_3$=$R_5$=$R_6$=$CH_3$)

(1) 4-chloromethylene-5,7-dihydroxy-coumarin (6-5, $R_1$=$ClCH_2$, $R_2$=H)

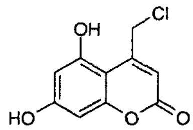

Using the same procedure as described in the preparative method of compound (6-1), except for using 7.5g (0.046mol) phloroglucinol and 7.6g (0.046mol) 4-chloro acetoacetic ester as starting material to obtain 10g of the title compound in 96% yield as a white powder crystalline. m.p. 228-230°C.
$^1$H-NMR (400MHz, DMSO-$d_6$, ppm): 10.881 (s, 1H, 7-OH), 10.414 (s, 1H, 5-OH), 6.273 (d, 1H, J=2.4Hz, 8-H), 6.211 (d, 1H, J=2.4Hz, 6-H), 5.023 (s, 2H, 4-$CH_2$);
ESI-MS (*m*/*z*): 227.1 $[M+H]^+$ (MW=226.62) ;

(2) benzo[1,2-b:3,4-b']-dipyranyl-5-hydroxyl-8-methyl-4-chloromethylene-2,10-dione (5a-5, $R_1$=$ClCH_2$, $R_2$=$R_4$=H, $R_3$=$CH_3$)

Using the same procedure as described in the preparative method of compound 5a-1, except for using 2.26g (10mmol) 4-chloromethylene-5,7-dihydroxy-coumarin (6-5) and 0.92g (10.7mmol) crotonic acid as starting material to obtain 2.5g of the title compound 5a-5 in 85% yield as a yellowish powder, m.p.230-233°C.
$^1$H-NMR (300MHz, DMSO-$d_6$, ppm): 6.496 (s, 1H, 6-H), 6.124 (s, 1H, 3-H), 5.724 (s, 2H, 4-$CH_2$-Cl), 4.707 (m, 1H, 8-H), 2.664 (m, 2H, 9-$CH_2$), 1.425 (d, 3H, J=6.3Hz, 8-$CH_3$);
ESI-MS (*m*/*z*): 295.0 $[M+H]^+$ (MW=294.69);

(3) 6,6,10-trimethyl-4-chloromethylene-2H,6H,12H-benzo[1,2-b:3,4-b':5,6-b"]-tripyranyl-2,12-d ione (4-5, $R_1$=$ClCH_2$, $R_2$=$R_4$=H, $R_3$=$R_5$=$R_6$=$CH_3$)

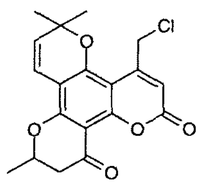

Using the same procedure as described in the preparative method of compound 4-1, except for using 29mg (0.1mmol) benzo[1,2-b:3,4-b']-dipyranyl-5-hydroxyl-8-methyl-4-chloromethylene-2,10-dione (5a-5) and 63mg (0.4mmol) 1,1-diethoxyl-3-methyl-2-butene to obtain 29 mg of the title compound in 80% yield as off-white powder, m.p.132-134°C.
$^1$H-NMR (300MHz, DMSO-$d_6$, ppm): 6.587 (d, 1H, J=10.2Hz, 8-H), 6.507 (s, 1H, 3-H), 5.820 (d, 1H, J=10.2Hz, 7-H),

4.998 (s 2H, 4-$CH_2Cl$), 4.720 (m, 1H, 10-H), 2.668 (m, 2H, 11-CH2), 1.527, 1.492 (2s, 6H, 6-$CH_3$), 1.450 (d, 3H, J=6.3Hz, 10-$CH_3$);
ESI-MS (*m*/*z*): 361.1 $[M+H]^+$ (MW=360.79);
Elemental analysis: Calculated for $C_{19}H_{17}ClO_5$ (%): C, 63.25; H, 4.75. Found (%): C, 63.23; H, 4.87.

Example 6

6,6,10-trimethyl-4-piperazidinylmethylene-10,11-dihydro-6H-benzo[2,3-f:2',3'-h]-tripyranyl-2,12-dione (4-6, $R_1$=piperazidinylmethylene, $R_2$=$R_4$=H, $R_3$=$R_5$=$R_6$=$CH_3$)

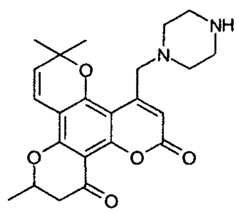

36mg (0.1mmol) 6,6,10-trimethyl-4-chloromethylene-2H,6H,12H-benzo[1,2-b:3,4-b':5,6-b"]-tripyranyl-2,12-dione was reacted with 17mg (0.2mmol) piperazidine for 10 hours at room temperature, after purification by silica-gel column chromatography, the title compound 4-6 was obtained in 64% yield as off-white powder, m.p.156-158°C.
$^1$H-NMR (400MHz, DMSO-$d_6$, ppm): 6.857 (s, 1H, NH), 6.576 (d, 1H, J=10.0Hz, 8-H), 6.467 (s, 1H, 3-H), 5.786 (d, 1H, J=10.0Hz, 7-H), 4.712 (m, 1H, 10-H), 4.309 (m, 4H, $CH_2$), 3.754 (s, 2H, 4-$CH_2$), 3.430 (m, 4H, $CH_2$), 2.754 (dd, 1H, J=11.6Hz, 16.4Hz, 11-He), 2.642 (dd, 1H, J=3.6Hz, 16.4Hz, 11-Ha), 1.502, 1.468 (2s, 6H, 6-$CH_3$), 1.444 (d, 3H, J=6.4Hz, 10-$CH_3$);
ESI-MS (*m*/*z*): 411.2 $[M+H]^+$ (MW=410.47);
High-resolution electrospray ionization mass spectrometry (HRESIMS): Calculated for $C_{23}H_{27}N_2O_5^+$ (*m*/*z*): 411.19199 , Found (*m*/*z*): 411.1921.

<u>Example 7</u>

6,6,10-trimethyl-4-p-methylpiperazidinylmethylene-10,11-dihydro-6H-[2,3-f:2',3'-h]-tri pyranyl-2,12-dione (4-7, $R_1$= p-methylpiperazidinylmethylene, $R_2$=$R_4$=H, $R_3$=$R_5$=$R_6$=$CH_3$)

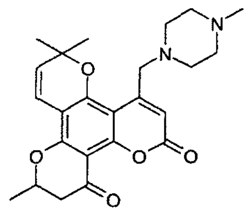

Using the same procedure as described in the preparative method of compound 4-6, except for using 36mg (0.1mmol) 6,6,10-trimethyl-chloromethylene-2H,6H,12H-benzo[1,2-b:3,4-b':5,6-b"]-tripyranyl-2,12-dio ne (4-5, $R_1$=$ClCH_2$, $R_2$=$R_4$=H, $R_3$=$R_5$=$R_6$=$CH_3$) and 20mg (0.2mmol) p-methylpiperazidine as starting material to obatin 32 mg of the title compound 4-7 in 75% yield as off-white powder, m.p.238-239°C.
$^1$H-NMR (400MHz, DMSO-$d_6$, ppm): 6.633 (d, 1H, J=10.0Hz, 8-H), 6.597 (s, 1H, 3-H), 5.605 (d, 1H, J=10.0Hz, 7-H), 4.644 (m, 1H, 10-H), 3.974 (s, 2H, 4-$CH_2$), 3.653 (m, 4H, $CH_2$), 2.977 (m, 4H, $CH_2$), 2.891 (s, 3H, N-$CH_3$), 2.698 (m, 2H, 11-$CH_2$), 1.546, 1.523 (2s, 6H, 6-$CH_3$), 1.254 (d, 3H, J=6.4Hz, 10-$CH_3$);
ESI-MS (*m*/*z*): 425.2 $[M+H]^+$ (MW=424.50);
HRESIMS: Calculated for $C_{24}H_{29}N_2O_5^+$ (*m*/*z*): 425.20764, found (*m*/*z*): 425.2077.

Example 8

6,6,10-trimethyl-4-trifluoromethyl-2H,6H,12H-benzo[1,2-b:3,4-b':5,6-b"]-tripyranyl-2,1 2-dione (4-8, $R_1$=$CF_3$, $R_2$=$R_4$=H, $R_3$=$R_5$=$R_6$=$CH_3$)

(1) 4-trifluoromethyl-5,7-dihydroxy-coumarin (6-8, $R_1$= $CF_3$, $R_2$=H)

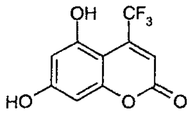

Using the same procedure as described in the preparative method of compound (6-1), except for using 7.5g (0.046mol) phloroglucinol and 8.47g (0.046mol) 4,4,4-trifluoro acetoacetic ester as staring material to obtain 10.4g of the title compound in 92% yield as a yellow powder crystalline. m.p.212-214°C;
$^1$H-NMR (300MHz, DMSO-$d_6$, ppm): 10.901 (s, 1H, 7-OH), 10.654 (s, 1H, 5-OH), 6.523 (s, 1H, 3-H), 6.313 (d, 1H, J=2.4Hz, 6-H), 6.276 (d, 1H, J=2.4Hz, 8-H);
ESI-MS (*m*/*z*): 247.1 $[M+H]^+$ (MW=246.14);

(2) benzo[1,2-b:3,4-b']-dipyranyl-5-hydroxyl-4-trifluoromethyl-8-methyl-2,10-dione (5a-8, $R_1$= $CF_3$, $R_2$=$R_4$=H, $R_3$=$CH_3$) and benzo[1,2-b:3,4-b']-dipyranyl-5-hydroxyl-4-trifluoromethyl-8-methyl-2,6-dione (5b-8 , $R_1$= $CF_3$, $R_2$=$R_4$=H, $R_3$=$CH_3$)

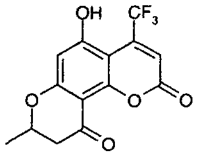
5a-8

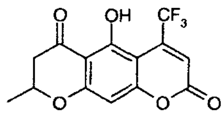
5b-8

Using the same procedure as described in the preparative method of compound 5a-1, except for using 2.47g (10mmol) 4-trifluoromethyl-5,7-dihydroxy-coumarin (6-8) and 0.92g (10.7mmol) crotonic acid as starting material to obtain 1.8g of the title compound 5a-8 in 58% yield as a yellowish powder (m.p.121-123°C) and 0.38g of the title compound 5b-8 in 12% yield as a yellowish powder(m.p.159-160°C).

Compound 5a-8: $^1$H-NMR (300MHz, DMSO-$d_6$, ppm): 12.108 (s, 1H, OH), 6.774 (s, 1H, 3-H), 6.340 (s, 1H, 6-H), 4.687 (qt, 1H, J=6.3Hz, 4.2Hz, 10.5Hz, 8-H), 2.694 (dd, 1H, J=10.5Hz, 16.2Hz, 9-He), 2.623 (dd, 1H, J=4.2Hz, 16.5Hz, 9-Ha), 1.405 (d, 3H, J=6.3Hz, 8-$CH_3$);
ESI-MS (*m*/*z*): 315.1 $[M+H]^+$ (MW=314.22)
Elemental analysis: Calculated for $C_{14}H_9F_3O_5$ (%): C, 53.51; H, 2.89. Found (%): C, 53.41; H, 3.04.

Compound 5b-8: $^1$H-NMR (300MHz, DMSO-$d_6$, ppm): 13.975 (s, 1H, OH), 6.799 (d, 1H, J=0.9Hz, 3-H), 6.600 (d, 1H, J=1.8Hz, 10-H), 4.806 (qt, 1H, J=6.3Hz, 3.3Hz, 12.3Hz, 8-H), 3.017 (dd, 1H, J=12.3Hz, 17.7Hz, 7-He), 2.828 (dd, 1H, J=3.3Hz, 17.7Hz, 7-Ha), 1.451 (d, 3H, J=6.3Hz, 8-$CH_3$);
ESI-MS (*m*/*z*): 315.1 $[M+H]^+$ (MW=314.22)

(3) 6,6,10-trimethyl-4-trifluoromethyl-2H,6H,12H-benzo[1,2-b:3,4-b':5,6-b"]-tripyranyl-2,12-di one (4-8, $R_1$=$CF_3$, $R_2$=$R_4$=H, $R_3$=$R_5$=$R_6$=$CH_3$)

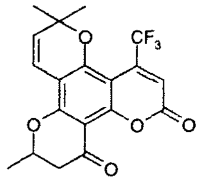

Using the same procedure as described in the preparative method of compound 4-1, except for using 31mg (0.1mmol) benzo[1,2-b:3,4-b']-dipyranyl-5-hydroxyl-4-trifluoromethyl-8-methyl-2,10-dione (5a-8) and 63mg (0.4mmol) 1,1-diethoxyl-3-methyl-2-butene as starting material to obtain 33 mg of the title compound in 87% yield as off-white powder, m.p.152-153°C.
$^1$H-NMR (300MHz, DMSO-$d_6$, ppm): 6.841 (s, 1H, 3-H), 6.591 (d, 1H, J=10.2Hz, 8-H), 5.838 (d, 1H, J=10.2Hz, 7-H), 4.763 (m, 1H, 10-H), 2.755 (dd, 1H, J=11.7Hz, . 16.5Hz, 11-He), 2.657 (dd, 1H, J=3.9Hz, 16.5Hz, 11-Ha), 1.462 (d, 3H, J=6.0Hz, 10-$CH_3$), 1.441,1.408 (2s, 6H, $CH_3$);
ESI-MS (*m*/*z*): 381.1 $[M+H]^+$ (MW=380.32)
Elemental analysis: Calculated for $C_{19}H_{15}F_3O_5$ (%): C, 60.00; H, 3.98. Found (%): C, 59.99; H, 4.18.

Example 9

6,6,10-trimethyl-4-ethyl-2H,6H,12H-benzo[1,2-b:3,4-b':5,6-b"]-tripyranyl-2,12-dione (4-9, $R_1$=$CH_2CH_3$, $R_2$=$R_4$=H, $R_3$=$R_5$=$R_6$=$CH_3$)

(1) 4-ethyl-5,7-dihydroxy-coumarin (6-9, $R_1$= $CH_2CH_3$, $R_2$=H)

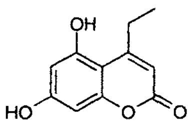

Using the same procedure as described in the preparative method of compound (6-1), except for using 7.5g (0.046mol) phloroglucinol and 6.63g (0.046mol) ethyl propionylacetate as starting material to obtain 9.2g of the title compound in 97% yield as a yellowish powder crystalline. m.p.241-243°C.
$^1$H-NMR (400MHz, DMSO-$d_6$, ppm): 10.556 (s, 1H, 7-OH), 10.266 (s, 1H, 5-OH), 6.264 (d, 1H, J=2.4Hz, 8-H), 6.169 (d, 1H, J=2.4Hz, 6-H), 5.822 (s, 1H, 3-H), 2.899 (q, 2H, J=7.2Hz, 4-C$\underline{H}_2$-$CH_3$), 1.152 (t, 3H, J=7.2Hz, 4-$CH_2$-C$\underline{H}_3$);
ESI-MS (*m*/*z*): 207.1 $[M+H]^+$ (MW=206.20);

(2) benzo[1,2-b:3,4-b']-dipyranyl-5-hydroxyl-4-ethyl-8-methyl-2,10-dione (5a-9 , $R_1$= $CH_2CH_3$, $R_2$=$R_4$=H, $R_3$=$CH_3$) and benzo[1,2-b:5,4-b']-dipyranyl-5-hydroxyl-4-ethyl-8-methyl-2,6-dione (5b-9 , $R_1$= $CH_2CH_3$, $R_2$=$R_4$=H, $R_3$=$CH_3$)

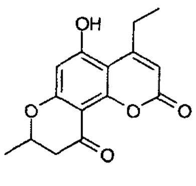
5a-9

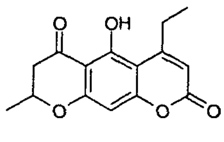
5b-9

Using the same procedure as described in the preparative method of compound 5a-1, except for using 2.06g (10mmol) 4-ethyl-5,7-dihydroxy-coumarin (6-9) and 0.92g (10.7mmol) crotonic acid as starting material to obtain 2.1g of the title compound 5a-9 in 77% yield as a white powder (m.p.165-166°C) and 0.52g of the title compound 5b-9 in 19% yield as a white powder(m.p.192-193°C).
Compound 5a-9 : $^1$HNMR (300MHz, DMSO-$d_6$, ppm): 6.291 (s, 1H, 6-H), 6.036 (s, 1H, 3-H), 4.637 (m, 1H, 8-H), 2.932 (q, 2H, J=7.2Hz, 4-C$\underline{H}_2$$CH_3$), 2.651 (dd, 1H, J=11.4Hz, 16.5Hz, 9-He), 2.564 (dd, 1H, J=3.9Hz, 16.5Hz, 9-Ha), 1.386 (d, 3H, J=6.0Hz, 8-$CH_3$), 1.150 (t, 3H, J=7.2Hz, 4-$CH_2$C$\underline{H}_3$);

ESI-MS (*m*/*z*): 275.1 $[M+H]^+$ (MW=274.27);
Elemental analysis: Calculated for $C_{15}H_{14}O_5 \cdot 2H_2O$ (%): C, 58.06; H, 5.84. Found (%): C, 58.33; H, 5.74.

Compound 5b-9: $^1$H-NMR (400MHz, DMSO-$d_6$, ppm): 13.904 (s, 1H, 5-OH), 6.462 (s, 1H, 10-H), 6.051 (s, 1H, 3-H), 4.743 (m, 1H, 8-H), 2.933 (m, 2H, 7-$CH_2$), 2.816 (q, 2H, J=6.2Hz, 4-C$\underline{H}_2$$CH_3$), 1.434 (d, 3H, J=4.8Hz, 8-$CH_3$), 1.176 (t, 3H, J=6.2Hz, 4-$CH_2$C$\underline{H}_3$); ESI-MS (*m*/*z*): 275.1 $[M+H]^+$ (MW=274.27);
Elemental analysis: Calculated for $C_{15}H_{14}O_5$ (%): C, 6.69; H, 5.15. Found (%): C, 65.60; H, 5.17.

(3) 6,6,10-trimethyl-4-ethyl-2H,6H,12H-benzo[1,2-b:3,4-b':5,6-b"]-tripyranyl-2,12-dione (4-9, $R_1$=$CH_2CH_3$, $R_2$=$R_4$=H, $R_3$=$R_5$=$R_6$=$CH_3$)

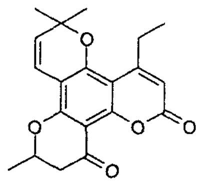

Using the same procedure as described in the preparative method of compound 4-1, except for using 27 mg (0.1mmol) benzo[1,2-b:5,4-b']-dipyranyl-5-hydroxyl-8-methyl-4-ethyl-2,10-dione (5a-9) and 63mg (0.4mmol) 1,1-diethoxyl-3-methyl-2-butene as starting material to obtain 28 mg of the title compound in 84% yield as off-white powder, m.p.133-135°C.
$^1$H-NMR (400MHz, DMSO-$d_6$): 6.593 (d, 1H, J=10.0Hz, 8-H), 6.116 (s, 1H, 3-H), 5.796 (d, 1H, J=10.0Hz, 7-H), 4.716 (m, 1H, 10-H), 2.935 (q, 2H, J=7.2Hz, 4-C$\underline{H}_2$$CH_3$), 2.723 (dd, 1H, J=11.6Hz, 16.4Hz, 11-He), 2.627 (dd, 1H, J=3.6Hz, 16.4Hz, 11-Ha), 1.504, 1.468 (2s, 6H, 6-$CH_3$), 1.447 (d, 3H, J=6.0Hz, 10-$CH_3$), 1.182 (t, 3H, J=7.2Hz, 4-$CH_2$C$\underline{H}_3$);
ESI-MS (*m*/*z*): 341.1 $[M+H]^+$ (MW=340.38);

Elemental analysis: Calculated for $C_{20}H_{20}O_5 \cdot \frac{1}{2} H_2O$ (%): C, 68.75; H, 6.05. Found (%): C, 68.67; H, 5.78.

<u>Example 10,</u>

6,6,10-trimethyl-4-ethyl-3-fluoro-2H,6H,12H-benzo[1,2-b:3,4-b':5,6-b"]-tripyranyl-2,1 2-dione (4-10, $R_1$=$CH_2CH_3$, $R_2$=F, $R_4$=H, $R_3$=$R_5$=$R_6$=$CH_3$)

(1) 4-ethyl-3-fluoro-5,7-dihydroxy-coumarin (6-10, $R_1$= $CH_2CH_3$, $R_2$=F)

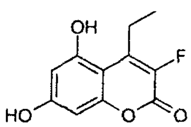

Using the same procedure as described in the preparative methods of compound (6-1), except for using 7.5 g (0.046mol) phloroglucinol and 7.5 g (0.046mol) Ethyl 2-fluoro propionylacetate as starting material to obtain 9.5g of the title compound in 92% yield as a yellowish powder crystalline. m.p.227-229 □.
$^1$H-NMR (400MHz, DMSO-$d_6$, ppm): 10.643 (s, 1H, OH), 10.286 (s, 1H, OH), 6.330 (d, 1H, J=2.4Hz, 8-H), 6.219 (d, 1H, J=2.4Hz, 6-H), 2.981 (dq, 2H, J=3.3Hz, 7.2Hz, 4-C$\underline{H}_2$$CH_3$), 1.177 (t, 3H, J=7.2Hz, 4-$CH_2$C$\underline{H}_3$);
ESI-MS (*m*/*z*): 225.1 $[M+H]^+$ (MW=224.18);

(2) benzo[1,2-b:3,4-b']-dipyranyl-5-hydroxyl-4-ethyl-3-fluoro-8-methyl-2,10-dione (5a-10, $R_1$= $CH_2CH_3$, $R_2$=F, $R_4$=H, $R_3$=$CH_3$) and benzo[1,2-b:5,4-b']-dipyranyl-5-hydroxyl-4-ethyl-3-fluoro-8-methyl-2,6-dione (5b-10 , $R_1$= $CH_2CH_3$, $R_2$=F, $R_4$=H, $R_3$=$CH_3$)

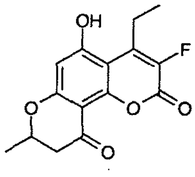

5a-10

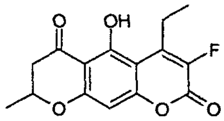

5b-10

Using the same procedure as described in the preparative method of compound 5a-1, except for using 2.24g (10mmol) 4-ethyl-3-fluoro-5,7-dihydroxy-coumarin (6-10) and 0.92g (10.7mmol) crotonic acid as starting material to obtain 1.9g of the title compound 5a-10 in 65% yield as a yellowish powder (m.p.138-141°C) and 0.23g of the title compound 5b-10 in 8% yield as a yellowish powder(m.p.151-152°C).

Compound 5a-10: $^1$H-NMR (300MHz, DMSO-$d_6$, ppm): 11.872 (s, 1H, OH), 6.349 (s, 1H, 6-H), 4.637 (m, 1H, 8-H), 2.982 (q, 2H, J=7.5Hz, 4-$C\underline{H}_2CH_3$), 2.664 (dd, 1H, J=11.1Hz, 16.5Hz, 9-He), 2.596 (dd, 1H, J=3.3Hz, 16.5Hz, 9-Ha), 1.390 (d, 3H, J=6.3Hz, 8-$CH_3$), 1.168 (t, 3H, J=7.5Hz, 4-$CH_2C\underline{H}_3$);

ESI-MS (*m*/*z*): 293.1 $[M+H]^+$ (MW=292.27);

Elemental analysis: Calculated for $C_{15}H_{13}FO_5\cdot\frac{1}{4}H_2O$ C, 60.71; H, 4.58. Found (%): C, 60.66; H, 4.44.

Compound 5b-10: $^1$H-NMR (300MHz, DMSO-$d_6$, ppm): 13.819 (s, 1H, OH), 6.537 (s, 1H, 10-H), 4.750 (m, 1H, 8-H), 2.990 (q, 2H, J=7.5Hz, 4-$C\underline{H}_2CH_3$), 2.965 (dd, 1H, J=12.0Hz, 17.7Hz, 7-He), 2.808 (dd, 1H, J=3.3Hz, 17.7Hz, 7-Ha), 1.440 (d, 3H, J=6.3Hz, 8-$C\underline{H_3}$), 1.201 (t, 3H, J=7.5Hz, 4-$CH_2C\underline{H}_3$);

ESI-MS (*m*/*z*): 293.1 $[M+H]^+$ (MW=292.27)

(3) 6,6,10-trimethyl-4-ethyl-3-fluoro-2H,6H,12H-benzo[1,2-b:3,4-b':5,6-b"]-tripyranyl-2,12-dio ne (4-10, $R_1$=$CH_2CH_3$, $R_2$=F, $R_4$=H, $R_3$=$R_5$=$R_6$=$CH_3$)

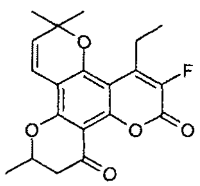

Using the same procedure as described in the preparative method of compound 4-1, except for using 29 mg (0.1mmol) benzo[1,2-b:3,4-b']-dipyranyl-5-hydroxyl-4-ethyl-3-fluoro-8-methyl-2,10-dione (5a-10) and 63mg (0.4mmol) 1,1-diethoxyl-3-methyl-2-butene as starting material to obtain 30 mg of the title compound in 85% yield as off-white powder, m.p.142-144°C.

$^1$H-NMR (300MHz, DMSO-$d_6$, ppm): 6.588 (d, 1H, J=10.2Hz, 8-H), 5.821 (d, 1H, J=10.2Hz, 7-H), 4.712 (m, 1H, 10-H), 2.972 (dq, 2H, J=7.2Hz, 3.6Hz, 4-$C\underline{H_2}CH_3$), 2.725 (dd, 1H, J=11.7Hz, 16.8Hz, 11-He), 2.629 (dd, 1H, J=3.6Hz, 16.8Hz, 11-Ha), 1.505,1.470 (2s, 6H, $CH_3$), 1.448 (d, 3H, J=6.3Hz, 10-$CH_3$), 1.200 (t, 3H, J=7.2Hz, 4-$CH_2C\underline{H}_3$);

ESI-MS (*m*/*z*): 359.2 $[M+H]^+$ (MW=358.37);

Elemental analysis: Calculated for $C_{20}H_{19}FO_5\cdot\frac{1}{5}H_2O$ C, 66.36; H, 5.40. Found (%): C, 66.40; H, 5.44.

Example 11

6,6,10-trimethyl-4-n-propyl-2H,6H,12H-benzo[1,2-b:3,4-b':5,6-b"]-tripyranyl-2,12-dio ne (4-11, $R_1$=n-$C_3H_7$, $R_2$= $R_4$=H, $R_3$=$R_5$=$R_6$=$CH_3$)

(1) 4-n-propyl-5,7-dihydroxy-coumarin (6-11, $R_1$=n-$C_3H_7$)

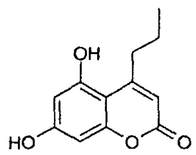

Using the same procedure as described in the preparative method of compound (6-1), except for using 7.5 g (0.046mol) phloroglucinol and 7.3 g (0.046mol) ethyl butyrylacetate as starting material to obtain 9.4g of the title compound in 93% yield as a yellowish powder crystalline. m.p.229-231°C.
$^1$H-NMR (300MHz, DMSO-$d_6$, ppm): 10.558 (s, 1H, OH), 10.270 (s, 1H, OH), 6.256 (d, 1H, J=2.4Hz, 8-H), 6.166 (d, 1H, J=2.4Hz, 6-H), 5.798 (s, 1H, 3-H), 2.824 (t, 2H, J=7.5Hz, 4-$C\underline{H}_2CH_2CH_3$), 1.557 (sex, 2H, J=7.5Hz, 7.2Hz, 4-$CH_2C\underline{H}_2CH_3$), 0.917 (t, 3H, J=7.2Hz, 4-$CH_2CH_2C\underline{H}_3$);
ESI-MS (*m*/*z*): 221.1 $[M+H]^+$ (MW=220.23)

(2) benzo[1,2-b:3,4-b']-dipyranyl-5-hydroxyl-4-n-propyl-8-methyl-2,10-dione (5a-11, $R_1$=n-$C_3H_7$, $R_2$=$R_4$=H, $R_3$=$R_5$=$R_6$=$CH_3$) and benzo[1,2-b:3,4-b']-dipyranyl -5-hydroxyl-4-n-propyl-8-methyl-2,6-dione (5b-11, $R_1$=n-$C_3H_7$, $R_2$=$R_4$=H, $R_3$=$R_5$=$R_6$=$CH_3$)

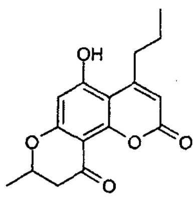

**5a-11**

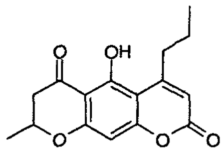

**5b-11**

Using the same procedure as described in the preparative method of compound 5a-1, except for using 2.24g (10mmol) 4-n-propyl-5,7-dihydroxy-coumarin (6-11) and 0.92g (10.7mmol) of crotonic acid as starting material to obtain 2.0g of the title compound 5a-11 in 70% yield as a white powder (m.p.137-138°C) and 0.26g of the title compound 5b-11 in 9% yield as a white powder(m.p.161-162°C).

Compound 5a-11: $^1$H-NMR (300MHz, DMSO-$d_6$, ppm): 11.760 (s, 1H, OH), 6.282 (s, 1H, 6-H), 6.025 (s, 1H, 3-H), 4.635 (m, 1H, 8-H), 2.863 (t, 2H, J=7.5Hz, 4-$C\underline{H}_2CH_2CH_3$), 2.648 (dd, 1H, J=11.1Hz, 16.5Hz, 9-He), 2.562 (dd, 1H, J=4.2Hz, 16.5Hz, 9-Ha), 1.552 (sex, 2H, J=7.5Hz, 7.2Hz, 4-$CH_2C\underline{H}_2CH_3$), 1.385 (d, 3H, J=6.0Hz, 8-$CH_3$), 0.924 (t, 3H, J=7.2Hz, 4-$CH_2CH_2C\underline{H}_3$);
ESI-MS (*m*/*z*): 289.1 $[M+H]^+$ (MW=288.30)

Compound 5b-11: $^1$H-NMR (300MHz, DMSO-$d_6$, ppm): 13.900 (s, 1H, OH), 6.450 (s, 1H, 10-H), 6.042 (s, 1H, 3-H), 4.753 (m, 1H, 8-H), 2.971 (dd, 1H, J=12.3Hz, 17.4Hz, 7-He), 2.875 (t, 2H, J=7.5Hz, 4-$C\underline{H}_2CH_2CH_3$), 2.793 (dd, 1H, J=3.0Hz, 17.4Hz, 7-Ha), 1.584 (sex, 2H, J=7.Hz, 7.2Hz, 4-$CH_2C\underline{H}_2CH_3$), 1.437 (d, 3H, J=6.3Hz, 8-$CH_3$), 0.948 (t, 3H, J=7.2Hz, 4-$CH_2CH_2C\underline{H}_3$);
ESI-MS (*m*/*z*): 289.1 $[M+H]^+$ (MW=288.30)

(3) 6,6,10-trimethyl-4-n-propyl-2H,6H,12H-benzo[1,2-b:3,4-b':5,6-b"]-tripyranyl-2,12-dione (4-11, $R_1$=n-$C_3H_7$, $R_2$= $R_4$=H, $R_3$=$R_5$=$R_6$=$CH_3$)

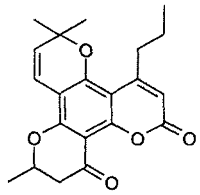

Using the same procedure as described in the preparative method of compound 4-1, except for using 29 mg (0.1mmol) benzo[1,2-b:3,4-b']-dipyranyl-5-hydroxyl-8-methyl-4-n-propyl-2,10-dione (5a-11) and 63mg (0.4mmol) 1,1-diethoxyl-3-methyl-2-butene as starting material to obtain 31 mg of the title compound in 88% yield as white powder, m.p.133-134°C.

$^1$H-NMR (300MHz, DMSO-$d_6$, ppm): $^1$H-NMR (300MHz, DMSO-$d_6$, ppm): 6.566 (d, 1H, J=9.9Hz, 8-H), 6.086 (s, 1H, 3-H), 5.774 (d, 1H, J=9.9Hz, 7-H), 4.707 (m, 1H, 10-H), 2.837 (t, 2H, J=7.5Hz, 4-C$\underline{H}_2$CH$_2$CH$_3$), 2.708 (dd, 1H, J=1.2.0Hz, 16.5Hz, 11-He), 2.606 (dd, 1H, J=3.9Hz, 16.5Hz, 11-Ha), 1.537 (sex, 2H, J=7.5Hz, 7.2Hz, 4-CH$_2$CH$_2$CH$_3$), 1.488, 1.450 (2s, 6H, CH$_3$), 1.439 (d, 3H, J=6.3Hz, 10-CH$_3$), 0.963 (t, 3H, J=7.2Hz, 4-CH$_2$CH$_2$C$\underline{H}_3$);

ESI-MS (*m/z*): 355.1 [M+H]$^+$ (MW=354.41);

Elemental analysis: Calculated for $C_{21}H_{22}O_5 \cdot \frac{1}{2} H_2O$ C, 69.41; H, 6.37. Found (%): C, 69.29; H, 6.03.

Example 12

6,6,10,10-trimethyl-4-n-propyl-2H,6H,12H-benzo[1,2-b:3,4-b':5,6-b"]-tripyranyl-2,12-dione (4-12, $R_1$=n-$C_3H_7$, $R_2$=$R_4$=H, $R_3$=di-CH$_3$, $R_5$=$R_6$=CH$_3$)

(1) benzo[1,2-b:3,4-b']-dipyranyl-5-hydroxyl-8,8-dimethyl-2,10-dione (5a-12, $R_1$=n-$C_3H_7$, $R_3$=di-CH$_3$, $R_2$=$R_4$=H)

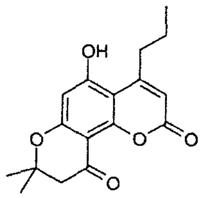

Using the same procedure as described in the preparative method of compound 5a-1, except for using 2.20g (10mmol) 4-n-propyl-5,7-dihydroxyl-coumarin (6-11) and 1.07g (10.7mmol) 3,3-dimethyl-propenoic acid as starting material to obtain 2.62g of the title compound 5a-12 in 87% yield as a white powder m.p.162-164°C.

$^1$H-NMR (400MHz, DMSO-$d_6$, ppm): 11.755 (s, 1H, 5-OH), 6.257 (s, 1H, 6-H), 6.026 (s, 1H, 3-H), 2.861 (t, 2H, J=7.4Hz, 4-C$\underline{H}_2$CH$_2$CH$_3$), 2.688 (s, 2H, 9-CH$_2$), 1.560 (m, 2H, J=7.4Hz, 4-CH$_2$C$\underline{H}_2$CH$_3$), 1.375 (s, 6H, 8-CH$_3$), 0.927 (t, 3H, J=7.4Hz, 4-CH$_2$CH$_2$C$\underline{H}_3$);

ESI-MS (*m/z*): 303.1 [M+H]$^+$ (MW=302.33);

Elemental analysis: Calculated for $C_{17}H_{18}O_5 \cdot \frac{1}{2} H_2O$ C, 65.58; H, 6.15. Found : C, 65.69; H, 5.73.

(2) 6,6,10,10-tetramethyl-4-n-propyl-2H,6H,12H-benzo[1,2-b:3,4-b':5,6-b"]-tripyranyl-2,12-dio ne (4-12, $R_1$=n-$C_3H_7$, $R_2$=$R_4$=H, $R_3$=di-CH$_3$, $R_5$=$R_6$=CH$_3$)

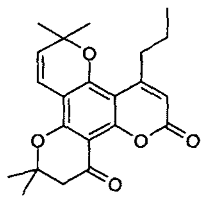

Using the same procedure as described in the preparative method of compound 4-1, except for using 30 mg (0.1mmol) benzo[1,2-b:3,4-b']-dipyranyl-5-hydroxyl-8,8-dimethyl-2,10-dione (5a-12) and 63mg (0.4mmol) 1,1-diethoxyl-3-methyl-2-butene as starting material to obtain 29 mg of the title compound in 80% yield as off-white powder, m.p. 110-112°C.
$^1$H-NMR (400MHz, DMSO-$d_6$, ppm): 6.585 (d, 1H, J=10.4Hz, 8-H), 6.100 (s, 1H, 3-H), 5.783 (d, 1H, J=10.4Hz, 7-H), 2.849 (t, 2H, J=7.6Hz, 4-C$\underline{H}_2$CH$_2$CH$_3$), 2.754 (s, 2H, 11-CH$_2$), 1.566 (m, 2H, J=7.6Hz, 7.4Hz, 4-CH$_2$C$\underline{H}_2$CH$_3$), 1.481 (s, 6H, 6-CH$_3$), 1.417 (s, 6H, 10-CH$_3$), 0.970 (t, 3H, J=7.4Hz, 4-CH$_2$CH$_2$C$\underline{H}_3$);
ESI-MS (*m/z*): 369.2 [M+H]$^+$ (MW=368.43);
Elemental analysis: Calculated for $C_{22}H_{24}O_5$ (%): C, 71.72; H, 6.57. Found (%): C, 71.65; H, 6.55.

Example 13,

6,6-dimethyl-4-n-propyl-2H,6H,12H-benzo[1,2-b:3,4-b':5,6-b"]-tripyranyl-2,12-dione (4-13, R$_1$=n-C$_3$H$_7$, R$_2$=R$_3$=R$_4$=H, R$_5$=R$_6$=CH$_3$,)

(1) 5-hydroxyl-4-n-propyl-benzo[2,3-f]-pyranyl-2,1.0-dione (5a-13, R$_1$=n-C$_3$H$_7$, R$_2$=R$_3$=R$_4$=H)

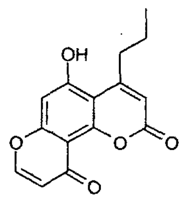

Using the same procedure as described in the preparative method of compound 5a-1, except for using 2.20g (10mmol) 4-n-propyl-5,7-dihydroxyl-coumarin (6-11) and 1.14g (10.7mmol) trans-3-chloro-propenoic acid as starting material to obtain 1.03g of the title compound 5a-13 in 28% yield as a white powder, m.p.116-118°C.
$^1$H-NMR (300MHz, DMSO-$d_6$): 11.861 (s, 1H, 5-OH), 8.134 (d, 1H, J=9.9Hz, 8-H), 6.841 (s, 1H, 6-H), 6.292 (d, 1H, J=9.9Hz, 9-H), 6.174 (s, 1H, 3-H), 2.924 (t, 2H, J=7.5Hz, 10-CH$_2$), 1.597 (sex, 2H, J=7.5Hz, 7.2Hz, 11-CH$_2$), 0.952 (t, 3H, J=7.2Hz, 12-CH$_3$);
ESI-MS (*m/z*): 273.1 [M+H]$^+$ (MW=272.26);

(2) 6,6-dimethyl-4-n-propyl-2H,6H,12H-benzo[2,3-f:2',3'-h]-tripyranyl-2,12-dione (4-13, R$_1$=n-C$_3$H$_7$, R$_2$=R$_3$=R$_4$=H, R$_5$=R$_6$=CH$_3$,)

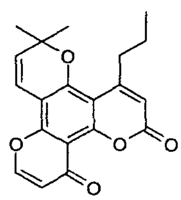

Using the same procedure as described in the preparative method of compound 4-1, except for using 27 mg (0.1mmol) 5-hydroxyl-4-n-propyl-benzo[2,3-f]-pyranyl-2,10-dione (5a-13) and 63mg (0.4mmol) 1,1-diethoxyl-3-methyl-2-butene as starting material to obtain 24 mg of the title compound in 71% yield as off-white powder, m.p.102-104°C. $^1$H-NMR (300MHz, DMSO-$d_6$): 8.142 (d, 1H, J=9.9Hz, 10-H), 6.692 (d, 1H, J=10.5Hz, 8-H), 6.374 (d, 1H, J=9.9Hz, 11-H), 6.238 (s, 1H, 3-H), 5.923 (d, 1H, J=9.9Hz, 7-H), 2.885 (t, 2H, J=7.5Hz, 13-$CH_2$), 1.591 (sex, 2H, J=7.5Hz, 7.2Hz, 14-$CH_2$), 1.505 (s, 6H, 6-$CH_3$), 0.993 (t, 3H, J=7.2Hz, 15-$CH_3$);
ESI-MS (*m*/*z*): 339.2 $[M+H]^+$ (MW=338.36);

Example 14

6,6-dimethyl-4,10-di-n-propyl-2H,6H,12H-benzo[1,2-b:3,4-b':5,6-b"]-tripyranyl-2,12-d ione

(1) benzo[1,2-b:3,4-b']-dipyranyl-5-hydroxyl-4,8-di-n-propyl-2,10-dione (5a-14, $R_1$=$R_3$=n-$C_3H_7$, $R_2$=$R_4$=H) and benzo [1,2-b:3,4-b']-dipyranyl-5-hydroxyl-4,8-di-n-propyl-2,6-dione (5b-14, $R_1$=$R_3$=n-$C_3H_7$, $R_2$=$R_4$=H)

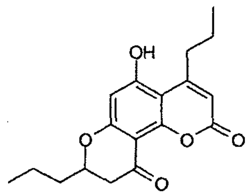

5a-14

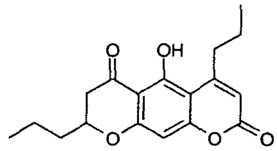

5b-14

Using the same procedure as described in the preparative method of compound 5a-1, except for using 2.24g (10mmol) 4-n-propyl-5,7-dihydroxyl-coumarin (6-11) and 1.22g (10.7mmol) trans-2-hydrosorbic acid as starting material to obtain 2.5g of the title compound 5a-14 in 79% yield as a white powder (m.p.121-123°C) and 0.32g of the title compound 5b-14 in 10% yield as a white powder(m.p.136-138°C).

Compound 5a-14: $^1$H-NMR (300MHz, DMSO-$d_6$): 11.768 (s, 1H, 5-OH), 6.284 (s, 1H, 6-H), 6.031 (s, 1H, 3-H), 4.514 (m, 1H, 8-H), 2.864 (t, 2H, J=7.5Hz, 11-$CH_2$), 2.653 (dd, 1H, J=11.4Hz, 16.5Hz, 9-He), 2.562 (dd, 1H, J=3.9Hz, 16.5Hz, 9-Ha), 1.714 (m, 2H 14-$CH_2$), 1.556 (sex, 2H, J=7.5Hz, 7.2Hz, 12-$CH_2$), 1.432 (m, 2H, 15-$CH_2$), 0.925 (t, 3H, J=7.2Hz, 13-$CH_3$), 0.900 (t, 3H, J=7.2Hz, 1.6-$CH_3$);
ESI-MS (*m*/*z*): 317.0 $[M+H]^+$ (MW=316.36);

Elemental analysis: Calculated for $C_{18}H_{20}O_5 \cdot \frac{1}{2} H_2O$ (%): C, 66.45; H, 6.50. Found (%): C, 66.44; H, 6.29.

Compound 5b-14: $^1$H-NMR (300MHz, DMSO-$d_6$): 13.876 (s, 1H, 5-OH), 6.427 (s, 1H, 10-H), 6.030 (s, 1H, 3-H), 4.626 (m, 1H, 8-H), 2.953 (dd, 1H, J=12.3Hz, 17.4Hz, 7-He), 2.862 (m, 2H, 11-$CH_2$), 2.782 (dd, 1H, J=3.3Hz, 17.4Hz, 7-Ha), 1.712 (m, 2H, 14-$CH_2$), 1.576 (sex, 2H, J=7.2Hz, 7.5Hz, 12-$CH_2$), 1.442 (m, 2H, 15-$CH_2$), 0.945 (t, 3H, J=7.2Hz, 13-$CH_3$), 0.922 (t, 3H, J=7.2Hz, 16-$CH_3$);
ESI-MS (*m*/*z*): 317.0 $[M+H]^+$ (MW=316.36);

(2) 6,6-dimethyl-4,10-n-propyl-2H,6H,12H-benzo[1,2-b:3,4-b':5,6-b"]-tripyranyl-2,12-dione (4-14, $R_1$=$R_3$=n-$C_3H_7$, $R_2$=$R_4$=H, $R_5$=$R_6$=$CH_3$)

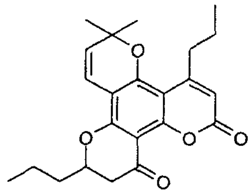

Using the same procedure as described in the preparative method of compound 4-1, except for using 32 mg (0.1 mmol) benzo[1,2-b:3,4-b']-dipyranyl-5-hydroxyl-4,8-di-n-propyl-2,10-dione (5a-14) and 63mg (0.4mmol) 1,1-diethoxyl-3-methyl-2-butene as starting material to obtain 31 mg of the title compound in 81% yield as off-white powder, m.p.141-143°C.

$^1$H-NMR (300MHz, DMSO-$d_6$): 6.549 (d, 1H, J=10.2Hz, 8-H), 6.082 (s, 1H, 3-H), 5.782 (d, 1H, J=10.2Hz, 7-H), 4.577 (o, 1H, J=3.6Hz, 11.7Hz, 4.2Hz, 10-H), 2.830 (t, 2H, J=7.8Hz, 12-$CH_2$), 2.704 (dd, 1H, J=11.7Hz, 16.5Hz, 11-He), 2.596 (dd, 1H, J=3.6Hz, 16.2Hz, 11-Ha), 1.766 (m, 2H, 15-$CH_2$), 1.673 (m, 2H, 13-$CH_2$), 1.561 (m, 2H, 16-$CH_2$), 1.490, 1.450 (2s, 6H, 6-$CH_3$), 0.962 (t, 3H, J=6.9Hz, 14-$CH_3$), 0.934 (t, 3H, J=7.2Hz, 17-$CH_3$);

ESI-MS (*m*/*z*): 383.1 $[M+H]^+$ (MW=382.46);

Elemental analysis: Calculated for $C_{23}H_{26}O_5\cdot\frac{1}{8}H_2O$ (%): C, 71.80; H, 6.87. Found (%): C, 71.75; H, 6.73.

Example 15

6,6-dimethyl-4-n-propyl-10-n-pentyl-2H,6H,12H-benzo[1,2-b:3,4-b':5,6-b"]-tripyranyl-2,12-dione (4-15, $R_1$=n-$C_3H_7$, $R_2$=$R_4$=H, $R_3$=n-$C_5H_{11}$, $R_5$=$R_6$= $CH_3$)

(1) benzo[1,2-b:3,4-b']-dipyranyl-5-hydroxyl-4-n-propyl-8-n-pentyl-2,10-dione (5a-15, $R_1$=n-$C_3H_7$, $R_2$=$R_4$=H, $R_3$=n-$C_5H_{11}$)

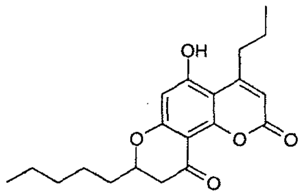

Using the same procedure as described in the preparative method of compound 5a-1, except for using 2.20g (10mmol) of 2.24g (10mmol) 4-n-propyl-5,7-dihydroxyl-coumarin (6-11) and 1.52g (10.7mmol) trans-2-n-octenoic acid as starting material to obtain 2.2g of the title compound 5a-15 in 64% yield as a yellowish powder, m.p.146-148°C.

$^1$H-NMR (300MHz, DMSO-$d_6$): 11.765 (s, 1H, 5-OH), 6.288 (s, 1H, 6-H), 6.036 (s, 1H, 3-H), 4.510 (m, 1H, 8-H), 2.868 (t, 2H, J=7.5Hz, 11-$CH_2$), 2.657 (dd, 1H, J=11.1Hz, 16.5Hz, 9-He), 2.566 (dd, 1H, J=3.9Hz, 16.5Hz, 9-Ha), 1.707 (m, 2H, 14-$CH_2$), 1.558 (sex, 2H, J=7.5Hz, 7.2Hz, 12-$CH_2$), 1.416 (m, 2H, 15-$CH_2$), 1.283 (m, 4H, 16,17-$CH_2$), 0.926 (t, 3H, J=7.2Hz, 13-$CH_3$), 0.868 (t, 3H, J=6.9Hz, 18-$CH_3$);

ESI-MS (*m*/*z*): 345.1 $[M+H]^+$ (MW=344.41);

Elemental analysis: Calculated for $C_{20}H_{24}O_5$ (%): C, 69.75; H, 7.02. Found (%): C, 69.52; H, 6.90.

(2) 6,6-dimethyl-4-n-propyl-10-n-pentyl-2H,6H,12H-benzo[1,2-b:3,4-b':5,6-b"]-tripyranyl-2,12-dione (4-15, $R_1$=n-$C_3H_7$, $R_2$=$R_4$=H, $R_3$=n-$C_5H_{11}$, $R_5$=$R_6$= $CH_3$)

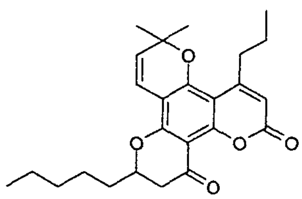

Using the same procedure as described in the preparative method of compound 4-1, except for using 34 mg (0.1mmol) benzo[1,2-b:3,4-b']-dipyranyl-5-hydroxyl-4-n-propyl-8-n-pentyl-2,10-dione (5a-15) and 63mg (0.4mmol) 1,1-diethoxyl-3-methyl-2-butene as starting material to obtain 33 mg of the title compound in 80% yield as off-white powder, m.p.128-131°C.

$^1$H-NMR (300MHz, DMSO-$d_6$): 6.558 (d, 1H, J=9.9Hz, 8-H), 6.099 (s, 1H, 3-H), 5.800 (d, 1H, J=10.2Hz, 7-H), 4.569 (o,

1H, J=3.9Hz, 12.0Hz, 4.2Hz, 10-H), 2.845 (dt, 2H, J=3.6Hz, 8.4Hz, 13-$CH_2$), 2.717 (dd, 1H, J=12.0Hz, 16.5Hz, 11-He), 2.609 (dd, 1H, J=3.9Hz, 16.5Hz, 11-Ha), 1.746 (m, 2H, 16-$CH_2$), 1.546 (sex, 2H, J=8.4Hz, 7.2Hz, 14-CH), 1.499, 1.458 (2s, 6H, 6-$CH_3$), 1.310 (m, 6H, 17,18,19-$CH_2$), 0.967 (t, 3H, J=7.2Hz, 15-$CH_3$), 0.871 (t, 3H, J=7.2Hz, 20-$CH_3$);

ESI-MS (*m*/*z*): 411.1 $[M+H]^+$ (MW=410.51);

Elemental analysis: Calculated for $C_{25}H_{30}O_5\cdot\frac{1}{6}H_2O$ (%): C, 72.62; H, 7.39. Found (%): C, 72.56; H, 7.49.

Example 16

6,6-dimethyl-4-n-propyl-10-phenyl-2H,6H,12H-benzo[1,2-b:3,4-b':5,6-b"]-tripyranyl-2,12-dione (4-16, $R_1$=n-$C_3H_7$, $R_2$=$R_4$=H, $R_3$=$C_6H_5$, $R_5$=$R_6$=$CH_3$)

(1) benzo[1,2-b:3,4-b']-dipyranyl-5-hydroxyl-8-phenyl-2,10-dione (5a-16, $R_1$=n-$C_3H_7$, $R_2$=$R_4$=H, $R_3$=$C_6H_5$) and benzo [1,2-b:3,4-b']-dipyranyl-5-hydroxyl-8-phenyl-2,6-dione (5a-16, $R_1$=n-$C_3H_7$, $R_2$=$R_4$=H, $R_3$=$C_6H_5$)

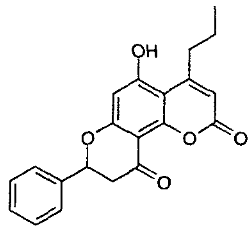

5a-16

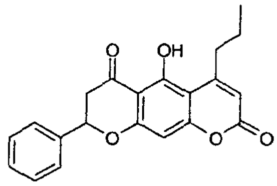

5b-16

Using the same procedure as described in the preparative method of compound 5a-1, except for using 2.20g (10mmol) 4-n-propyl-5,7-dihydroxyl-coumarin (6-11) and 1.59g (10.7mmol) trans-cinnamic acid as starting material to obtain 2.32g of the title compound 5a-16 in 66% yield as a white powder m.p.171-173°C, and 0.52g of the title compound 5b-16 in 15% yield as a white powder m.p.182-183°C.

Compound 5a-16: $^1$H-NMR (300MHz, DMSO-$d_6$): 11.878 (s, 1H, 5-OH), 7.445 (m, 5H, 8-Ph), 6.373 (s, 1H, 6-H), 6.066 (s, 1H, 3-H), 5.668 (dd, 1H, J=3.3Hz, 12.6Hz, 8-H), 3.156 (dd, 1H, J=1.2.3Hz, 16.5Hz, 9-He), 2.886 (t, 2H, J=7.2Hz, 11-$CH_2$), 2.756 (dd, 1H, J=3.3Hz, 16.5Hz, 9-Ha), 1.572 (sex, 2H, J=7.2Hz, 7.5Hz, 12-$CH_2$), 0.936 (t, 3H, J=7.5Hz, 13-$CH_3$);

ESI-MS (*m*/*z*): 351.0 $[M+H]^+$ (MW=350.37);

Elemental analysis: Calculated for $C_{21}H_{18}O_5\cdot\frac{1}{3}H_2O$ (%): C, 70.77; H, 5.27. Found (%): C, 70.69; H, 4.96.

Compound 5b-16: $^1$H-NMR (300MHz, DMSO-$d_6$): 13.871 (s, 1H, 5-OH), 7.445 (m, 5H, 8-Ph), 6.556 (s, 1H, 10-H), 6.072 (s, 1H, 3-H), 5.768 (dd, 1H, J=3.0Hz, 12.9Hz, 8-H), 3.498 (dd, 1H, J=12.9Hz, 17.4Hz, 7-He), 2.986 (dd, 1H, J=3.0Hz, 17.4Hz, 7-Ha), 2.883 (dt, 2H, J=7.5Hz, 3.3Hz, 11-$CH_2$), 1.601 (sex, 2H, J=7.2Hz, 7.5Hz, 12-$CH_2$), 0.961 (t, 3H, J=7.2Hz, 13-$CH_3$);
ESI-MS (*m*/*z*): 351.0 $[M+H]^+$ (MW=350.37);

(2) 6,6-dimethyl-4-n-propyl-10-phenyl-2H,6H,12H-benzo[1,2-b:3,4-b':5,6-b"]-tripyranyl-2,12-di one (4-16, $R_1$=n-$C_3H_7$, $R_2$=$R_4$=H, $R_3$=$C_6H_5$, $R_5$=$R_6$=$CH_3$)

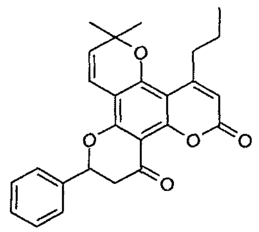

Using the same procedure as described in the preparative method of compound 4-1, except for using 35 mg (0.1mmol) benzo[1,2-b:3,4-b']-dipyranyl-5-hydroxyl-8-phenyl-2,10-dione (5a-16) and 63mg (0.4mmol) 1,1-diethoxyl-3-methyl-2-butene as starting material to obtain 34 mg of the title compound in 82% yield as off-white powder, m.p. 151-153°C.

$^1$H-NMR (300MHz, DMSO-$d_6$): 7.456 (m, 5H, 10-Ph), 6.559 (d, 1H, J=9.9Hz, 8-H), 6.113 (s, 1H, 3-H), 5.776 (d, 1H, J=9.9Hz, 7-H), 5.750 (dd, 1H, J=3.0Hz, 12.3Hz, 10-H), 3.1.75 (dd, 1H, J=12.3Hz, 16.2Hz, 11-He), 2.858 (m, 2H, 13-$CH_2$), 2.815 (dd, 1H, J=3.0Hz, 16.2Hz, 11-Ha), 1.564 (sex, 2H, J=7.2Hz, 7.5Hz, 14-$CH_2$), 1.493, 1.466 (2s, 6H, 6-$CH_3$), 0.970 (t, 3H, J=7.2Hz, 15-CH3);

ESI-MS (*m*/*z*): 417.1 $[M+H]^+$ (MW=416.48);

Elemental analysis: Calculated for $C_{26}H_{24}O_5\cdot\frac{1}{2}H_2O$ (%): C, 73.39; H, 5.92.

Found (%): C, 73.47; H, 5.61.

Example 17

6,6-dimethyl-4-n-propyl-10-p-methyl-phenyl-2H,6H,12H-benzo[1,2-b:3,4-b':5,6-b"]-tri pyranyl-2,12-dionc (4-17, $R_1$=n-$C_3H_7$, $R_2$=$R_4$=H, $R_3$=*p*-$CH_3C_6H_4$, $R_5$=$R_6$=$CH_3$,)

(1) benzo[1,2-b:3,4-b']-dipyranyl-5-hydroxyl-8-p-methyl-phenyl-2,10-dione (5a-17, $R_1$=n-$C_3H_7$, $R_2$=$R_4$=H, $R_3$=*p*-$CH_3C_6H_4$)

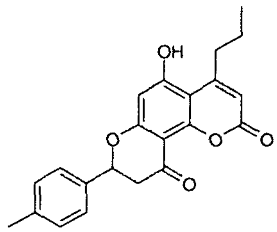

Using the same procedure as described in the preparative method of compound 5a-1, except for using 2.20g (10mmol) of 4-n-propyl-5,7-dihydroxyl-coumarin (6-11) and 1.74g (10.7mmol) of trans-p-methylphenyl propenoic acid as starting material to obtain 2.48g of the title compound 5a-17 in 68% yield as a white powder, m.p.169-171°C.
$^1$H-NMR (300MHz, DMSO-$d_6$): 11.176 (s, 1H, 5-OH), 7.094 (d, 2H, J=8.1Hz, 8-Ar-H), 6.968 (d, 2H, J=8.1Hz, 8-Ar-H), 6.578 (s, 1H, 6-H), 6.037 (s, 1H, 3-H), 4.665 (d, 1H, J=6.0Hz, 8-H), 3.336 (q, 2H, J=7.2Hz, 16.2Hz, 9-$CH_2$), 2.906 (m, 2H, 11-$CH_2$), 2.219 (s, 3H, 14-$CH_3$), 1.598 (sex, 2H, J=7.2Hz, 7.5Hz, 12-$CH_2$), 0.938 (t, 3H, J=7.2Hz, 13-$CH_3$);
ESI-MS (*m*/*z*): 365.1 $[M+H]^+$ (MW=364.40);
Elemental analysis: Calculated for $C_{22}H_{20}O_5$ (%): C, 72.52; H, 5.53. Found (%): C, 72.47; H, 5.43.

(2) 6,6-dimethyl-4-n-propyl-10-p-methyl-phenyl-2H,6H,12H-benzo[1,2-b:3,4-b':5,6-b"]-tripyran yl-2,12-dione (4-17, $R_1$=n-$C_3H_7$, $R_2$=$R_4$=H, $R_3$=*p*-$CH_3C_6H_4$, $R_5$=$R_6$=$CH_3$)

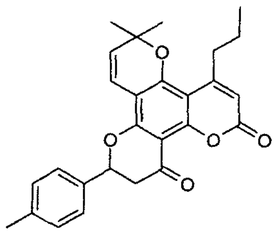

Using the same procedure as described in the preparative method of compound 4-1, except for using 36 mg (0.1mmol) benzo[1,2-b:3,4-b']-dipyranyl-5-hydroxyl-8-p-methyl-phenyl-2,10-dione (5a-17) and 63mg (0.4mmol) 1,1-diethoxyl-3-methyl-2-butene as starting material to obtain 36 mg of the title compound in 85% yield as off-white powder, m.p.174-176°C.

$^1$H-NMR (300MHz, DMSO-$d_6$): 7.105 (d, 2H, J=7.8Hz, 10-Ar-H), 6.991 (d, 2H, J=8.1Hz, 10-Ar-H), 6.123 (s, 1H, 3-H), 6.622 (d, 1H, J=9.9Hz, 8-H), 5.889 (d, 1H, J=9.9Hz, 7-H), 4.703 (d, 1H, J=6.0Hz, 10-H), 3.377 (dd, 2H, J=7.2Hz, 16.2Hz, 11-$CH_2$), 2.886 (m, 2H, 13-$CH_2$), 2.226 (s, 3H, 16-$CH_3$), 1.596 (sex, 2H, J=7.2Hz, 7.5Hz, 14-$CH_2$), 1.513, 1.489 (2s, 6H, 6-$CH_3$), 0.986 (t, 3H, J=7.2Hz, 15-$CH_3$);

ESI-MS (*m*/*z*): 431.1 [M+H]$^+$ (MW=430.51);

Elemental analysis: Calculated for $C_{27}H_{26}O_5 \cdot \frac{1}{8} H_2O$ (%): C, 74.94; H, 6.11. Found (%): C, 75.02; H, 6.14.

Example 18

6,6-dimethyl-4-n-propyl-10,11-trans-cyclohexyl-2H,6H,12H-benzo[1,2-b:3,4-b':5,6-b"] -tripyranyl-2,12-dione (4-18, $R_1$=n-$C_3H_7$, $R_2$=H, $R_3$,$R_4$= trans-cyclohexyl, $R_5$=$R_6$=$CH_3$)

(1) benzo[1,2-b:3,4-b']-dipyranyl-5-hydroxyl-4-n-propyl-8,9-trans-cyclohexyl-2,10-dione (5a-18, $R_1$=n-$C_3H_7$, $R_3$, $R_4$= trans-cyclohexyl, $R_2$=H)

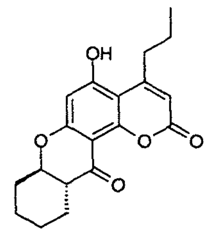

Using the same procedure as described in the preparative method of compound 5a-1, except for using 2.20g (10mmol) 4-n-propyl-5,7-dihydroxyl-coumarin (6-11) and 1.35g (10.7mmol) trans-cyclohexenoic acid as starting material to obtain 1.38g of the title compound 5a-18 in 42% yield as a white powder, m.p.160-163°C.

$^1$H-NMR (300MHz, DMSO-$d_6$): 11.781 (s, 1H, 5-OH), 6.283 (s, 1H, 6-H), 6.032 (s, 1H, 3-H), 4.162 (dt, 1H, J=4.5Hz, 11.4Hz, 8-H), 2.865 (t, 2H, J=7.5Hz, 11-$CH_2$), 2.485 (m, 1H, 9-H), 2.114 (m, 2H, 14-$CH_2$), 1.721 (m, 2H, 17-$CH_2$), 1.544 (m, 6H, 12,15,16-$CH_2$), 0.924 (t, 3H, J=7.2Hz, 13-$CH_3$);

ESI-MS (*m*/*z*): 329.1 [M+H]$^+$ (MW=328.37);

(2) 6,6-dimethyl-4-n-propyl-10,11-trans-cyclohexyl-2H,6H,12H-benzo[1,2-b:3,4-b':5,6-b"]-tripy ranyl-2,12-dione (4-18, $R_1$=n-$C_3H_7$, $R_2$=H, $R_3$,$R_4$= trans-cyclohexyl, $R_5$=$R_6$=$CH_3$)

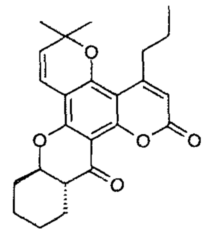

Using the same procedure as described in the preparative method of compound 4-1, except for using 33 mg (0.1mmol) benzo[1,2-b:3,4-b']-dipyranyl-5-hydroxyl-4-n-propyl-8,9-trans-cyclohexyl-2,10-dione (5a-18) and 63mg (0.4mmol) 1,1-diethoxyl-3-methyl-2-butene as starting material to obtain 32 mg of the title compound in 80% yield as white powder, m.p.151-153°C.
$^1$H-NMR (300MHz, DMSO-$d_6$): 6.577 (d, 1H, J=10.2Hz, 8-H), 6.104 (s, 1H, 3-H), 5.592 (d, 1H, J=9.9Hz, 7-H), 4.238 (dt, 1H, J=4.5Hz, 11.4Hz, 10-H), 2.853 (dt, 2H, J=3.6Hz, 8.4Hz, 13-$CH_2$), 2.561 (m, 1H, 11-H), 2.169 (m, 2H, 16-$CH_2$), 1.710 (m, 2H, 19-$CH_2$), 1.580 (m, 6H, 14,17,18-$CH_2$), 1.503,1.458 (2s, 6H, 6-$CH_3$), 0.972 (t, 3H, J=7.2Hz, 15-$CH_3$);
ESI-MS (*m/z*): 395.1 $[M+H]^+$ (MW=394.47);

Elemental analysis: Calculated for $C_{24}H_{26}O_5 \cdot \frac{2}{3} H_2O$ (%): C, 70.92; H, 6.78. Found (%): C, 71.10; H, 6.79.

Example 19

6,6-dimethyl-4-n-propyl-1.0,11-cis-cyclohexyl-2H,6H,12H-benzo[1,2-b:3,4-b':5,6-b"]-tr ipyranyl-2,12-dione (4-19, $R_1$=n-$C_3H_7$, $R_2$=H, $R_3$,$R_4$= cis-cyclohexyl, $R_5$=$R_6$=$CH_3$)

(1) benzo[1,2-b:3,4-b']-dipyranyl-5-hydroxyl-8,9-cis-cyclohexyl-2,10-dione (5a-19, $R_1$=n-$C_3H_7$, $R_3$, $R_4$= cis-cyclohexyl, $R_2$=H)

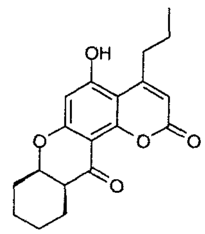

Using the same procedure as described in the preparative method of compound 5a-1, except for using 2.20g (10mmol) 4-n-propyl-5,7-dihydroxyl-coumarin (6-11) and 1.35g (10.7mmol) cyclohexenoic acid as starting material to obtain 1.42g of the title compound 5a-19 in 43% yield as a white powder, m.p.159-161°C ;
$^1$H-NMR (300MHz, DMSO-$d_6$): 11.818 (s, 1H, 5-OH), 6.307 (s, 1H, 6-H), 6.021 (s, 1H, 3-H), 4.642 (m, 1H, 8-H), 2.860 (t, 2H, J=7.5Hz, 11-$CH_2$), 2.502 (m, 2H, 14-$CH_2$), 1.922 (m, 1H, 9-H), 1.566 (m, 8H, 12,15,16,17-$CH_2$), 0.923 (t, 3H, J=7.2Hz, 13-$CH_3$);
ESI-MS (*m/z*): 329.1 $[M+H]^+$ (MW=328.37);
Elemental analysis: Calculated for $C_{19}H_{20}O_5$ (%): C, 69.50; H, 6.14.Found (%): C, 69.44; H, 5.88.

(2) 6,6-dimethyl-4-n-propyl-10,11-cis-cyclohexyl-2H,6H,12H-benzo[1,2-b:3,4-b':5,6-b"]-tripyra nyl-2,12-dione (4-19, $R_1$=n-$C_3H_7$, $R_2$=H, $R_3$,$R_4$= cis-cyclohexyl, $R_5$=$R_6$=$CH_3$)

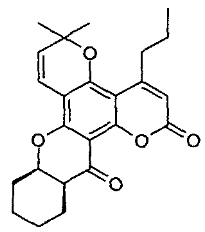

Using the same procedure as described in the preparative method of compound 4-1, except for using 33 mg (0.1mmol) benzo[1,2-b:3,4-b']-dipyranyl-5-hydroxyl-8,9-cis-cyclohexyl-2,10-dione (5a-19) and 63mg (0.4mmol) 1,1-diethoxyl-3-methyl-2-butene as starting material to obtain 33 mg of the title compound in 84% yield as off-white powder, m.p.156-159°C.
$^1$H-NMR (300MHz, DMSO-$d_6$): 6.622 (d, 1H, J=9.9Hz, 8-H), 6.095 (s, 1H, 3-H), 5.785 (d, 1H, J=10.2Hz, 7-H), 4.727 (m, 1H, 10-H), 2.843 (t, 2H, J=7.5Hz, 13-$CH_2$), 2.544 (m, 1H, 11-H), 2.166 (m, 2H, 16-$CH_2$), 1.573 (m, 6H, 14,17,18-$CH_2$), 1.722 (m, 2H, 19-$HC_2$), 1.488,1.479 (2s, 6H, 6-$CH_3$), 0.965 (t, 3H, J=7.2Hz, 15-$CH_3$);
ESI-MS (*m*/*z*): 395.1 $[M+H]^+$ (MW=394.47);

Elemental analysis: Calculated for $C_{24}H_{26}O_5 \cdot \frac{1}{6} H_2O(\%)$: C, 72.52; H, 6.68. Found (%): C, 72.46; H, 6.80

Example 20

6,6,10-trimethyl-3-chiolo-4-n-propyl-2H,6H,12H-benzo[1,2-b:3,4-b':5,6-b"]-tripyranyl-2,12-dione (4-20, $R_1$=n-$C_3H_7$, $R_2$=Cl, $R_3$=$R_5$=$R_6$=$CH_3$, $R_4$=H)

(1) 3-chloro-4-n-propyl-5,7-dihydroxy-coumarin (6-20, $R_1$=n-$C_3H_7$, $R_2$=Cl)

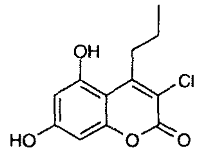

Using the same procedure as described in the preparative method of compound (6-1), except for using 7.5 g (0.046mol) phloroglucinol and 8.86 g (0.046mol) 2-chloroethyl butyryl acetate as starting material to obtain 10.6g of the title compound in 90% yield as a yellow powder crystalline. m.p. 242-245°C.
$^1$H-NMR (400MHz, DMSO-$d_6$, ppm): 10.811 (s, 1H, 7-OH), 10.434 (s, 1H, 5-OH), 6.307 (d, 1H, J=2.4Hz, 8-H), 6.197 (d, 1H, J=2.4Hz, 6-H), 3.135 (m, 2H, 4-C$\underline{H}_2$-$C_2H_5$), 1.559 (m, 2H, 4-$CH_2$-C$\underline{H}_2$-$CH_3$), 0.976 (t, 3H, J=7.2Hz, 4-$CH_2$-$CH_2$-C$\underline{H}_3$);
ESI-MS (*m*/*z*): 255.1 $[M+H]^+$ (MW=254.67);

(2) benzo[1,2-b:3,4-b']-dipyranyl-5-hydroxyl-8-methyl-3-chloro-4-n-propyl-2,10-dione (5a-20, $R_1$=n-$C_3H_7$, $R_2$=Cl, $R_3$=$CH_3$, $R_4$=H) and benzo[1,2-b:5,4-b']-dipyranyl-5-hydroxyl-8-methyl-3-chloro-4-n-propyl-2,6-dione (5b-20, $R_1$=n-$C_3H_7$, $R_2$=Cl, $R_3$=$CH_3$, $R_4$=H)

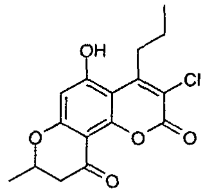

5a-20

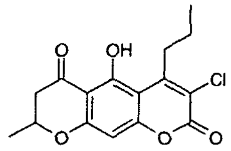

5b-20

Using the same procedure as described in the preparative method of compound 5a-1, except for using 2.55g (10mmol) 3-chloro-4-n-propyl-5,7-dihydroxy-coumarin (6-20) and 0.92g (10.7mmol) crotonic acid as starting material to obtain 2.4g of the title compound 5a-20 in 75% yield as a white powder, m.p.164-165°C, and 0.30g of the title compound 5b-20 in 9.3% yield as a white powder m.p.188-189°C.

Compound 5a-20: $^1$HNMR (300MHz, DMSO-$d_6$, ppm): 12.016 (s, 1H, OH), 6.345 (s, 1H, 6-H), 4.657 (m, 1H, 8-H), 3.155 (t, 2H, J=7.5Hz, 4-C$\underline{H}_2$$CH_2CH_3$), 2.658 (dt, 2H, J=3.3Hz, 11.1Hz, 9-$CH_2$), 1.558 (sex, 2H, J=7.5Hz, 7.2Hz, 4-$CH_2$C$\underline{H}_2$$CH_3$), 1.392 (d, 3H, J=6.0Hz, 8-CH), 0.985 (t, 3H, J=7.2Hz, 4-$CH_2CH_2$C$\underline{H}_3$);

ESI-MS (*m*/*z*): 323.1 $[M]^+$ (MW=322.75);

Elemental analysis: Calculated for $C_{16}H_{15}ClO_5$ (%): C, 59.54; H, 4.68. Found (%): C, 59.44; H, 4.69.

Compound 5b-20: $^1$H-NMR (400MHz, DMSO-$d_6$, ppm): 14.134 (s, 1H, 5-OH), 6.531 (s, 1H, 10-H), 4.774 (dq, 1H, J=6.4Hz, 3.2Hz, 12.4Hz, 8-H), 3.154 (m, 2H, 4-C$\underline{H}_2$$CH_2CH_3$), 3.002 (dd, 1H, J=12.4Hz, 17.6Hz, 7-He), 2.822 (dd, 1H, J=3.2Hz, 17.2He, 7-Ha), 1.595 (dt, 1H, J=7.2Hz, 8.0Hz, 4-$CH_2$C$\underline{H}_2$$CH_3$), 1.442 (d, 3H, J=6.4Hz, 8-$CH_3$), 1.016 (t, 3H, J=7.2Hz, 4-$CH_2CH_2$C$\underline{H}_3$);

ESI-MS (*m*/*z*): 323.1 $[M]^+$ (MW=322.75);

(3) 6,6,10-trimethyl-3-chrolo-4-n-propyl-2H,6H,12H-benzo[1,2-b:3,4-b':5,6-b"]-tripyranyl-2,12-dione (4-20, $R_1$=n-$C_3H_7$, $R_2$=Cl, $R_3$=$R_5$=$R_6$=$CH_3$, $R_4$=H)

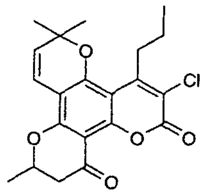

Using the same procedure as described in the preparative method of compound 4-1, except for using 32 mg (0.1 mmol) benzo[1,2-b:3,4-b']-dipyranyl-5-hydroxyl-8-methyl-3-chloro-4-n-propyl-2,10-dione (5a-20) and 63mg (0.4mmol) 1,1-diethoxyl-3-methyl-2-butene as starting material to obtain 33 mg of the title compound in 85% yield as off-white powder, m.p.112-114°C.

$^1$H-NMR (400MHz, DMSO-$d_6$): 6.607 (d, 1H, J=10.0Hz, 8-H), 5.833 (d, 1H, J=10.0Hz, 7-H), 4.734 (m, 1H, 10-H), 3.122 (t, 2H, J=8.0Hz, 4-C$\underline{H}_2$$CH_2CH_3$), 2.737 (dd, 1H, J=12.0Hz, 16.4Hz, 11-He), 2.645 (dd, 1H, J=3.6Hz, 16.4Hz, 11-Ha), 1.581 (m, 2H, 4-$CH_2$C$\underline{H}_2$$CH_3$), 1.520, 1.480 (2s, 6H, 6-$CH_3$), 1.452 (d, 3H, J=6.0Hz, 10-$CH_3$), 1.045 (t, 3H, J=7.2Hz, 4-$CH_2CH_2$C$\underline{H}_3$);

ESI-MS (*m*/*z*): 389.1 $[M+H]^+$ (MW=388.85);

Elemental analysis: Calculated for $C_{21}H_{21}ClO_5\cdot\frac{1}{4}H_2O$ (%): C, 64.12; H, 5.64. Found (%): C, 64.09; H, 5.82.

Example 21

6,6,10-trimethyl-4-phenyl-2H,6H,12H-benzo[1,2-b:3,4-b':5,6-b"]-tripyranyl-2,12-dione (4-21, $R_1$=$C_6H_5$, $R_2$=$R_4$=H, $R_3$=$R_5$=$R_6$=$CH_3$)

(1) 4-phenyl-5,7-dihydroxy-coumarin (6-21 , $R_1$=$C_6H_5$ , $R_2$=H)

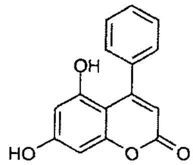

Using the same procedure as described in the preparative method of compound (6-1), except for using 7.5 g (0.046mol) pholoroglucinol and 8.84 g (0.046mol) ethyl benzyl acetate as starting material to obtain 11.5g of the title compound in 98% yield as a yellow powder crystalline. m.p. 226-228C.
$^1$H-NMR (300MHz, DMSO-$d_6$, ppm): 10.371 (s, 1H, 7-OH,), 10.085 (s, 1H, 5-OH,), 7.334 (m, 5H, 4-Ph), 6.253 (d, 1H, J=2.1Hz, 8-H), 6.147 (d, 1H, J=2.1Hz, 6-H), 5.729 (s, 1H, 3-H);
ESI-MS (*m*/*z*): 255.2 $[M+H]^+$ (MW=254.24)

(2) benzo[1,2-b:3,4-b']-dipyranyl-5-hydroxyl-8-methyl-4-phenyl-2,10-dione (5a-21, $R_1$=$C_6H_5$, $R_2$=$R_4$=H, $R_3$=$CH_3$) and benzo[1,2-b:5,4-b']-dipyranyl-5-hydroxyl-8-methyl-4-phenyl-2,6-dione (5b-21, $R_1$=$C_6H_5$, $R_2$=$R_4$=H, $R_3$=$CH_3$)

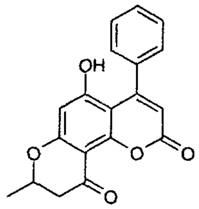

5a-21

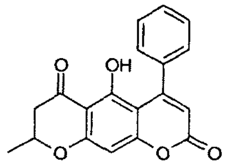

5b-21

Using the same procedure as described in the preparative method of compound 5a-1, except for using 2.55g (10mmol) 4-phenyl-5,7-dihydroxy-coumarin (6-21) and 0.92g (10.7mmol) crotonic acid as starting material to obtain 1.74g of the title compound 5a-21 in 54% yield as a white powder m.p.126-129□, and 0.39g of the title compound 5b-21 in 12% yield as a white powder m.p.151-154°C.

Compound 5a-21: $^1$H-NMR (300MHz, DMSO-$d_6$, ppm): 11.327 (s, 1H, 5-OH,), 7.319 (m, 5H, 4-Ph), 6.161 (s, 1H, 6-H), 5.960 (s, 1H, 3-H), 4.666 (m, 1H, 8-H), 2.630 (m, 2H, 9-$CH_2$), 1.396 (d, 3H, J=6.3Hz, 8-$CH_3$);
ESI-MS (m/z): 323.1 $[M+H]^+$ (MW=322.32);
IR (KBr, $cm^{-1}$): 3059, 2970,2725,1745,1610, 1550,1367,1242,1147, 854,825;
Elemental analysis: Calculated for $C_{19}H_{14}O_5$ (%): C, 70.80; H, 4.38. Found (%): C, 70.98; H, 4.66.

Compound 5b-21: $^1$H-NMR (300MHz, DMSO-$d_6$, ppm): 1.3.273 (s, 1H, 5-OH,), 7.400 (m, 5H, 4-Ph), 6.536 (s, 1H, 10-H), 5.967 (s, 1H, 3-H), 4.738 (m, 1H, 8-H), 2.921 (dd, 1H, J=17.4Hz, 12.2Hz, 7-He), 2.748 (dd, 1H, J=17.4Hz, 3.3Hz, 7-Ha), 1.429 (d, 3H, J=6.3Hz, 8-$CH_3$);
ESI-MS (*m*/*z*): 323.1 $[M+H]^+$ (MW=322.32);

Elemental analysis: Calculated for $C_{19}H_{14}O_5 \cdot \frac{1}{2} H_2O$ (%): C, 68.87; H, 4.56. found (%): C, 68.73; H, 4.66

(3) 6,6,10-trimethyl-4-phenyl2H,6H,12H-benzo[1,2-b:3,4-b':5,6-b"]-tripyranyl-2,12-dione (4-21, $R_1=C_6H_5$, $R_2=R_4=H$, $R_3=R_5=R_6=CH_3$)

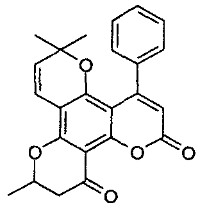

Using the same procedure as described in the preparative method of compound 4-1, except for using 32 mg (0.1 mmol) benzo[1,2-b:3,4-b']-dipyranyl-5-hydroxyl-8-methyl-4-phenyl-2,10-dione (5a-21) and 63mg (0.4mmol) 1,1-diethoxyl-3-methyl-2-butene as starting material to obtain 32 mg of the title compound in 83% yield as white powder, m.p.141-144°C.
$^1$H-NMR (300MHz, $CDCl_3$, ppm): 7.270 (m, 5H, 4-Ph), 6.556 (d, 1H, J=9.9Hz, 8-H), 6.063 (s, 1H, 3-H), 5.429 (d, 1H, J=9.9Hz, 7-H), 4.667 (m, 1H, 10-H), 2.717 (m, 2H, 11-CH2), 1.549 (d, 3H, J=6.0Hz, 10-$CH_3$), 0.987, 0.944 (2s, 6H, 6-$CH_3$);
ESI-MS (*m*/*z*): 389.4 $[M+H]^+$ (MW=388.42);
Elemental :Calculated for $C_{24}H_{20}O_5$ (%): C, 74.21; H, 5.19. Found (%): C, 74.40; H, 5.22.

Example 22

6,6,10-trimethyl-4-p-nitrophenyll-2H,6H,12H-benzo[1,2-b:3,4-b':5,6-b"]-tripyranyl-2,1 2-dione (4-22, $R_1$=*p*-$NO_2C_6H_4$, $R_2=R_4=H$, $R_3=R_5=R_6=CH_3$)

(1) 4-p-nitrophenyl-5,7-dihydroxy-coumarin (**6-22**, $R_1$=p-$NO_2C_6H_4$, $R_2$=H)

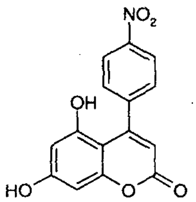

Using the same procedure as described in the preparative method of compound (6-1), except for using 7.5 g (0.046mol) phloroglucinol and 10.91 g (0.046mol) p-nitrobenzoyl acetic ester as starting material to obtain 10.5g of the title compound in 76% yield as a yellow powder crystalline. m.p. 268-270°C.
$^1$H-NMR (300MHz, DMSO-$d_6$): 10.494 (s, 1H, OH), 10.289 (s, 1H, OH), 8.222 (d, 2H, J=9.0Hz, Ar-H), 7.615 (d, 2H, J=9.0Hz, Ar-H), 6.279 (d, 2H, J=2.1Hz, 8-H), 6.151 (d, 2H, J=2.1Hz, 6-H), 5.844 (s, 1H, 3-H);
ESI-MS (*m*/*z*): 298.1 [M-H] - (MW=299.24)

(2) benzo[1,2-b:3,4-b']-dipyranyl-5-hydroxyl-8-methyl-4-p-nitrophenyl-2,10-dione (**5a-22**, $R_1$=*p*-$NO_2C_6H_4$, $R_3=CH_3$, $R_2=R_4=H$) or benzo[1,2-b:5,4-b']-dipyranyl-5-hydroxyl-8-methyl-4-p-nitrophenyl-2,6-dione (**5b-22**, $R_1$=*p*-$NO_2C_6H_4$, $R_3=CH_3$, $R_2=R_4=H$)

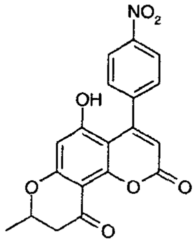

5a-22

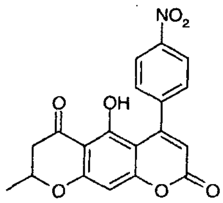

5b-22

Using the same procedure as described in the preparative method of compound 5a-1, except for using 2.99g (10mmol) of 4-p-nitrophenyl-5,7-dihydroxy-coumarin (**6-22**) and 0.92g (10.7mmol) crotonic acid as starting material to obtain 2.1g of the title compound **5a-22** in 57% yield as a white powder m.p.207-209°C, and 0.68g of the title compound **5b-22** in 18% yield as a white powder m.p.196-198°C.

Compound **5a-22**: $^1$HNMR (300MHz, DMSO-$d_6$, ppm): 11.489 (s, 1H, OH), 8.238 (d, 2H, J=8.7Hz, Ar-H), 7.616 (dd, 2H, J=2.1Hz, 8.7Hz, Ar-H), 6.117 (s, 1H, 6-H), 6.090 (s, 1H, 3-H), 4.667 (m, 1H, 8-H), 2.691 (dt, 2H, J=4.2Hz, 10.5Hz, 9-$CH_2$), 1.406 (d, 3H, J=6.3Hz, 8-$CH_3$);
ESI-MS (*m*/*z*): 366.2 $[M-H]^-$ (MW=367.32);
Elemental analysis: Calculated for $C_{19}H_{13}NO_7$ (%): C, 62.13; H, 3.57; N, 3.81. Found (%): C, 61.98; H, 3.75; N, 3.93.

Compound **5b-22**: $^1$H-NMR (300MHz, DMSO-$d_6$): 13.267 (s, 1H, OH), 8.273 (m, 2H, 4-Ar-H), 7.652 (m, 2H, 4-Ar-H), 6.608 (s, 1H, 10-H), 6.126 (s, 1H, 3-H), 4.769 (m, 1H, 8-H), 2.953 (dd, 1H, J=12.3Hz, 17.7Hz, 7-He), 2.771 (dd, 1H, J=3.3Hz, 17.4Hz, 7-Ha), 1.444 (d, 3H, J=6.3Hz, 8-$CH_3$);
ESI-MS (m/z): 366.2 $[M-H]^-$ (MW=367.32);

(3) 6,6,10-trimethyl-4-p-nitrophenyl-2H,6H,12H-benzo[1,2-b:3,4-b':5,6-b"]-tripyranyl-2,12-dion e (4-22, $R_1$=*p*-$NO_2C_6H_4$, $R_2$=$R_4$=H, $R_3$=$R_5$=$R_6$=$CH_3$)

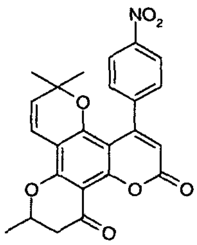

Using the same procedure as described in the preparative method of compound **4-1**, except for using 37 mg (0.1mmol) benzo[1,2-b:3,4-b']-dipyranyl-5-hydroxyl-8-methyl-4-p-nitrophenyl-2,10-dione (**5a-22**) and 63mg (0.4mmol) 1,1-diethoxyl-3-methyl-2-butene as starting material to obtain 32 mg of the title compound in 75% yield as white powder, m.p.165-167°C.
$^1$H-NMR (300MHz, DMSO-$d_6$): 8.284 (m, 2H, 4-Ar-H), 7.621 (m, 2H, 4-Ar-H), 6.509 (d, 1H, J=9.9Hz, 8-H), 6.123 (s, 1H, 3-H), 5.613 (d, 1H, J=9.9Hz, 7-H), 4.743 (m, 1H, 10-H), 2.754 (dd, 1H, J=11.4Hz, 16.5Hz, 11-He), 2.691 (dd, 1H, J=3.6Hz, 16.5Hz, 11-Ha), 1.458 (d, 3H, J=6.0Hz, 10-$CH_3$), 0.926, 0.875 (2s, 6H, 6-$CH_3$);
ESI-MS *(m/z):* 434.2 $[M+H]^+$ (MW=433.42);
Elemental analysis: Calculated for $C_{24}H_{19}NO_7$ (%): C, 66.51; H, 4.42; N, 3.23. Found (%): C, 66.35; H, 4.28; N, 3.26.

Example 23

6,6,10-trimethyl-4-p-aminophenyl-2H,6H,12H-benzo[1,2-b:3,4-b':5,6-b"]-tripyranyl-2,12-di one (4-23, $R_1$=*p*-$NH_2C_6H_4$, $R_2$=$R_4$=H, $R_3$=$R_5$=$R_6$=$CH_3$)

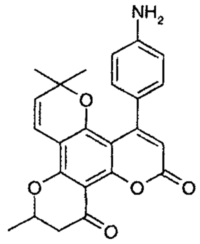

10 ml concentrated hydrochloric acid and 0.19g $SnCl_2$ (Immol) was added into the solution of 22mg (0.05mmol) 6,6,10-trimethyl-4-p-nitrophenyl-2H,6H,12H-benzo[1,2-b:3,4-b':5,6-b"]-tripyranyl-2,12-dion e (**4-22**) in 1.0 ml EtOH. The reaction mixture was stirred in oil-bath for 2 hours at 60°C and then cooled to room temperature, poured into the solution of 40% NaOH in protion with stirring. After extraction with dichloromethane (DCM), concentrated under reduce pressure, dried with dry $NaSO_4$ and purification by silica-gel column chromatography , 13mg of the title compound **4-23** was obtained as off-white powder in 63% yield, m.p.149-151°C.
$^1$H-NMR (400MHz, DMSO-$d_6$): 7.089 (m, 2H, 4-Ar-H), 6.492 (m, 2H, 4-Ar-H), 6.477 (d, 1H, J=10.0Hz, 8-H), 6.275 (d, 1H, J=8.4Hz, 3-H), 5.577 (d, 1H, J=10.0Hz, 7-H), 5.495 (d, 2H, J=6.0Hz, $NH_2$), 4.647 (m, 1H, 10-H), 2.840 (dd, 1H, J=12.4Hz, 15.6Hz, 11-He), 2.677 (dd, 1H, J=3.2Hz, 15.6Hz, 11-Ha), 1.453 (d, 3H, J=6.0Hz, 10-$CH_3$), 1.273, 1.224 (2s, 6H, 6-$CH_3$);
ESI-MS (*m*/*z*): 404.1. $[M+H]^+$ (MW=403.44);
HRESIMS: Calculated for $C_{24}H_{22}NO_5^+$ *(m/z):* 404.14979 found (*m*/*z*): 404.1495

Example 24

6,6,10-trimethyl-4-(3,4,5-trimethoxyl)-phenyl-2H,6H,12H-benzo[1,2-b:3,4-b':5,6-b"]-tr ipyanyl-2,12-dione (**4-24**, $R_1$= (3,4,5-trimethoxyl)-phenyl, $R_2$=$R_4$=H, $R_3$=$R_5$=$R_6$=$CH_3$)

(1) 4-(3,4,5-trimethoxyl)-phenyl-5,7-dihydroxy-coumarin (**6-24**, $R_1$ =(3,4,5-trimethoxyl)-phenyl, $R_2$=H)

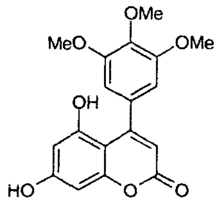

Using the same procedure as described in the preparative method of compound (6-1), except for using 7.5 g (0.046mol) phloroglucinol and 12.98 g (0.046mol) 3,4,5-trimethoxy-benzoyl acetic ester as starting material to obtain 14.9g of the title compound in 97% yield as a white powder crystalline. m.p.218-220°C.
$^1$H-NMR (400MHz, DMSO-$d_6$, ppm): 10.368 (s, 1H, 7-OH), 10.127 (s, 1H, 5-OH), 6.624 (s, 2H, 4-Ph), 6.248 (d, 1H, J=2.4Hz, 8-H), 6.159 (d, 1H, J=2.4Hz, 6-H), 5.827 (s, 1H, 3-H), 3.756 (s, 6H, -$OCH_3$), 3.692 (s, 3H, -$OCH_3$);
ESI-MS (m/z): 345.1 $[M+H]^+$ (MW=344.32);

(2) benzo[1,2-b:3,4-b']-dipyranyl-5-hydroxyl-8-methyl-4-(3,4,5-trimethoxyl)-phenyl--2,10-dione (**5a-24**, $R_1$=(3,4,5-trimethoxyl)-phenyl, $R_3$=$CH_3$, $R_2$=$R_4$=H)

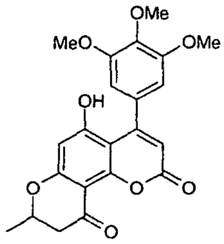

Using the same procedure as described in the preparative method of compound 5a-1, except for using 3.44g (10mmol) 4-(3,4,5-trimethoxyl)-phenyl-5,7-dihydroxy-coumarin (**6-24**) and 0.92g (10.7mmol) crotonic acid as starting material to obtain 1.9g of the title compound **5a-24** in 46% yield as a white powder m.p.178-180°C.
$^1$H-NMR (400MHz, DMSO-$d_6$, ppm): 11.363 (s, 1H, 5-OH), 6.624 (s, 2H, 4-Ar-H), 6.181 (s, 1H, 6-H), 6.061 (s, 1H, 3-H), 4.641 (m, 1H, 8-H), 3.753 (s, 6H, $OCH_3$), 3.679 (s, 3H, $OCH_3$), 2.641 (m, 2H, 9-$CH_2$), 1.404 (d, 3H, J=6.0Hz, 8-$CH_3$);
ESI-MS (*m*/*z*): 413.1[M+H]$^+$ (MW=412.40);
Elemental analysis: Calculated for $C_{22}H_{20}O_5$ (%): C, 64.07; H, 4.89. Found (%): C, 64.23; H, 4.47.

**(3)** 6,6,10-trimethyl-4-(3,4,5-trimethoxyl)-phenyl-2H,6H,12H-benzo[1,2-b:3,4-b':5,6-b"]-tripyra nyl-2,12-dione (**4-24**, $R_1$= (3,4,5-trimethoxyl)-phenyl, $R_2$=$R_4$=H, $R_3$=Rs=$R_6$=$CH_3$)

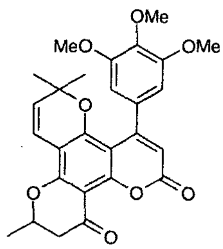

Using the same procedure as described in the preparative method of compound **4-1**, except for using 41 mg (0.1mmol) benzo[1,2-b:3,4-b']-dipyranyl-5-hydroxyl-8-methyl-4-(3,4,5-trimethoxyl)-phenyl--2,10-dione (**5a-24-** and 63mg (0.4mmol) 1,1-diethoxyl-3-methyl-2-butene as starting material to obtain 37 mg of the title compound in 77% yield as white powder, m.p.1.29-131 °C.
$^1$H-NMR (400M.Hz, DMSO-$d_6$): 6.622 (s, 2H, 4-Ar-H), 6.512 (d, 1H, J=10.0Hz, 8-H), 6.072 (s, 1H, 3-H), 5.625 (d, 1H, J=10.0Hz, 7-H), 4.729 (m, 1H, 10-H), 3.741 (s, 6H, $OCH_3$), 3.689 (s, 3H, $OCH_3$), 2.691 (m, 2H, 11-$CH_2$), 1.493, 1.459 (2s, 6H, 6-$CH_3$), 1.453 (d, 3H, J=5.2Hz, 10-$CH_3$);
ESI-MS (*m*/*z*): 479.1 [M+H]$^+$ (MW=478.50);
Elemental analysis: Calculated for $C_{27}H_{26}O_8 \cdot H_2O$ (%): C, 65.31; H, 5.68. Found (%): C, 65.09; H, 5.92.

<u>Example25</u>

6,6,10-trimethyl-4-(3-(2,6-dichloro-5-fluoro)pyridinyl)-2H,6H,12H-benzo[1,2-b:3,4-b':5,6-b' ']-tripyranyl-2,12-dione (**4-25**, $R_1$=3-(2,6-dichloro-5-fluoro)pyridinyl, $R_2$=$R_4$=H, $R_3$=$R_5$=$R_6$=$CH_3$)

(1) 4-(3-(2,6-dichloro-5-fluoro)pyridinyl)-5,7-dihydroxy-coumarin (**6-25** $R_1$=3-(2,6-dichloro-5-fluoro)pyridinyl, $R_2$=H)

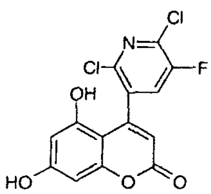

Using the same procedure as described in the preparative method of compound (6-1), except for using 7.5 g (0-046mol) phloroglucinol and 12.88 g (0.046mol) 3-(2,6-dichloro-5-fluoro)pyridinyl acetic ester as starting material to obtain 15.1.g of the title compound in 96% yield as a yellowish powder crystalline. m.p. >300°C.
$^1$H-NMR (400MHz, DMSO-$d_6$, ppm): 10.535 (s, 1H, 7-OH), 10.473 (s, 1H, 5-OH), 8.215 (d, 1H, J=8.0Hz, 4-Ar-H), 6.284 (d, 1H, J=1.6Hz, 8-H), 6.142 (d, 1H, J=1.6Hz, 6-H), 6.041 (s, 1H, 3-H);
ESI-MS (*m*/*z*): 342.1 $M^+$ (MW=342.11);

(2) benzo[1,2-b:3,4-b']-dipyranyl-5-hydroxyl-8-methyl-4-(3-(2,6-dichloro-5-fluoro)pyridinyl) -2,10-dione (**5a-25**, $R_1$=3-(2,6-dichloro-5-fluoro)pyridinyl, $R_3$=$CH_3$, $R_2$=$R_4$=H)

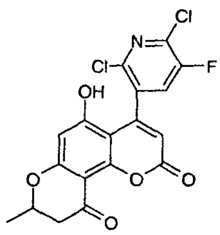

Using the same procedure as described in the preparative method of compound 5a-1, except for using 3.42g (10mmol of 4-(3-(2,6-dichloro-5-fluoro)pyridinyl)-5,7-dihydroxy-coumarin (**6-25**) and 0.76g (10.7mmol) crotonic acid as starting material to obtain 3.2g of the title compound 5a-25 in 78% yield as a white powder, m.p.197-199°C.
$^1$H-NMR (400MHz, DMSO-$d_6$, ppm): 11.727 (s, 1H, 5-OH), 8.219 (dd, 1H, J=1.2H.z, 8.0Hz, CH), 6.301 (s, 1H, 6-H), 6.181 (d, 1H, J=1.2Hz, 3-H), 4.697 (m, 1H, 8-H), 2.685 (m, 2H, 9-$CH_2$), 1.395 (d, 3H, J=6.0Hz, 8-$CH_3$);
ESI-MS (m/z): 410.2[M]$^+$ (MW=410.18);

(3) 6,6,10-trimethyl-4-(3-(2,6-dichloro-5-fluoro)pyridinyl)-2H,6H,1,2H-benzo[1,2-b:3,4-b':5,6-b' ']-tripyranyl-2,12-dione (**4-25**, $R_1$=3-(2,6-dichloro-5-fluoro)pyridinyl, $R_2$=$R_4$=H, $R_3$=$R_5$=$R_6$=$CH_3$)

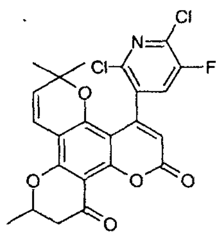

Using the same procedure as described in the preparative method of compound 4-1, except for using 41 mg (0.1 mmol) benzo[1,2-b:3,4-b']-dipyranyl-5-hydroxyl-8-methyl-4-(3-(2,6-dichloro-5-fluoro)pyridinyl) -2,10-dione (5a-25) and 63mg (0.4mmol) 1,1-diethoxyl-3-methyl-2-butene as starting material to obtain 38 mg of the title compound in 81% yield as white powder, m.p.158-160°C.
$^1$H-NMR (400MHz, DMSO-$d_6$): 8.256 (dd, 1H, J=2.8Hz, 8.4Hz, 4-Ar-H), 6.525 (d, 1H, J=10.4Hz, 8-H), 6.386 (s, 1H, 3-H), 5.680 (d, 1H, J=10.4Hz, 7-H), 4.793 (m, 1H, 10-H), 2.719 (m, 2H, 11-$CH_2$), 1.464, 1.449 (2s, 6H, 6-$CH_3$), 1.451 (d, 3H, J=6.4Hz, 10-$CH_3$);
ESI-MS (*m*/*z*): 476.1 (478.1) $M^+$ (MW=476.29);
Elemental analysis: Calculated for $C_{23}H_{16}Cl_2FNO_5$ (%): C, 58.00; H, 3.39; N, 2.94. Found (%): C, 57.98; H, 3.66; N, 3.16.

Example 26

6,6,10-trimethyl-3,4-cyclopentyl-2H,6H,12H-benzo[1,2-b:3,4-b':5,6-b"]-tripyranyl-2,12 -dione (**4-26**, $R_1$,$R_2$=3,4-cyclopentyl, $R_3$=$R_5$=$R_6$=$CH_3$, $R_4$=H)

(1) 3,4-cyclopentyl-5,7-dihydroxy-coumarin (**6-26** , $R_1$,$R_2$=3,4-cyclopentyl)

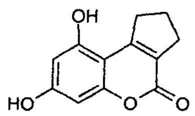

Using the same procedure as described in the preparative method of compound (6-1), except for using 7.5 g (0.046mol) phloroglucinol and 7.18 g (0.046mol) ethyl 2-oxo-cyclopentonate as starting material to obtain 9.7g of the title compound in 96% yield as a white powder crystalline. m.p. 216-218°C.
$^1$H-NMR (400MHz, DMSO-$d_6$, ppm): 10.394 (s, 1H, OH), 6.250 (d, 1H, J=2.4Hz, 8-H), 6.180 (d, 1H, J=2.4Hz, 6-H), 3.198 (t, 2H, J=7.8Hz, 4-$CH_2$), 2.582 (t, 2H, J=7.5Hz, 3-$CH_2$), 1.972 (m, 2H, J=7.5Hz, 7.8Hz, $CH_2$);
ESI-MS (*m*/*z*): 219.1 $[M+H]^+$ (MW=218.21);

(2) benzo[1,2-b:3,4-b']-dipyranyl-5-hydroxyl-8-methyl-3,4-cyclopentyl-2,10-dione (**5a-26**, $R_1$,$R_2$=3,4-cyclopentyl $R_3$=$CH_3$, $R_4$=H)

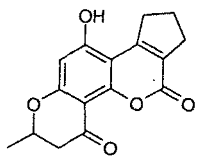

Using the same procedure as described in the preparative method of compound 5a-1, except for using 2.18g (10mmol) 3,4-cyclopentyl-5,7-dihydroxy-coumai-in (6-26) and 0.92g (10.7mmol) crotonic acid as starting material to obtain 2.1g of the title compound 5a-26 in 73% yield as a yellowish powder, m.p.154-155°C.
$^1$HNMR (300MHz, DMSO-$d_6$, ppm): 11.547 (s, 1H, OH), 6.236 (s, 1H, 6-H), 4.629 (m, 1H, 8-H), 3.228 (t, 2H, J=7.5Hz, $CH_2$), 2.650 (m, 2H, 9-$CH_2$), 2.584 (m, 2H, $CH_2$), 1.989 (m, 2H, $CH_2$), 1.387 (d, 3H, J=6.0Hz, 8-$CH_3$);
ESI-MS (m/z): 287.1 $[M+H]^+$ (MW=286.28);

Elemental analysis: Calculated for $C_{16}H_{14}O_5 \cdot \frac{2}{3} H_2O$ (%): C, 64.42; H, 5.18. found (%): C, 64.20; H, 5.39.

(3) 6,6,10-trimethyl-3,4-cyclopentyl-2H,6H,12H-benzo[1,2-b:3,4-b':5,6-b"]-tripyranyl-2,12-dion e (**4-26**, $R_1$,$R_2$=3,4-cyclopentyl, $R_3$=$R_5$=$R_6$=$CH_3$, $R_4$=H)

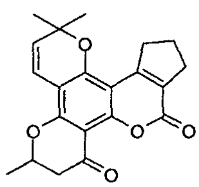

Using the same procedure as described in the preparative method of compound **4** in example 1, except for using 28 mg (0.1mmol) benzo[1,2-b:3,4-b']-dipyranyl-5-hydroxyl-8-methyl-3,4-cyclopentyl-2,10-dione **(5a-26)** and 63mg

(0.4mmol) 1,1-diethoxyl-3-methyl-2-butene as starting material to obtain 25 mg of the title compound in 71% yield as white powder, m.p.108-109°C.
$^1$H-NMR (400MHz, *DMSO-d$_6$):* 6.559 (d, 1H, J=10.0Hz, 8-H), 5.768 (d, 1H, J=10.0Hz, 7-H), 4.713 (m, 1H, 10-H), 3.258 (m, 2H, $CH_2$), 2.633 (m, 2H, $CH_2$), 2.025 (m, 2H, $CH_2$), 1.472 (m, 2H, $CH_2$), 1.450, 1.439 (2s, 6H, 6-$CH_3$), 1.388 (d, 3H, J=6.4Hz, 10-$CH_3$);
ESI-MS (m/z): 353.1 $[M+H]^+$ (MW=352.39);

Elemental analysis: Calculated for $C_{21}H_{20}O_5\cdot\frac{4}{3}H_2O$ (%): C, 67.01; H, 6.06. Found (%): C, 66.99; H, 5.67.

Example 27

6,6,10-trimethyl-3,4-cyclohexyl-2H,6H,12H-benzo[1,2-b:3,4-b':5,6-b"]-tripyranyl-2,12-dione (4-27, 3,4-cyclohexyl, $R_3=R_5=R_6=CH_3$, $R_4$=H)

(1) 3,4-cyclohexyl-5,7-dihydroxy-coumarin (**6-27** , $R_1$,$R_2$=3,4- cyclohexyl)

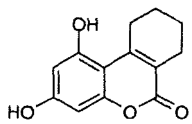

Using the same procedure as described in the preparative method of compound (6-1), except for using 7.5 g (0.046mol) phloroglucinol and 7.83 g (0.046mol) ethyl 2-oxo-cyclohexanoate as starting material to obtain 9.6g of the title compound in 90% yield as a white powder crystalline. m.p. 224-226°C.
$^1$H-NMR (400MHz, DMSO-d$_6$, ppm): 10.336 (s, 1H, 7-OH), 10.082 (s, 1H, 5-OH), 6.239 (d, 1H, J=2.4Hz, 8-H), 6.125 (d, 1H, J=2.4Hz, 6-H), 3.017 (m, 2H, 4-$CH_2$), 2.326 (m, 2H, 3-$CH_2$), 1.632 (m, 4H, $CH_2$);
ESI-MS (*m*/*z*): 233.2 $[M+H]^+$ (MW=232.23);

(2) benzo[1,2-b:3,4-b']-dipyranyl-5-hydroxyl-8-methyl-3,4-cyclohexyl-2,10-dione (**5a-27**, $R_1$, $R_2$=3,4-cyclohexyl, $R_3=CH_3$, $R_4$=H)

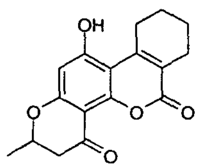

Using the same procedure as described in the preparative method of compound 5a-1, except for using 2.32g (10mmol) 3,4-cyclohexyl-5,7-dihydroxy-coumarin (**6-27**) and 0.92g (10.7mmol)) crotonic acid as starting material to obtain 2.28g of the title compound **5a-27** in 76% yield as a yellowish powder, m.p.149-151°C.
$^1$H NMR (300MHz, DMSO-d$_6$, ppm): 11.493 (s, 1H, OH), 6.238 (s, 1H, 6-H), 4.611 (m, 1H, 8-H), 2.985 (br, 2H, $CH_2$), 2.638 (dd, 1H, J=11.4Hz, 16.2Hz, 9-He), 2.568 (dd, 1H, J=3.9Hz, 16.2Hz, 9-Ha), 2.344 (br, 2H, $CH_2$), 1.632 (br, 4H, $CH_2$), 1.378 (d, 3H, J=6.6Hz, 8-$CH_3$);
ESI-MS (*m*/*z*): 301.1 $[M+H]^+$ (MW=300.31);

Elemental analysis: Calculated for $C_{17}H_{16}O_5\cdot\frac{1}{4}H_2O$ (%): C, 66.99; H, 5.46. Found : C, 67.08; H, 5.60.

(3) 6,6,10-trimethyl-3,4-cyclohexyl-2H,6H,12H-benzo[1,2-b:3,4-b':5,6-b"]-tripyranyl-2,12-dion e (**4-27**, $R_1$, $R_2$=cy-clohexyl, $R_3$=$R_5$=$R_6$=$CH_3$, $R_4$=H)

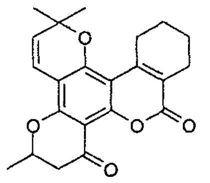

Using the same procedure as described in the preparative method of compound **4-1**, except for using 30 mg (0.1mmol) benzo[1,2-b:3,4-b']-dipyranyl-5-hydroxyl-8-methyl-3,4-cyclohexyl-2,10-dione (**5a-27**) and 63mg (0.4mmol) 1,1-diethoxyl-3-methyl-2-butene as starting material to obtaind 29 mg of the title compound in 79% yield as white powder, m.p.121-123°C.
$^1$H-NMR (400MHz, DMSO-$d_6$): 6.564 (d, 1H, J=10.0Hz, 8-H), 5.758 (d, 1H, J=10.0Hz, 7-H), 4.684 (m, 1H, 10-H), 3.006 (m, 2H, $CH_2$), 2.701 (m, 2H, 11-$CH_2$), 2.371 (m, 2H, $CH_2$), 1.653 (m, 4H, $CH_2$), 1.483, 1.464 (2s, 6H, 6-$CH_3$), 1.437 (d, 3H, J=6.8Hz, 10-$CH_3$);
ESI-MS (*m/z*): 367.1 $[M+H]^+$ (MW=366.41);
Elemental analysis: Calculated for $C_{22}H_{22}O_5 \cdot H_2O$ (%): C, 68.73; H, 6.29. Found (%): C, 68.70; H, 6.07.

<u>Example 28</u>

2,10,10-trimethyl-8-n-propyl-6-thio-2,3-dihydro-6H,10H-benzo[2,3-f:2',3'-h]-tripyranyl -4-one (7, $R_1$=n-$C_3H_7$, $R_2$=$R_4$=H, $R_3$=$R_5$=$R_6$=$CH_3$)

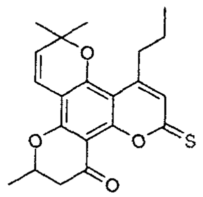

(1) 4-n-propyl-5,7-di(p-methylphenylsulfonyl)-coumarin (8, $R_1$=n-$C_3H_7$, $R_2$=H)

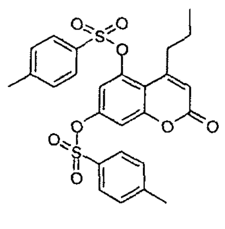

2.20g (10mmol) 4-n-propyl-5,7-dihydroxy-coumarin (6-11) was dissolved in 20 ml pyridine and was cold in ice-water bath, then 11.44g (60 mmol) p-methylphenylsulfonic acid chloride was added. The resulting reaction solution was stirred at room temperature overnight, then was poured into concentrated HCl/cracked ice with viorous stirring. The solid product was filtered and washed with water and then dried to obtain 4.9g of the title compound in 93% yield as a white powder, m.p.239-241 °C.
$^1$H-NMR (300MHz, DMSO-$d_6$): 7.762 (m, 4H, 12,15-Ar-H), 7.499 (m, 4H, 13,16-Ar-H), 7.150 (d, 1H, J=2.4Hz, 8-H), 6.781 (d, 1H, J=2.4Hz, 6-H), 6.322 (s, 1H, 3-H), 2.691 (t, 2H, J=7.5Hz, 9-$CH_2$), 2.429,2.408 (2s, 6H, 14,17-$CH_3$), 1.413 (sex, 2H, J=7.5Hz, 7.2Hz, 10-$CH_2$), 0.792 (t, 3H, J=7.2Hz, 11-$CH_3$);
ESI-MS (m/z): 529.0 $[M+H]^+$ (MW=528.60);

IR (KBr) $cm^{-1}$: 3087, 2962, 2877, 1743, 1612, 1421, 1379, 1194;

(2) 4-n-propyl-5,7-di(p-methylphenyl-sulfonyl)-2-thio-coumarin (**9**, $R_1$=n-$C_3H_7$, $R_2$=H)

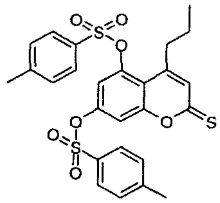

2.64g (5mmol) 4-n-propyl-5,7-di(p-methylphenylsulfonyl)-coumarin (8) was dissolved in xylene and 11g (50mmol) $P_2S_5$ was added. The reaction mixture was refluxed for 12 hours and then cooled to room temperature. The excess $P_2S_5$ was filtered off and the filtrate was concentrated in vacuo. The residue was purified by silica gel column chromatography to afford 1.9g of the title compound in 70% yield as yellow powder, m.p.198-201°C.
$^1$H-NMR (300MHz, DMSO-$d_6$): 7.768 (dd, 4H, J=8.4Hz, 6.9Hz, Ar-H), 7.496 (dd, 4H, J=8.1Hz, 6.6Hz, Ar-H), 7.345 (d, 1H, J=2.7Hz, 8-H), 7.082 (s, 1H, 3-H), 6.859 (d, 1H, J=2.4Hz, 6-H), 2.637 (t, 2H, J=7.5Hz, 9-$CH_2$), 2.426, 2.403 (2s, 6H, 14,17-$CH_3$), 1.403 (sex, 2H, J=7.5Hz, 7.2Hz, 10-$CH_2$), 0.785 (t, 3H, J=7.2Hz, 11-$CH_3$);
ESI-MS (*m/z*): 545.0 $[M+H]^+$ (MW=544.67);
IR (KBr) $cm^{-1}$: 3087, 2974, 2881, 1614, 1597, 1385, 1144.

(3) 4-n-propyl-5,7-dihydroxy-2-thio-coumarin (**10** , $R_1$=n-$C_3H_7$, R2=H)

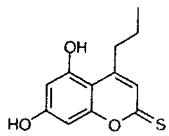

1.36g (2.5mmol) 4-n-propyl-5,7-di(p-methylphenylsulfonyl)-2-thio-coumarin (9) was dissolved in 20 ml THF, 1.18g (3.75mmol) TBAF was added and the reaction mixture was refluxed for 10 hours and then was concentrated in vacuo. The residue was purified by silica gel column chromatography to afford 0.44g of the title compound in 75% yield as yellow powder, m.p.204-206°C.
$^1$H-NMR (300MHz, DMSO-$d_6$): 10.901 (s, 1H, 7-OH), 10.634 (s, 1H, 5-OH), 6.766 (s, 1H, 3-H), 6.358 (d, 1H, J=2.4Hz, 8-H), 6.344 (d, 1H, J=2.4Hz, 6-H), 2.826 (t, 2H, J=7.5Hz, 9-$CH_2$), 1.578 (sex, 2H, J=7.5Hz, 7.2Hz, 10-$CH_2$), 0.932 (t, 3H, J=7.2Hz, 11-$CH_3$);
ESI-MS (*m/z*): 237.1 $[M+H]^+$ (MW=236.29);
Elemental analysis: Calculated for $C_{12}H_{12}O_3S$ (%): C, 61.00; H, 5.12. Found (%): C, 60.84; H, 5.14.

(4) 5-hydroxyl-8-methyl-4-n-propyl-2-thio-2H-benzo[2,3-f]-dipyranyl-10-one (11, $R_1$=n-$C_3H_7$, $R_2$=$R_4$=H, $R_3$=$CH_3$)

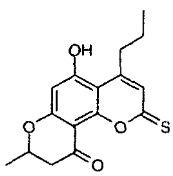

Using the same procedure as described in the preparative method of compound 5a-1. Using 118mg (0.5mmol) of 4-n-propyl-5,7-dihydroxy-2-thio-coumarin (10) and 43mg (0.5mmol) of crotonic acid as starting material to obtain 95mg of the title compound in 63% yield as a brown yellow powder, m.p.161-163°C.

$^1H$ NMR (300MHz, DMSO-$d_6$, ppm): 6.957 (s, 1H, 3-H), 6.365(s, 1H, 6-H), 4.693(m, 1H, 8-H), 2.848(t, 2H, J=7.5Hz, 11-$CH_2$), 2.696(dd, 1H, J=11.4Hz, 16.5Hz, 9-He), 2.608(dd, 1H, J=3.9Hz, 16.5Hz, 9-Ha), 1.564(sex, 2H, J=7.5Hz, 7.2Hz, 12-$CH_2$), 1.406(d, 3H, J=6.3Hz, 8-$CH_3$), 0.928(t, 3H, J=7.2Hz, 13-$CH_3$);
ESI-MS (m/z): 305.1 [M+H]$^+$ (MW=304.37);
HRESIMS: Calculated for $C_{16}H_{17}O_4S^+$ (m/z): 305.08475 , found (m/z): 305.0845.

(5) 2,10,10-trimethyl-8-n-propyl-6-thio-2,3-dihydro-6H,10H-benzo[2,3-f:2',3'-h]-tripyran yl-4-one (7 , $R_1$=n-$C_3H_7$, $R_2$=$R_4$=H, $R_3$=$R_5$=$R_6$=$CH_3$)

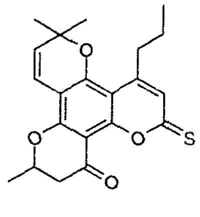

Using the same procedure as described in the preparative method of compound 4-1, except for using 76 mg (0.25mmol) 5-hydroxyl-8-methyl-4-n-propyl-2-thio-2H-benzo[2,3-f]-dipyranyl-10-one (11) and 158mg (1mmol) 1,1-diethoxyl-3-methyl-2-butene as starting material to obtain 58 mg of the title compound in 63% yield as brown yellow powder, m.p.139-141°C.
$^1H$ NMR (300MHz, DMSO-$d_6$, ppm): 7.003(s, 1H, CH), 6.606(d, 1H, J=9.9Hz, CH), 5.839(d, 1H, J=10.2Hz, CH), 5.311 (m, 1H, CH), 2.825(m, 2H, $CH_2$), 2.716(m, 2H, $CH_2$), 1.580(m, 2H, $CH_2$), 1.516, 1.478(2s, 6H, $CH_3$), 1.462(d, 3H, J=6.3Hz, $CH_3$), 0.974(t, 3H, J=7.2Hz, $CH_3$);
ESI-MS (*m/z*): 371.1 [M+H]$^+$ (MW=370.47);
Elemental analysis: Calculated for $C_{21}H_{22}O_4S \cdot 3H_2O$ (%): C, 59.42; H, 6.64. Found (%): C, 59.50; H, 6.32.

Example 29

6,6,10-trimethyl-4-n-propyl-2H,6H,12H-benzo[1,2-b:3,4-b':5,6-b"]-tripyranyl-2,12-dio ne (4-29, $R_1$=n-$C_3H_7$, $R_2$= R4=H, $R_3$=$R_5$=$R_6$=$CH_3$)

(1) 5-hydroxy-4-n-propyl-7-p-methylphenylsulfonyl-coumarin (12, $R_1$=n-$C_3H_7$, $R_2$=H)

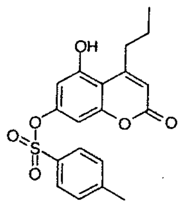

2.64g (5mmol) 4-n-propyl-5,7-di(p-methylsulfonyl)-coumarin (8) was dissolved in 20 ml THF and 1.58g (5mmol) TBAF was added in order to deprotect the 5-hydroxy protective group. The reaction mixture was stirred for 10 hours at room temperature and then was concentrated in vacuo. The residue was purified by silica gel column chromatography to afford 1.65g of the title compound in 88% yield as white powder, m.p.155-157°C.
$^1H$ NMR (300MHz, DMSO-$d_6$, ppm): 11.261 (s, 1H, OH), 7.790 (m, 2H, Ar-H), 7.499 (m, 2H, Ar-H), 6.521 (d, 1H, J=2.4Hz, 8-H), 6.472 (d, 1H, J=2.4Hz, 6-H), 6.104 (s, 1H, 3-H), 2.859 (t, 2H, J=7.5Hz, $CH_2$), 2.422 (s, 3H, $CH_3$), 1.547 (sex, 2H, J=7.5Hz, 7.2Hz, $CH_2$), 0.921 (t, 3H, J=7.2Hz, $CH_3$);
ESI-MS (*m/z*): 375.1 [M+H]$^+$ (MW=374.42);

(2) 2,2-dimethyl-8-oxo-10-n-propyl-2H,8H-benzo[2,3-f]dipyranyl-5-p-methylphenylsulfate (13, $R_1$=n-$C_3H_7$, $R_2$=H, $R_5$=$R_6$=$CH_3$)

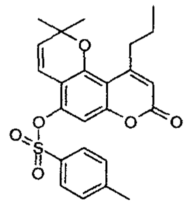

Using the same procedure as described in the preparative method of compound 4-1, except for using 374 mg (1mmol) 5-hydroxy-4-n-propyl-7-p-methylphenylsulfonyl-coumarin (12) and 633mg (4mmol) 1,1-diethoxyl-3-methyl-2-butene as starting material to obtain 385 mg of the title compound in 87% yield as white powder, m.p.141-1.43°C.
$^1$H NMR (300MHz, DMSO-$d_6$, ppm): 7.778 (m, 2H, Ar-H), 7.465 (m, 2H, Ar-H), 6.643 (s, 1H, 10-H), 6.179 (s, 1H, 3-H), 6.170 (d, 1H, J=9.9Hz, 8-H), 5.645 (d, 1H, J=9.9Hz, 7-H), 2.810 (t, 2H, J=7.5Hz, $CH_2$), 2.399 (s, 3H, $CH_3$), 1.534 (sex, 2H, J=7.5Hz, 7.2Hz, $CH_2$), 1.319 (s, 6H, CH3), 0.952 (t, 3H, J=7.2Hz, $CH_3$);
ESI-MS (*m/z*): 441.1 $[M+H]^+$ (MW=440.52);
Elemental analysis: Calculated for $C_{24}H_{24}O_6S$ (%): C, 65.44; H, 5.49. Found (%): C, 65.43; H, 5.42.

(3) 5-hydroxy-2,2-dimethyl-10-n-propyl-2H-benzo[2,3-f]dipyranyl-8-one (14, $R_1$=n-$C_3H_7$, $R_2$=H, $R_5$=$R_6$=$CH_3$)

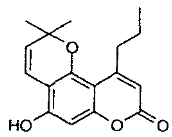

Using the same procedure as described in the preparative method of compound 10 in example 28, except for using 220mg (0.5mmol) 2,2-dimethyl-8-oxo-10-n-propyl-2H,8H-benzo[2,3-f]dipyranyl-5-p-methylphenylsulfate (13) and 236mg (0.75mmol) TBAF as starting material to obtain 95 mg of the title compound in 66% yield as yellow powder, m.p. 128-131°C.
$^1$H NMR (300MHz, DMSO-*$d_6$*, ppm): 10.771 (s, 1H, OH), 6.554 (d, 1H, J=9.9Hz, 8-H), 6.338 (s, 1H, 10-H), 5.907 (s, 1.H, 3-H), 5.652 (d, 1H, J=9.9Hz, 7-H), 2.826 (t, 2H, J=7.5Hz, $CH_2$), 1.579 (sex, 2H, J=7.5Hz, 7.2Hz, $CH_2$), 1.433 (s, 6H, $CH_3$), 0.976 (t, 3H; J=7.2Hz, $CH_3$);
ESI-MS (*m*/*z*): 287.2 $[M+H]^+$ (MW=286.33);

Elemental analysis: Calculated for $C_{17}H_{18}O_4\cdot\frac{1}{4}H_2O$ (%): C, 70.21; H, 6.41. Found (%): C, 70.31; H 6.39.

(4) 6,6,10-trimethyl-4-n-propyl-2H,6H,12H-benzo[1,2-b:3,4-b':5,6-b"]-tripyranyl-2,12-dione (4-29, $R_1$=n-$C_3H_7$, $R_2$= $R_4$=H, $R_3$=Rs=$R_6$=$CH_3$)

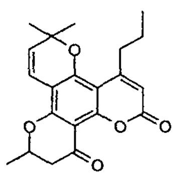

14.3mg (0.05mmol) 5-hydroxy-2,2-dimethyl-10-n-propyl-2H-benzo[2,3-f]dipyranyl-8-one was dissolved in 10

ml $BF_3.Et_2O$ solution. 10mg (0.1mmol) crotonyl chloride was added dropwhise. The resulting reaction solution was allowed to 60 °C for two hours, then cooled to room temperature whereupon it was poured into cracked ice-water and extracted with ethyl acetate (3×20). The organic layer was concentrated and the residue was purified by silica gel column chromatography to afford 18mg of the title compound in 51% yield as white powder, m.p.112-115°C.
$^1$H-NMR (300MHz, DMSO-$d_6$, ppm): 6.566 (d, 1H, J=9.9Hz, 8-H), 6.086 (s, 1H, 3-H), 5.774 (d, 1H, J=9.9Hz, 7-H), 4.707 (m, 1H, J=6.3Hz, 12.0Hz, 3.9Hz, 10-H), 2.837 (t, 2H, J=7.5Hz, 4-C$\underline{H}_2$CH$_2$CH$_3$), 2.708 (dd, 1H, J=12.0Hz, 16.5Hz, 11-He), 2.606 (dd, 1H, J=3.9Hz, 16.5Hz, 11-Ha), 1.537 (sex, 2H, J=7.5Hz, 7.2Hz, 4-CH$_2$CH$_2$CH$_3$), 1.488, 1.450 (2s, 6H, CH$_3$), 1.439 (d, 3H, J=6.3Hz, 10-CH$_3$), 0.963 (t, 3H, J=7.2Hz, 4-CH$_2$CH$_2$C$\underline{H}_3$);
ESI-MS (*m/z*): 355.1 [M+H]$^+$ (MW=354.41);

Example 30

6,6,11-trimethyl-4-n-propyl-2H,6H,12H-benzo[1,2-b:3,4-b':5,6-b"]-tripyranyl-2,12-dio ne (4-30, $R_1$=n-$C_3H_7$, $R_2$=$R_3$=H, $R_4$=$R_5$=$R_6$=CH$_3$)

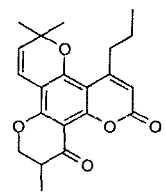

14.3mg (0.05mmol) 5-hydroxy-2,2-dimethyl-10-n-propyl-2H-benzo[2,3-f]dipyranyl-8-one (14) was dissolved in 10 ml $BF_3.Et_2O$ solution. 10 ml (01.1mol) 2-methyl acryloyl chloride was added dropwhise. The resulting reaction solution was allowed to 60 °C for two hours, then cooled to room temperature whereupon it was poured into cracked ice-water and extracted with ethyl acetate(3×20). The organic layer was concentrated and the residue was purified by silica gel column chromatography to afford 19mg of the title compound in 54% yield as white powder, m.p.133-134°C.
$^1$H-NMR (300MHz, DMSO-$d_6$, ppm): 6.565 (d, 1H, J=9.9Hz, 8-H), 6.105 (s, 1H, 3-H), 5.789 (d, 1H, J=9.9Hz, 7-H), 4.630 (dd, 1H, J=5.1Hz, 11.1Hz, 10-He), 4.256 (t, 1H, J=11.1Hz, 10-Ha), 2.846 (m, 3H, 11-CH, 4-C$\underline{H}_2$CH$_2$CH$_3$), 1.562 (sex, 2H, J=7.5Hz, 7.2Hz, 4-CH$_2$C$\underline{H}_2$CH$_3$), 1.492, 1.469 (2s, 6H, CH$_3$), 1.052 (d, 3H, J=6.9Hz, 11-CH$_3$), 0.968 (t, 3H, J=7.2Hz, 4-CH$_2$CH$_2$C$\underline{H}_3$);
ESI-MS (*m/z*): 355.1 [M+H]$^+$ (MW=354.41);
HRESIMS: Calculated for $C_{21}H_{23}O_5^+$ (*m/z*): 355.15455, found (*m/z*): 355.15497.

Example 31

6,6,10-trimethyl-3-fluoro-4-n-propyl-2H,6H,12H-benzo[1,2-b:3,4-b':5,6-b"]-tripyranyl-2,12-dione (**4-31**, $R_1$=n-$C_3H_7$, $R_2$=F, $R_3$=$R_5$=$R_6$=CH$_3$, $R_4$=H)

(1) 3-fluoro-4-n-propyl-5,7-dihydroxy-coumarin (**6-31**, $R_1$=n-$C_3H_7$, $R_2$=F)

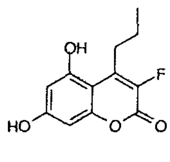

Using the same procedure as described in the preparative method of compound (6-1), except for using 7.5 g (0.046mol) phloroglucinol and 8.1 g (0.046mol) ethyl 2-fluoro butyrylacetate as starting material to obtain 10.6g of the title compound in 97% yield as a yellow powder crystalline. m.p. 228-229°C.
$^1$H-NMR (300MHz, DMSO-*$d_6$*, ppm): 10.632 (s, 1H, OH), 10.270 (s, 1H, OH), 6.313 (d, 1H, J=2.4Hz, 8-H), 6.213 (d, 1H, J=2.4Hz, 6-H), 2.935 (dt, 2H, J=3.3Hz, 8.1Hz, 4-C$\underline{H}_2$CH$_2$CH$_3$), 1.596 (sex, 2H, J=8.1Hz, 7.2Hz, 4-CH$_2$C$\underline{H}_2$CH$_3$), 0.936 (t, 3H, J=7.2Hz, 4-CH$_2$CH$_2$C$\underline{H}_3$);

ESI-MS (*m*/*z*): 239.1 $[M+H]^+$ (MW=238.22);

(2) benzo[1,2-b:3,4-b']-dipyranyl-5-hydroxyl-8-methyl-3-fluoro-4-n-propyl-2,10-dione (**5a-31**, $R_1$=n-$C_3H_7$, $R_2$=F, $R_3$=$CH_3$, $R_4$=H) and benzo[1,2-b:5,4-b']-dipyranyl-5-hydroxyl-8-methyl-3-fluoro-4-n-propyl-2,6-dione (**5b-31**, $R_1$=n-$C_3H_7$, $R_2$=F, $R_3$=$CH_3$, $R_4$=H)

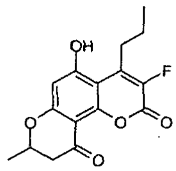

5a-31

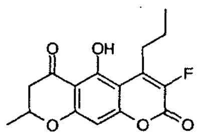

5b-31

Using the same procedure as described in the preparative method of compound 5a-1, except for using 2.38g (10mmol) 3-fluoro-4-n-propyl-5,7-dihydroxy-coumarin (6-31) and 0.92g (10.7mmol) crotonic acid as starting material to obtain 2.1g of the title compound 5a-31 in 68% yield as a yellowish powder (m.p.169-171°C) and 0.34g of the title compound 5b-31 in 11% yield as a yellowish powder(m.p.188-189°C).

Compound 5a-31: $^1$HNMR (300MHz, DMSO-*d*$_6$, ppm): 11.882 (s, 1H, OH), 6.351 (s, 1H, 6-H), 4.638 (dt, 1H, J=6.6Hz, 4.5Hz, 10.8Hz, 8-H), 2.960 (dt, 2H, J=3.0Hz, 7.5Hz, 4-C$\underline{H}_2$CH$_2$CH$_3$), 2.660 (dd, 1H, J=1.0.8Hz, 16.2Hz, 9-He), 2.580 (dd, 1H, J=4.5Hz, 16.5Hz, 9-Ha), 1.583 (sex, 2H, J=7.5Hz, 7.2Hz, 4-CH$_2$C$\underline{H}_2$CH$_3$), 1.389 (d, 3H, J=6.6Hz, 8-CH$_3$), 0.938 (t, 3H, J=7.2Hz, 4-CH$_2$CH$_2$C$\underline{H}_3$);
ESI-MS (*m*/*z*): 307.1 $[M+H]^+$ (MW=306.29);
Elemental analysis: Calculated for $C_{16}H_{15}FO_5$ (%): C, 62.74; H, 4.94. Found (%): C, 62.89; H, 5.02.

Compound 5b-31: $^1$H-NMR (300MHz, DMSO-d$_6$, ppm): 13.814 (s, 1H, OH), 6.530 (s, 1H, 10-H), 4.750 (dq, 1H, J=3.0Hz, 12.3Hz, 6.3Hz, 8-H), 2.990 (dd, 1H, J=12.3Hz, 17.4Hz, 7-He), 2.931 (t, 2H, J=7.5Hz, 4-C$\underline{H}_2$CH$_2$CH$_3$), 2.802 (dd, 1H, J=3.0Hz, 17.4Hz, 7-Ha), 1.612 (sex, 2H, J=7.5Hz, 7.2Hz, 4-CH$_2$C$\underline{H}_2$CH$_3$), 1.436 (d, 3H, J=6.3Hz, 8-CH$_3$), 0.959 (t, 3H, J=7.2Hz, 4-CH$_2$CH$_2$C$\underline{H}_3$);
ESI-MS (*m*/*z*): 307.1 $[M+H]^+$ (MW=306.29);

(3) 6,6,10-trimethyl-3-fluoro-4-n-propyl-2H,6H,12H-benzo[1,2-b:3,4-b':5,6-b"]-tripyranyl-2,12-dione (**4-31**, $R_1$=n-$C_3H_7$, $R_2$=F, $R_3$=$R_5$=$R_6$=$CH_3$, $R_4$=H)

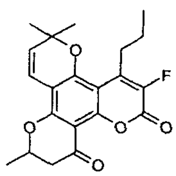

Using the same procedure as described in the preparative method of compound 4-1, except for using 31 mg (0.1 mmol) benzo[1,2-b:3,4-b':]-dipyranyl-5-hydroxyl-8-methyl-3-fluoro-4-n-propyl-2,10-dione (5a-31) and 63mg (0.4mmol) 1,1-diethoxyl-3-methyl-2-butene as starting material to obtain 32 mg of the title compound in 86% yield as off-white powder, m.p.11.4-116°C.
$^1$H-NMR (300MHz, DMSO-*d*$_6$): 6.587 (d, 1H, J=9.9Hz, 8-H), 5.819 (d, 1H, J=9.9Hz, 7-H), 4.711 (m, 1H, 10-H), 2.917 (dt, 2H, J=3.6Hz, 7.5Hz, 4-C$\underline{H}_2$CH$_2$CH$_3$), 2.724 (dd, 1H, J=11.4Hz, 16.5Hz, 11-He), 2.629 (dd, 1H, J=3.9Hz, 16.5Hz, 11-Ha), 1.587 (sex, 2H, J=7.5Hz, 7.2Hz, 4-CH$_2$C$\underline{H}_2$CH$_3$), 1.501,1.464 (2s, 6H, CH$_3$), 1.446 (d, 3H, J=6.3Hz, 10-CH$_3$), 0.990 (t, 3H, J=7.2Hz, 4-CH$_2$CH$_2$C$\underline{H}_3$);
ESI-MS (*m*/*z*): 373.2 $[M+H]^+$ (MW=372.40);
Elemental analysis: Calculated for $C_{21}H_{21}FO_5$ (%): C, 67.73; H, 5.68. Found (%): C, 67.53; H, 5.89.

Example 32

10-methyl-3-fluoro-4,6-di(-n-propyl)-2H,6H,12H-benzo[1,2-b:3,4-b':5,6-b"]-tripyranyl-2,12-dione (**4-32**, $R_1$=$R_5$=n-$C_3H_7$, $R_2$=F, $R_3$=$CH_3$, $R_4$=$R_6$=H)

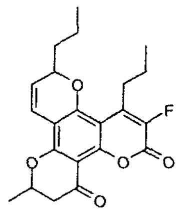

Using the same procedure as described in the preparative method of compound 4-1, except for using 31 mg (0.1mmol) benzo[1,2-b:3,4-b']-dipyranyl-5-hydroxyl-8-methyl-3-fluoro-4-n-propyl-2,10-dione (5a-31) and 69mg (0.4mmol) 1,1-diethoxyl-3-methyl-2-hexene as starting material to obtain 24 mg of the title compound in 62% yield as off-white powder, m.p.123-124°C.
$^1$H-NMR (300MHz, DMSO-$d_6$, ppm): 6.665 (dt, 1H, J=9.9Hz, 3.0Hz, 8-H), 5.866 (dt, 1H, J=9.9Hz, 3.6Hz, 7-H), 5.181 (m, 1H, 6-H), 4.712 (m, 1H, 10-H), 2.926 (m, 2H, $CH_2$), 2.729 (dd, 1H, J=11.1Hz, 16.5Hz, 11-He), 2.639 (dd, 1H, J=3.9Hz, 16.5Hz, 11-Ha), 1.761 (m, 2H, $CH_2$), 1.562 (m, 2H, $CH_2$), 1.441 (d, 3H, J=6.3Hz, 10-$CH_3$), 1.410 (m, 2H, $CH_2$), 0.955 (t, 3H, J=7.2Hz, $CH_3$), 0.917 (t, 3H, J=7.2Hz, $CH_3$);
ESI-MS (*m*/*z*): 387.0 $[M+H]^+$ (MW=386.42);

Elemental analysis: Calculated for $C_{22}H_{23}FO_5 \cdot \frac{1}{2} H_2O$ (%): C, 66.82; H, 6.11. Found (%): C, 66.58; H, 5.94.

Example 33

10-methyl-3-fluoro-4-n-propyl-6-n-butyl-2H,6H,12H-benzo[1,2-b:3,4-b':5,6-b"]-tripyr anyl-2,12-dione (**4-33**, $R_1$=n-$C_3H_7$, $R_2$=F, $R_3$=$CH_3$, $R_4$=$R_6$=H, $R_5$=n-$C_4H_9$)

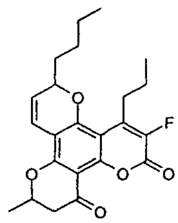

Using the same procedure as described in the preparative method of compound 4-1, except for using 31 mg (0.1mmol) benzo[1,2-b:3,4-b']-dipyranyl-5-hydroxyl-8-methyl-3-fluoro-4-n-propyl-2,10-dione (5a-31) and 74mg (0.4mmol) 1,1-diethoxyl-2-heptene as starting material to obtain 23 mg of the title compound in 58% yield as off-white powder, m.p.119-121°C.
$^1$H-NMR (300MHz, DMSO-$d_6$, ppm): 6.679 (d, 1H, J=10.2Hz, 8-H), 5.841 (m, 1H, 7-H), 5.128 (m, 1H, 6-H), 4.694 (m, 1H, 10-H), 2.926 (m, 2H, $CH_2$), 2.705 (m, 2H, $CH_2$), 2.664 (m, 2H, 11-$CH_2$), 1.788 (m, 2H, $CH_2$), 1.559 (m, 2H, $CH_2$), 1.438 (d, 3H, J=5.4Hz, 10-$CH_3$), 1.213 (m, 2H, $CH_2$), 0.923 (t, 3H, J=7.5Hz, $CH_3$), 0.886 (t, 3H, J=7.2Hz, $CH_3$);
ESI-MS (*m*/*z*): 401.1 $[M+H]^+$ (MW=400.45);

Elemental analysis: Calculated for $C_{23}H_{25}FO_5 \cdot \frac{1}{3} H_2O$ (%): C, 67.96; H, 6.80. Found (%): C, 67.91; H, 7.02.

Example 34

10-methyl-4,6-di(-n-propyl)-2H,6H,12H-benzo[1,2-b:3,4-b':5,6-b"]-tripyranyl-2,12-dio ne (**4-34**, $R_1$=$R_5$=n-$C_3H_7$, $R_3$=$CH_3$, $R_2$=$R_4$=$R_6$=H)

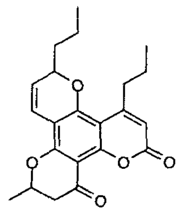

Using the same procedure as described in the preparative method of compound 4-1, except for using 29 mg (0.1 mmol) benzo[1,2-b:3,4-b']-dipyranyl-5-hydroxyl-8-methyl-4-n-propyl-2,10-dione (5a-11) and 69mg (0.4mmol) 1,1-diethoxyl-2-hexene as starting material to obtain 20 mg of the title compound in 55% yield as off-white powder, m.p. 158-160°C.
$^1$H-NMR (300MHz, DMSO-$d_6$, ppm): 6.659 (dt, 1H, J=10.2Hz, 2.4Hz, 8-H, 6.112 (s, 1H, 3-H), 5.835 (dt, 1H, J=10.2Hz, 3.3Hz, 7-H), 5.120 (m, 1H, 6-H), 4.727 (m, 1H, 10-H), 2.859 (dt, 2H, J=7.5Hz, 3.9Hz, 4-C$\underline{H}_2$$CH_2CH_3$), 2.723 (m, 2H, 6-C$\underline{H}_2$$CH_2CH_3$), 2.676 (dd, 1H, J=12.0Hz, 16.5Hz, 11-He), 2.623 (dd, 1H, J=3.9Hz, 16.5Hz, 11-Ha), 1.788 (m, 2H, 4-$CH_2$C$\underline{H}_2$$CH_3$), 1.537 (m, 2H, 6-$CH_2$C$\underline{H}_2$$CH_3$), 1.440 (d, 3H, J=6.3Hz, 10-CH), 0.961 (t, 3H, J=7.2Hz, 4-$CH_2CH_2$C$\underline{H}_3$), 0.903 (t, 3H, J=6.9Hz, 6-$CH_2CH_2$C$\underline{H}_3$);
ESI-MS (*m*/*z*): 369.1 [M+H]$^+$ (MW=368.43);

Elemental analysis: Calculated for $C_{22}H_{24}O_5 \cdot \frac{1}{3} H_2O$ (%): C, 70.57; H, 6.64. Found (%): C, 70.51; H, 6.92.

Example 35

10-methyl-4-n-propyl-6-n-butyl-2H,6H,12H-benzo[1,2-b:3,4-b':5,6-b"]-tripyranyl-2,12-dione (**4-35**, $R_1$=n-$C_3H_7$, $R_3$=$CH_3$, $R_2$=$R_4$=$R_6$=H, $R_5$=n-$C_4H_9$)

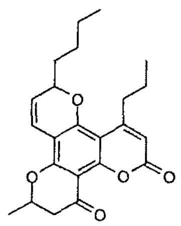

Using the same procedure as described in the preparative method of compound 4-1, except for using 29 mg (0.1mmol)) benzo[1,2-b:3,4-b']-dipyranyl-5-hydroxyl-8-methyl-4-n-propyl-2,10-dione (5a-11) and 74mg (0.4mmol) 1,1-diethoxyl-2-heptene as starting material to obtain 21 mg of the title compound in 56% yield as off-white powder, m.p. 138-139°C.
$^1$H-NMR (300MHz, DMSO-$d_6$, ppm): 6.630 (dt, 1H, J=10.2Hz, 5.4Hz, 8-H), 6.083 (s, 1H, 3-H), 5.807 (dt, 1H, J=10.2Hz, 3.3Hz, 7-H), 5.095 (m, 1H, 6-H), 4.676 (m, 1H, 10-H), 2.895 (m, 2H, $CH_2$), 2.797 (m, 2H, $CH_2$), 2.627 (m, 2H, 11-$CH_2$), 1.769 (m, 2H, $CH_2$), 1.547 (m, 2H, $CH_2$), 1.435 (d, 3H, J=6.3Hz, 10-$CH_3$), 1.299 (m, 2H, $CH_2$), 0.945 (t, 3H, J=7.5Hz, $CH_3$), 0.882 (t, 3H, J=7.2Hz, $CH_3$);
ESI-MS (*m*/*z*): 383.1 [M+H]$^+$ (MW=382.46);
Elemental analysis: Calculated for $C_{23}H_{26}O_5 \cdot 2H_2O$ (%): C, 66.01;. H, 7.47. Found (%): C, 65.97; H, 7.69.

Example 36

6,6,10-trimethyl-4-n-butyl-2H,6H,12H-benzo[1,2-b:3,4-b':5,6-b"]-tripyranyl-2,12-dion e (**4-36**, $R_1$=n-$C_4H_9$, $R_2$=$R_4$=H, $R_3$=$R_5$=$R_6$=$CH_3$)

(1) 4-n-butyl-5,7-dihydroxy-coumarin (**6-36** , $R_1$=n-$C_4H_9$ , $R_2$=H)

Using the same procedure as described in the preparative method of compound (6-1), except for using 7.5 g (0.046mol) phloroglucinol and 7.92 g (0.046mol) ethyl pentanoyl acetate as starting material to obtain 9.37g of the title compound in 87% yield as a yellowish powder crystalline. m.p. 236-237°C,
$^1$H-NMR (300MHz, DMSO-$d_6$, ppm): 10.569 (s, 1H, OH), 10.271 (s, 1H, OH), 6.251 (d, 1H, J=2.4Hz, 8-H), 6.163 (d, 1H, J=2.4Hz, 6-H), 5.811 (s, 1H, 3-H), 2.854 (t, 2H, J=7.5Hz, 4-C$\underline{H}_2$CH$_2$CH$_2$CH$_3$), 1.528 (m, 2H, 4-CH$_2$C$\underline{H}_2$CH$_2$CH$_3$), 1.350 (sex, 2H, J=7.2Hz, 7.5Hz, 4-CH$_2$CH$_2$C$\underline{H}_2$CH$_3$), 0.891 (t, 3H, J=7.2Hz, 4-CH$_2$CH$_2$CH$_2$C$\underline{H}_3$);
ESI-MS (*m*/*z*): 235.1 $[M+H]^+$ (MW=234.25);

(2) benzo[1,2-b:3,4-b']-dipyranyl-5-hydroxyl-8-methyl-4-n-butyl-2,10-dione (**5a-36**, $R_1$=n-$C_4H_9$, $R_2$=$R_4$=H, $R_3$=$CH_3$) and benzo[1,2-b:3,4-b']-dipyranyl-5-hydroxyl-4-n-butyl-8-methyl-2,6-dione (**5b-36**, $R_1$=n-$C_4H_9$, $R_2$=$R_4$=H, $R_3$=$CH_3$)

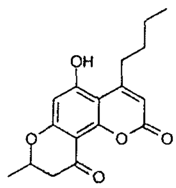

5a-36

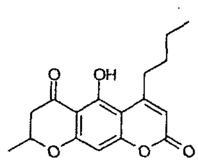

5b-36

Using the same procedure as described in the preparative method of compound 5a-1, except for using 2.34g (10mmol) 4-n-butyl-5,7-dihydroxy-coumarin (6-36) and 0.92g (10.7mmol) crotonic acid as starting material to obtain 1.96g of the title compound 5a-36 in 65% yield as a white powder (m.p.145-147°C) and 0.45g of the title compound 5b-36 in 15% yield as a white powder(m.p.178-179°C).

Compound 5a-36: $^1$H NMR (300MHz, DMSO-$d_6$, ppm): 11.765 (s, 1H, OH), 6.281 (s, 1H, 6-H), 6.024 (s, 1H, 3-H), 4.634 (m, 1H, 8-H), 2.878 (t, 2H, J=7.5Hz; 4-C$\underline{H}_2$CH$_2$CH$_2$CH$_3$), 2.646 (dd, 1H, J=11.4Hz, 16.5Hz, 9-He), 2.561 (dd, 1H, J=3.9Hz, 16.5Hz, 9-Ha), 1.507 (m, 2H, 4-CH$_2$C$\underline{H}_2$CH$_2$CH$_3$), 1.385 (d, 3H, J=6.3Hz, 8-CH$_3$), 1.335 (m, 2H, 4-CH$_2$CH$_2$C$\underline{H}_2$CH$_3$), 0.890 (t, 3H, J=7.2Hz, 4-CH$_2$CH$_2$CH$_2$C$\underline{H}_3$);
ESI-MS (*m*/*z*): 303.2 $[M+H]^+$ (MW=302.33);
Elemental analysis: Calculated for $C_{17}H_{18}O_5$ (%): C, 67.54; H, 6.00. Found (%): C, 67.64; H, 6.07.

Compound 5b-36: $^1$H NMR (300MHz, DMSO-$d_6$, ppm): 13.913 (s, 1H, OH), 6.466 (s, 1H, 10-H), 6.054 (s, 1H, 3-H), 4.755 (dq, 1H, J=6.3Hz, 3.0Hz, 12.3Hz, 8-H), 2.981 (dd, 1H, J=12.3Hz, 17.4Hz, 7-He), 2.887 (t, 2H, J=7.5Hz, 4-C$\underline{H}_2$CH$_2$CH$_2$CH$_3$), 2.795 (dd, 1H, J=3.0Hz, 17.4Hz, 7-Ha), 1.546 (m, 2H, 4-CH$_2$C$\underline{H}_2$CH$_2$CH$_3$), 1.438 (d, 3H, J=6.3Hz, 8-CH$_3$), 1.361 (sex, 2H, J=7.5Hz, 7.2Hz, 4-CH$_2$CH$_2$C$\underline{H}_2$CH$_3$), 0.901 (t, 3H, J=7.2Hz, 4-CH$_2$CH$_2$CH$_2$C$\underline{H}_3$);
ESI-MS (*m*/*z*): 303.2 $[M+H]^+$ (MW=302.33);

(3) 6,6,10-trimethyl-4-n-butyl-2H,6H,12H-benzo[1,2-b:3,4-b':5,6-b"]-tripyranyl-2,12-dione (**4-36**, $R_1$=n-$C_4H_9$, $R_2$=$R_4$=H, $R_3$=$R_5$=$R_6$=$CH_3$)

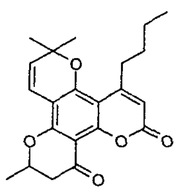

Using the same procedure as described in the preparative method of compound 4-1, except for using 30 mg

(0.1mmol) benzo[1,2-b:3,4-b']-dipyranyl-5-hydroxyl-8-methyl-4-n-butyl-2,10-dione (5a-36) and 63mg (0.4mmol) 1,1-diethoxyl-3-methyl-2-butene as starting material to obtain 33 mg of the title compound in 89% yield as off-white powder, m.p.121-122°C.
$^1$H-NMR (300MHz, DMSO-$d_6$, ppm): 6.574 (d, 1H, J=10.2Hz, 8-H), 6.088 (s, 1H, 3-H), 5.779 (d, 1H, J=10.2Hz, 7-H), 4.709 (m, 1H, 10-H), 2.864 (t, 2H, J=7.5Hz, $CH_2$), 2.712 (dd, 1H, J=11.4Hz, 16.5Hz, 11-He), 2.611 (dd, 1H, J=3.9Hz, 16.5Hz, 11-Ha), 1.541 (m, 2H, $CH_2$), 1.489, 1.452 (2s, 6H, $CH_3$), 1.376 (m, 2H, $CH_2$), 1.441 (d, 3H, J=6.6Hz, 10-$CH_3$), 0.903 (t, 3H, J=7.2Hz, $CH_3$);
ESI-MS (*m*/*z*): 369.1 $[M+H]^+$ (MW=368.43);
Elemental analysis: Calculated for $C_{22}H_{24}O_5$ (%): C, 71.72; H, 6.57. Found (%): C, 71.59; H, 6.70.

Example 37

10-methyl-6-n-propyl-4-n-butyl-2H,6H,12H-benzo[1,2-b:3,4-b':5,6-b"]-tripyranyl-2,12 -dione (**4-37**, $R_1$=n-$C_4H_9$, $R_2$=$R_4$=$R_6$=H, $R_3$=$CH_3$, $R_5$=n-$C_3H_7$)

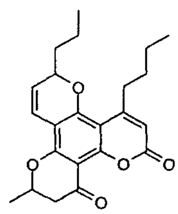

Using the same procedure as described in the preparative method of compound 4-1, except for using 30 mg (0.1mmol) benzo[1,2-b:3,4-b']-dipyranyl-5-hydroxyl-8-methyl-4-n-butyl-2,10-dione (5a-36) and 69mg (0.4mmol) 1,1-diethoxyl-2-hexene as starting material to obtain 29 mg of the title compound in 76% yield as off-white powder, m.p. 135-138°C.
$^1$H-NMR (300MHz, DMSO-$d_6$, ppm): 6.635 (d, 1H, J=9.9Hz, 8-H), 6.083 (s, 1H, 3-H), 5.817 (dt, 1H, J=9.9Hz, 3.0Hz, 7-H), 5.108 (m, 2H, $CH_2$), 4.702 (m, 1H, 10-H), 2.855 (m, 2H, $CH_2$), 2.645 (m, 2H, 11-$CH_2$), 1.762 (m, 2H, $CH_2$), 1.436 (d, 3H, J=6.3Hz, 10-$CH_3$), 1.358 (m, 2H, $CH_2$), 1.087 (m, 2H, $CH_2$), 0.922 (t, 3H, J=7.2Hz, $CH_3$), 0.873 (t, 3H, J=7.2Hz, $CH_3$);
ESI-MS (*m*/*z*): 383.1 $[M+H]^+$ (MW=382.46);
Elemental analysis: Calculated for $C_{23}H_{26}O_5$ (%): C, 72.23; H, 6.85. Found (%): C, 72.16; H, 7.15.

Example 38

10-methyl-4,6-di(n-butyl)-2H,6H,12H-benzo[1,2-b:3,4-b':5,6-b"]-tripyranyl-2,12-dione (**4-38**, $R_1$=$R_5$=n-$C_4H_9$, $R_2$=$R_4$=$R_6$=H, $R_3$=$CH_3$)

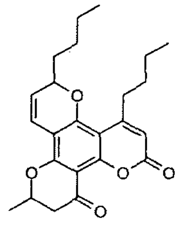

Using the same procedure as described in the preparative method of compound 4-1, except for using 30 mg (0.1 mmol) benzo[1,2-b:3,4-b']-dipyranyl-5-hydroxyl-8-methyl-4-n-butyl-2,10-dione (5a-36) and 74mg (0.4mmol) 1,1-diethoxyl-2-heptene as starting material to obtain 31 mg of the title compound in 79% yield as off-white powder, m.p. 146-148°C.
$^1$H-NMR (300MHz, DMSO-$d_6$, ppm): 6.643 (dd, 1H, J=2.1Hz, 9.9Hz, 8-H), 6.089 (s, 1H, 3-H), 5.820 (dt, 1H, J=3.0Hz, 9.9Hz, 7-H), 5.108 (m, 2H, $CH_2$), 4.706 (m, 2H; $CH_2$), 2.879 (m, 4H, $CH_2$), 2.665 (m, 2H, 11-$CH_2$), 1.768 (m, 2H, $CH_2$),

1.496 (m, 2H, $CH_2$), 1.436 (m, 2H, $CH_2$), 0.921 (t, 3H, J=7.2Hz, $CH_3$), 0.874 (t, 3H, J=7.2Hz, $CH_3$);
ESI-MS (m/z): 397.1 $[M+H]^+$ (MW=396.49);
Elemental analysis: Calculated for $C_{24}H_{28}O_5$ (%): 72.70; H, 7.12. Found (%): C, 72.47; H, 7.14.

Example 39

10-methyl-4-ethyl-6-n-propyl-3-fluoro-2H,6H,12H-benzo[1,2-b:3,4-b':5,6-b"]-tripyran yl-2,12-dione (**4-39**, $R_1$=$C_2H_5$, $R_2$=F, $R_3$=$CH_3$, $R_5$=n-$C_3H_7$, $R_4$=$R_6$=H)

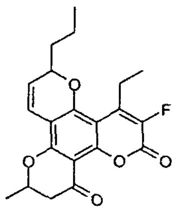

Using the same procedure as described in the preparative method of compound 4-1, except for using 29 mg (0.1mmol) benzo[1,2-b:3,4-b']-dipyranyl-5-hydroxyl-8-methyl-4-ethyl-3-fluoro-2,10-dione (5a-10) and 69mg (0.4mmol) 1,1-diethoxyl-2-hexene as starting material to obtain 25 mg of the title compound in 68% yield as off-white powder, m.p. 151-153°C.
$^1$H-NMR (300MHz, DMSO-$d_6$, ppm): 6.641 (dd, 1H, J=1.8Hz, 10.2Hz, 8-H), 5.842 (dt, 1H, J=3.3Hz, 10.2Hz, 7-H), 5.1.48 (m, 1H, 6-H), 4.700 (m, 1H, 10-H), 2.955 (m, 2H, 4-$CH_2CH_3$), 2.694 (m, 2H, 11-$CH_2$), 1.783 (m, 2H, $CH_2$), 1.440 (d, 3H, J=6.3Hz, 10-$CH_3$), 1.768 (m, 2H, $CH_2$), 0.937 (t, 3H, J=7.2Hz, $CH_3$), 0.891 (t, 3H, J=7.2Hz, $CH_3$);
ESI-MS (*m*/*z*): 373.1 [M+H] (MW=372.40);
Elemental analysis: Calculated for $C_{21}H_{21}FO_5$ (%): C, 67.73; H, 5.68. Found (%): C, 67.76; H, 5.61.

Example 40

10-methyl-4-ethyl-3-fluoro-6-n-butyl-2H,6H,12H-benzo[1,2-b:3,4-b':5,6-b"]-tripyranyl-2,12-dione (**4-40**, $R_1$=$C_2H_5$, $R_2$=F, $R_3$=$CH_3$, $R_5$=n-$C_4H_9$, $R_4$=$R_6$=H)

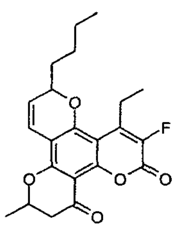

Using the same procedure as described in the preparative method of compound 4-1, except for using 29 mg (0.1 mmol) benzo[1,2-b:3,4-b']-dipyranyl-5-hydroxyl-8-methyl-4-ethyl-3-fluoro-2,10-dione (5a-10) and 74mg (0.4mmol) 1,1-diethoxyl-2-heptene as starting material to obtain 22 mg of the title compound in 56% yield as off-white powder, m.p. 142-143°C.
$^1$H-NMR (300MHz, DMSO-$d_6$, ppm): 6.660 (dt, 1H, J=1.BHz, 10.2Hz, 8-H), 5.849 (dt, 1H, J=3.3Hz, 10.2Hz, 7-H), 5.155 (m, 1H, 6-H), 4.707 (m, 1H, 10-H), 2.965 (m, 2H, $CH_2$), 1.791 (m, 2H, $CH_2$), 1.442 (d, 3H, J=6.0Hz, 10-$CH_3$), 1.331 (m, 2H, $CH_2$), 1.144 (m, 2H, $CH_2$), 0.894 (t, 3H, J=7.2Hz, $CH_3$), 0.872 (t, 3H, J=7.2Hz, $CH_3$);
ESI-MS (m/z): 373.1 $[M+H]^+$ (MW=372.40);
Elemental analysis: Calculated for $C_{22}H_{23}FO_5 \cdot \frac{1}{3} H_2O$ (%): C, 67.34; H, 6.08. Found (%): C, 67.41; H, 6.09.

Example 41,

10-methyl-4-n-propyl-6-phenyl-2H,6H,12H-benzo[1,2-b:3,4-b':5,6-b"]-tripyranyl-2,12-dione (**4-41**, $R_1$=n-$C_3H_7$, $R_3$=$CH_3$, $R_5$=$C_6H_5$, $R_2$=$R_4$=$R_6$=H)

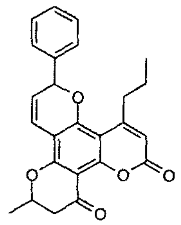

Using the same procedure as described in the preparative method of compound 4-1, except for using 31 mg (0.1mmol) benzo[1,2-b:3,4-b']-dipyranyl-5-hydroxyl-8-methyl-4-n-propyl-2,10-dione (5a-11) and 82mg (0.4mmol) 1,1-diethoxyl-3-phenyl-2-propene as starting material to obtain 27 mg of the title compound in 66% yield as off-white powder, m.p.96-99°C.
$^1$H-NMR (300MHz, DMSO-$d_6$, ppm): 7.451 (m, 5H, 6-Ph), 6.862 (dd, 1H, J=1.8Hz, 9.9Hz, 8-H), 6.257 (dd, 1H, J=1.8Hz, 3.9Hz, 6-H), 6.052 (s, 1H, 3-H), 5.952 (dd, 1H, J=3.9Hz, 9.9Hz, 7-H), 4.783 (m, 1H, 10-H), 2.947 (t, 2H, J=7.5Hz, $CH_2$), 2.669 (m, 2H, 11-$CH_2$), 1.647 (sex, 2H, J=7.5Hz, 7.2Hz, $CH_2$), 1.474 (d, 3H, J=6.3Hz, 10-$CH_3$), 0.973 (t, 3H, J=7.2Hz, $CH_3$);
ESI-MS (*m*/*z*): 403.1 $[M+H]^+$ (MW=402.45);
Elemental analysis: Calculated for $C_{25}H_{22}O_5 \cdot H_2O$ (%): C, 71.41; H, 5.75. Found (%): C, 71.25; H, 5.97.

Example 42

10-methyl-4-ethyl-6-phenyl-3-fluoro-2H,6H,12H-benzo[1,2-b:3,4-b':5,6-b"]-tripyranyl-2,12-dione (**4-42**, $R_1$=$C_2H_5$, $R_2$=F, $R_3$=$CH_3$, $R_5$=$C_6H_5$, $R_4$=$R_6$=H)

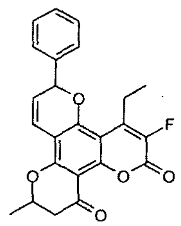

Using the same procedure as described in the preparative method of compound 4-1, except for using 29 mg (0.1mmol) benzo[1,2-b:3,4-b']-dipyranyl-5-hydroxyl-8-methyl-4-ethyl-3-fluoro-2,10-dione (5a-10) and 82mg (0.4mmol) 1,1-diethoxyl-3-phenyl-2-propene as starting matrial to obtain 30 mg of the title compound in 75% yield as off-white powder, m.p. 109-111 °C.
$^1$H-NMR (300MHz, DMSO-$d_6$, ppm): 7.445 (m, 5H, 6-Ph), 6.890 (dd, 1H, J=1.8Hz, 9.9Hz, 8-H), 6.289 (dd, 1H, J=1.8Hz, 3.9Hz, 6-H), 6.015 (dd, 1H, J=3.9Hz, 9.9Hz, 7-H), 4.756 (m, 1H, 10-H), 3.060 (m, 2H, $CH_2$), 2.703 (m, 2H, 11-$CH_2$), 1.481 (d, 3H, J=6.3Hz, 10-$CH_3$), 1.040 (t, 3H, J=7.2Hz, $CH_3$);
ESI-MS (*m*/*z*): 407.1 $[M+H]^+$ (MW=406.41);
Elemental analysis: Calculated for $C_{24}H_{19}FO_5 \cdot \frac{1}{2} H_2O$ (%): C, 69.39; H, 4.85. Found (%): C, 69.62; H, 4.97.

Example 43

10-methyl-4-n-propyl-6-phenyl-3-fluoro-2H,6H,12H-benzo[1,2-b:3,4-b':5,6-b"]-tripyra nyl-2,12-dione (**4-43**, $R_1$=n-$C_3H_7$, $R_2$=F, $R_3$=$CH_3$, $R_5$=$C_6H_5$, $R_4$=$R_6$=H)

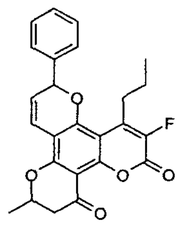

Using the same procedure as described in the preparative method of compound 4-1, except for using 31 mg (0.1mmol) benzo[1,2-b:3,4-b']-dipyranyl-5-hydroxyl-8-n-propyl-3-fluoro-2,10-dione (5a-31) and 82mg (0.4mmol) 1,1-diethoxyl-3-phenyl-2-propene as starting material to obtain 28 mg of the title compound in 68% yield as off-white powder, m.p.128-129°C.
$^1$H-NMR (300MHz, DMSO-$d_6$, ppm): 7.457 (m, 5H, 6-Ph), 6.867 (dd, 1H, J=1.8Hz, 9.9Hz, 8-H), 6.267 (dd, 1H, J=1.8Hz, 3.3Hz, 6-H), 5.958 (dd, 1H, J=3.3Hz, 9.9Hz, 7-H), 4.770 (m, 1H, 10-H), 2.801 (m, 2H, $CH_2$), 2.688 (m, 2H, 11-$CH_2$), 1.747 (m, 2H, $CH_2$), 1.485 (d, 3H, J=6.3Hz, 10-$CH_3$), 0.674 (t, 3H, J=7.2Hz, $CH_3$);
ESI-MS (*m*/*z*): 421.1 $[M+H]^+$ (MW=420.44);
Elemental analysis: Calculated for $C_{25}H_{21}FO_5 \cdot \frac{2}{3} H_2O$ (%): C, 69.44; H, 5.21. Found (%): C, 69.60; H, 5.41.

Example 44

10-methyl-4-n-butyl-6-phenyl-2H,6H,12H-benzo[1,2-b:3,4-b':5,6-b"]-tripyranyl-2,12-d ione (**4-44**, $R_1$=n-$C_4H_9$, $R_3$=$CH_3$, $R_5$=$C_6H_5$, $R_2$=$R_4$=$R_6$=H)

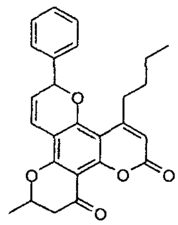

Using the same procedure as described in the preparative method of compounds 4-1, except for using 30 mg (0.1mmol) benzo[1,2-b:3,4-b']-dipyranyl-5-hydroxyl-8-methyl-4-n-butyl-2,10-dione (5a-36) and 82mg (0.4mmol) 1,1-diethoxyl-3-phenyl-2-propene as starting material to obtain 26 mg of the title compound in 62% yield as off-white powder, m.p.137-139°C.
$^1$H-NMR (300MHz, DMSO-$d_6$, ppm): 7.534 (m, 5H, 6-Ph), 6.857 (dd, 1H, J=1.8Hz, 9.9Hz, 8-H), 6.243 (dd, 1H, J=1.8Hz, 3.3Hz, 6-H), 6.043 (s, 1H, 3-H), 5.925 (dd, 1H, J=3.3Hz, 9.9Hz, 7-H), 4.746 (m, 1H, 10-H), 2.748 (m, 2H, $CH_2$), 2.684 (m, 2H, $CH_2$), 1.470 (dd, 3H, J=1.2Hz, 6.3Hz, 10-$CH_3$), 1.401. (m, 2H, $CH_2$), 1.224 (m, 2H, $CH_2$), 0.658 (t, 3H, J=7.2Hz, $CH_3$);
ESI-MS (*m*/*z*): 417.1 $[M+H]^+$ (MW=416'.48);
Elemental analysis: Calculated for $C_{26}H_{24}O_5 \cdot \frac{1}{3} H_2O$ (%): C, 73.92; H, 5.88. Found (%): C, 73.93; H, 6.02.

Example 45

1.0-methyl-4-ethyl-6-phenyl-2H,6H,12H-benzo[1,2-b:3,4-b':5,6-b"]-tripyranyl-2,12-dio ne (**4-45**, $R_1$=$C_2H_5$, $R_3$=$CH_3$, $R_5$=$C_6H_5$, $R_2$=$R_4$=$R_6$=H)

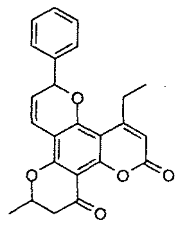

Using the same procedure as described in the preparative method of compound 4-1, except for using 27 mg (0.1mmol) benzo[1,2-b:3,4-b']-dipyranyl-5-hydroxyl-8-methyl-4-ethyl-2,10-dione (5a-9) and 82mg (0.4mmol) 1,1-diethoxyl-3-phenyl-2-propene as starting material to obtain 25 mg of the title compound in 64% yield as off-white powder, m.p.126-128°C.
$^1$H-NMR (300MHz, DMSO-$d_6$, ppm): 7.437 (m, 5H, 6-Ph), 6.862 (dd, 1H, J=1.8Hz, 9.9Hz, 8-H), 6.286 (dd, 1H, J=1.8Hz, 3.3Hz, 6-H), 6.060 (s, 1H, 3-H), 5.979 (dd, 1H, J=3.3Hz, 9.9Hz, 7-H), 4.753 (m, 1H, 10-H), 3.040 (m, 2H, $CH_2$), 2.690 (m, 2H, $CH_2$), 1.471 (dd, 3H, J=2.1Hz, 6.3Hz, 10-$CH_3$), 1.221 (t, 3H, J=7.2Hz, $CH_3$);
ESI-MS (*m*/*z*): 389.1 $[M+H]^+$ (MW=388.42);

Elemental analysis: Calculated for $C_{24}H_{20}O_5\cdot\frac{1}{2}H_2O$ (%): C, 72.53; H, 5.33. Found (%): C, 72.60; H, 5.83.

Example 46

10-methyl-4-ethyl-6-n-butyl-2H,6H,12H-benzo[1,2-b:3,4-b':5,6-b"]-tripyranyl-2,12-dio ne (**4-46**, $R_1$=$C_2H_5$, $R_3$=$CH_3$, $R_5$=n-$C_4H_9$, $R_2$=$R_4$=$R_6$=H)

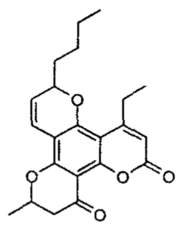

Using the same procedure as described in the preparative method of compound 4-1, except for using 27 mg (0.1mmol) benzo[1,2-b:3,4-b']-dipyranyl-5-hydroxyl-8-methyl-4-ethyl-2,10-dione (5a-9) and 74mg (0.4mmol) 1,1-diethoxyl-2-heptene as starting material to obtain 29 mg of the title compound in 78% yield as off-white powder, m.p. 119-121°C.
$^1$H-NMR (300MHz, DMSO-$d_6$, ppm): 6.632 (dt, 1H, J=1.8Hz, 9.9Hz, 8-H), 6.096 (s, 1H, 3-H), 5.809 (dt, 1H, J=3.3Hz, 9.9Hz, 7-H), 5.467 (m, 1H, 6-H), 4.688 (m, 1H, 10-H), 2.915 (m, 2H, $CH_2$), 2.654 (m, 2H, 11-$CH_2$), 1.790 (m, 2H, $CH_2$), 1.435 (dd, 3H, J=3.6Hz, 6.0Hz, 10-$CH_3$), 1.340 (m, 4H, $CH_2$), 1.168 (t, 3H, J=7.2Hz, $CH_3$), 0.881 (t, 3H, J=7.2Hz, $CH_3$);
ESI-MS (*m*/*z*): 369.1 $[M+H]^+$ (MW=368.43);

Elemental analysis: Calculated for $C_{22}H_{24}O_5\cdot\frac{1}{2}H_2O$ (%): C, 70.01; H, 6.68. Found (%): C, 69.98; H, 6.89.

Example 47

10-methyl-4-ethyl-6-n-propyl-2H,6H,12H-benzo[1,2-b:3,4-b':5,6-b"]-tripyranyl-2,12-di one (**4-47**, $R_1$=$C_2H_5$, $R_3$=$CH_3$, $R_5$=n-$C_3H_7$, $R_2$=$R_4$=$R_6$=H)

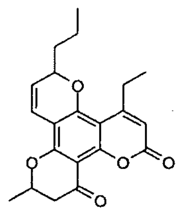

Using the same procedure as described in the preparative method of compound 4-1, except for using 27 mg (0.1 mmol) benzo[1,2-b:3,4-b']-dipyranyl-5-hydroxyl-8-methyl-4-ethyl-2,10-dione (5a-9) and 69mg (0.4mmol) 1,1-diethoxyl-2-hexene as starting material to obtain 29 mg of the title compound in 78% yield as off-white powder, m.p. 119-121°C.
$^1$H-NMR (300MHz, DMSO-$d_6$, ppm): 6.637 (dt, 1H, J=1.8Hz, 9.9Hz, 8-H), 6.098 (s, 1H, 3-H), 5.819 (dt, 1H, J=3.3Hz, 9.9Hz, 7-H), 5.477 (m, 1H, 6-H), 4.701 (m, 1H, 10-H), 2.949 (m, 2H, $CH_2$), 2.665 (m, 2H, 11-$CH_2$), 1.764 (m, 2H, $CH_2$), 1.438 (dd, 3H, J=3.0Hz, 6.3Hz, 10-$CH_3$), 1.170 (t, 3H, J=7.2Hz, $CH_3$), 1.116 (m, 4H, $CH_2$), 0.899 (t, 3H, J=7.2Hz, $CH_3$);
ESI-MS (*m*/*z*): 355.1 $[M+H]^+$ (MW=354.41);
Elemental analysis: Calculated for $C_{21}H_{22}O_5 \cdot \frac{1}{2} H_2O$ (%): C, 69.41; H, 6.38. Found (%): C, 69.62; H, 6.75.

Example 48

10-methyl-4,6-di(-n-propyl)-3,-chloro-2H,6H,12H-benzo[1,2-b:3,4-b':5,6-b"]-tripyrany 1-2,12-dione (**4-48**, $R_1$=$R_5$=n-$C_3H_7$, $R_2$=Cl, $R_3$=$CH_3$, $R_4$=$R_6$=H)

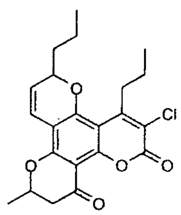

Using the same procedure as described in the preparative method of compound 4-1, except for using 32 mg (0.1mmol) benzo[1,2-b:3,4-b']-dipyranyl-5-hydroxyl-8-methyl-4-n-propyl-3-chloro-2,10-dione (5a-20) and 69mg (0.4mmol) 1,1-diethoxyl-2-hexene as starting material to obtain 29 mg of the title compound in 73% yield as off-white powder, m.p.140-142°C.
$^1$H-NMR (300MHz, DMSO-$d_6$, ppm): 6.671 (dt, 1H, J=1.8Hz, 9.9Hz, 8-H), 5.878 (dt, 1H, J=2.7Hz, 9.9Hz, 7-H), 5.177 (m, 1H, 6-H), 4.734 (m, 1H, 10-H), 3.118 (t, 2H, J=7.2Hz, $CH_2$), 2.662 (m, 2H, 11-$CH_2$), 1.785 (m, 2H, $CH_2$), 1.551 (m, 2H, $CH_2$), 1.447 (d, 3H, J=6.3Hz, 10-$CH_3$), 1.002 (t, 3H, J=7.2Hz, $CH_3$), 0.939 (t, 3H, J=7.2Hz, $CH_3$);
ESI-MS (*m*/*z*): 403.1 $M^+$ (MW=402.88);
Elemental analysis: Calculated for $C_{22}H_{23}ClO_5$ (%): C, 65.59; H, 5.75. Found (%): C, 65.36; H, 5.74.

Example 49

10-methyl-4-n-propyl-3-chloro-6-n-butyl-2H,6H,12H-benzo[1,2-b:3,4-b':5,6-b"]-tripyra nyl-2,12-dione (**4-49**, $R_1$=n-$C_3H_7$, $R_2$=Cl, $R_3$=$CH_3$, $R_5$=n-$C_4H_9$, $R_4$=$R_6$=H)

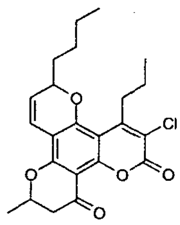

Using the same procedure as described in the preparative method of compound 4-1, except for using 32 mg (0.1mmol) benzo[1,2-b:3,4-b']-dipyranyl-5-hydroxyl-8-methyl-4-n-propyl-3-chloro-2,10-dione (5a-20) and 74mg (0.4mmol) 1,1-diethoxyl-2-heptene as starting material to obtain 30 mg of the title compound in 71% yield as off-white powder, m.p.146-149°C.
$^1$H-NMR (300MHz, DMSO-$d_6$, ppm): 6.664 (dt, 1H, J=1.8Hz, 10.2Hz, 8-H), 5.871 (dt, 1H, J=3.3Hz, 10.2Hz, 7-H), 5.175 (m, 1H, 6-H), 4.730 (m, 1H, 10-H), 3.110 (t, 2H, J=7.8Hz, $CH_2$), 2.673 (m, 2H, 11-$CH_2$), 1.803 (m, 2H, $CH_2$), 1.550 (m, 2H, $CH_2$), 1.446 (d, 3H, J=6.3Hz, 10-$CH_3$), 1.343 (m, 4H, $CH_2$), 1.003 (t, 3H, J=7.2Hz, $CH_3$), 0.890 (t, 3H, J=6.6Hz, $CH_3$);
ESI-MS (*m*/*z*): 417.1 $M^+$ (MW=416.91);
Elemental analysis: Calculated for $C_{23}H_{25}ClO_5\cdot\frac{1}{8}H_2O$ (%): C, 65.91; H, 6.07. Found (%): C, 65.90; H, 6.23.

Example 50

10-methyl-4-n-propyl-3-chloro-6-phenyl-2H,6H,12H-benzo[1,2-b:3,4-b':5,6-b"]-tripyra nyl-2,12-dione (**4-50**, $R_1$=n-$C_3H_7$, $R_2$=Cl, $R_3$=$CH_3$, $R_5$=$C_6H_5$, $R_4$=$R_6$=H)

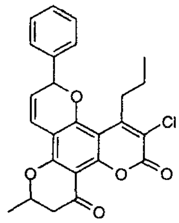

Using the same procedure as described in the preparative method of compound 4-1, except for using 32 mg (0.1mmol) benzo[1,2-b:3,4-b']-dipyranyl-5-hydroxyl-8-methyl-4-n-propyl-3-chloro-2,10-dione (5a-20) and 82mg (0.4mmol) 1,1-diethoxyl-3-phenyl-2-propene as starting material to obtain 30 mg of the title compound in 71% yield as off-white powder, m.p.152-154°C.
$^1$H-NMR (300MHz, DMSO-$d_6$, ppm): 7.449 (m, 5H, 6-Ph), 6.852 (dq, 1H, J=1.8Hz, 10.2Hz, 8-H), 6.275 (dq, 1H, J=3.0Hz, 1.8Hz, 6-H), 5.952 (dq, 1H, J=3.0Hz, 10.2Hz, 7-H), 4.771 (m, 1H, 10-H), 2.967 (m, 2H, $CH_2$), 2.715 (m, 2H, 11-$CH_2$), 1.480 (dd, 3H, J=0.9Hz, 6.3Hz, 10-$CH_3$), 1.318 (m, 2H, $CH_2$), 0.675 (t, 3H, J=7.2Hz, $CH_3$);
ESI-MS (*m*/*z*): 437.1 $M^+$ (MW=436.90);
Elemental analysis: Calculated for $C_{25}H_{21}ClO_5$ (%): C, 68.73; H, 4.84. Found (%): C, 68.53; H, 5.14.

Example 51

11-methyl-4,6-di(-n-propyl)-2H,6H,12H-benzo[1,2-b:3,4-b':5,6-b"]-tripyranyl-2,12-dio ne (**4-51**, $R_1$=$R_5$=n-$C_3H_7$, $R_2$=$R_3$=$R_6$=H, $R_4$=$CH_3$)

(1) benzo[1,2-b:3,4-b']-dipyranyl-5-hydroxyl-9-methyl-4-n-propyl-2,10-dione (5a-51 $R_1$=n-$C_3H_7$, $R_2$=$R_3$=H, $R_4$=$CH_3$)

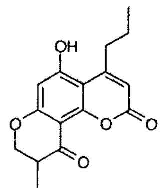

Using the same procedure as described in the preparative method of compound 5a-1, except for using 2.20.g (10mmol) 4-n-propyl-5,7-dihydroxy-coumarin (6-11) and 0.92g (10.7mmol) 2-methylpropenoic acid as starting material to obtain 1.93g of the title compound 5a-11 in 67% yield as a off-white powder, m.p.164-166°C.
$^1$H-NMR (300MHz, DMSO-$d_6$, ppm): 10.961 (s, 1H, OH), 6.450 (s, 1H, 6-H), 6.052 (s, 1H, 3-H), 3.154 (dd, 1H, J=6.6Hz, 15.6Hz, 10-He), 2.907 (m, 3H, 4-$CH_2$, 11-CH), 2.642 (dd, 1H, J=12.3Hz, 15.6Hz, 10-Ha), 1.588 (sex, 2H, J=7.5Hz, 7.2Hz, 4-$CH_2C\underline{H}_2CH_3$), 1.250 (d, 3H, J=6.6Hz, 11-$CH_3$), 0.939 (t, 3H, J=7.2Hz, 4-$CH_2CH_2C\underline{H}_3$);
ESI-MS (*m*/*z*): 289.1 $[M+H]^+$ (MW=288.30);
Elemental analysis: Calculated for $C_{16}H_{16}O_5$ (%): C, 66.66; H, 5.59. Found (%): C, 66.55; H, 6.10.

(2) 11-methyl-4,6-di(-n-propyl)-2H,6H,12H-benizo[1,2-b:3,4-b':5,6-b"]-tripyranyl-2,1.2-dione (**4-51**, $R_1$=$R_5$=n-$C_3H_7$, $R_2$=$R_3$=$R_6$=H, $R_4$=$CH_3$)

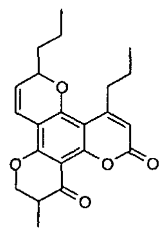

Using the same procedure as described in the preparative method of compound 4-1, except for using 28 mg (0.1mmol) benzo[1,2-b:3,4-b']-dipyranyl-5-hydroxyl-9-methyl-4-n-propyl-2,10-dione (5a-51) and 69mg (0.4mmol) 1,1-diethoxyl-2-hexene as starting material to obtain 25 mg of the title compound in 69% yield as off-white powder, m.p. 148-150°C.
$^1$H-NMR (300MHz, DMSO-$d_6$, ppm): 6.633 (d, 1H, J=10.2Hz, 8-H), 6.101 (s, 1H, 3-H), 5.840 (d, 1H, J=10.2Hz, 7-H), 5.124 (m, 1H, 6-H), 4.613 (m, 1H, 10-He), 4.258 (m, 1H, 10-Ha), 2.857 (m, 3H, 11-CH, 4-$C\underline{H}_2CH_2CH_3$), 1.765 (m, 2H, 6-$C\underline{H}_2CH_2CH_3$), 1.535 (m, 4H, $CH_2$), 1.059 (d, 3H, J=6.9Hz, 11-$CH_3$), 0.933 (m, 6H, $CH_3$);
ESI-MS (*m*/*z*): 369.1 $[M+H]^+$ (MW=368.43);
Elemental analysis: Calculated for $C_{22}H_{24}O_5 \cdot \frac{4}{3} H_2O$ (%): C, 67.33; H, 6.85. Found (%): C, 67.42; H, 6.58.

Example 52

10-methyl-4-n-propyl-6-ethyl-2H,6H,12H-benzo[1,2-b:3,4-b':5,6-b"]-tripyranyl-2,12-di one (**4-52**, $R_1$=n-$C_3H_7$, $R_3$=$CH_3$, $R_5$=$C_2H_5$, $R_2$=$R_4$=$R_6$=H)

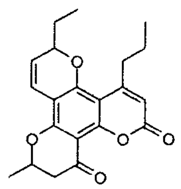

Using the same procedure as described in the preparative method of compound 4-1, except for using 28 mg (0.1mmol) benzo[1,2-b:3,4-b']-dipyranyl-5-hydroxyl-8-methyl-4-n-propyl-2,10-dione (5a-11) and 63mg (0.4mmol) 1,1-diethoxyl-2-pentene as starting material to obtain 24 mg of the title compound in 68% yield as off-white powder, m.p. 139-141°C.

$^1$H-NMR (300MHz, DMSO-$d_6$, ppm): 6.669 (dt, 1H, J=2.4Hz, 9.9Hz, 8-H), 6.098 (s, 1H, 3-H), 5.821 (dt, 1H, J=3.3Hz, 9.9Hz, 7-H), 5.478 (m, 1H, 6-H), 4.695 (m, 1H, 10-H), 2.959 (m, 2H, $CH_2$), 2.661 (m, 2H, 11-$CH_2$), 1.823 (m, 2H, $CH_2$), 1.562 (m, 2H, $CH_2$), 1.433 (d, 3H, J=6.3Hz, 10-$CH_3$), 0.973 (t, 3H, J=7.2Hz, $CH_3$), 0.925 (t, 3H, J=7.2Hz, $CH_3$);

ESI-MS (*m*/*z*): 355.1 $[M+H]^+$ (MW=354.41);

Elemental analysis: Calculated for $C_{21}H_{22}O_5 \cdot \frac{1}{2} H_2O$ (%): C, 69.41; H, 6.38. found (%): C, 69.27; H, 6.81.

Example 53

10-methyl-4,6-diethyl-2H,6H,12H-benzo[1,2-b:3,4-b':5,6-b"]-tripyranyl-2,12-dione (**4-53**, $R_1$=$R_5$=$C_2H_5$, $R_3$=$CH_3$, $R_2$=$R_4$=$R_6$=H)

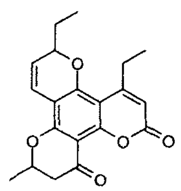

Using the same procedure as described in the preparative method of compound 4-1, except for using 27 mg (0.1mmol) benzo[1,2-b:3,4-b']-dipyranyl-5-hydroxyl-8-methyl-4-ethyl-2,10-dione (5a-9) and 63mg (0.4mmol) 1,1-diethoxyl-2-pentene as starting material to obtain 22 mg of the title compound in 64% yield as off-white powder, m.p. 129-131°C.

$^1$H-NMR (300MHz, DMSO-$d_6$, ppm): 6.664 (d, 1H, J=9.9Hz, 8-H), 6.107 (s, 1H, 3-H), 5.819 (m, 1H, 7-H), 5.480 (m, 1H, 6-H), 4.780 (m, 1H, 10-H), 2.932 (m, 2H, $CH_2$), 2.663 (m, 2H, 11-$CH_2$), 1.771 (m, 2H, $CH_2$), 1.434 (d, 3H, J=6.3Hz, 10-$CH_3$), 1.151 (m, 6H, $CH_3$);

ESI-MS (*m*/*z*): 341.1 $[M+H]^+$ (MW=340.38);

Elemental analysis: Calculated for $C_{20}H_{20}O_5 \cdot \frac{5}{12} H_2O$ (%): C, 69.05; H, 6.04. Found (%): C, 69.16; H, 6.42.

Example 54

10-methyl-4-n-propyl-6-ethyl-3-chloro-2H,6H,12H-benzo[1,2-b:3,4-b':5,6-b"]-tripyran yl-2,12-dione (**4-54**, $R_1$=n-$C_3H_7$, $R_2$=Cl, $R_3$=$CH_3$, $R_5$=$C_2H_5$, $R_4$=$R_6$=H)

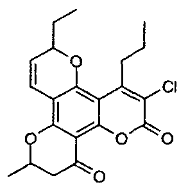

Using the same procedure as described in the preparative method of compound 4-1, except for using 32 mg (0.1mmol) benzo[1,2-b:3,4-b']-dipyranyl-5-hydroxyl-8-methyl-4-n-propyl-3-chloro-2,10-dione (5a-20) and 63mg (0.4mmol) 1,1-diethoxyl-2-pentene as starting material to obtain 27 mg of the title compound in 71% yield as off-white powder, m.p.145-147°C.

$^1$H-NMR (300MHz, DMSO-$d_6$, ppm): 6.670 (d, 1H, J=10.2Hz, 8-H), 5.853 (m, 1H; 7-H), 5.128 (m, 1H, 6-H), 4.724 (m, 1H, 10-H), 3.097 (t, 2H, J=7.8Hz, $CH_2$), 2.715 (m, 2H, 11-$CH_2$), 1.818 (m, 2H, $CH_2$), 1.545 (m, 2H, $CH_2$), 1.446 (d, 3H, J=6.3Hz, 10-$CH_3$), 0.987 (t, 3H, J=7.2Hz, $CH_3$), 0.937 (t, 3H, J=7.5Hz, $CH_3$);

ESI-MS (*m*/*z*): 389.1 $M^+$ (MW=388.85);

Elemental analysis: Calculated for $C_{21}H_{21}ClO_5 \cdot \frac{1}{4} H_2O$ , calculated (%): C, 64.12; H, 5.51. Found (%): C, 64.11; H, 5.43.

Example 55

10-methyl-4,6-diethyl-3-fluoro-2H,6H,12H-benzo[1,2-b:3,4-b':5,6-b"]-tripyranyl-2,12-dione (**4-55**, $R_1$=$R_5$=$C_2H_5$, $R_2$=F, $R_3$=$CH_3$, $R_4$=$R_6$=H)

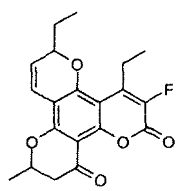

Using the same procedure as described in the preparative method of compound 4-1, except for using 29 mg (0.1mmol) benzo[1,2-b:3,4-b']-dipyranyl-5-hydroxyl-8-methyl-4-ethyl-3-fluoro-2,10-dione (5a-10) and 63mg (0.4mmol) 1,1-diethoxyl-2-pentene as starting material to obtain 25 mg of the title compound in 70% yield as off-white powder, m.p. 144-146°C.

$^1$H-NMR (300MHz, DMSO-$d_6$, ppm): 6.662 (dt, 1H, J=2.4Hz, 10.2Hz, 8-H), 5.840 (dt, 1H, J=2.7Hz, 10.2Hz, 7-H), 5.114 (m, 1H, 6-H), 4.712 (m, 1H, 10-H), 2.965 (m, 2H, $CH_2$), 2.669 (m, 2H, 11-$CH_2$), 1.817 (m, 2H, $CH_2$), 1.441 (d, 3H, J=6.0Hz, 10-$CH_3$), 1.193 (t, 3H, J=7.2Hz, $CH_3$), 1.138 (t, 3H, J=7.2Hz, $CH_3$);

ESI-MS (*m*/*z*): 359.1 $[M+H]^+$ (MW=358.37);

Elemental analysis: Calculated for $C_{20}H_{19}FO_5 \cdot \frac{1}{3} H_2O$ (%): C, 65.93; H, 5.44. Found (%): C, 65.88; H, 5.60.

Example 56

10-methyl-4-n-propyl-6-ethyl-3-fluoro-2H,6H,12H-benzo[1,2-b:3,4-b':5,6-b"]-tripyrany l-2,12-dione (**4-56**, $R_1$=n-$C_3H_7$, $R_2$=F, $R_3$=$CH_3$, $R_5$=$C_2H_5$, $R_4$=$R_6$=H)

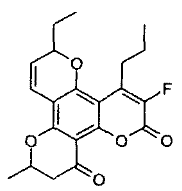

Using the same procedure as described in the preparative method of compound 4-1, except for using 31 mg (0.1mmol) benzo[1,2-b:3,4-b']-dipyranyl-5-hydroxyl-8-methyl-4-n-propyl-3-fluoro-2,10-dione (5a-31) and 63mg (0.4mmol) 1,1-diethoxyl-2-pentene as starting material to obtain 24 mg of the title compound in 65% yield as off-white powder, m.p.147-149°C.
$^1$H-NMR (300MHz, DMSO-$d_6$, ppm): 6.675 (dt, 1H, J=1.BHz, 10.2Hz, 8-H), 5.850 (dt, 1H, J=3.3Hz, 1.0.2Hz, 7-H), 5.122 (m, 1H, 6-H), 4.706 (m, 1H, 1.0-H), 2.922 (m, 2H, $CH_2$), 2.658 (m, 2H, 11-$CH_2$), 1.831 (m, 2H, $CH_2$), 1.577 (m, 2H, $CH_2$), 1.441 (d, 3H, J=6.0Hz, 10-$CH_3$), 0.989 (t, 3H, J=7.2Hz, $CH_3$), 0.926 (t, 3H, J=7.2Hz. $CH_3$);
ESI-MS (*m*/*z*): 373.1 $[M+H]^+$ (MW=372.40);
Elemental: $C_{21}H_{21}FO_5$, calculated (%): C. 67.73; H, 5.68. Found (%): C, 67.54; H, 5.72.

Example 57 of the invention

6,6,10-trimethyl-4-n-propyl-10-bromomethyl-2H,6H,12H-benzo[1,2-b:3,4-b':5,6-b"]-tri pyranyl-2,12-dione (**4-57**, $R_1$=n-$C_3H_7$, $R_3$=$BrCH_2$, $R_5$=$R_6$=$CH_3$, $R_2$=$R_4$=H)

(1) benzo[1,2-b:3,4-b']-dipyranyl-5-hydroxyl-8-bromomethyl-4-n-propyl-2,10-dione (**5a-57**, $R_1$=n-$C_3H_7$, $R_2$=H, $R_3$=$BrCH_2$, $R_4$=H) and benzo[1,2-b:5,4-b']-dipyranyl-5-hydroxyl-8-bromomethyl-4-n-propyl-2,6-dione (5b-57, $R_1$=n-$C_3H_7$, $R_2$=H, $R_3$=$BrCH_2$, $R_4$=H)

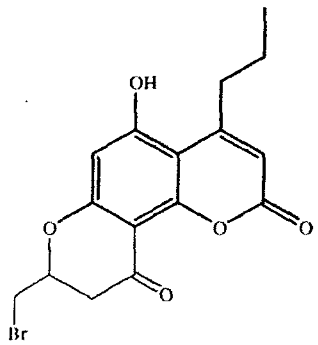

**5a-57**

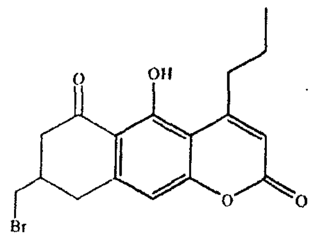

**5b-57**

Using the same procedure as described in the preparative method of compound 5a-1, except for using 0.44g (2mmol) 4-n-propyl-5,7-dihydroxyl-coumarin (6-11) and 0.445g (3mmol) 4-bromo-crotonic acid as starting material to obtain 0.157g of the title compound 5a-57 in 43% yield as a yellowish powder m.p.201-203°C, and 0.136g of the title compound 5b-57 in 37% yield as a white powder m.p.228-229°C.

Compound 5a-57: $^1$H-NMR (300MHz, DMSO-$d_6$, ppm): 11.892 (s, 1H, OH); 6.337 (s, 1H, 6-H), 6.062 (s, 1H, 3-H), 4.787 (m, 1H, 8-H), 3.893 (dd, 1H, J=3.3Hz, 11.4Hz, $BrCH_2$), 3.795 (dd, 1H, J=6.3Hz, 11.4Hz, $BrCH_2$), 2.881 (t, 2H, J=6.3Hz, 4-$C\underline{H}_2CH_2CH_3$), 2.823 (dd, 1H, 12.3Hz, 16.5Hz, 9-CH), 2.629 (dd, 1H, J=3.3Hz, 16.5Hz, 9-CH). 1.562 (sex, 2H, J=7.2Hz, 7.5Hz, 4-$CH_2C\underline{H}_2CH_3$), 0.931 (t, 3H, J=7.2Hz, 4-$CH_2CH_2C\underline{H}_3$);
ESI-MS (*m*/*z*): 366.8/368.8 $M^+$ (MW=367.20);

Anal. Calcd. for $C_{16}H_{15}BrO_5$: C, 52.34; H, 4.12. Found: C, 52.23; H, 4.04.

Compound 5b-57: $^1$H-NMR (300MHz, DMSO-$d_6$, ppm): 13.806 (s, 1H, 5-OH), 6.527 (s, 1H, 10-H), 6.081 (s, 1H, 3-H), 4.943 (m, 1H, 8-H), 3.949 (dd, 1H, J=3.3Hz, 11.4Hz, $BrCH_2$), 3.851 (dd, 1H, J=5.4Hz, 11.4Hz, $BrCH_2$), 3.148 (dd, 1H, J=12.3Hz, 17.1Hz, 7-CH), 2.890 (m, 2H, 4-$C\underline{H}_2CH_2CH_3$), 2.856 (dd, 1H; J=3.3Hz, 17.1Hz, 7-CH), 1.594 (sex, 2H, J=7.2Hz, 7.5Hz, 4-$CH_2C\underline{H}_2CH_3$), 0.953 (t, 3H, J=7.2Hz, 4-$CH_2CH_2C\underline{H}_3$);
ESI-MS (*m*/*z*): 366.8/368.8 $M^+$ (MW=367.20);
HRESIMS obsd. m/z : 367.02119, calcd. for $C_{16}H_{16}BrO_5^{+}$: 367.01811.

(2) 6,6,10-trimethyl-4-n-propyl-10-bromomethyl-2H,6H,12H-benzo[1,2-b:3,4-b':5,6-b"]-tripyran yl-2,12-dione (**4-57**, $R_1$=n-$C_3H_7$, $R_3$=$BrCH_2$, $R_5$=$R_6$=$CH_3$, $R_2$=$R_4$=H)

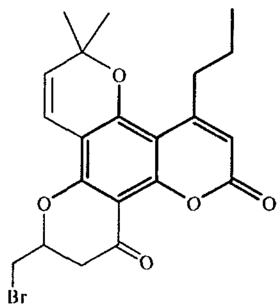

To a solution of 0.37 g (1mmol) compound 5a-57 in microwave reaction tube, 2ml toluene solvent and 1 ml acetal (13) and two drops of pyridine was added. The reaction solution was irradiated with microwave at 150 watt , 140 °C for 20 min. Then purification by silica-gel column chromatography, the title compound 4-57 was obtainede in 87% yield as off-white powder, m. p. 136-138□; $^1$H-NMR (300MHz, DMSO-*d6*, ppm): 6.617 (d, 1H, J=9.9Hz, 8-H), 6.126 (s, 1H, 3-H), 5.836 (d, 1H, J=9.9Hz, 7-H), 4.840 (m, 1H, 10-H), 3.940 (dd, 1H, J=3.3Hz, 11.4Hz, BrCH2), 3.838 (dd, 1H, J=6.6Hz, 11.4Hz, BrCH2), 2.858 (t, 2H, J=7.2Hz, 4-CH2CH2CH3), 2.825 (m, 1H, 11-CH2), 2.687 (dd, 1H, J=3.0Hz, 16.2Hz, 11-CH2), 1.575 (m, 2H, 4-CH2CH2CH3), 1.512, 1.469 (2s, 6H, 6-CH3), 0.969 (t, 3H, J=7.2Hz, 4-CH2CH2CH3);
ESI-MS (m/z): 433.1/435.1 M+ (MW=433.30);
HRESIMS obsd. m/z 433.0627, calcd. for C21H22BrO5+433.0650.

<u>Example 58</u>

6,6,10-trimethyl-4-n-propyl-10-azidomethyl-2H,6H,12H-benzo[1,2-b:3,4-b':5,6-b"]-tri pyranyl-2,12-dione (4-58,$R_1$=n-$C_3H_7$, $R_3$= $N_3CH_2$, $R_5$=$R_6$=$CH_3$, $R_2$=$R_4$=H)

(1) benzo[1,2-b:3,4-b']-dipyranyl-5-hydroxyl-8-azidomethyl-4-n-propyl-2,10-dione (5a-58, $R_1$=n-$C_3H_7$, $R_2$=H, $R_3$=$N_3CH_2$, $R_4$=H)

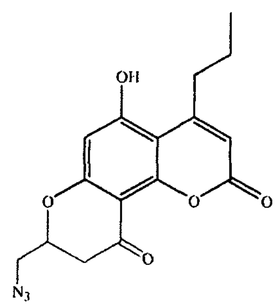

Compound 5a-57 (0.37 g, 1 mmol) was dissolved in 6 ml DMF, then 0.13 g (2 mmol) $NaN_3$ was added. The resulting reaction mixture was heated to 70 □ and stirred for 1 hour and then concentrated to give viscose oil which was purified by silica gel column chromatography to afford 0.28g of the title compound in 85% yield as white powder, m. p.

208-209.5°C.
$^1H$ NMR (300M Hz, DMSO-$d_6$, ppm):11.867 (br, 1H, OH), 6.322 (S, 1H, Ph-H), 6.051 (s, 1H, 3-H), 4.768 (m, 1H, 10-H), 3.666 (d, 2H, J=8.4Hz, NCH2), 2.859 (m, 2H, 4-CH2CH2CH3), 2.792 (d, 1H, J=3Hz, 11-CH2), 2.596 (d, 1H, J=3Hz, 11-CH2), 1.560 (m, 2H, 4-CH2CH2CH3), 0.928 (t, 3H, J=7.5Hz, 4-CH2CH2CH3);
ESI-MS (m/z): 330.01 [M+H$^+$] (MW=329.31).

(2) 6,6,10-trimethyl-4-n-propyl-10-azidomethyl-2H,6H,12H-benzo[1,2-b:3,4-b':5,6-b"]-tripyran yl-2,12-dione (4-58, $R_1$=n-$C_3H_7$, $R_3$= $N_3CH_2$, $R_5$=$R_6$=$CH_3$, $R_2$=$R_4$=H)

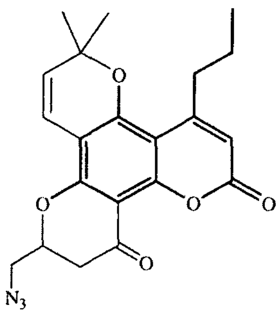

Using the same procedure as described in the preparative method of compound 4-57, except for using 165 mg (0.5mmol) Compound 5a-58 and 1 ml acetal as starting material to obtain 150mg of the title compound in 68% yield as yellowish powder, m.p.116-117°C ; $^1H$ NMR (300M Hz, DMSO-$d_6$, ppm):6.608 (d, 1H, J=10Hz, 8-H), 6.133 (s, 1H, 3-H), 5.840 (d, 1H, J=10Hz, 7-H), 4.629 (m, 1H, 10-H), 3.716 (m, 2H, NCH2), 2.859 (m, 2H, 4-<u>CH2</u>CH2CH3), 2.822 (1H, 11-CH2), 2.628 (d, 1H, J=3Hz, 11-CH2), 1.580 (m, 2H, 4-CH2CH2CH3), 1.510, 1.471 (2s, 6H, 6-CH3), 0.973 (t, 3H, J=7.5Hz, 4-CH2CH2CH3);
ESI-MS (m/z): 396.14 [M+H$^+$] (MW=395.41).

Example 59

6,6,10-trimethyl-4-n-propyl-10-aminomethyl-2H,6H,12H-benzo[1,2-b:3,4-b':5,6-b"]-tr ipyranyl-2,12-dione (4-59, $R_1$=n-$C_3H_7$, $R_3$= $NH_2CH_2$, $R_5$=$R_6$=$CH_3$, $R_2$=$R_4$=H)

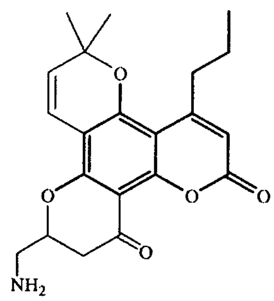

0.2 g compound 4-58 (0.5 mmol) was dissolved in 20 ml THF and then 0.45 g $SnCl_2 \cdot 2H_2O$ (2 mmol) and 1 ml concentrated hydrochloric acid were added. The resulting mixture was refluxed with stirring for 3 hours and then 10 ml water was added to quench the reaction, then concentrated ammonia water was added dropwise to adjust the PH of reaction solution to 8-9. The pale white solid was filtrated off and the filtrate was extracted 3 times with ethyl acetate. The combined organic phase was dried and concentrated to give brown oil which was purified by anti-phase C-18 column chromatography to afford 0.11g of the title compound 4-59 in 59.5% yield as yellowish powder, m. p. > 220°C , decomposition.
$^1H$ NMR (500M Hz, DMSO-$d_6$, ppm): 6.841 (d, 1H, J=10Hz, 8-H), 6.535 (br, 1H, NH), 6.131 (s, 1H, 3-H), 5.806 (d, 1H, J=10Hz, 7-H), 4.634 (m, 1H, 10-H), 3.086 (m, 2H, NCH2), 2.854 (m, 2H, 4-<u>CH2</u>CH2CH3), 2.820 (1H, 11-CH2,), 2.618 (d, 1H, J=6Hz, 11-CH2), 1.561 (m, 2H, 4-CH2CH2CH3), 1.514, 1.478 (2s, 6H, 6-CH3), 0.974 (t, 3H, J=7.5Hz, 4-CH2CH2CH3);
ESI-MS (m/z): 370.26 [M+H$^+$] (MW=369.41).

Example 60

6,6-dimethyl-4-n-propyl-10-(p-fluorophenylureido)methylene-2H,6H,12H-benzo[1,2-b :3,4-b':5,6-b"]-tripyranyl-2,12-dione (4-60,$R_1$=n-$C_3H_7$, $R_5$=$R_6$=$CH_3$, $R_2$=$R_4$=H)

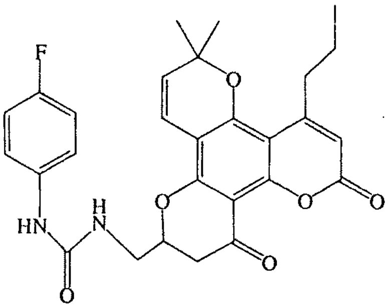

18.5 mg compound 4-59 (0.05 mmol) was dissolved in 10 ml THF and then 14 mg (0.1 mmol) 4-fluorophenyl isocyanate was added and refluxed with stirring for 3 hours. Then the reaction mixture was purified by silica gel column chromatography to afford 11.8mg of the title compound 4-60 in 60.8% yield as white powder, m. p. 119-120.5°C ; $^1$H-NMR (300MHz, DMSO-$d_6$, ppm): 8.623 (s, 1H, NH), 7.392(pent, 2H, Ar-H, J=4.8Hz, J=2.1Hz), 7.056 (t, 2H, J=9Hz, Ar-H), 6.696 (d, 1H, J=10.2Hz, 8-H), 6.468(t, 1H, J=5.7Hz, NH), 6.119 (s, 1H, 3-H), 5.794 (d, 1H, J=10.2Hz, 7-H), 4.643 (m, 1H, 10-H), 3.506 (m, 2H, NCH2), 2.856 (m, 2H, 4-CH2CH2CH3), 2.760 (dd, 1H, J=14.4Hz, J=3.6Hz, 11-CH2), 2.580 (dd, 1H, J=14.4Hz, J=3Hz, 11-CH2), 1.564 (m, 2H, 4-CH2CH2CH3), 1.504, 1.467 (2s, 6H, 6-CH3), 0.970 (t, 3H, J=7.2Hz, 4-CH2CH2CH3); ESI-MS (m/z): 507.18 [M+H$^+$] (MW=506.52);
HRESIMS obsd. m/z 507.1925, calcd. for $C_{28}H_{28}N_2O_6F^+$ 507.1920.

Example 61

6,6-dimethyl-4-n-propyl-10-(p-acetylaminophenylsulfonamido)methylene-2H,6H,12H-b enzo[1,2-b:3,4-b':5,6-b"]-tripyranyl-2,12-dione (4-61, $R_1$=n-$C_3H_7$, $R_5$=$R_6$=$CH_3$, $R_2$=$R_4$=H)

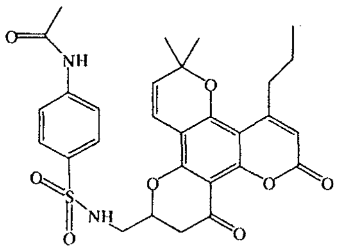

Using the same procedure as described in the preparative method of compound 4-60, except for using compound 4-59 (18.5 mg, 0.05 mmol), 0.1 mmol 4-acetylaminophenylsulfonyl chloride and 0.1 mmol pyridine as starting material to obtain the title compound in 61% yield as white powder, m. p. 127-128.5°C ; $^1$H NMR (600M Hz, DMSO-$d_6$, ppm): 10.343 (s, 1H, NH), 8.01 (s, 1H, NH), 7.95 (s, 2H, Ph-H), 7.93 (s, 2H, Ph-H), 6.661 (d, 1H, J=10Hz, 8-H), 6.105 (s, 1H, 3-H), 5.796 (d, 1H, J=10Hz, 7-H), 4.541 (m, 1H, 10-H), 3.226 (d, 1H, J=14.4Hz, NCH), 3.157 (m, 1H, NCH), 2.848 (m, 2H, J=6Hz, 4-CH2CH2CH3), 2.752 (d, 1H, J=3Hz, 11-CH2), 2.543 (d, 1H, J=3Hz, 11-CH2), 2.066 (s, 3H, CH3), 1.560 (m, 2H, 4-CH2CH2CH3), 1.500, 1.464 (2s, 6H, 6-CH3), 0.969 (t, 3H, J=7.2Hz, 4-CH2CH2CH3);
ESI-MS (m/z): 567.74 [M+H$^+$] (MW=566.62).

Example 62

6,6-dimethyl-4-n-propyl-10-(p-fluorophenylsulfonamido)methylene-2H,6H,12H-benzo[ 1,2-b:3,4-b':5,6-b"]-tripyranyl-2,12-dione (4-62,$R_1$=n-$C_3H_7$, $R_5$=$R_6$=$CH_3$, $R_2$=$R_4$=H)

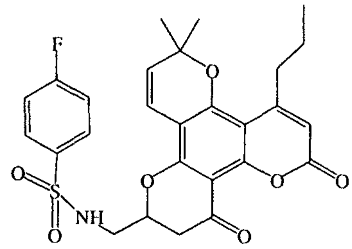

Using the same procedure as described in the preparative method of compound 4-60, except for using 18.5 mg (0.05mmol) compound 4-59 and 0.1mmol 4-fluorophenylsulfonyl chloride as starting material to obtain the title compound in 46.6% yield as white powder, m. p. 129-131°C ; $^1$H-NMR (300MHz, DMSO-$d_6$, ppm): 8.191 (t, 1H, NH), 7.904(dd, 2H, J=8.7Hz, J=5.1Hz, Ar-H), 7.427 (t, 2H, J=9Hz, Ar-H), 6.657 (d, 1H, J=10.2Hz, 8-H), 6.118 (s, 1H, 3-H), 5.792 (d, 1H, J=9.9Hz, 7-H), 4.576 (m, 1H, 10-H), 3.179 (m, 2H, NCH2), 2.840 (t, 2H, 4-CH2CH2CH3), 2.742 (d, 1H, J=14.5Hz, 11-CH2), 2.564 (1H, 11-CH2), 1.562 (m, 2H, 4-CH2CH2CH3), 1.503, 1.469 (2s, 6H, 6-CH3), 0.969 (t, 3H, J=7.2Hz, 4-CH2CH2CH3);
ESI-MS (m/z): 528.28[M+H$^+$] (MW=527.56);
HRESIMS obsd. m/z 528.1490, calcd. for $C_{27}H_{27}NO_7FS^+$ 528.1492.

Example 63

6,6-dimethyl-4-n-propyl-10-(p-methoxylphenylsulfonamido)methylene-2H,6H,12H-ben zo[1,2-b:3,4-b':5,6-b"]-tripyranyl-2,12-dione (**4-63**, $R_1$=n-$C_3H_7$, $R_5$=$R_6$=$CH_3$, $R_2$=$R_4$=H)

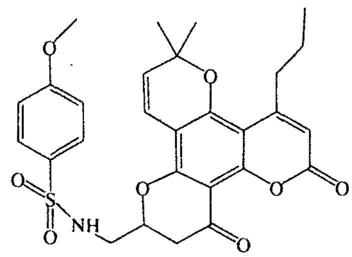

Using the same procedure as described in the preparative method of compound 4-60, except for using 18.5 mg (0.05mmol) compound 4-59 and 0.1mmol 4-methoxylphenylsulfonyl chloride as starting material to obtain the title compound in 54.8% yield as off-white powder, m. p. 108-109°C ; $^1$H-NMR (300MHz, DMSO-$d_6$, ppm): 7.981 (t, 1H, J=6.3Hz, NH), 7.747(dd, 2H, J=9Hz, Ph-H), 7.082 (dd, 2H, J=9Hz, Ph-H), 6.672 (d, 1H, J=10Hz, 8-H), 6.116 (s, 1H, 3-H), 5.774 (d, 1H, J=10Hz, 7-H), 4.559 (m, 1H, 10-H), 3.814 (s, 3H, $OCH_3$), 3.142 (m, 2H, NCH2), 2.860 (t, 2H, J=7.2Hz, 4-CH2CH2CH3), 2.758 (d, 1H, J=3.9Hz, 11-CH2), 2.562 (d, 1H, J=3.3Hz, 11-CH2), 1.551 (m, 2H, 4-CH2CH2CH3), 1.505, 1.470 (2s, 6H, 6-CH3), 0.971 (t, 3H, J=7.2Hz, 4-CH2CH2CH3);
ESI-MS (m/z): 540.23 [M+H$^+$] (MW=539.6);
HRESIMS obsd m/z 540.1705, calcd for $C_{28}H_{30}NO_4S^+$ 540.1692

Example 64

6,6-dimethyl-4-n-propyl-10-(o-methoxyl-phenylthioureido)methylene-2H,6H,12H-benz o[1,2-b:3,4-b':5,6-b"]-tripyranyl-2,12-dione (**4-64**, $R_1$=n-$C_3H_7$, $R_5$=$R_6$=$CH_3$, $R_2$=$R_4$=H)

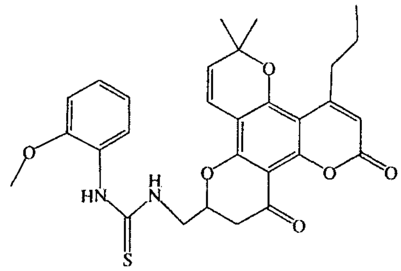

Using the same procedure as described in the preparative method of compound 4-60, except for using 18.5 mg (0.05mmol) compound 4-59 and 0.1mmol 2-methoxylphenylisocyanate as starting material to obtain the title compound in 55.8% yield as white powder, m. p. 193-194°C ; $^1$H-NMR (300MHz, DMSO-$d_6$, ppm): 9.148 (s, 1H, NH), 8.076 (s, 1H, NH), 7.758(s, 1H, Ar-H), 7.153(t, 1H, J=8Hz, Ar-H), 7.045 (d, 1H, J=8.5Hz, Ar-H), 6.910(t, 1H, J=7.5Hz, Ar-H), 6.687 (d, 1H, J=10Hz, 8-H), 6.124 (s, 1H, 3-H), 5.819 (d, 1H, J=10Hz, 7-H), 4.799 (m, 1H, 10-H), 3.952 (m, 2H, NCH2), 3.785 (s, 3H, $OCH_3$), 2.857 (t, 2H, J=6Hz, 4-CH2CH2CH3), 2.815 (dd, 1H, J=14.4Hz, J=4Hz, 11-CH2), 2.659 (dd, 1H, J=14.4Hz, J=4Hz, 11-CH2), 1.747 (m, 2H, 4-CH2CH2CH3), 1.514, 1.477 (2s, 6H, 6-CH3), 0.974 (t, 3H, J=7.5Hz, 4-CH2CH2CH3);
ESI-MS (m/z): 535.41 [M+H$^+$] (MW=534.62);
HRESIMS obsd. m/z 535.1906, calcd. for $C_{29}H_{31}N_2O_6S^+$ 535.1897.

Example 65

6,6-dimethyl-4-n-propyl-10-tetrahydropyrrolylmethylene-2H,6H,12H-benzo[1,2-b:3,4-b ':5,6-b"]-tripyranyl-2,12-dione (**4-65**, $R_1$=n-$C_3H_7$, $R_5$=$R_6$=$CH_3$, $R_2$=$R_4$=H)

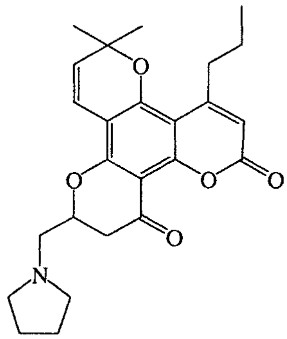

2 equivlent cyclopentylamine was added into the solution of compound **4-57** (0.02mmol) in THF and the mixture was stirred overnight at room temperature. After concentration and purification by silica gel column chromatography with petroleum ether/ethyl acetate as the eluent, 6.1 mg comopound **4-65** was obtained as brown-yellow powder in 72% yield, m. p. 148-149°C. $^1$H-NMR (500MHz, DMSO-$d_6$, ppm): 6.442 (dd, 1H, J=2.0Hz, 10.0Hz, 8-H), 6.172 (s, 1H, 3-H), 5.579 (dd, 1H, J=2.5Hz, 10.0Hz, 7-H), 4.776 (m, 1H, 10-H), 3.457 (m, 2H, $CH_2$), 3.114 (m, 2H, 4-$C\underline{H}_2CH_2CH_3$), 2.355 (m, 4H, $CH_2$), 2.221 (m, 2H, 11-$CH_2$), 1.846 (m, 4H, $CH_2$), 1.723 (m, 2H, 4-$CH_2C\underline{H}_2CH_3$), 1.263, 1.250 (2s, 6H, 6-$CH_3$), 0.873 (t, 3H, J=7.0Hz, 4-$CH_2CH_2C\underline{H}_3$);
ESI-MS (*m*/*z*): 424.1 [M+H]$^+$ (MW=423.51) ;
HRESIMS obsd. m/z 424.2125, calcd. for $C_{25}H_{30}NO_5^+$ 424.21239.

Example 66

6,6-dimethyl-4-n-propyl-10-piperidinylmethylene-2H,6H,12H-benzo[1,2-b:3,4-b':5,6-b"]-tripyranyl-2,12-dione (**4-66**, $R_1$=n-$C_3H_7$, $R_5$=$R_6$=$CH_3$, $R_2$=$R_4$=H)

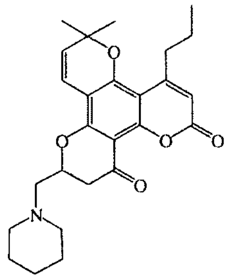

Using the same procedure as described in the preparative method of compound 4-65, except for using 4-57 and 2 equivlent cyclohexylamine as starting material to obtain 6mg of the title compound 4-66 in 69% yield as off-white powder, m. p. 154-155°C. $^1$H-NMR (500MHz, DMSO-$d_6$, ppm): 6.448 (d, 1H, J=10.0Hz, 8-H), 6.149 (d, 1H, J=9.5Hz, 3-H), 5.696 (d, 1H, J=10.0Hz, 7-H), 4.786 (m, 1H, 10-H), 3.418 (m, 2H, $CH_2$), 3.067 (m, 2H, 4-$C\underline{H}_2CH_2CH_3$), 2.286 (m, 2H, 11-$CH_2$), 2.083 (m, 4H, 2$CH_2$), 1.471 (m, 2H, 4-$CH_2C\underline{H}_2CH_3$), 1.344, 1.310 (2s, 6H, 6-$CH_3$), 1.296 (m, 6H, 3$CH_2$), 0.864 (t, 3H, J=7.5Hz, 4-$CH_2CH_2C\underline{H}_3$);
ESI-MS (*m/z*): 438.1 $[M+H]^+$ (MW=437.54) ;
HRESIMS obsd. m/z 438.2282, calcd. for $C_{26}H_{32}NO_5^+$ 438.22804.

Example 67

5-hydroxy-6-(1'-isopropyl-1'H-pyrrolyl-2'-ene)-2,2-dimethyl-10-n-propyl-2H-benzo[ 2,3-f]-pyranyl-8-one (**4-67**,$R_1$=n-$C_3H_7$, $R_5$=$R_6$=$CH_3$, $R_2$=H , Y= i-$C_3H_7$)

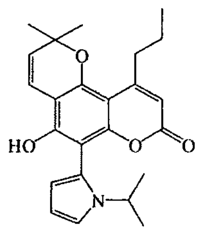

Using the same procedure as described in the preparative method of compound 4-65, except for using 4-57 and 2 equivlent isopropylamine as starting material to obtain 4.4mg of the title compound 4-67 in 56% yield as yellow powder, m. p. 161-162°C. $^1$H-NMR (500MHz, DMSO-$d_6$, ppm): 7.014 (t, 1H, J=3.0Hz, 1.5Hz, 5-$H_{pyrrole}$), 6.659 (d, 1H, J=10.0Hz, 8-H), 6.156 (t, 1H, J=3.0Hz, 3.5Hz, 4-$H_{pyrrole}$), 5.920 (s, 1H, 3-H), 5.884 (dd, 1H, J=2.0Hz, 3.5Hz, 3-$H_{pyrrole}$), 5.703 (d, 1H, J=10.0Hz, 7-H), 3.784 (sept, 1H, J=7.0Hz, CH), 2.869 (m, 2H, 4-$CH_2CH_2CH_3$), 1.605 (m, 2H, 4-$CH_2CH_2CH_3$), 1.477, 1.453 (2s, 6H, 6-$CH_3$), 1.278 (d, 3H, J=7.0Hz, $CH_3$), 1.228 (d, 3H, J=7.0Hz, $CH_3$), 0.988 (t, 3H; J=7.5Hz, 4-$CH_2CH_2C\underline{H}_3$); $^{13}$C-NMR (125MHz, DMSO-$d_6$, ppm): 159, 157, 155, 154, 151, 127, 120, 117, 116, 110, 109, 107, 105, 102, 102, 77, 47, 37, 27, 27, 23, 23, 23, 13.
ESI-MS (*m/z*): 394.2 $[M+H]^+$ (MW=393.49) ;
HRESIMS obsd. m/z 394.2021, calcd. for $C_{27}H_{28}NO_4^+$ 394.2018.

Example 68

5-hydroxy-6-(1'-(2-pyridyl)methylene-1'H-pyrrolyl-2'-ene)-2,2-dimethyl-10-n-propyl-2 H-benzo-[2,3-f]-pyranyl-8-one (**4-68**, $R_1$=n-$C_3H_7$, $R_5$=$R_6$=$CH_3$, $R_2$=$R_4$=H)

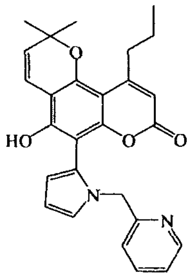

Using the same procedure as described in the preparative method of compound 4-65, except for using 4-57 and 2 equivlent 2-aminomethylpyridine as starting material to obtain 4.3mg of the title compound 4-68 in 49% yield as off-yellow powder, m. p. 171-173°C. $^1$H-NMR (300MHz, *DMSO-$d_6$*, ppm): 8.308 (m, 1H, Ar-H), 7.573 (dt, 1H, J=1.8Hz, 7.8Hz, Ar-H), 7.140 (m, 1H, Ar-H), 6.940 (t, 1H, J=2.4Hz, 5-$H_{pyrrole}$), 6.892 (m, 1H, Ar-H), 6.629 (d, 1H, J=9.9Hz, 8-H), 6.177 (t, 1H, J=3.0Hz, 4-$H_{pyrrole}$), 6.022 (dd, 1H, J=1.8Hz, 3.6Hz, 3-$H_{pyrrole}$), 5.832 (s, 1H, 3-H), 5.671 (d, 1H, J=9.9Hz, 7-H), 4.941 (s, 2H, $CH_2$), 2.806 (m, 2H, 4-$C\underline{H}_2CH_2CH_3$), 1.552 (m, 2H, 4-$CH_2CH_2CH_3$), 1.438 (s, 6H, 6-$CH_3$), 0.967 (t, 3H, J=7.2Hz, 4-$CH_2CH_2C\underline{H}_3$);
ESI-MS (m/z): 443.1 $[M+H]^+$ (MW=442.52) ;
HRESIMS obsd. m/z 443.1973, calcd. for $C_{27}H_{27}N_2O_4^+$ 443.19708.

Example 69

5-hydroxy-6-[1'-(2-furanyl)methylene-1'H-pyrrolyl-2'-ene]-2,2-dimethyl-10-n-propyl-2 H-laenzo[2,3-f]-pyranyl-8-one (**4-69**, $R_1$=n-$C_3H_7$, $R_5$=$R_6$=$CH_3$, $R_2$=$R_4$=H)

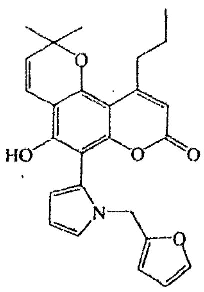

Using the same procedure as described in the preparative method of compound 4-65, except for using 0.02mmol compound 4-57 and 2 equivlent 2-aminomethylfuran as starting material to obtain 5.2mg of the title compound 4-69 in 61% yield as off-white powder, m. p. 157-159°C. $^1$H-NMR (500MHz, DMSO-$d_6$, ppm): 7.397 (d, 1H, J=1.0Hz, CH), 6.884 (dd, 1H, J=1.5Hz, 2.5Hz, CH), 6.673 (d, 1H, J=10.0Hz, 8-H), 6.219 (dd, 1H, J=1.5Hz, 3.5Hz, CH), 6.120 (t, 1H, J=3.5Hz, CH), 5.984 (dd, 1H, J=1.5Hz, 3.5Hz, CH), 5.945 (d, 1H, J=3.5Hz, CH), 5.900 (s, 1H, 3-H), 5.721 (d, 1H, J=10.0Hz, 7-H), 4.789 (q, 2H, J=16.0Hz, 18.5Hz, $CH_2$), 2.871 (m, 2H, 4-$C\underline{H}_2CH_2CH_3$), 1.614 (m, 2H, 4-$CH_2C\underline{H}_2CH_3$), 1.483, 1.464 (2s, 6H, 6-$CH_3$), 0.992 (t, 3H, J=7.5Hz, 4-$CH_2CH_2C\underline{H}_3$);
ESI-MS (*m/z*): 432.1 $[M+H]^+$ (MW=431.49) ;
HRESIMS obsd. m/z 432.1810, calcd. for $C_{26}H_{26}NO_5^+$ 432.18109.

Example 70 of the invention

6,6,-dimethyl-4-propyl-10,11-cis-cyclopropyl-2H,6H,12H-benzo[1,2-b:3,4-b':5,6-b"] tripyranyl-2,12-dione (**4-70**, $R_1$=n-$C_3H_7$, $R_5$=$R_6$=$CH_3$, $R_2$=$R_4$=$CH_2$)

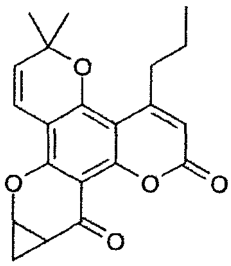

The title compound 4-70 can be synthesized in two methos:

Method 1:

(1) benzo[1,2-b:3,4-b']dipyranyl-5-hydroxyl-8,9-cis-cyclopropyl-4-n-propyl-2,10-dione (**5a-70**, $R_1$=n-$C_3H_7$, $R_2$H, $R_3$=$R_4$=$CH_2$)

20 mg (excess) NaH was added into the solution of 38 mg compound **5a-57** in 10 ml absolute anhydrous THF and the reaction mixture was stirred for 48 hours at room temperature. After filtration, the resulted yellowish powder was dissolved in 2 ml methonal and then acidified with 1M hydrochloric acid. Extracted 3 times with EtOAc, the combined organic phase was dried, filtrated and purified through chromatography on silica gel eluting with petroleum-EtOAC(2:1) to afford ompound **5a-70** in 69% yield as white powder. $^1$H NMR (300M Hz, DMSO-$d_6$, ppm): 11.708 (s, 1H, OH), 6.283 (S, 1H, Ph-H), 6.050 (s, 1H, 3-H), 4.731 (m, 1H, 8-H), 2.860 (t, 2H, J=7.8Hz, 4-CH2CH2CH3), 2.118 (m, 1H, 9-H), 1.615-1.443(m, 4H, 11-CH2, 4-CH2CH2CH3), 0.923 (t, 3H, J=7.5Hz, 4-CH2CH2CH3); ESI-MS (m/z): 287.24 $[M+H]^+$ (MW=286.28) ; HRESIMS obsd. m/z 287.0914, calcd. for $C_{16}H_{15}O_5^+$ 287.0914.

(2) 6,6,-dimethyl-4-n-propyl-10,11-cis-cyclopropyl-2H,6H,12H-benzo[1,2-b:3,4-b':5,6-b"]tripyr anyl-2,12-dione (**4-70**, $R_1$=n-$C_3H_7$, $R_5$=$R_6$=$CH_3$, $R_2$=$R_4$=$CH_2$)

Using the same procedure as described in the preparative method of compound 4-1, except for using 19 mg (0.05mmol) compound 5a-70 and 1,1-diethoxyl-3-methyl-2-butene as starting material to obtain 11mg of the title compound 4-70 in 48% yield as off-white powder, m. p. 113-115°C. $^1$H-NMR (300MHz, DMSO-$d_6$, ppm): 6.554 (d, 1H, J=9.9Hz, 8-H), 6.123 (s, 1H, 3-H), 5.800 (d, 1H, J=9.9Hz, 7-H), 4.862 (m, 1H, 10-H), 2.844 (t, 2H, J=7.5Hz, 4-$CH_2CH_2CH_3$), 2.181 (m, 1H, 11-CH), 1.568 (m, 4H, 13-$CH_2$, 4-$CH_2CH_2CH_3$), 1.490,1.456 (2s, 6H, 6-$CH_3$), 0.967 (t, 3H, J=7.5Hz, 4-$CH_2CH_2CH_3$); ESI-MS (*m/z*): 353.1 $[M+H]^+$ (MW=352.39) ; HRESIMS obsd. m/z 353.1392, calcd. for $C_{21}H_{21}O_5^+$ 353.1389.

Method 2:

Using the method for synthesizing compound 4-67(or 4-68, 4-69), compound 4-70 can be obtained simutaiously in 15-20% yield as yellow powder, m. p. 113-115°C. $^1$H-NMR (500MHz, DMSO-$d_6$, ppm): 6.557 (d, 1H, J=10.0Hz, 8-H), 6.125 (s, 1H, 3-H), 5.801 (d, 1H, J=10.0Hz, 7-H), 4.866 (m, 1H, 10-H), 2.847 (t, 2H, J=7.5Hz, 4-$CH_2CH_2CH_3$), 2.182 (dt, 1H, J=6.5Hz, 9.0Hz, 11-CH), 1.570 (m, 4H, 13-$CH_2$, 4-$CH_2CH_2CH_3$), 1.492,1.458 (2s, 6H, 6-$CH_3$), 0.969 (t, 3H, J=7.5Hz, 4-$CH_2CH_2CH_3$); ESI-MS (*m/z*): 353.1 $[M+H]^+$ (MW=352.39) ; HRESIMS obsd. m/z 353.1392, calcd. for $C_{21}H_{21}O_5^+$ 353.1389.

Example 71

6,6,10-trimethyl-4-(3,3',3"-trifluoromethyl)-2H,6H,12H-benzo[1,2-b:3,4-b':5,6-b" ]-trip yranyl-2,12-dione (**4-71**)

(1) 4-(3,3',3"-trifluoro)-n-propyl-5,7-dihydroxy-coumarin (**6-71**)

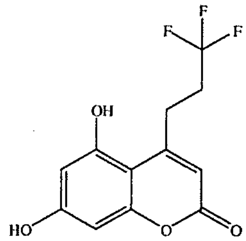

Using the same procedure as described in the preparative method of compound (6-1), except for using phloroglucinol and Ethyl 4-trifluorobutyryl acetate as starting material to obtain the title compound in 78% yield as a brown yellow powder. m. p. 205-207°C ; $^1$H-NMR (300MHz, DMSO-$d_6$, ppm): 10.821 (s, 1H, OH), 10.366 (s, 1H, OH), 6.277 (d, 1H, J=2.4Hz, 8-H), 6.193 (d, 1H, J=2.4Hz, 6-H), 5.970 (s, 1H, 3-H), 3.096 (t, 2H, J=7.8Hz, 4-C$\underline{H}_2$CH$_2$CF$_3$), 2.572 (m, 2H, 4-CH$_2$C$\underline{H}_2$CF$_3$);
$^{19}$F-NMR (400MHz, DMSO-$d_6$, ppm): -65.129 (t, J=11.2Hz);
ESI-MS (*m/z*): 273.5 [M-H]$^-$ (MW=274.20) ;
HRESIMS obsd. m/z 273.0400, calcd. for $C_{12}H_8F_3O_4^-$ 273.03746.

(2) benzo[1,2-b:3,4-b']-dipyranyl-5-hydroxyl-80methyl-4-((3,3',3"-trifluoro)-n-propyl)-2,10-dio ne(5a-71)

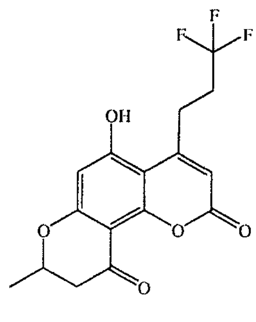

Using the same procedure as described in the preparative method of compound 5a-1, except for using compound 4-(γ-trifluoropropyl)-5,7-dihydroxy-coumarin (6-71) and crotonic acid as starting material to obtain of the title compound 5a-71 in 77% yield as a brown yellow powder, m. p. 127-129°C. $^1$H-NMR (300MHz, DMSO-*d*$_6$, ppm): 12.676 (s, 1H, OH, 6.269 (s, 1H, 6-H), 5.987 (s, 1H, 3-H), 4.631 (m, 1H, 8-H), 3.135 (t, 2H, J=7.5Hz, 4-C$\underline{H}_2$CH$_2$CF$_3$), 2.637 (m, 2H, 9-CH$_2$), 2.584 (m, 2H, 4-C$\underline{H}_2$CH$_2$CF$_3$), 1.385 (d, 3H, J=6.0Hz, 8-CH$_3$);
$^{19}$F-NMR (400MHz, DMSO-*d*$_6$, ppm): -64.971 (t, J=11.2Hz);
ESI-MS (m/z): 341.2 [M-H]$^-$ (MW=342.27);
HRESIMS obsd. m/z 341.0643, calcd. for $C_{16}H_{12}F_3O_5^-$ 341.06368.

(3) 6,6,10-trimethyl-4-(3,3',3"-trifluoromethyl)-2H,6H,12H-benzo[1,2-b:3,4-b':5,6-b"]-tripyran yl-2,12-dione (**4-71**, $R_1$=n-$C_2H_4CF_3$, $R_2$=H, $R_3$=$R_5$=$R_6$=$CH_3$, $R_4$=H)

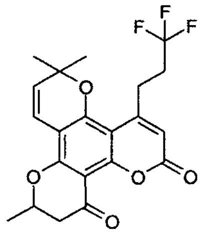

Using the same procedure as described in the preparative method of compound 4-1, except for using compound 5a-71 and 1,1-diethoxyl-3-methyl-2-butene as starting material to obtain the title compound in 72% yield as brown-yellow powder, m. p. 133-134°C. $^1$H-NMR (500MHz, DMSO-$d_6$, ppm): 6.602 (d, 1H, J=10.0Hz, 8-H), 6.266 (s, 1H, 3-H), 5.810 (d, 1H, J=10.0Hz, 7-H), 4.723 (m, 1H, 10-H), 3.164 (m, 2H, 4-C<u>H</u>$_2$CH$_2$CF$_3$), 2.661 (m, 2H, 11-CH$_2$), 2.568 (m, 2H, 4-CH$_2$C<u>H</u>$_2$CF$_3$), 1.497, 1.464 (2s, 6H, 6-CH$_3$), 1.449 (d, 3H, J=6.0Hz, 10-CH$_3$);
$^{19}$F-NMR (400MHz, DMSO-$d_6$, ppm): -64.764 (t, J=11.2Hz);
ESI-MS (m/z): 409.1 [M+H]$^+$ (MW=408.38) ;
HRESIMS obsd. m/z 409.1271, calcd. for $C_{21}H_{20}F_3O_5^+$ 409.12628.

Example 72

4-(3,3',3"-trifluoro-n-propyl)-6-propenyl-10-methyl-2H,6H,12H-benzo[ 1,2-b:3,4-b' :5,6 -b"]-tripyranyl-2,12-dione (**4-72** , $R_1$=n-$C_2H_4CF_3$, $R_2$=H, $R_3$=$CH_3$, $R_5$=$C_3H_5$, $R_4$=$R_6$=H)

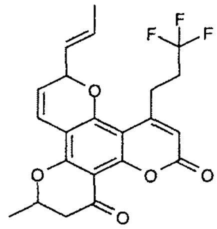

Using the same procedure as described in the preparative method of compound 4-1, except for using compound 5a-71 and 1,1-diethoxyl-2,4-hexadiene as starting material to obtain the title compound 4-72 in 56% yield as brown-yellow powder, m. p. 147-149°C.
$^1$H-NMR (400MHz, DMSO-$d_6$, ppm): 6.744 (d, 1H, J=12.3Hz, CH), 6.511 (m, 1H, CH), 6.207 (s, 1H, 3-H), 6.027 (m, 1H, CH), 5.845 (m, 1H, CH), 5.596 (m, 1H, CH), 4.710 (m, 1H, 10-H), 3.100 (t, 2H, J=7.6Hz, 4-C<u>H</u>$_2$CH$_2$CF$_3$), 2.655 (m, 2H, 11-CH$_2$), 2.015 (m, 2H, 4-CH$_2$C<u>H</u>$_2$CF$_3$), 1.681 (d, 3H, J=6.4Hz, CH$_3$), 1.388 (dd, 3H, J=4.4Hz, 6.0Hz, 10-CH$_3$);
ESI-MS *(m/z):* 421.1 [M+H]$^+$ (MW=420.39) ;
HRESIMS obsd. m/z 421.1263, calcd. for $C_{22}H_{20}F_3O_5^+$ 421.12628.

Example 73

4-(3,3',3"-trifluoro-n-propyl)-6-n-butyl-10-methyl-2H,6H,12H-benzo[1,2-b:3,4-b':5, 6-b"]-tripyranyl-2,12-dione (**4-73** , $R_1$=n-$C_2H_4CF_3$, $R_2$=H, $R_3$= $CH_3$, $R_5$=$C_4H_9$ , $R_4$=$R_6$=H)

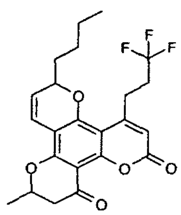

Using the same procedure as described in the preparative method of compound 4-1, except for using compound 5a-71 and 1,1-diethoxyl-2-heptene as starting material to obtain the title compound 4-73 in 74% yield as yellow powder, m. p. 129-130°C.
$^1$H-NMR (500MHz, DMSO-$d_6$, ppm): 6.658 (dt, 1H, J=2.0Hz, 10.0Hz, 8-H), 6.241 (d, 1H, J=3.0Hz, 3-H), 5.847 (dq, 1H, J=3.5Hz, 10.0Hz, 7-H), 5.155 (m, 1H, 6-H), 4.717 (m, 1 H, 10-H), 3.189 (m, 2H, $CH_2$), 3.091 (m, 2H, 4-C$\underline{H}_2$$CH_2CF_3$), 2.657 (m, 2H, 11-$CH_2$), 1.997 (m, 2H, 4-$CH_2$C$\underline{H}_2$$CF_3$), 1.791 (m, 2H, $CH_2$), 1.446 (d, 3H, J=6.5Hz, 10-$CH_3$), 1.370 (m, 2H, $CH_2$), 0.870 (t, 3H, J=7.5Hz, $CH_3$);
ESI-MS (*m*/*z*): 437.1 $[M+H]^+$ (MW=436.43) ;
HRESIMS obsd. m/z 437.1577, calcd. for $C_{23}H_{24}F_3O_5^+$ 437.15758.

Example 74

4-(3,3',3"-trifluoro-n-propyl)-6-phenyl-10-methyl-2H,6H,12H-benzo[1 ,2-b:3,4-b' :5,6-b "]-tripyranyl-2,12-dione (**4-74** , $R_1$=n-$C_2H_4CF_3$, $R_2$=H, $R_3$=$CH_3$, $R_5$=Ph , $R_4$=$R_6$=H)

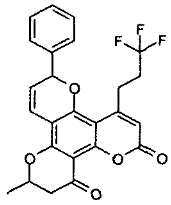

Using the same procedure as described in the preparative method of compound 4-1, except for using compound 5a-71 and 1,1-diethoxyl-3-pheny-propene as starting material to obtain the title compound 4-74 in 58% yield as yellowish powder, m. p. 171-173°C.
$^1$H-NMR (400MHz, DMSO-$d_6$, ppm): 7.411 (m, 5H, 6-Ph), 6.861 (dt, 1H, J=2.0Hz, 10.0Hz, 8-H), 6.283 (m, 1H, 6-H), 6.186 (d, 1H, J=2.0Hz, 3-H), 5.970 (dq, 1H, J=4.5Hz, 10.0Hz, 7-H), 4.766 (m, 1H, 10-H), 2.931 (m, 2H, 4-C$\underline{H}_2$$CH_2CF_3$), 2.703 (m, 2H, 11-$CH_2$), 1.978 (m, 2H, 4-$CH_2CH_2CF_3$), 1.479 (dd, 3H, J=5.5Hz, 7.5Hz, 10-$CH_3$);
ESI-MS (m/z): 457.1 $[M+H]^+$ (MW=456.42) ;
HRESIMS obsd. m/z 457.1264, calcd. for $C_{25}H_{20}F_3O_5^+$ 457.12628.

Example 75

6,11-dimethyl-4-n-propyl-2H,6H,12H-benzo[1,2-b:3,4-b':5,6-b"]-tripyranyl-2,12-dione (**4-75**, $R_1$=n-$C_3H_7$, $R_2$=H, $R_4$=$R_5$=$CH_3$, $R_3$=$R_6$=H)

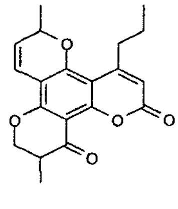

Using the same procedure as described in the preparative method of compound 4-1, except for using compound 5a-51 and 1,1-diethoxyl-2-butene as starting material to obtain the title compound 4-75 in 79% yield as off-white powder, m. p. 135-137°C.
$^1$H-NMR (300MHz, DMSO-$d_6$, ppm): 6.611 (dd, 1H, J=1.8Hz, 9.9Hz, 8-H), 6.107 (s, 1H, 3-H), 5.812 (dq, 1H, J=3.3Hz, 9.9Hz, 7-H), 5.204 (m, 1H, 6-H), 4.640 (dt, 1H, J=2.1Hz, 12.3Hz, CH), 4.252 (dq, 1H, J=3.0Hz, 12.3Hz, CH), 2.829 (m, 3H, 4-$CH_2$, 11-CH), 1.559 (m, 2H, 4-$CH_2$C$\underline{H}_2$$CH_3$), 1.460 (t, 3H, J=6.6Hz, $CH_3$), 1.331 (d, 3H, J=6.3Hz, $CH_3$), 0.950 (t, 3H, J=7.2Hz, 4-$CH_2CH_2$C$\underline{H}_3$);
ESI-MS (*m*/*z*): 340.9 $[M+H]^+$ (MW=340.38) ;
HRESIMS obsd m/z 341.1386, calcd for $C_{20}H_{21}O_5^+$ 341.1389.

Example 76

4-n-propyl-6-propenyl-11-methyl-2H,6H,12H-benzo[1,2-b:3,4-b':5,6-b"]-tripyranyl-2,1 2-dione (**4-76**, $R_1$=n-$C_3H_7$, $R_2$=H, $R_4$=$CH_3$, $R_3$=$R_6$=H)

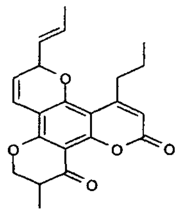

Using the same procedure as described in the preparative method of compound 4-1, except for using compound 5a-51 and 1,1-diethoxyl-2,4-hexadiene as starting material to obtain the title compound 4-76 in 65% yield as orange powder, m. p. 165-166°C.
$^1$H-NMR (300MHz, DMSO-$d_6$, ppm): 6.764 (dd, 1H, J=4.8Hz, 12.3Hz, CH), 6.305 (m, 1H, CH), 6.078 (s, 1H, 3-H), 5.979 (m, 1H, CH), 5.568 (m, 2H, 2CH), 4.589 (m, 2H, 10-$CH_2$), 2.782 (m, 3H, 4-C$\underline{H}_2$$CH_2CH_3$, 11-CH), 1.676 (m, 3H, $CH_3$), 1.497 (m, 2H, 4-$CH_2$C$\underline{H}_2$$CH_3$),1.043 (m, 3H, $CH_3$), 0.874 (t, 3H, J=7.2Hz, 4-$CH_2CH_2CH_3$);
ESI-MS (*m*/*z*): 366.9 $[M+H]^+$ (MW=366.42) ;
HRESIMS obsd. m/z 367.15399, calcd. for $C_{22}H_{23}O_5^+$ 367.15455.

Example 77

4-n-propyl-6-n-butyl-11-methyl-2H,6H,12H-benzo[1,2-b:3,4-b':5,6-b"]-tripyranyl-2,12 -dione (**4-77**, $R_1$=n-$C_3H_7$, $R_2$=H, $R_4$=$CH_3$,$R_5$=n-$C_4H_9$, $R_3$=$R_6$=H)

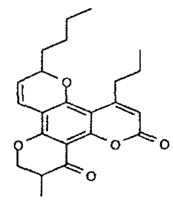

Using the same procedure as described in the preparative method of compound 4-1, except for using compound 5a-51 and 1,1-diethoxyl-2-heptene as starting material to obtain the title compound 4-77 in 71% yield as off-white powder, m. p. 144-145°C.
$^1$H-NMR (300MHz, DMSO-$d_6$, ppm): 6.630 (dd, 1H, J=1.8Hz, 9.9Hz, 8-H), 6.098 (s, 1H, 3-H), 5.814 (dq, 1H, J=3.0Hz, 9.9Hz, 7-H), 5.114 (m, 1H, 6-H), 4.625 (dd, 1 H, J=5.1Hz, 11.1Hz, CH), 4.246 (dq, 1H, J=3.0Hz, 11.1Hz, CH), 2.853 (m, 3H, 4-$CH_2$, 11-CH), 1.790 (m, 2H, 6-$CH_2$), 1.556 (m, 2H, 4-$CH_2$C$\underline{H}_2$$CH_3$), 1.341 (d, 3H, J=6.3Hz, $CH_3$), 1.085 (m, 4H, $CH_2$), 0.947 (m, 6H, $CH_3$);
ESI-MS (*m*/*z*): 382.9 $[M+H]^+$ (MW=382.46) ;
HRESIMS obsd. m/z 383.18972, calcd. for $C_{23}H_{27}O_5^+$ 383.18585.

Example 78

4-n-propyl-6-phenyl-11-methyl-2H,6H,12H-benzo[1,2-b:3,4-b':5,6-b"]-tripyranyl-2,12-dione (**4-78**, $R_1$=n-$C_3H_7$, $R_2$=H, $R_4$=$CH_3$,$R_5$=Ph, $R_3$=$R_6$=H)

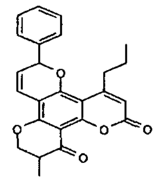

Using the same procedure as described in the preparative method of compound 4-1, except for using compound 5a-51 and 1,1-diethoxyl-3-phenyl-propene as starting material to obtain the title compound 4-78 in 70% yield as off-white powder, m. p. 187-189°C.

$^1$H-NMR (300MHz, DMSO-$d_6$, ppm): 7.426 (m, 5H, 6-Ph), 6.816 (dq, 1H, J=1.8Hz, 9.9Hz, 8-H), 6.223 (dq, 1H, J=1.8Hz, 8.7Hz, 6-H), 6.038 (s, 1H, 3-H), 5.923 (dq, 1H, J=1.8Hz, 9.9Hz, 7-H), 4.668 (dq, 1H, J=5.1Hz, 11.1Hz, CH), 4.294 (dt, 1H, J=1.2Hz, 11.1Hz, CH), 2.894 (m, 1H, 11-CH), 2.640 (m, 2H, 4-$CH_2$), 1.312 (m, 2H, 4-$CH_2C\underline{H}_2CH_3$), 1.066 (d, 3H, J=6.9Hz, 11-$CH_3$), 0.621 (t, 3H, J=7.2Hz, 4-$CH_2CH_2C\underline{H}_3$);

ESI-MS (*m/z*): 402.9 $[M+H]^+$ (MW=402.45) ;

HRESIMS obsd. m/z 403.1541, calcd. for $C_{25}H_{23}O_5^+$ 403.15455.

Example 79

6,10-dimethyl-4-ethyl-2H,6H,12H-benzo[1,2-b:3,4-b':5,6-b"]-tripyranyl-2,12-dione (**4-79**, $R_1$=$C_2H_5$, $R_2$=H, $R_3$= $R_5$=$CH_3$, $R_4$=$R_6$=H)

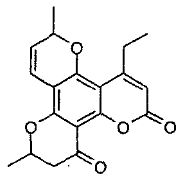

Using the same procedure as described in the preparative method of compound 4-1, except for using compound 5a-9 and 1,1-diethoxyl-2-butene as starting material to obtain the title compound 4-79 in 59% yield as off-white powder, m. p. 133-134°C.

$^1$H-NMR (300MHz, DMSO-$d_6$, ppm): 6.625 (dt, 1H, J=1.8Hz, 9.9Hz, 8-H), 6.101 (s, 1H, 3-H), 5.818 (dt, 1H, J=3.3Hz, 9.9Hz, 7-H), 5.514 (m, 1H, 6-H), 4.716 (m, 1H, 10-H), 2.997 (m, 2H, 4-$C\underline{H}_2CH_3$), 2.667 (m, 2H, 11-$CH_2$), 1.441 (d, 3H, J=6.0Hz, 6-$CH_3$), 1.435 (d, 3H, J=6.3Hz, 10-$CH_3$), 1.174 (t, 3H, J=7.2Hz, 4-$CH_2C\underline{H}_3$);

ESI-MS (*m/z*): 326.9 $[M+H]^+$ (MW=326.35) ;

*Anal.* Calcd. for $C_{19}H_{18}O_5 \cdot \frac{2}{3} H_2O$: C, 67.44; H, 5.76. Found: C, 67.45; H, 6.24.

Example 80

6,10-dimethyl-4-n-propyl-2H,6H,12H-benzo[1 ,2-b:3,4-b':5,6-b"]-tripyranyl-2,12-dione (**4-80**, $R_1$=n-$C_3H_7$, $R_2$=H, $R_3$=$R_5$=$CH_3$, $R_4$=$R_6$=H)

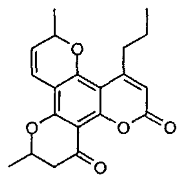

Using the same procedure as described in the preparative method of compound 4-1, except for using compound

5a-11 and 1,1-diethoxyl-2-butene as starting material to obtain the title compound 4-80 in 62% yield as off-white powder, m. p. 125-127°C.

$^1$H-NMR (300MHz, DMSO-$d_6$, ppm): 6.628 (dt, 1H, J=1.8Hz, 9.9Hz, 8-H), 6.107 (s, 1H, 3-H), 5.813 (dt, 1H, J=3.3H.z, 9.9Hz, 7-H), 5.217 (m, 1H, 6-H), 4.726 (m, 1H, 10-H), 2.820 (t, 2H, J=7.5Hz, 4-C$\underline{H}_2$CH$_2$CH$_3$), 2.659 (m, 2H, 11-CH$_2$), 1.558 (m, 2H, 4-CH$_2$C$\underline{H}_2$CH$_3$), 1.478 (d, 3H, J=6.6Hz, 6-CH$_3$), 1.440 (d, 3H, J=6.3Hz, 10-CH$_3$), 0.951 (t, 3H, J=7.2Hz, 4-CH$_2$CH$_2$C$\underline{H}_3$);

ESI-MS (*m*/*z*): 340.9 [M+H]$^+$ (MW=340.38) ;

*Anal.* Calcd. for $C_{20}H_{20}O_5 \cdot \frac{1}{2} H_2O$: C, 68.75; H, 6.06. Found: C, 68.93; H, 6.50.

Example 81

6,10-dimethyl-4-n-propyl-3-chloro-2H,6H,12H-benzo[1,2-b:3,4-b':5,6-b"]-tripyranyl-2, 12-dione (**4-81**, R$_1$=n-C$_{3H7}$, R$_2$=Cl, R$_3$=R$_5$=CH$_3$, R$_4$=R$_6$=H)

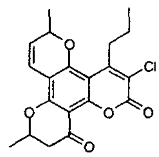

Using the same procedure as described in the preparative method of compound 4-1, except for using compound 5a-20 and 1,1-diethoxyl-2-butene as starting material to obtain the title compound 4-81 in 64% yield as off-white powder, m. p. 129-131°C.

$^1$H-NMR (300MHz, DMSO-$d_6$, ppm): 6.646 (dt, 1H, J=1.8Hz, 9.9Hz, 8-H), 5.856 (dt, 1H, J=3.0Hz, 9.9Hz, 7-H), 5.240 (m, 1H, 6-H), 4.738 (m, 1H, 10-H), 3.097 (t, 2H, J=7.5Hz, 4-C$\underline{H}_2$CH$_2$CH$_3$), 2.693 (m, 2H, 11-CH$_2$), 1.573 (m, 2H, 4-CH$_2$C$\underline{H}_2$CH$_3$), 1.516 (d, 3H, J=6.6Hz, 6-CH$_3$), 1.448 (d, 3H, J=6.3Hz, 10-CH$_3$), 1.023 (t, 3H, J=7.2Hz, 4-CH$_2$CH$_2$C$\underline{H}_3$);
ESI-MS (*m*/*z*): 374.9 M$^+$ (MW=374.82) ;
*Anal.* Calcd. for $C_{20}H_{19}ClO_5$: C, 64.09; H, 5.11. Found: C, 63.82; H, 5.25.

Example 82

6,1.0-dimethyl-4-n-butyl-2H,6H,12H-benzo[1,2-b:3,4-b':5,6-b"]-tripyranyl-2,12-dione (**4-82**, R$_1$=n-C$_4$H$_9$, R$_2$=H, R$_3$=R$_5$=CH$_3$, R$_4$=R$_6$=H)

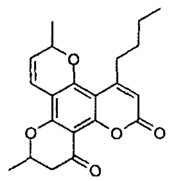

Using the same procedure as described in the preparative method of compound 4-1, except for using compound 5a-36 and 1,1-diethoxyl-2-butene as starting material to obtain the title compound 4-82 in 61% yield as yellowish powder, m. p. 128-129°C.

$^1$H-NMR (300MHz, DMSO-$d_6$, ppm): 6.634 (dt, 1H, J=1.8Hz, 9.9Hz, 8-H), 6.109 (s, 1H, 3=H), 5.818 (dt, 1H, J=3.0Hz, 9.9Hz, 7-H), 5.198 (m, 1H, 6-H), 4.716 (m, is, 10-H), 2.843 (m, 2H, 4-C$\underline{H}_2$CH$_2$CH$_2$CH$_3$), 2.657 (m, 2H, 11-CH$_2$), 1.530 (m, 2H, 4-CH$_2$C$\underline{H}_2$CH$_2$CH$_3$), 1.470 (d, 3H, J=6.6Hz, 6-CH$_3$), 1.440 (d, 3H, J=6.3Hz, 10-CH$_3$), 1.384 (m, 2H, 4-CH$_2$CH$_2$C$\underline{H}_2$CH$_3$), 0.904 (t, 3H, J=7.2Hz, 4-CH$_2$CH$_2$CH$_2$C$\underline{H}_3$);

ESI-MS (*m*/*z*): 354.9 [M+H]$^+$ (MW=354.41) ;

*Anal.* Calcd. for $C_{21}H_{22}O_5 \cdot \frac{1}{4} H_2O$: C, 70.28; H, 6.32. Found: C, 70.33; H, 6.29.

Example 83

6,10-dimethyl-4-ethyl-3-fluoro-2H,6H,12H-benzo[1,2-b:3,4-b':5,6-b"]-tripyranyl-2,12-dione (**4-83**, $R_1=C_2H_5$, $R_2=F$, $R_3=R_5=CH_3$, $R_4=R_6=H$)

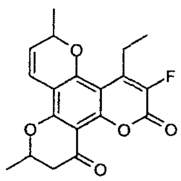

Using the same procedure as described in the preparative method of compound 4-1, except for using compound 5a-10 and 1,1-diethoxyl-2-butene as starting material to obtain the title compound 4-83 in 65% yield as off-white powder, m. p. 141-142°C.

$^1$H-NMR (300MHz, DMSO-$d_6$, ppm): 6.639 (dt, 1H, J=1.8Hz, 9.9Hz, 8-H), 5.852 (dt, 1H, J=3.3Hz, 9.9Hz, 7-H), 5.547 (m, 1H, 6-H), 4.715 (m, 1H, 10-H), 2.968 (m, 2H, 4-C$\underline{H}_2$CH$_3$), 2.677 (m, 2H, 11-CH$_2$), 1.483 (d, 3H, J=6.6Hz, 6-CH$_3$), 1.444 (d, 3H, J=6.3Hz, 10-CH$_3$), 1.198 (t, 3H, J=7.2Hz, 4-CH$_2$C$\underline{H}_3$);

ESI-MS (*m/z*): 344.9 [M+H]$^+$ (MW=344.34) ;

*Anal.* Calcd. for $C_{19}H_{17}FO_5 \cdot \frac{1}{2} H_2O$: C, 64.58; H, 5.13. Found: C, 64.51; H, 5.39.

Example 84

6,10-dimethyl-4-n-propyl-3-fluoro-2H,6H,12H-benzo[1,2-b:3,4-b':5,6-b"]-tripyranyl-2, 12-dione (**4-84**, $R_1$=n-$C_3H_7$, $R_2=F$, $R_3=R_5=CH_3$, $R_4=R_6=H$)

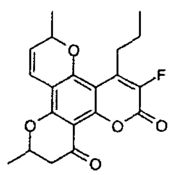

Using the same procedure as described in the preparative method of compound 4-1, except for using compound 5a-31 and 1,1-diethoxyl-2-butene as starting material to obtain the title compound 4-84 in 66% yield as off-white powder, m. p. 150-153C.

$^1$H-NMR (300MHz, DMSO-$d_6$, ppm): 6.636 (dt, 1H, J=2.1Hz, 9.9Hz, 8-H), 5.844 (dt, 1H, J=3.3Hz, 7-H), 5.190 (m, 1H, 6-H), 4.713 (m, 1H, 10-H), 2.902 (m, 2H, 4-C$\underline{H}_2$CH$_2$CH$_3$), 2.676 (m, 2H, 11-CH$_2$), 1.586 (m, 2H, 4-CH$_2$C$\underline{H}_2$CH$_3$), 1.483 (d, 3H, J=6.6Hz, 6-CH$_3$), 1.442 (d, 3H, J=6.3Hz, 10-CH$_3$), 0.970 (t, 3H, J=7.2Hz, 4-CH$_2$CH$_2$C$\underline{H}_3$);

ESI-MS (*m/z*): 358.9 [M+H]$^+$ (MW=358.37) ;

HRESIMS obsd. m/z 359.1291, calcd. for $C_{20}H_{20}FO_5^+$ 359.12947.

Example 85

4-n-propyl-6-propenyl-10-methyl-2H,6H,12H-benzo[1,2-b:3,4-b':5,6-b"]-tripyranyl-2,1 2-dione (**4-85**, $R_1$=n-$C_3H_7$, $R_3$=$CH_3$, $R_4$=$R_6$=H)

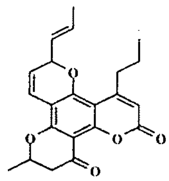

Using the same procedure as described in the preparative method of compound 4-1, except for using compound 5a-11 and 1,1-diethoxyl-2,4-hexdiene as starting material to obtain the title compound 4-85 in 63% yield as orange powder, m. p. 136-138°C.
$^1$H-NMR (300MHz, DMSO-$d_6$, ppm): 6.652 (dq, 1H, J=1.5Hz, 9.9Hz, 8-H), 6.074 (s, 1H, 3-H), 5.904 (m, 1H, CH), 5.805 (dd, 1H, J=3.9Hz, 9.9Hz, 7-H), 5.702 (dq, 1H, J=6.0Hz, 13.5Hz, CH), 5.506 (m, 1H, 6-H), 4.715 (m, 1H, 10-H), 2.761 (m, 2H, 4-C$\underline{H}_2$CH$_2$CH$_3$), 2.650 (m, 2H, 11-CH$_2$), 1.682 (dt, 3H, J=1.2Hz, 6.0Hz, CH$_3$), 1.505 (m, 2H, 4-CH$_2$C$\underline{H}_2$CH$_3$), 1.440 (dd, 3H, J=2.7Hz, 6.3Hz, 10-CH$_3$), 0.901 (t, 3H, J=7.2Hz, 4-CH$_2$CH$_2$C$\underline{H}_3$);
ESI-MS (*m*/*z*): 366.9 [M+H]$^+$ (MW=366.42) ;
*Anal*. Calcd. for $C_{22}H_{22}O_5.H_2O$: C, 68.74; H, 6.29. Found: C, 68.53; H, 6.22.

Example 86

4-n-propyl-6-propenyl-3-chloro-10-methyl-2H,6H,12H-benzo[1,2-b:3,4-b':5,6-b"]-tripy ranyl-2,12-dione (**4-86**, $R_1$=n-$C_3H_7$, $R_2$=Cl, $R_3$=$CH_3$, $R_4$=$R_6$=H)

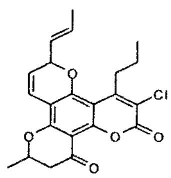

Using the same procedure as described in the preparative method of compound 4-1, except for using compound 5a-20 and 1,1-diethoxyl-2,4-hexdiene as starting material to obtain the title compound 4-86 in 58% yield as off-white powder, m. p. 142-144°C.
$^1$H-NMR (300MHz, DMSO-$d_6$, ppm): 6.690 (dq, 1H, J=1.2Hz, 9.9Hz, CH), 5.931 (m, 1H, CH), 5.854 (m, 1H, CH), 5.738 (m, 1H, CH), 5.580 (m, 1H, CH), 4.750 (m, 1H, 10-H), 3.063 (m, 2H, 4-C$\underline{H}_2$CH$_2$CH$_3$), 2.671 (m, 2H, 11-CH$_2$), 1.690 (dt, 3H, J=1.5Hz, 6.6Hz, CH$_3$), 1.528 (m, 2H, 4-CH$_2$C$\underline{H}_2$CH$_3$), 1.452 (dd, 3H, J=3.0Hz, 6.3Hz, 10-CH$_3$), 0.984 (dt, 3H, J=3.6Hz, 7.2Hz, 4-CH$_2$CH$_2$C$\underline{H}_3$);
ESI-MS (*m*/*z*): 400.9 M$^+$ (MW=400.86);
HRESIMS obsd. *m*/*z* 401.1163, calcd. for $C_{22}H_{22}ClO_5^+$ 401.11557.

Example 87

4-ethyl-6-propenyl-10-methyl-2H,6H,12H-benzo[1,2-b:3,4-b':5,6-b"]-tripyranyl-2,12-d ione (**4-87**, $R_1$=$C_2H_5$, $R_2$=H $R_3$=$CH_3$, $R_4$=$R_6$=H)

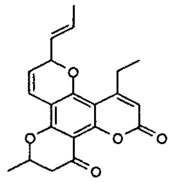

Using the same procedure as described in the preparative method of compound 4-1, except for using compound 5a-9 and 1,1-diethoxyl-2,4-hexdiene as starting material to obtain the title compound 4-87in 67% yield as orange powder, m. p. 153-155°C.
$^1$H-NMR (300MHz, DMSO-$d_6$, ppm): 6.762 (dd, 1H, J=3.9Hz, 12.6Hz, CH), 6.340 (m, 1H, CH), 6.069 (s, 1H, 3-H), 5.823 (m, 1H, CH), 5.596 (m, 2H, 2CH), 4.601 (m, 1H, 10-H), 2.858 (m, 2H, 4-C$\underline{H}_2$CH$_3$), 2.606 (m, 2H, 11-CH$_2$), 1.677 (m, 3H, CH$_3$), 1.340 (m, 3H, CH$_3$), 1.198 (t, 3H, J=7.2Hz, 4-CH$_2$C$\underline{H}_3$);
ESI-MS (*m*/*z*): 352.9 $[M+H]^+$ (MW=352.39) ;
*Anal.* Calcd. for $C_{21}H_{20}O_5.2H_2O$: C, 64.94; H, 6.23. Found: C, 65.15; H, 6.18.
HRESIMS obsd. m/z 353.1398, calcd. for $C_{21}H_{21}O_5^+$ 353.1389.

Example 88

4-ethyl-6-propenyl-3-fluoro-10-methyl-2H,6H,12H-benzo[1,2-b:3,4-b':5,6-b"]-tripyran yl-2,12-dione (**4-88**, $R_1$=$C_2H_5$, $R_2$=F, $R_3$=$CH_3$, $R_4$=$R_6$=H)

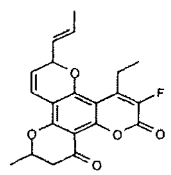

Using the same procedure as described in the preparative method of compound 4-1, except for using compound 5a-10 and 1,1-diethoxyl-2,4-hexdiine as starting material to obtain the title compound 4-88 in 66% yield as yellow powder, m. p. 158-161°C.
$^1$H-NMR (300MHz, DMSO-$d_6$, ppm): 6.773 (dd, 1H, 3.3Hz, 12.3Hz, CH), 6.361 (m, 1H, CH), 6.044 (m, 1H, CH), 5.598 (m, 2H, 2CH), 4.648 (m, 1H, 10-H), 2.940 (m, 2H, 4-C$\underline{H}_2$CH$_3$), 2.620 (m, 2H, 11-CH$_2$), 1.701 (m, 3H, CH$_3$), 1.375 (d, 3H, J=6.3Hz, 10-CH$_3$), 1.129 (t, 3H, J=7.2Hz, 4-CH$_2$C$\underline{H}_3$);
ESI-MS (*m*/*z*): 370.9 $[M+H]^+$ (MW=370.38) ;
*Anal.* Calcd. for $C_{21}H_{19}FO_5\cdot\frac{5}{3}H_2O$: C, 62.99; H, 5.62. Found: C, 62.83; H, 5.78.

Example 89

4-ethyl-7,10-dimethyl-2H,6H,12H-benzo[1,2-b:3,4-b':5,6-b"]-tripyranyl-2,12-dione (**4-89** , $R_1$=$C_2H_5$, $R_2$=H, $R_3$=$CH_3$, $R_4$=$R_5$=$R_6$=H, $R_7$=$CH_3$, $R_8$=H)

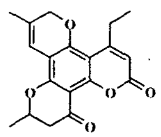

Using the same procedure as described in the preparative method of compound 4-1, except for using compound 5a-9 and 1,1-diethoxyl-2-methylpropene as starting material to obtain the title compound 4-89 in 49% yield as white

powder, m. p. 121-123°C.
$^1$H-NMR (300MHz, DMSO-$d_6$, ppm): 6.400 (q, 1H, J=1.8Hz, 8-H), 6.107 (s, 1H, 3-H), 4.852 (s, 2H, 6-$CH_2$), 4.697 (m, 1H, 10-H), 2.875 (q, 2H, J=7.2Hz, 4-C<u>H</u>$_2$$CH_3$), 2.697 (m, 2H, 11-$CH_2$), 1.827 (d, 3H, J=1.8Hz, 7-$CH_3$), 1.442 (d, 3H, J=6.3Hz, 10-$CH_3$), 1.145 (d, 3H, J=7.2Hz, 4-$CH_2CH_3$);
ESI-MS (*m*/*z*): 326.9 $[M+H]^+$ (MW=326.35) ;

*Anal.* Calcd. for $C_{19}H_{18}O_5\cdot\frac{3}{4}H_2O$: C, 67.15; H, 5.78. Found: C, 67.21; H, 5.58.

Example 90

4-n-propyl-7,10-dimethyl-2H,6H,12H-benzo[1,2-b:3,4-b':5,6-b"]-tripyranyl-2,12-dione (**4-90**, $R_1$=n-$C_3H_7$, $R_2$=H, $R_3$=$CH_3$, $R_4$=$R_5$=$R_6$=H, $R_7$=$CH_3$, $R_8$=H)

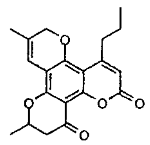

Using the same procedure as described in the preparative method of compound 4-1, except for using compound 5a-11 and 1,1-diethoxyl-2-methylpropene as starting material to obtain the title compound 4-90 in 53% yield as white powder, m. p. 127-128°C.
$^1$H-NMR (300MHz, DMSO-$d_6$, ppm): 6.401 (m, 1H, 8-H), 6.104 (s, 1H, 3-H), 4.851 (s, 2H, 6-$CH_2$), 4.697 (m, 1H, 10-H), 2.799 (t, 2H, J=7.5Hz, 4-C<u>H</u>$_2$$CH_2CH_3$), 2.659 (m, 2H, 11-$CH_2$), 1.828 (s, 3H, 7-$CH_3$), 1.546 (m, 2H, 4-$CH_2$C<u>H</u>$_2$$CH_3$), 1.441 (d, 3H, J=6.3Hz, 10-$CH_3$), 0.941 (t, 3H, J=7.2Hz, 4-$CH_2CH_2$C<u>H</u>$_3$);
ESI-MS (*m*/*z*): 340.9 $[M+H]^+$ (MW=340.38) ;

*Anal.* Calcd. for $C_{20}H_{20}O_5\cdot\frac{3}{4}H_2O$: C, 67.88; H, 6.12. Found: C, 67.96; H, 6.44.

Example 91

4-n-propyl-3-chloro-7,10-dimethyl-2H,6H,12H-benzo[1,2-b:3,4-b':5,6-b"]-tripyranyl-2,12-dione (**4-91**, $R_1$=n-$C_3H_7$, $R_2$=Cl, $R_3$=$CH_3$, $R_4$=$R_5$=$R_6$=H, $R_7$=$CH_3$, $R_8$=H)

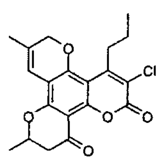

Using the same procedure as described in the preparative method of compound 4-1, except for using compound 5a-20 and 1,1-diethoxyl-2-methylpropene as starting material to obtain the title compound 4-91 in 51 % yield as white powder, m. p. 129-131°C.
$^1$H-NMR (300MHz, DMSO-$d_6$, ppm): 6.415 (q, 1H, J=1.5Hz, 8-H), 4.882 (s, 2H, 6-$CH_2$), 4.719 (m, 1H, 10-H), 3.075 (t, 2H, J=7.5Hz, 4-C<u>H</u>$_2$$CH_2CH_3$), 2.678 (m, 2H, 11-$CH_2$), 1.844 (d, 3H, J=1.5Hz, 7-$CH_3$), 1.556 (m, 2H, 4-$CH_2$C<u>H</u>$_2$$CH_3$), 1.448 (d, 3H, J=6.3Hz, 10-$CH_3$), 1.009 (t, 3H, J=7.2Hz, 4-$CH_2CH_2$C<u>H</u>$_3$);
ESI-MS (*m*/*z*): 374.9 $M^+$ (MW=374.82) ;

*Anal.* Calcd. for $C_{20}H_{19}ClO_5\cdot\frac{1}{2}H_2O$: C, 62.58; H, 5.25. Found: C, 62.56; H, 5.72.

Example 92

4-n-propyl-3-fluoro-7,10-dimethyl-2H,6H,12H-benzo[1,2-b:3,4-b':5,6-b"]-tripyranyl-2, 12-dione (**4-92**, $R_1=C_2H_5$, $R_2$=F, $R_3=CH_3$, $R_4=R_5=R_6$=H, $R_7=CH_3$, $R_8$=H)

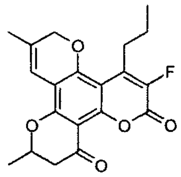

Using the same procedure as described in the preparative method of compound 4-1, except for using compound 5a-31 and 1,1-diethoxyl-2-methylpropene as starting material to obtain the title compound 4-92 in 47% yield as brown powder, m. p. 122-124°C.
$^1$H-NMR (300MHz, DMSO-$d_6$, ppm): 6.408 (s, 1H, 8-H), 4.863 (s, 2H, 6-$CH_2$), 4.697 (m, 1H, 10-H), 2.897 (m, 2H, 4-$C\underline{H}_2CH_2CH_3$), 2.671 (m, 2H, 11-$CH_2$), 1.836 (s, 3H, 7-$CH_3$), 1.570 (m, 2H, 4-$CH_2C\underline{H}_2CH_3$), 1.443 (d, 3H, J=6.0Hz, 10-$CH_3$), 0.958 (t, 3H, J=7.2Hz, 4-$CH_2CH_2C\underline{H}_3$);
ESI-MS (*m*/*z*): 358.9 [M+H]$^+$ (MW=358.37) ;
*Anal*. Calcd. for $C_{20}H_{19}FO_5\cdot\frac{2}{3}H_2O$: C, 64.86; H, 5.53. Found: C, 64.72; H, 5.96.

Example 93

4-ethyl-3-fluoro-7,10-dimethyl-2H,6H,12H-benzo[1,2-b:3,4-b':5,6-b"]-tripyranyl-2,12-dione (**4-93**, $R_1=C_2H_5$, $R_2$=F, $R_3=CH_3$, $R_4=R_5=R_6$=H, $R_7=CH_3$, $R_8$=H)

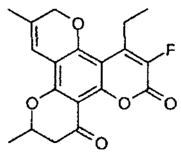

Using the same procedure as described in the preparative method of compound 4-1, except for using compound 5a-10 and 1,1-diethoxyl-2-methylpropene as starting material to obtain the title compound 4-93 in 49% yield as yellowish powder, m. p. 118-120°C.
$^1$H-NMR (300MHz, DMSO-$d_6$, ppm): 6.382 (d, 1H, J=1.5Hz, 8-H), 4.857 (s, 2H, 6-$CH_2$), 4.668 (m, 1H, 10-H), 2.899 (q, 2H, J=7.2Hz, 4-$C\underline{H}_2CH_3$), 2.687 (m, 2H, 11-$CH_2$), 1.828 (s, 3H, 7-$CH_3$), 1.442 (d, 3H, J=6.0Hz, 10-$CH_3$), 1.149 (t, 3H, J=7.2Hz, 4-$CH_2C\underline{H}_3$);
ESI-MS (*m*/*z*): 344.9 [M+H]$^+$ (MW=344.34) ;
And the title compounds **4-94, 4-95, 4-96** were simutaiously obtained respectively when the compounds **4-89, 4-90** and **4-91** were produced in this method.

Example 94

8-ethyoxyl-7,10-dimethyl-4-ethyl-7,8,10,11-tetrahydro-6H-benzo[2,3-f;2',3'-h]-tripyran yl-2,12-dione (**4-94**, $R_1=C_2H_5$, $R_2$=H, $R_3=CH_3$, $R_4=R_5=R_6$=H, $R_7=CH_3$, $R_8=OC_2H_5$)

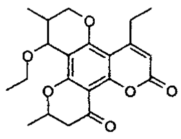

It is white powder, yield 19%, m. p. 174-176°C.

$^1$H-NMR (300MHz, DMSO-$d_6$, ppm): 6.106 (s, 1H, 3-H), 5.169 (dd, 1H, J=5.4Hz, 4.8Hz, 8-H), 4.703 (m, 1H, 10-H), 3.850 (dq, 1H, J=7.2Hz, 14.1Hz, 6-CH), 3.701 (dq, 1H, J=2.7Hz, 14.1Hz, 6-CH), 3.013 (sex, 1H, J=7.2Hz, 1.4.7Hz, $OC\underline{H}_2CH_3$), 2.898 (q, 2H, J=7.2Hz, 4-$C\underline{H}_2CH_3$), 2.691 (m, 2H, 11-$CH_2$), 2.324 (dt, 1H, J=6.9Hz, 14.7Hz, $OC\underline{H}_2CH_3$), 2.047 (m, 1H, 7-H), 1.434 (d, 3H, J=6.3Hz, 10-$CH_3$), 1.173 (dd, 3H, J=6.9Hz, 7.2Hz, $OCH_2C\underline{H}_3$), 1.148 (t, 3H, J=7.2Hz, 4-$CH_2C\underline{H}_3$), 0.982 (d, 3H, J=6.9Hz, 7-$CH_3$);

ESI-MS (*m*/*z*): 372.9 $[M+H]^+$ (MW=372.42) ;

*Anal*. Calcd. for $C_{21}H_{24}O_6\cdot\frac{1}{3}H_2O$: C, 66.65; H, 6.56. Found: C, 66.43; H, 6.33.

Example 95

8-ethyoxyl-7,10-dimethyl-4-n-propyl-7,8,10,11-tetrahydro-6H-benzo[2,3-f;2',3'-h']-trip yranyl-2,12-dione (**4-95**, $R_1$=n-$C_3H_7$, $R_2$=H, $R_3$=$CH_3$, $R_4$=$R_5$=$R_6$=H, $R_7$=$CH_3$, $R_8$=$OC_2H_5$)

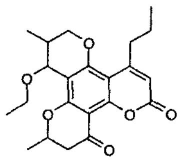

It is white powder, yield 26%, m. p. 198-201°C.

$^1$H-NMR (300MHz, DMSO-$d_6$, ppm): 6.097 (s, 1H, 3-H), 5.154 (dd, 1H, J=5.4Hz, 8-H), 4.703 (m, 1H, 10-H), 3.864 (dq, 1H, J=7.2Hz, 14.1Hz, 6-CH), 3.709 (dq, 1H, J=2.7Hz, 14.1Hz, 6-CH), 2.956 (dd, 1H, J=7.2Hz, 13.8Hz, $OC\underline{H}_2CH_3$), 2.866 (t, 2H, J=7.5Hz, 4-$C\underline{H}_2CH_2CH_3$), 2.783 (dd, 1H, J=8.1Hz, 14.1Hz, 11-CH), 2.646 (dd, 1H, J=3.9Hz, 14.1Hz, 11-CH), 2.301 (dd, 1H, J=6.9Hz, 13.8Hz, $OC\underline{H}_2CH_3$), 2.035 (qu, 1H, J=5.7Hz, 6.3Hz, 7-H), 1.568 (sex, 2H, J=7.5Hz, 7.2Hz, 4-$CH_2C\underline{H}_2CH_3$), 1.433 (d, 3H, J=6.3Hz, 10-$CH_3$), 1.162 (t, 3H, J=7.2Hz, $OCH_2C\underline{H}_3$), 0.954 (d, 3H, J=5.7Hz, 7-CH), 0.944 (t, 3H, J=7.2Hz, , 4-$CH_2CH_2CH_3$);

ESI-MS (*m*/*z*): 386.9 $[M+H]^+$ (MW=386.45) ;

*Anal.* Calcd. for $C_{22}H_{26}O_6\cdot\frac{1}{4}H_2O$: C, 67.59; H, 6.83. Found: C, 67.48; H, 6.68.

Example 96

8-ethoxyl-7,10-dimethyl-4-n-propyl-3-chloro-7,8,10,11-tetrahydro-6H-benzo[2,3-f;2',3' -h]-tripyranyl-2,12-dione (**4-96**, $R_1$=n-$C_3H_7$, $R_2$=Cl, $R_3$=$CH_3$, $R_4$=$R_5$=$R_6$=H, $R_7$=$CH_3$, $R_8$=$OC_2H_5$)

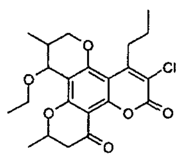

It is yellow powder, yield 21%, m. p. 187-189°C.
$^1$H-NMR (300MHz, DMSO-$d_6$, ppm): 5.223 (dd, 1H, J=8.1Hz, 4.8Hz, 8-H), 4.724 (m, 1H, 10-H), 3.846 (dq, 1H, J=2.7Hz, 7.2Hz, 6-CH), 3.732 (dq, 1H, J=3.3Hz, 7.2Hz, 6-CH 3.176 (m, 2H, 4-C$\underline{H}_2$CH$_2$CH$_3$), 2.777 (m, 1 H, OC$\underline{H}_2$CH$_3$), 2.680 (m, 2H, 11-CH$_2$), 2.354 (dt, 1H, J=6.0Hz, 16.8Hz, OC$\underline{H}_2$CH$_3$), 2.090 (m, 1H., 7-H), 1.576 (m, 2H, 4-CH$_2$C$\underline{H}_2$CH$_3$), 1.440 (d, 3H, J=6.3Hz, 10-CH$_3$), 1.153 (q, 3H, J=7.2Hz, OCH$_2$C$\underline{H}_3$), 1.007 (t, 3H, J=7.5Hz, 4-CH$_2$CH$_2$C$\underline{H}_3$), 0.957 (t, 3H, J=6.6Hz, 7-CH$_3$);
ESI-MS (*m*/*z*): 420.9 M$^+$ (MW=420.89) ;
*Anal.* Calcd. for $C_{22}H_{25}ClO_6$: C, 62.78; H, 5.99. Found: C, 62.90; H, 5.96.

Example 97

4-n-propyl-6-(o-nitrophenyl)-10-methyl-2H,6H,12H-benzo[1,2-b:3,4-b':5,6-b"]-tripyr anyl-2,12-dione (**4-97**, $R_1$=n-$C_3H_7$, $R_2$=H, $R_3$=$CH_3$, $R_4$=$R_6$=H, $R_5$=Ph-$NO_2$)

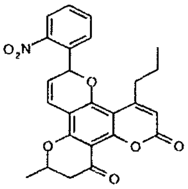

Using the same procedure as described in the preparative method of compound 4-1, except for using compound 5a-11 and o-nitrophenyl(ethyl)acetal as starting material to obtain the title compound 4-97 in 44% yield as orange powder, m. p. 216-218°C.
$^1$H-NMR (300MHz, DMSO-$d_6$, ppm): 8.125 (m, 1H, Ar-H), 7.819 (m, 2H, Ar-H), 7.689 (m, 1H, Ar-H), 6.877 (m, 1H, CH), 6.620 (m, 1H, CH), 6.081 (d, 1H, J=1.SHz, 3-H), 5.926 (dq, 1H, J=3.3Hz, 9.0Hz, CH), 4.774 (m, 1H, 10-H), 2.695 (m, 2H, 4-C$\underline{H}_2$CH$_2$CH$_3$), 1.996 (m, 2H, 11-CH$_2$), 1.476 (d, 3H, J=6.3Hz, 10-CH$_3$), 1.301 (m, 2H, 4-CH$_2$C$\underline{H}_2$CH$_3$), 0.551 (m, 3H, 4-CH$_2$CH$_2$C$\underline{H}_3$);
ESI-MS (m/z): 448.2 [M+H]$^+$ (MW=447.45) ;
*Anal.* Calcd. for $C_{25}H_{21}NO_7 \cdot 5H_2O$: C, 55.86; H, 5.81. Found: C, 56.05; H, 5.89.
And the title compound **4-98** was obtained simutaiously in this method.

Example 98

8-ethoxyl-4-n-propyl-6-(o-nitrophenyl)-10-methyl-4-n-propyl-7,8,10,11-tetrahydro-6H -benzo[2,3-f;2',3'-h]-tripyranyl-2,12-dione (**4-98**, $R_1$=$C_2H_5$, $R_2$=H, $R_3$=$CH_3$, $R_4$=$R_6$=H, $R_5$=Ph-$NO_2$, $R_7$=H, $R_8$=$OC_2H_5$)

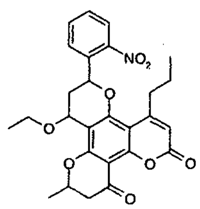

It is white powder, yield 25%, m. p. 237-240°C.
$^1$H-NMR (300MHz, DMSO-$d_6$, ppm): 7.874 (m, 1H, Ar-H), 7.469 (m, 2H, Ar-H), 7.204 (m, 1H, Ar-H), 6.165 (d, 1H, J=4.2Hz, 3-H), 5.513 (m, 1H, CH), 4.584 (m, 1H, CH), 4.484 (m, 1H, 10-H), 3.895 (m, 1H, CH), 3.685 (m, 1H, CH), 2.957 (m, 2H, 4-CH$_2$CH$_2$CH$_3$), 2.541 (m, 2H, 11-CH), 2.198 (m, 2H, CH$_2$), 1.657 (m, 2H, 4-CH$_2$CH$_2$CH$_3$), 1.138 (m, 3H, CH$_3$), 0.983 (t, 3H, J=7.2Hz, CH$_3$), 0.814 (d, 3H, J=6.3Hz, CH$_3$);
ESI-MS (m/z): 494.1 [M+H]$^+$ (MW=493.52) ;

Example 99

4-n-propyl-6-(o-aminophenyl)-10-methyl-2H,6H,12H-benzo[1,2-b:3,4-b':5,6-b"]-tripyr anyl-2,12-dione (**4-99**, $R_1$=n-$C_3H_7$, $R_2$=H, $R_3$=$CH_3$, $R_4$=$R_6$=H, $R_5$=Ph-$NH_2$)

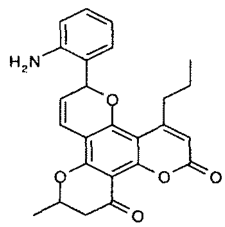

Using the same procedure as described in the preparative method of compound 4-23. Compound 4-97 was treated with 15 equivlent $SnCl_2$. The title compound 4-99 can be produced as yellow powder in 81% yield, m. p. 175-177°C. $^1$H-NMR (300MHz, DMSO-$d_6$, ppm): 7.762 (m, 1H, Ar-H), 7.258 (m, 1H, Ar-H), 6.944 (m, 1H, Ar-H), 6.594 (m, 2H, Ar-H), 6.055 (s, 1H, 3-H), 5.936 (br, $NH_2$), 4.800 (m, 1H, CH), 4.358 (m, 1H, CH), 2.893 (t, 2H, J=7.5Hz, 4-C<u>H</u>$_2$CH$_2$CH$_3$), 2.633 (m, 2H, 11-$CH_2$), 2.108 (m, 2H, $CH_2$), 1.569 (dd, 3H, J=6.3Hz, 13.2Hz, $CH_3$), 0.973 (t, 3H, J=7.2Hz, 4-CH$_2$CH$_2$C<u>H</u>$_3$);
ESI-MS (*m/z*): 418.1 $[M+H]^+$ (MW=417.47);
HRESIMS obsd. m/z 418.1652, calcd. for $C_{25}H_{24}NO_5^+$ 418.16544.

Example 100

10,10-dimethyl-8-n-propyl-3,4-dihydro-10H-benzo[2,3-f;2',3'-h]-dipyranyl-2,6-dione (**4-100**, $R_1$=n-$C_3H_7$, $R_2$=H, $R_5$=$R_6$=$CH_3$)

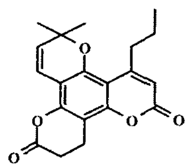

(1) 5-hydroxy-4-n-propyl-9,10-dihydro-benzo[2,3-f]-pyranyl-2,8-dione (5a-100)

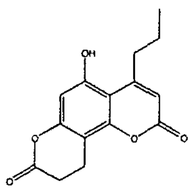

Compound 6-11 was mixed with 1 equivlent acrylic acid and $CF_3SO_3H$, the mixture was stirred fou 4 hours at 60 °C in oil-bath. After cooled, the reaction solution was poured into ice-water, stirred, extrated with ethyl acetate, concentrated and dried, then purified through chromatography on silica gel to obtained 5-hydroxy-4-n-propyl-9,10-dihydro-benzo[2,3-f]-pyranyl-2,8-dione (5a-100) as off-white powder in 65% yield, m. p. 166-167°C.
$^1$H-NMR (400MHz, DMSO-$d_6$, ppm): 11.034 (s, 1H, 5-OH), 6.600 (s, 1H, 6-H), 6.053 (s, 1H, 3-H), 2.852 (in, 6H, 4,9,10-$CH_2$), 1.571 (m, 2H, 4-CH$_2$C<u>H</u>$_2$CH$_3$), 0.969 (t, 3H, J=7.2Hz, 4-CH$_2$CH$_2$C<u>H</u>$_3$);
ESI-MS (*m/z*): 275.1 $[M+H]^+$ (MW=274.27) ;
*Anal.* Calcd. for $C_{15}H_{14}O_5$: C, 65.69; H, 5.15. Found: C, 65.45; H, 5.20.

HRESIMS obsd. $m/z$ 275.0918, calcd. for $C_{15}H_{15}O_5^+$ 275.09195.

(2) 10,10-dimethyl-8-n-propyl-3,4-dihydro-10H-benzo[2,3-f;2',3'-h]-dipyranyl-2,6-dione (**4-100**, $R_1$=n-$C_3H_7$, $R_2$=H, $R_5$=$R_6$=$CH_3$)

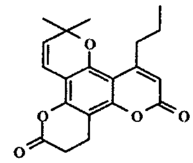

Using the same procedure as described in the preparative method of compound 4-1, except for using compound 5a-100 and o-nitrophenyl(ethyl)acetal as starting material to obtain the title compound 4-100 in 72% yield as off-white powder, m. p. 132-135°C.
$^1$H-NMR (400MHz, DMSO-d6, ppm): 6.841 (d, 1H, J=10.0Hz, 8-H), 6.104 (s, 1H, 3-H), 5.684 (d, 1H, J=10.0Hz, 7-H), 2.968 (m, 4H, $CH_2$), 2.786 (m, 2H, 11-$CH_2$), 1.623 (m, 2H, 4-$CH_2C\underline{H}_2CH_3$), 1.508, 1.480 (2s, 6H, $CH_3$), 1.047 (t, 3H, J=6.4Hz, 4-$CH_2CH_2C\underline{H}_3$);
ESI-MS ($m/z$): 341.1 $[M+H]^+$ (MW=340.37) ;
HRESIMS obsd m/z 341.1370, calcd for $C_{20}H_{21}O_5^+$ 341.1389.

5a-101 5-(p-tolyl)sulfonyloxy-8-methyl-4-n-propyl-2H-benzo[2,3-f]dipyranyl-2,10-dione (R1=$R_1$=n-$C_3H_7$, R3=CH3,)

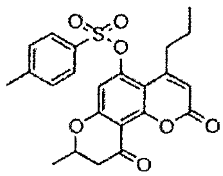

Compound 5a-11 (1 mmol) was reacted with 3 equivlent p-tolyl-sulfonyl chloride, the title compound 5a-101 was obtained in 84% yield as white powder crystalline, m. p. 258-260°C.
$^1$H NMR (300MHz, DMSO-$d_6$, ppm): 7.897(dd, 2H, J=1.8Hz, 7.8Hz, 14-Ar-H), 7.550(d, 2H, J=7.8Hz, 15-Ar-H), 6.696(s, 1H, 6-H), 6.230(s, 1H, 3-H), 4.797(m, 1H, J=3.6Hz, 6.3Hz, 11.7Hz, 8-H), 2.805 (dd, 1H, J=11.7Hz, 16.5Hz, 9-He), 2.688 (dd, 1H, J=3.6Hz, 16.5Hz, 9-Ha), 2.661(dt, 2H, J=3.3Hz, 8.1Hz, 11-$CH_2$), 2.441(s, 3H, 16-$CH_3$), 1.418(d, 3H, J=6.3Hz, 8-$CH_3$), 1.390(m, 2H, 12-$CH_2$), 0.758(t, 3H, J=7.2Hz, 13-$CH_3$);
ESI-MS (m/z): 443.1 $[M+H]^+$ (MW=442.49) ;

5a-102 5-(p-tolyl)sulfonyloxy-8-methyl-4-n-propyl-2-thio-2H-benzo[2,3-f]dipyranyl-2,10-dione

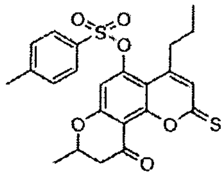

Compound 5a-101 (0.5 mmol) was reacted with 5 equivlent $P_2S_5$, the title compound 5a-102 was obtained in 74% yield as brown-yellow powder, m. p. 224-226°C.
$^1$H NMR (300MHz, DMSO-$d_6$, ppm): 7.910(dd, 2H, J=1.8Hz, 6.6Hz, 14-Ar-H), 7.551(dd, 2H, J=0.6Hz, 8.4Hz, 15-Ar-H), 7.054(s, 1H, 3-H), 6.785(s, 1H, 6-H), 4.840(m, 1H, J=11.7Hz, 3.6Hz, 6.3Hz, 8-H), 2.836(dd, 1H, J=11.7Hz, 16.2Hz, 9-He), 2.718(dd, 1H, J=3.6Hz, 16.2Hz, 9-Ha), 2.619(dt, 2H, J=2.4Hz, 7.2Hz, 11-$CH_2$), 2.440(s, 3H, 16-$CH_3$), 1.434(d, 3H,

J=6.3Hz, 8-$CH_3$), 1.384(m, 2H, 12-$CH_2$), 0.754(t, 3H, J=7.2Hz, 13-$CH_3$);
ESI-MS (*m*/*z*): 458.9 $[M+H]^+$ (MW=458.56).

**Pharmacological experiment**

Experiment 1

Inhibitory activity of the coumarin derivatives of the present invention against HIV-1.

Inhibitory activity of the compounds against HIV-1 is assayed using the literature method. The experimental conditons, procedures, reagents and so on of the experiment are refered to the reference of "ZHIWEI CHEN, PEI ZHOU, DAVID D. HO, et al. Genetically divergent strains of simian immuno - deficiency virus use CCR5 as a coreceptor for entry. Journal of Virology, 1997, 71(4): 2705-2714".

The results of the anti-HIV-1 activity of some compounds described in the present invention are shown in the table 1. It is indicated that some compounds give similar activity potency against HIV-1 in vitro to the parent natural compound (+)-calanolide A. It is especially interesting that three compounds (example 14, 57 and 70) exhibit the much higher inhibitory activity campared to the natural product of (+)-calanolide A and also with high therapeutic index.

Table 1, inhibitory activity of compounds against HIV-1.

| Compd | Anti-HIV (%, 1.0μM) | Cell toxicity (10μM) | $EC_{50}$ (TI) |
|---|---|---|---|
| (+)-11-demethyl Calanolide A | 75% | 54 (++) | - |
| (+)-Calanolide A | 86% | 6 (+) | 100-200* |
| Example 7 | 48.3 | - | - |
| Example 9 | 73.2 | - | 0.27μM(>1000) |
| Example 11 | 73.1 | - | 0.11μM(818) |
| Example 12 | 68.3 | | 0.64μM (47-140) |
| Example 13 | 64.9 | - | - |
| Example 14 | 61.9 | + | 32.5 nM (102-308) |
| Example 15 | 46.4 | + | - |
| Example 16 | 62.7 | + | - |
| Example 17 | 57 | - | - |
| Example 18 | 40 | + | - |
| Example 19 | 59.3 | + | - |
| Example 20 | 68.1 | - | 0.37μM (243-730) |
| Example 22 | 51.9 | - | - |
| Example 24 | 58 | - | - |
| Example 27 | 67 | - | - |
| Example 29 | 73.1 | - | 0.11μM(818) |
| Example 30 | 38.9 | ++ | - |
| Example 31 | 45.3 | ++ | - |
| Example 34 | 54. | - | - |
| Example 44 | 96.4 | ++ | - |
| Example 45 | 96.4 | ++ | - |
| Example 46 | 96.7 | ++ | - |
| Example 47 | 97 | ++ | - |
| Example 50 | 96.3 | +++ | - |
| Example 57 of the invention | 93.5 | - | 2.85 nM (>10526) |
| Example 70 of the invention | 68.6 | - | 14.1 nM (709-2127) |
| Example 73 | 53.6 | - | - |
| Example 74 | 47.6 | - | - |
| Example 75 | 62.4 | ++ | - |
| Example 76 | 37.8 | - | - |
| Example 77 | 35.2 | + | - |

(continued)

| Compd | Anti-HIV (%, 1.0μM) | Cell toxicity (10μM) | $EC_{50}$ (TI) |
|---|---|---|---|
| Example 79 | 57.4 | - | - |
| Example 81 | 55.7 | - | - |
| Example 82 | 75.4 | - | - |
| Example 83 | 39.5 | - | - |
| Example 86 | 57.3 | - | - |
| Example 87 | 56.4 | - | - |
| Example 88 | 46.2 | - | - |
| Example 91 | 49.1 | - | - |
| Example 92 | 47.1 | - | - |
| Example 96 | 51.7 | - | - |
| Example 97 | 52.6 | - | - |
| Example 98 | 61 | + | - |

Therapeutic index (TI) = 50% cytotoxicity doses / 50% effective dose; "+", "++", "+++" indicate that < 10%, 10-80%, > 80% of the host cells of the virus died at the concentration of 10 μmol of the tested compounds, respectively; * J. Med. Chem., 1996, 39(6):) 303-13.

Experiment 2

Activity of the compounds of the present invention against Mycobacterium tuberculosis

The results of the anti-Mycobacterium tuberculosis activity of the novel synthetic dipyranyl coumarin compounds and some of their synthetic intermediate described in the present invention are shown in the table 2 ("Antimycobacterial data were provided by the Tuberculosis Antimicrobial Acquisition and Coordinating Facility (TAACF) through a rescarch and development contract with the U.S. National Institute of Allergy and Infectious Diseases". The screen was conducted against Mycobacterium tuberculosis H37Rv (ATCC 27294) in BACTEC 12B medium using the Microplate Alamar Blue Assay (MABA), Control compound(Amikacin): Activity: Avg $MIC_{50}$ = 0.09 μg/mL - Avg $MIC_{90}$ = 0.12 μg/mL).

It is defined that the compound which $MIC_{90}$ value is less than 10μg/mL is indicated as anti-TB active compound. Wherein examples 57, 70, are novel anti-TB active compounds.

Table 2. Activity of the compounds against TB.

| Examples | Assay | $MIC_{90}$(μg/mL) | $IC_{50}$(μg/mL) |
|---|---|---|---|
| Example 1 | MABA | 59.459 | 39.192 |
| Example 2 | MABA | 35.17 | 20.319 |
| Example 3 | MABA | >100 | 33.37 |
| Example 4 | MABA | >100 | 7.517 |
| Example 6 | MABA | >100 | 52.447 |
| Example 14 | MABA | 6.437 | 5.723 |
| Example 16 | MABA | >100 | 2.912 |
| Example 18 | MABA | >100 | 46.75 |
| Example 19 | MABA | 11.246 | 7.946 |
| Example 21 | MABA | >100 | 16.626 |
| Example 23 | MABA | 27.6 | 24.193 |
| Example 25 | MABA | 3.898 | 2.858 |
| Example 26 | MABA | 43.608 | 23.88 |
| Example 32 | MABA | >100 | 78.299 |
| Example 34 | MABA | 3.183 | 2.875 |
| Example 36 | MABA | 36.586 | 24.248 |
| Example 37 | MABA | 8.36 | 5.332 |
| Example 38 | MABA | 14.419 | 10.417 |
| Example 39 | MABA | 9.936 | 5.089 |
| Example 40 | MABA | 2.47 | 1.584 |

(continued)

| Examples | Assay | $MIC_{90}$(μg/mL) | $IC_{50}$(μg/mL) |
|---|---|---|---|
| Example 41 | MABA | 3.268 | 2.918 |
| Example 42 | MABA | 6.807 | 5.561 |
| Example 43 | MABA | 10.791 | 6.42 |
| Example 45 | BABA | 2.726 | 2.012 |
| Example 46 | MABA | 0.716 | 0.276 |
| Example 47 | MABA | 1.729 | 1.493 |
| Example 49 | MABA | 6.396 | 4.619 |
| Example 50 | MABA | 6.695 | 6.072 |
| Example 51 | MABA | 0.496 | <0.2 |
| Example 52 | MABA | 13.402 | 11.778 |
| Example 53 | MABA | 6.847 | 6.096 |
| Example 54 | MABA | 61.792 | 36.518 |
| Example 55 | MABA | 1.07 | <0.2 |
| Example 56 | MABA | >100 | 62.338 |
| Example 57 of the invention | MABA | 1.754 | 0.825 |
| Example 58 | MABA | 54.902 | 34.498 |
| Example 60 | MABA | >100 | 32.547 |
| Example 61 | MABA | >100 | 17.198 |
| Example 68 | MABA | 44.884 | 32.994 |
| Example 70 of the invention | MABA | 5.543 | 3.307 |
| Example 74 | MABA | 6.379 | 5.701 |
| Example 75 | MABA | 1.891 | 1.005 |
| Example 78 | MABA | >100 | 98.967 |
| Example 79 | MABA | 6.837 | 6.067 |
| Example 80 | MABA | 5.458 | 3.42 |
| Example 82 | MABA | 56.155 | 36.254 |
| Example 83 | MABA | 27.67 | 24.407 |
| Example 84 | MABA | 27.688 | 17.292 |
| Example 85 | MABA | 4.861 | 2.08 |
| Example 87 | MABA | >100 | 51.239 |
| Example 88 | MABA | >100 | 70.653 |
| Example 93 | MABA | >100 | 76.155 |
| Example 94 | MABA | 16.67 | 10.005 |
| Example 95 | MABA | 34.469 | 20.105 |
| Example 96 | MABA | 22.233 | 11.104 |
| Example 99 | MABA | 15.494 | 7.486 |
| 6-71 | MABA | 4.06 | 2.526 |
| 5b-16 | MABA | 5.406 | 4.312 |
| 6-36 | MABA | 14.434 | 11.348 |
| 5b-21 | MABA | 21.303 | 14.865 |
| 5a-31 | MABA | 23.472 | 16.991 |
| 6-26 | MABA | 24.358 | 9.97 |
| 5a-16 | MABA | 25.136 | 22.507 |
| 5a-71 | MABA | 29.922 | 15.447 |
| 5a-20 | MABA | 41.962 | 26.946 |
| 5a-13 | MABA | 53.43 | 46.748 |
| 5a-14 | MABA | 85.832 | 60.245 |
| 5a-7 | MABA | 94.977 | 59.798 |

(continued)

| Examples | Assay | $MIC_{90}$($\mu$g/mL) | $IC_{50}$($\mu$g/mL) |
|---|---|---|---|
| 5b-14 | MABA | >100 | 6.586 |
| 5a-15 | MABA | >100 | 66.008 |
| 5a-19 | MABA | >100 | 83.08 |
| 5b-11 | MABA | >100 | 95.261 |
| 5b-1 | MABA | >100 | 43.07 |
| 5a-27 | MABA | >100 | 94.349 |
| 5b-3 | MABA | >100 | 90.516 |
| 5a-101 | MABA | 6.663 | 4.2 |
| 5a-102 | MABA | 6.241 | 5.48 |

## Claims

1. A compound of general formula (I):

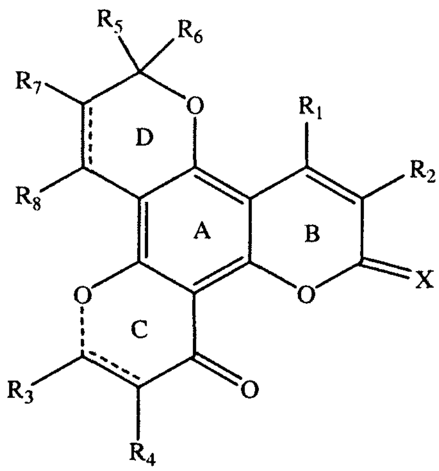

**(I)**

or an optical isomer, pharmaceutically acceptable salt or solvate thereof, wherein:

<u>----</u> in ring C is a carbon-carbon single bond; --- in ring C is a carbon-oxygen single bond;
<u>----</u> in ring D is either a carbon-carbon single bond or a carbon-carbon double bond;
X is O or S;
$R_1$ is $C_{1-6}$ alkyl;
$R_2$ is H, **<u>or</u>** halogen
$R_3$ is a methylene substituted by one or more halogen atoms;
$R_4$ is H,
$R_5$ and $R_6$ are **<u>both methyl groups</u>**;
$R_7$ is H or $C_{1-6}$ alkyl; $R_8$ is H or $C_{1-6}$ alkoxyl; with the proviso that when <u>----</u> in ring D is a carbon-carbon single bond, $R_8$ is alkoxyl; when $R_7$ and $R_8$ are H, <u>----</u> in ring D is a carbon-carbon double bond.

2. The compound of claim 1, or a optical isomer, pharmaceutically acceptable salt or solvate thereof, wherein X is S, both $R_5$ and $R_6$ are methyl groups, and the bond in ring C between the carbon connected with $R_3$ and the carbon connected with $R_4$ is a carbon-carbon single bond.

3. The compound or an optical isomer, pharmaceutically acceptable salt or solvate thereof, selected from the group consisting of:

6,6-dimethyl-4-n-propyl-10-bromomethyl-2H,6H,12H-benzo[1,2-b:3,4-b':5,6-b']-trip yranyl-2,12-dione,
6,6-dimethyl-4-n-propyl-10,11-cis-cyclopropyl-2H,6H,12H-benzo[1,2-b:3,4-b':5,6-b'] tripyranyl-2,12-dione.

4. A pharmaceutical composition comprising a pharmaceutically effective amount of a compound of any one of claims 1 to 3 or an optically active substance, pharmaceutically acceptable salt or solvate therof, and a pharmaceutically acceptable carrier or excipient.

5. A compound of any one of claims 1 to 3 for use in a method of treatment or prevention of a disease related to HIV-1 infection.

6. A compound of any one of claims 1 to 3 for use in a method of treatment or prevention of a disease related to TB infection.

7. A process for preparing a tetracyclic coumarin compound of the general formula below, which is **characterized in that** the compound with ring D is obtained from a tricyclic coumarin compound with rings A, B and C under microwave irradiation according to the chemical reaction below:

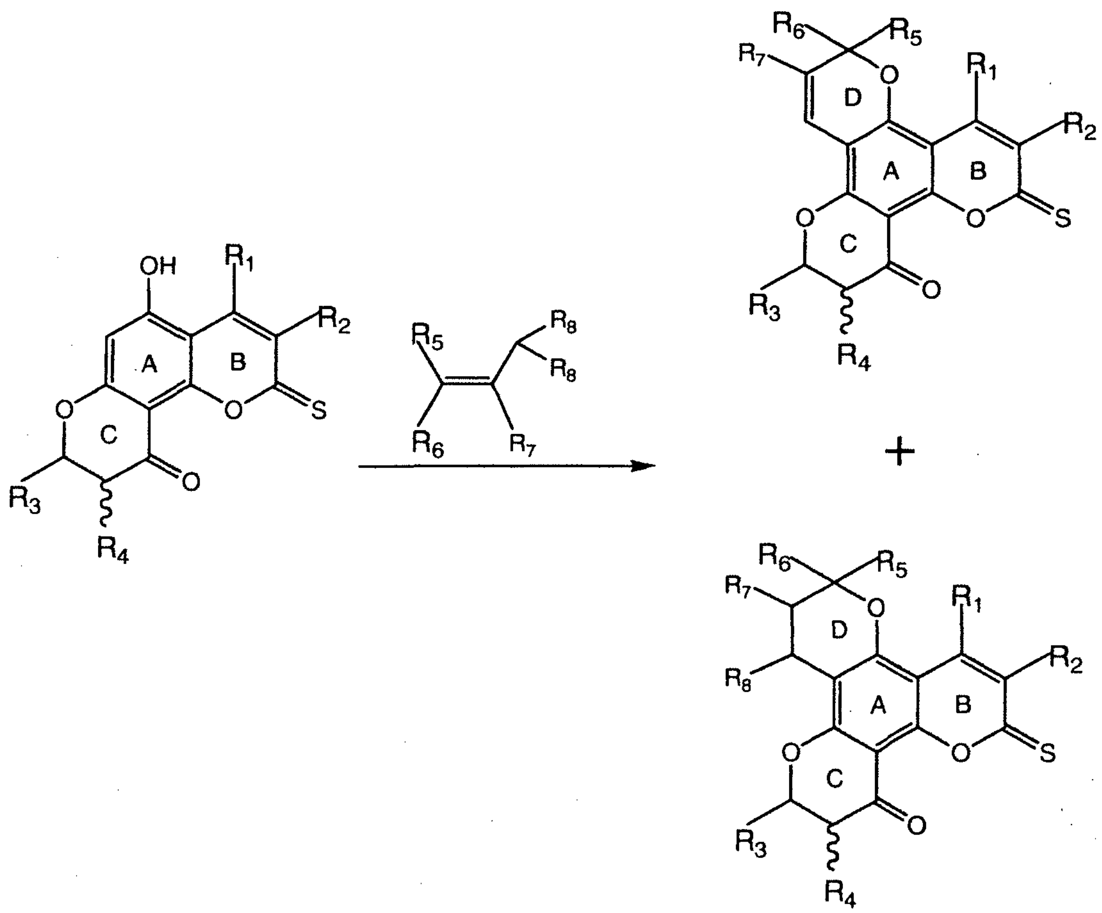

wherein R)-$R_8$ are as defined in claim 1.

8. A process for preparing tetracyclocoumarin compound (**4**), which is **characterized in that** tricyclic coumarin compound **14** with rings A, B and D is reacted with an acyl halide of an α, β-unsaturated acid to form ring C:

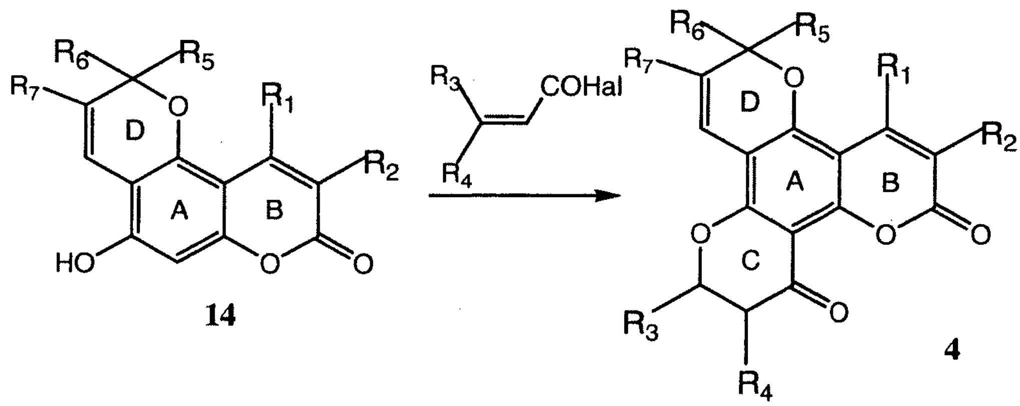

14

4

wherein $R_1$-$R_7$ are as defined in claim 1.

**9.** A process for preparing the compound of claim 1 in which X is S, which is **characterized in that** the compound is synthesized through the construction of ring D from a tricyclic coumarin compound with rings A, B and C according to the process of claim 8:

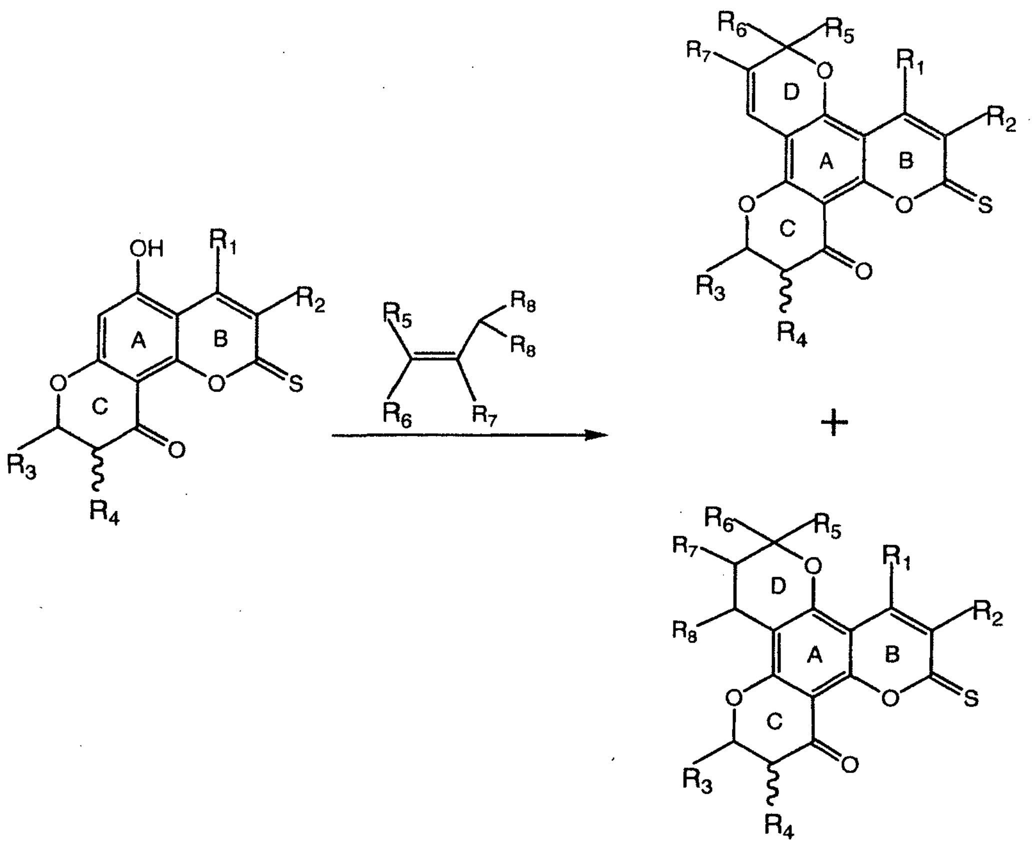

wherein $R_1$-$R_8$ are as defined in claim 1.

**Patentansprüche**

**1.** Verbindung mit der allgemeinen Formel (I):

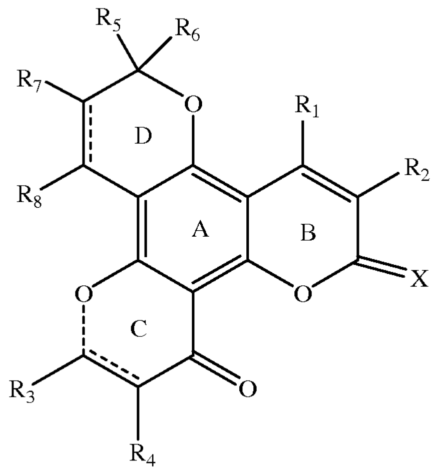

(I)

oder ein optisches Isomer, ein pharmazeutisch verträgliches Salz oder Solvat davon, wobei

---- in Ring C eine Kohlenstoff-Kohlenstoff-Einfachbindung darstellt,
--- in Ring C eine Kohlenstoff-Sauerstoff-Einfachbindung darstellt,
---- in Ring D entweder eine Kohlenstoff-Kohlenstoff-Einfachbindung oder eine Kohlenstoff-Kohlenstoff-Doppelbindung darstellt,
X O oder S ist,
$R_1$ ein $C_{1-6}$-Alkyl ist,
$R_2$ H oder ein Halogen ist,
$R_3$ ein Methylen ist, das mit einem oder mehreren Halogenatom/en substituiert ist,
$R_4$ H ist,
$R_5$ und $R_6$ Methylgruppen sind,
$R_7$ H oder ein $C_{1-6}$-Alkyl ist, $R_8$ H oder ein $C_{1-6}$-Alkoxyl ist, unter der Bedingung, dass, wenn ---- in Ring D eine Kohlenstoff-Kohlenstoff-Einfachbindung darstellt, $R_8$ ein Alkoxyl ist, wenn $R_7$ und $R_8$ H sind, ---- in Ring D eine Kohlenstoff-Kohlenstoff-Doppelbindung darstellt.

2. Verbindung nach Anspruch 1 oder ein optisches Isomer, ein pharmazeutisch verträgliches Salz oder Solvat davon, wobei X S ist, $R_5$ und $R_6$ Methylgruppen sind und die Bindung in Ring C zwischen dem mit $R_3$ verbundenen Kohlenstoff und dem mit $R_4$ verbundenen Kohlenstoff eine Kohlenstoff-Kohlenstoff-Einfachbindung ist.

3. Verbindung oder ein optisches Isomer, ein pharmazeutisch verträgliches Salz oder Solvat davon, das aus der Gruppe ausgewählt ist, die besteht aus:

6,6-Dimethyl-4-n-propyl-10-brommethyl-2H,6H,12H-benzo[1,2-b:3,4-b':5,6-b']-tripyranyl-2,12-dion,
6,6-Dimethyl-4-n-propyl-10,11-cis-cyclopropyl-2H,6H,12H-benzo[1,2-b:3,4-b':5,6-b']tripyranyl-2,12-dion.

4. Pharmazeutische Zusammensetzung, umfassend eine pharmazeutisch wirksame Menge einer Verbindung nach einem der Ansprüche 1 bis 3 oder eine optisch aktive Substanz, ein pharmazeutisch verträgliches Salz oder Solvat davon und einen pharmazeutisch verträglichen Träger oder Hilfsstoff.

5. Verbindung nach einem der Ansprüche 1 bis 3 zur Verwendung in einem Verfahren zur Behandlung oder Vorbeugung einer Erkrankung in Bezug auf eine HIV-1-Infektion.

6. Verbindung nach einem der Ansprüche 1 bis 3 zur Verwendung in einem Verfahren zur Behandlung oder Vorbeugung einer Erkrankung in Bezug auf eine TB-Infektion.

7. Verfahren zum Herstellen einer tetrazyklischen Cumarinverbindung der unten gezeigten allgemeinen Formel, die **dadurch gekennzeichnet ist, dass** die Verbindung mit Ring D aus einer trizyklischen Cumarinverbindung mit den Ringen A, B und C unter Mikrowellenbestrahlung gemäß der unten gezeigten chemischen Reaktion erhalten wird:

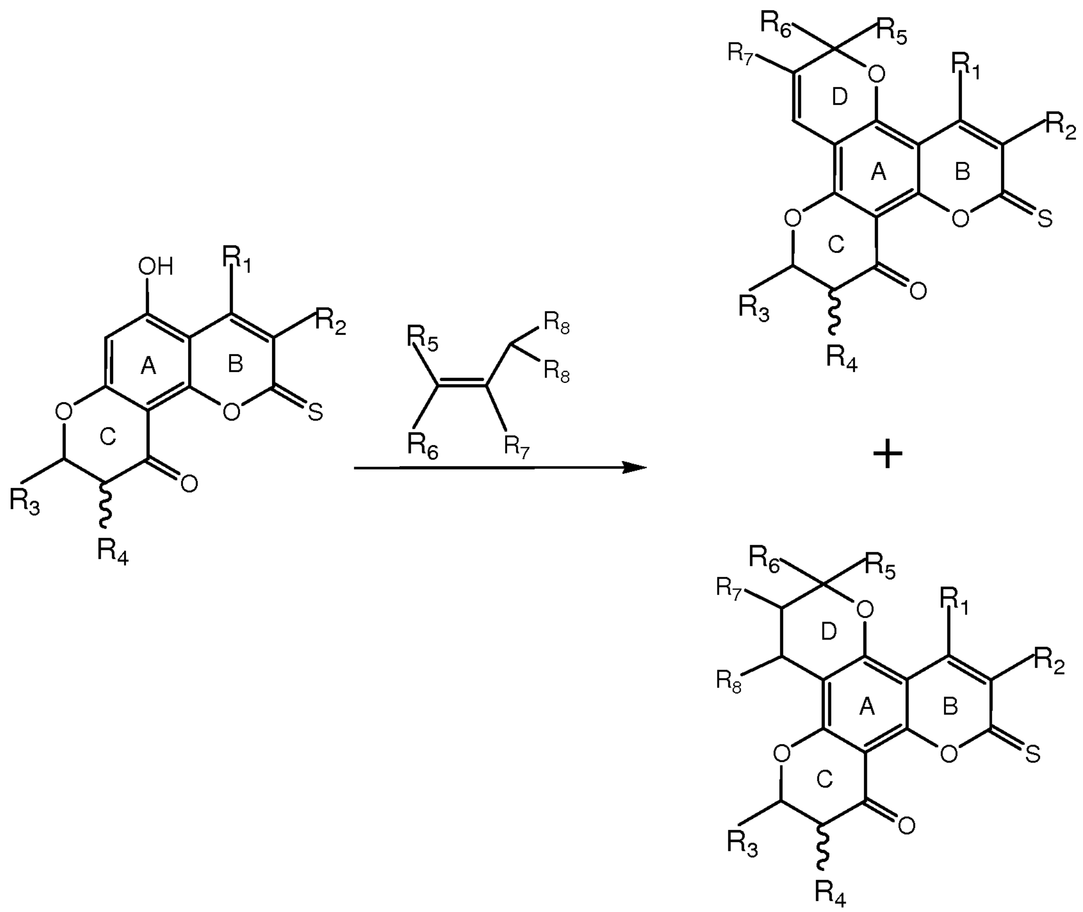

wobei $R_1$ bis $R_8$ wie in Anspruch 1 definiert sind.

**8.** Verfahren zum Herstellen einer Tetrazyklocumarinverbindung (4), die **dadurch gekennzeichnet ist, dass** die trizyklischen Cumarinverbindung 14 mit den Ringen A, B und D mit einem Alcylhalid einer $\alpha$, $\beta$-ungesättigten Säure reagiert, um Ring C zu bilden:

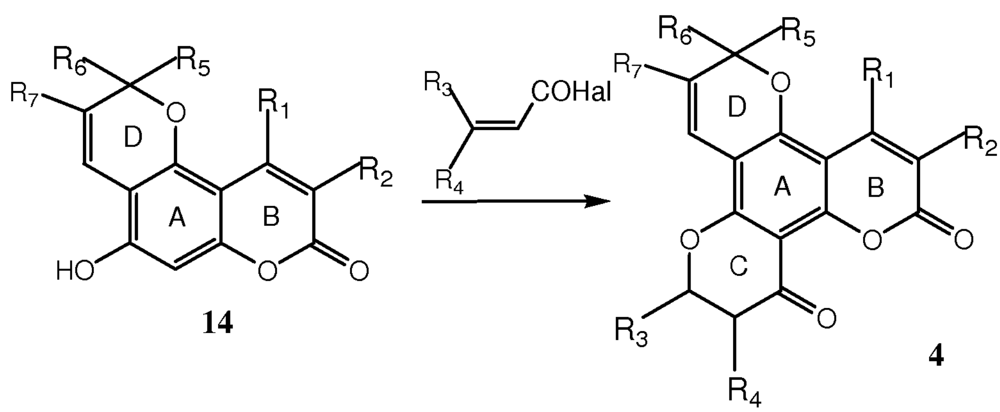

14

4

wobei $R_1$ bis $R_7$ wie in Anspruch 1 definiert sind.

**9.** Verfahren zum Herstellen der Verbindung nach Anspruch 1, in der X S ist, die **dadurch gekennzeichnet ist, dass** die Verbindung durch die Konstruktion des Rings D aus einer trizyklischen Cumarinverbindung mit den Ringen A, B und C gemäß dem Verfahren nach Anspruch 8 synthetisiert wird:

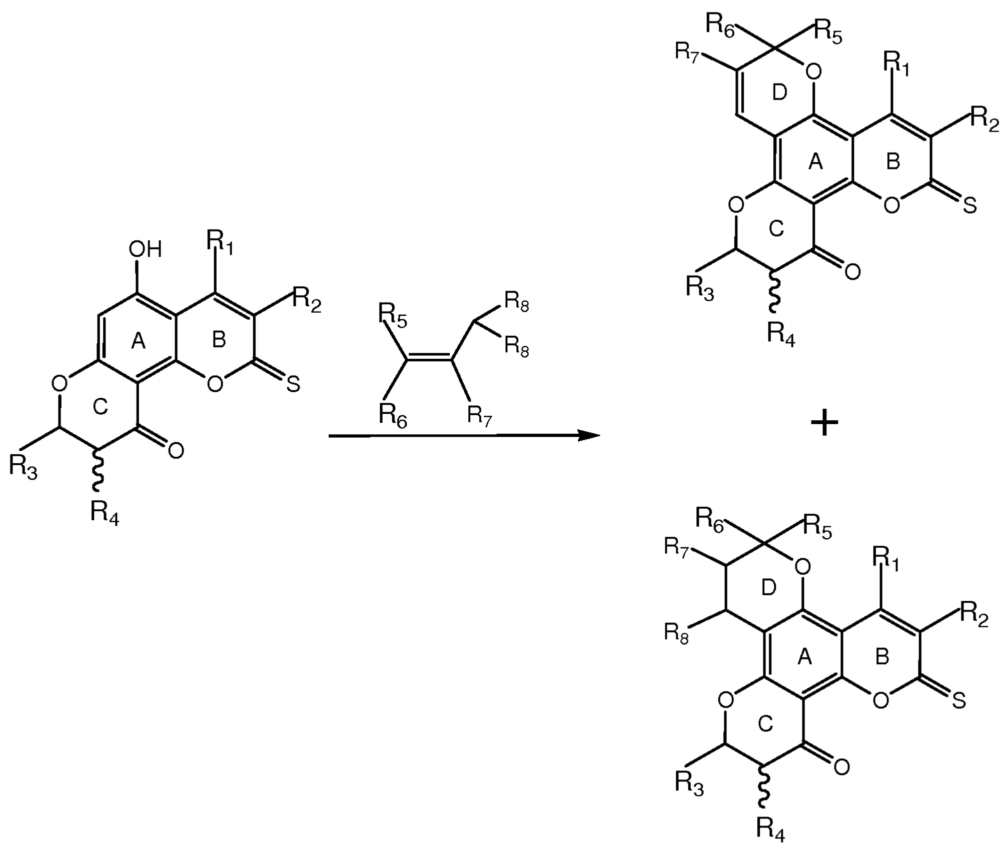

wobei $R_1$ bis $R_8$ wie in Anspruch 1 definiert sind.

**Revendications**

**1.** Composé de la formule générale (I) :

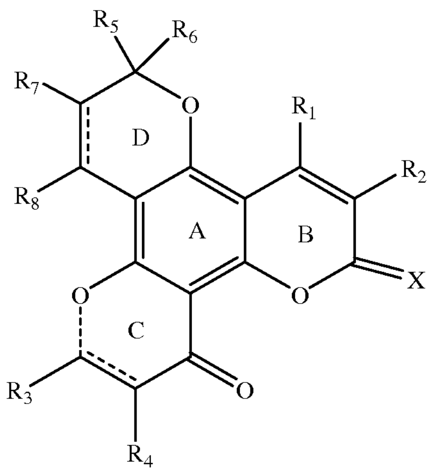

(I)

ou isomère optique, sel ou solvate pharmaceutiquement acceptable de celui-ci, dans lequel :

---- dans le cycle C est une liaison simple carbone-carbone ;
---- dans le cycle C est une liaison simple carbone-oxygène ;
---- dans le cycle D est soit une liaison simple carbone-carbone soit une liaison double carbone-carbone ;
X est O ou S ;
$R_1$ est un groupe alkyle en $C_{1-6}$ ;
$R_2$ est H ou un atome d'halogène ;
$R_3$ est un groupe méthylène substitué par un ou plusieurs atomes d'halogène ;
$R_4$ est H,
$R_5$ et $R_6$ sont tous deux des groupes méthyle ;
$R_7$ est H ou un groupe alkyle en $C_{1-6}$ ; $R_8$ est H ou un groupe alcoxyle en $C_{1-6}$ ; à condition que lorsque ---- dans le cycle D est une liaison simple carbone-carbone, $R_8$ est un groupe alcoxyle ; lorsque $R_7$ et $R_8$ sont H, ---- le cycle D est une double liaison carbone-carbone.

**2.** Composé selon la revendication 1, ou isomère optique, sel ou solvate pharmaceutiquement acceptable de celui-ci, dans lequel X est S, à la fois $R_5$ et $R_6$ sont des groupes méthyle, et la liaison dans le cycle C entre le carbone connecté à $R_3$ et le carbone connecté à $R_4$ est une liaison simple carbone-carbone.

**3.** Composé ou isomère optique, sel ou solvate pharmaceutiquement acceptable de celui-ci, choisi dans le groupe constitué par :

la 6,6-diméthyl-4-n-propyl-10-bromométhyl-2H,6H,12H-benzo-[1,2-b:3,4-b':5,6-b']-tripyranyl-2,12-dione,
la 6,6-diméthyl-4-n-propyl-10,11-cis-cyclopropyl-2H,6H,12H-benzo[1,2-b:3,4-b':3,6-b']tripyranyl-2,12-dione.

**4.** Composition pharmaceutique comprenant une quantité pharmaceutiquement efficace d'un composé selon l'une quelconque des revendications 1 à 3 ou d'une substance optiquement active, d'un sel ou d'un solvate pharmaceutiquement acceptable de celui-ci, et un support ou un excipient pharmaceutiquement acceptable.

**5.** Composé selon l'une quelconque des revendications 1 à 3 pour une utilisation dans un procédé de traitement ou de prévention d'une maladie liée à une infection par le VIH-1.

**6.** Composé selon l'une quelconque des revendications 1 à 3 pour une utilisation dans un procédé de traitement ou de prévention d'une maladie liée à une infection par la TB.

**7.** Procédé de préparation d'un composé de coumarine tétracyclique de la formule générale ci-dessous, lequel est **caractérisé en ce que** le composé avec le cycle D est obtenu à partir d'un composé de coumarine tricyclique avec les cycles A, B et C sous une irradiation de micro-ondes selon la réaction chimique ci-dessous :

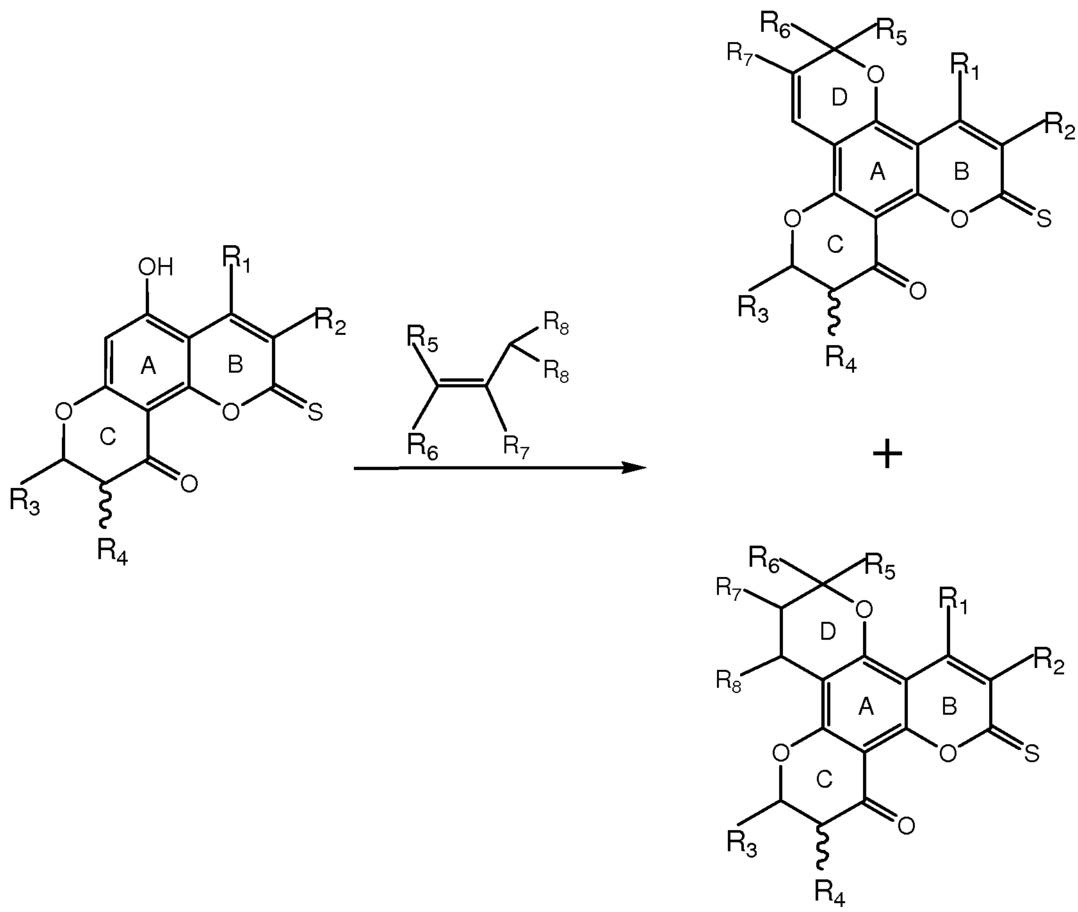

où $R_1$-$R_8$ sont comme définis dans la revendication 1.

8. Procédé de préparation d'un composé de tétracyclocoumarine (4), lequel est **caractérisé en ce qu'**un composé de coumarine tricyclique (14) avec les cycles A, B et D réagit avec un halogénure d'acyle d'un acide $\alpha$, $\beta$-insaturé pour former le cycle C :

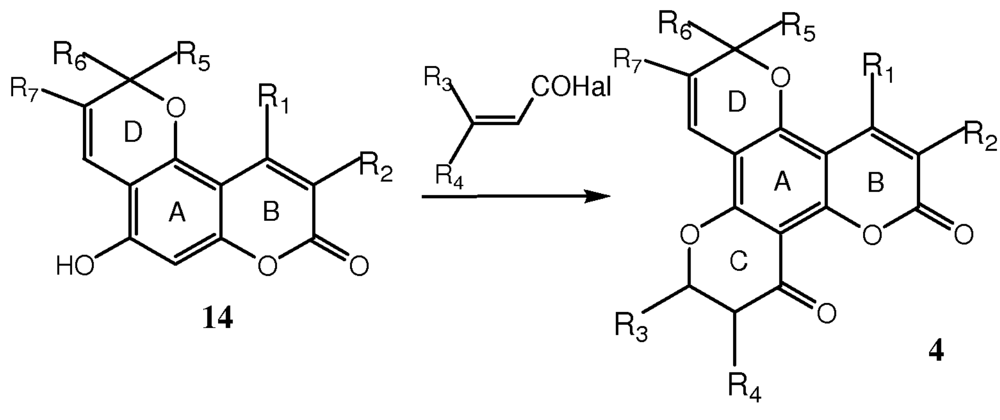

14

4

où $R_1$-$R_7$ sont comme définis dans la revendication 1.

9. Procédé de préparation du composé selon la revendication 1, dans lequel X est S, lequel est **caractérisé en ce que** le composé est synthétisé par la construction du cycle D à partir d'un composé de coumarine tricyclique avec les cycles A, B et C selon le procédé de la revendication 8 :

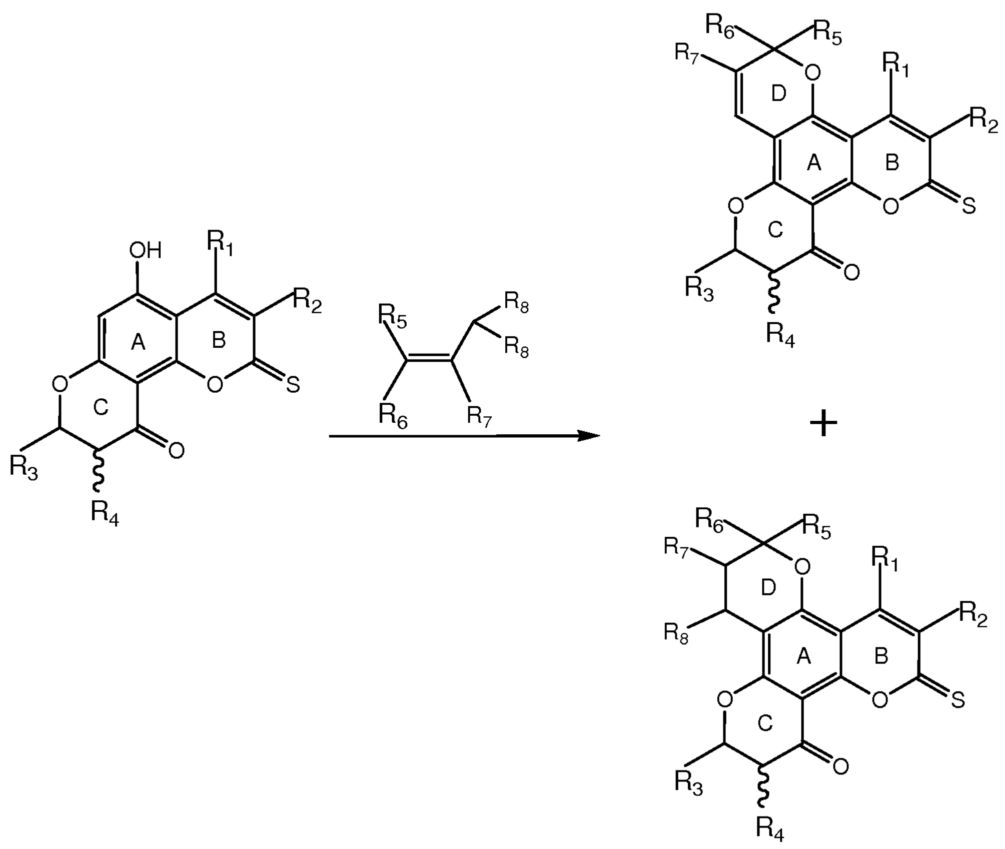

où $R_1$-$R_8$ sont comme définis dans la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 03123628
- CN 154849 A
- US 5859050 A
- US 6670383 B2
- US 6277879 B1
- CN 1548439 A


### Non-patent literature cited in the description


- *J. MED. CHEM.,* 1992, vol. 35, 2735
- *J. MED. CHEM.,* 1996, vol. 39, 1303
- *Bioorg. Med. Chem.,* 2004, 1199-1207
- *J Org Chem,* 1993, vol. 58, 5605
- *Tetrahedron Lett,* 1995, vol. 36, 5475
- *Acta Pharmaceutica Sinica,* 1999, vol. 34 (9), 673
- *Chinese Chem. Lett.,* 1997, vol. 8, 859
- *Chinese Chem. Lett.,* 1998, vol. 9, 433
- **XUE H. et al.** Highly suppressing wild-type HIV-1 and Y181C mutant HIV-1 strains by 10-chloromethy-11-demethyl-12-oxo-calanolide A with druggable profile. *Jomal of Medicinal Chemistry,* 2010, vol. 53 (3), 1397-1401
- **CABON O. ; BUISSON D. ; LARCHEVEQUE M. et al.** *Tetrahedron Asymmetry,* 1995, vol. 6 (9), 2199-2210
- **LERMER L. ; NEELAND E. G ; OUNSWORTH J. P. et al.** *Can J Chem,* 1992, vol. 70 (5), 1427-1445
- *J. Med. Chem.,* 1996, vol. 39, 1303-1313
- **T. GREENE.** Protecting Croups in Organic. Synthesis. John Wiley & Sons, Inc,
- **ZHIWEI CHEN ; PEI ZHOU ; DAVID D. HO et al.** Genetically divergent strains of simian immuno - deficiency virus use CCR5 as a coreceptor for entry. *Journal of Virology,* 1997, vol. 71 (4), 2705-2714
- *J. Med. Chem.,* 1996, vol. 39 (6), 303-13